# EUROPEAN PATENT SPECIFICATION

(11) **EP 4 061 817 B1**
(45) Date of publication and mention of the grant of the patent: **07.10.2026**
(21) Application number: 20890594.3
(22) Date of filing: 19.11.2020
(51) Int. Cl.: C07D 473/32, C07D 487/04, A61K 31/519, A61K 31/52, A61P 35/00

(54) **ADENOSINE RECEPTOR ANTAGONIST COMPOUNDS**
ADENOSINREZEPTOR-ANTAGONIST-VERBINDUNGEN
COMPOSÉS ANTAGONISTES DU RÉCEPTEUR DE L'ADÉNOSINE

(30) Priority: 19.11.2019 KR 20190149117
(43) Date of publication of application: 28.09.2022
(73) Proprietor: Ildong Pharmaceutical Co., Ltd., Seocho-gu, Seoul 06752 (KR)
(72) Inventor: LEE, Yoon-Suk, Hwaseong-si, Gyeonggi-do 18449 (KR); KWON, Sung-Wook, Hwaseong-si, Gyeonggi-do 18449 (US); KIM, Kyung-Sun, Hwaseong-si, Gyeonggi-do 18449 (KR); KIM, Jeong-Geun, Hwaseong-si, Gyeonggi-do 18449 (KR); KIM, Jeong-Ah, Hwaseong-si, Gyeonggi-do 18449 (KR); MOON, An-Na, Hwaseong-si, Gyeonggi-do 18449 (KR); PARK, Sun-Young, Hwaseong-si, Gyeonggi-do 18449 (KR); BAN, Jun-Su, Hwaseong-si, Gyeonggi-do 18449 (KR); SONG, Dong-Keun, Hwaseong-si, Gyeonggi-do 18449 (KR); JUNG, Ju-Young, Hwaseong-si, Gyeonggi-do 18449 (KR); LEE, Soo-Jin, Hwaseong-si, Gyeonggi-do 18449 (KR)
(74) Representative: Marks & Clerk LLP
(86) International application number: PCT/IB2020/000971
(87) International publication number: WO 2021/099838

(56) References cited:
- EP-B1- 1 349 857
- WO-A1-01/02400
- WO-A1-2009/156737
- WO-A1-2011/050160
- US-A- 6 124 300
- US-A1- 2006 111 373

## Description

### INTRODUCTION

Adenosine carries out many biological functions through particular cell receptors and is associated with a variety of biological activities including immune functions and inflammation.

There are four types of adenosine receptor (A1, A2A, A2B, and A3), and they are connected to the heteromeric G protein. Each of the A2A receptor and A2B receptor is connected to Gs subtype of Gα proteins. When these receptors are stimulated, the receptor format changes, and such a change induces the discharge of the Gs subunit activated by the Gβ ρ dimer, and then hydrolysis of the adenosine triphosphate (ATP) in the cell to produce cyclic adenosine monophosphate (cAMP). The cAMP synthesis activates protein kinase A (PKA) and phosphorylation of other proteins. In the T cell, mainly type I PKA isoform exists around the T cell receptor (TCR), and if activation of PKA increases due to the increased cAMP levels, the TCR signal transmission process is inhibited, thereby contributing to the occurrence of various illnesses.

Cancer cells produce significantly more adenosine than is produced by normal cells. In cancer cells, high-density adenosine induces activation of the A2A receptor to inhibit the immune system thereby and protect the cells. In the micro-environment of a tumor, the concentration of adenosine can be at 50 µM, an increase over normal cells, and lead to immunosuppression of T-cell function and activation. An A2A receptor antagonist can be used to adjust the cancer cell's inhibition of the immune system to induce anticancer effects.

A2A receptor antagonists are in development for immuno-oncology therapies. A2A receptor antagonists can enhance antitumor immunity. The A2A receptor is widely produced in white blood cells. When a T cell's A2A receptor becomes activated, TCR-mediated cytotoxicity and production of cytokines decreases, proliferation of T cells is inhibited, and expansion of Treg cells is induced. In immuno-oncology, the immunity checkpoint inhibitors of PD-1 or PD-L1 (e.g., antibody inhibitors) are widely used. However, statistically only 20% to 30% of patients produce PD-1/PD-L1, and thus there are many patients who do not benefit from the efficacy of such inhibitors. Treatments involving immunity checkpoint inhibitors in combination with A2A receptor antagonists are of interest.

Control of adenosine receptors is of interest for treatment of various indications. Modulating activity of the adenosine A1 receptor is of interest for treatment of nervous system disorders, asthma, heart failure, renal failure, and the like; antagonizing the adenosine A2A receptor is of interest for the treatment of Parkinson's disease, and the like; modulating activity of the adenosine A2B receptor is of interest for the treatment of chronic lung disorders such as asthma, cancer, in immuno-oncology and the like; and modulating the adenosine A3 receptor is of interest for treatment of asthma and chronic obstructive lung disorders, glaucoma, cancer, cerebral apoplexy, and the like.

### SUMMARY

The present disclosure provides adenosine receptor (e.g., A2A and/or A1 receptor) antagonist compounds and compositions including said compounds. The present disclosure also provides methods of using said compounds and compositions for modulating (e.g., inhibiting or antagonizing) A2A and/or A1 receptor in a biological system. The compounds and compositions find use in various therapeutic applications including the treatment of cancer and in immuno-oncology. The compounds and compositions find use in various therapeutic applications including the treatment of central nervous system or neurodegenerative diseases, such as Parkinson's disease.

The invention as set out in the appended set of claims.

### DETAILED DESCRIPTION

### Adenosine Receptor Antagonist Compounds

As summarized above, the present disclosure provides A2A and/or A1 receptor antagonist compounds and compositions. The compounds can modulate adenosine A2A and/or A1 receptor in cells and biological systems of interest. The compounds find use in a variety of therapeutic applications, including treatment of cancer and in immuno-oncology, and the treatment of central nervous system or neurodegenerative diseases, such as Parkinson's Disease.

The compounds of this disclosure can be described as cyano-substituted fused pyrimidine compounds that include a core structure having a 2-amino-pyrimidine ring fused to a five-membered heterocycle ring. The core structure is further substituted with a benzonitrile substituent (a 3-cyano-phenyl) or a derivative thereof. The fused five membered heterocycle ring can be further substituted with an optionally substituted benzyl group. The compounds have a 1H-pyrazolo[3,4-d]pyrimidin-6-amine core structure that is further substituted as described herein.

The compound is of formula (IIb): 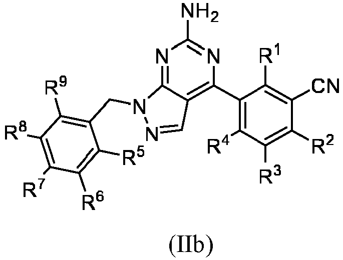 wherein:
R¹ to R⁹ are independently selected from H, (C₁-C₈)alkyl, substituted (C₁-C₈)alkyl, (C₂-C₈)alkenyl, substituted (C₂-C₈)alkenyl, (C₂-C₈)alkynyl, substituted (C₂-C₈)alkynyl, (C₁-C₃)haloalkyl, (C₁-C₈)alkoxy, substituted (C₁-C₈)alkoxy, -CONH₂, substituted amido, - NH₂, substituted amino, -CO₂H, cyano, halogen, hydroxyl, -NO₂, -SO₃H, -SO₂NH₂, substituted sulfonamide, and thiol;
or a solvate, a hydrate and/or a stereoisomer thereof, or a pharmaceutically acceptable salt thereof.

In some embodiments of formula (IIb), R¹ to R⁹ are independently selected from H, (C₁-C₅)alkyl, substituted (C₁-C₅)alkyl, (C₁-C₃)haloalkyl, (C₁-C₅)alkoxy, substituted (C₁-C₅)alkoxy, -NH₂, substituted amino, halogen, and hydroxyl. In some embodiments of formula (IIb), R¹ to R⁹ are independently selected from H, NH₂, F, CH₃, and CF₃.

In some embodiments of formula (IIb), the compound is of formula (IIIb): 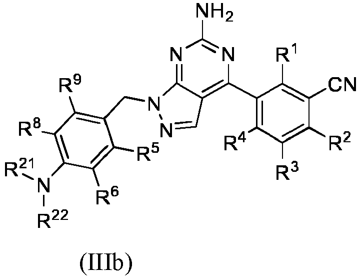 wherein:
R²¹ and R²² are independently selected from H, (C₁-C₈)alkyl, substituted (C₁-C₈)alkyl, SO₂R³⁰, and COR³⁰ , wherein R³⁰ is (C₁-C₈)alkyl, or substituted (C₁-C₈)alkyl;
or a solvate, a hydrate and/or a stereoisomer thereof, or a pharmaceutically acceptable salt thereof.

In some embodiments of formula (IIIb), R²¹ and R²² are each H.

In some embodiments of formula (IIIb), R⁵, R⁶, R⁸ and R⁹ are independently selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl. In some embodiments of formula (IIIb), R⁵, R⁶, R⁸ and R⁹ are independently selected from H, F, CH₃, and CF₃. In some embodiments of formula (IIIb), R⁵, R⁶, R⁸ and R⁹ are each H.

In some embodiments of formula (IIIb), R² to R⁴ are each H, and R¹ is selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl. In some embodiments of formula (IIIb), R¹ is selected from H, F, CH₃, and CF₃.

In some embodiments of formula (IIIb), the compound is selected from: 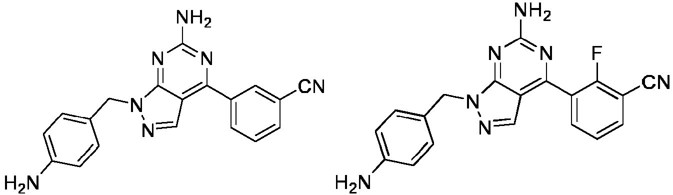 and 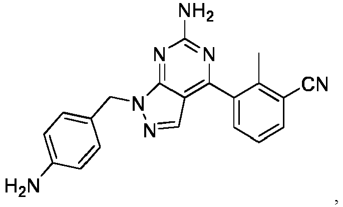 or a solvate, a hydrate and/or a stereoisomer thereof, or a pharmaceutically acceptable salt thereof.

In some embodiments of formula (IIIb), the compound is of formula (IVb): 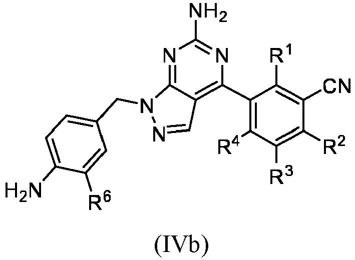 or a solvate, a hydrate and/or a stereoisomer thereof, or a pharmaceutically acceptable salt thereof.

In some embodiments of formula (IVb), R⁶ is selected from (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl. In some embodiments of formula (IVb), R⁶ is selected from (C₁-C₅)alkyl, substituted (C₁-C₅)alkyl, and (C₁-C₃)haloalkyl. In some embodiments of formula (IVb), R⁶ is CH₃ or CF₃.

In some embodiments of formula (IVb), R² to R⁴ are each H, and R¹ is selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl. In some embodiments of formula (IVb), R¹ is selected from H, F, CH₃, and CF₃.

In some embodiments of formula (IVb), the compound is selected from: 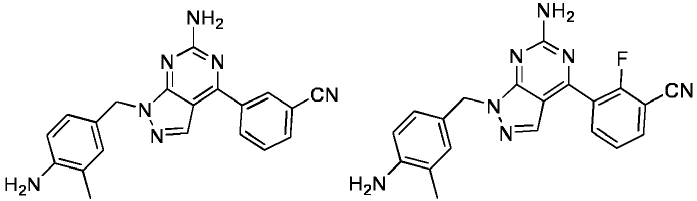 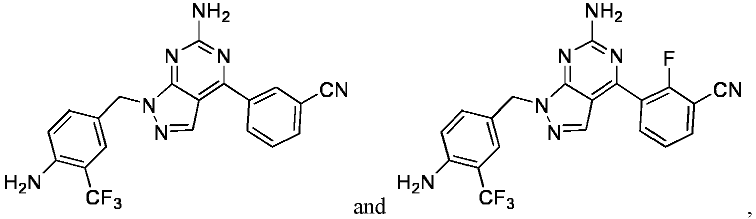 or a solvate, a hydrate and/or a stereoisomer thereof, or a pharmaceutically acceptable salt thereof.

In some embodiments of formula (IIIb), the compound is of formula (Vb): 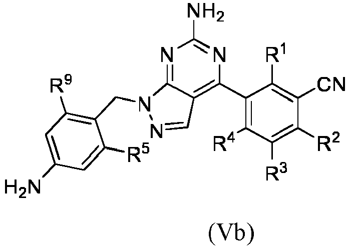 or a solvate, a hydrate and/or a stereoisomer thereof, or a pharmaceutically acceptable salt thereof.

In some embodiments of formula (Vb), R⁵ and R⁹ are independently selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl. In some embodiments of formula (Vb), R⁵ and R⁹ are independently selected from H and halogen. In some embodiments of formula (Vb), R⁵ is F. In some embodiments of formula (Vb), R⁹ is F. In some embodiments of formula (Vb), R⁵ and R⁹ are each F. In some embodiments of formula (Vb), R⁵ is H and R⁹ is F.

In some embodiments of formula (Vb), the compound is selected from: 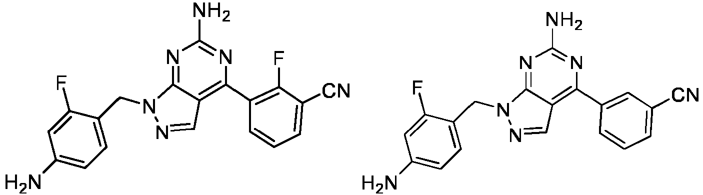 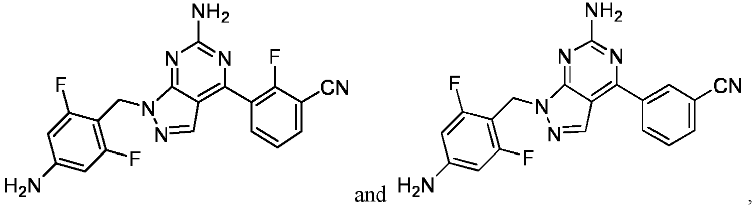 or a solvate, a hydrate and/or a stereoisomer thereof, or a pharmaceutically acceptable salt thereof.

In some embodiments of formula (IIb), the compound is of formula (VIb): 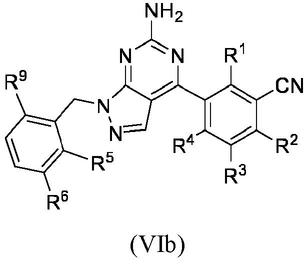 or a solvate, a hydrate and/or a stereoisomer thereof, or a pharmaceutically acceptable salt thereof.

In some embodiments of formula (VIb), R⁵, R⁶ and R⁹ are independently selected from H, (C₁-C₅)alkyl, substituted (C₁-C₅)alkyl, (C₁-C₃)haloalkyl, (C₁-C₅)alkoxy, substituted (C₁-C₅)alkoxy, -NH₂, substituted amino, halogen, and hydroxyl. In some embodiments of formula (VIb), R⁶ is selected from H, (C₁-C₅)alkyl, substituted (C₁-C₅)alkyl, and (C₁-C₃)haloalkyl. In some embodiments of formula (VIb), R⁶ is CH₃ or CF₃. In some embodiments of formula (VIb), R⁶ is H.

In some embodiments of formula (VIb), R⁵ and R⁹ are independently selected from H and halogen. In some embodiments of formula (VIb), R⁵ is F. In some embodiments of formula (VIb), R⁹ is F. In some embodiments of formula (VIb), R⁵ and R⁹ are each F. In some embodiments of formula (VIb), R⁵ is H and R⁹ is F.

In some embodiments of formula (VIb), the compound is selected from: 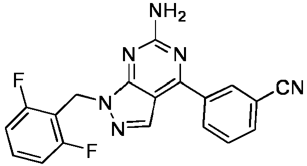 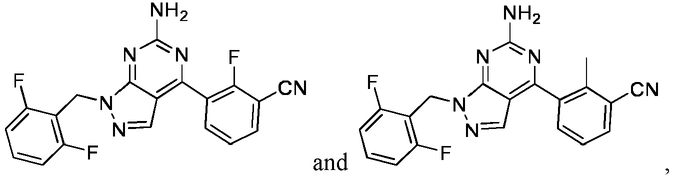 or a solvate, a hydrate and/or a stereoisomer thereof, or a pharmaceutically acceptable salt thereof.

In some embodiments of formula (IIb)-(VIb), R¹ to R⁴ are independently selected from H, (C₁-C₅)alkyl, substituted (C₁-C₅)alkyl, (C₁-C₃)haloalkyl, (C₁-C₅)alkoxy, substituted (C₁-C₅)alkoxy, -NH₂, substituted amino, halogen, and hydroxyl. In some embodiments of formula (IIb)-(VIb), R¹ to R⁴ are independently selected from H, (C₁-C₅)alkyl, substituted (C₁-C₅)alkyl, (C₁-C₃)haloalkyl and halogen.

In some embodiments of formula (IIb)-(VIb), R¹ is H. In some embodiments of formula (IIb)-(VIb), R¹ is selected from (C₁-C₅)alkyl, substituted (C₁-C₅)alkyl, (C₁-C₃)haloalkyl and halogen. In some embodiments of formula (IIb)-(VIb), R¹ is F, CH₃ or CF₃. In some embodiments of formula (IIb)-(VIb), R², R³ or R⁴ is selected from (C₁-C₅)alkyl, substituted (C₁-C₅)alkyl, (C₁-C₃)haloalkyl and halogen. In some embodiments of formula (IIb)-(VIb), R², R³ and R⁴ are each H.

In some embodiments, the compound is of formula (XIVb): 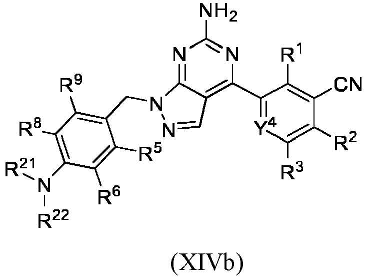 wherein:
Y⁴ is CR⁴;
R¹ to R⁴ are independently selected from H, (C₁-C₈)alkyl, substituted (C₁-C₈)alkyl, (C₂-C₈)alkenyl, substituted (C₂-C₈)alkenyl, (C₂-C₈)alkynyl, substituted (C₂-C₈)alkynyl, (C₁-C₃)haloalkyl, (C₁-C₈)alkoxy, substituted (C₁-C₈)alkoxy, -CONH₂, substituted amido, - NH₂, substituted amino, -CO₂H, cyano, halogen, hydroxyl, -NO₂, -SO₃H, -SO₂NH₂, substituted sulfonamide, and thiol; and
R²¹ and R²² are independently selected from H, (C₁-C₈)alkyl, substituted (C₁-C₈)alkyl, SO₂R³⁰, and COR³⁰ , wherein R³⁰ is (C₁-C₈)alkyl, or substituted (C₁-C₈)alkyl,
or a solvate, a hydrate and/or a stereoisomer thereof, or a pharmaceutically acceptable salt thereof.

In some embodiments of formula (XIVb), R⁵, R⁶, R⁸ and R⁹ are independently selected from H, (C₁-C₅)alkyl, substituted (C₁-C₅)alkyl, (C₁-C₃)haloalkyl, (C₁-C₅)alkyl, substituted (C₁-C₅)alkyl, halogen, and hydroxyl.

In some embodiments of formula (XIVb), R²¹ and R²² are each H.

In some embodiments of formula (XIVb), R² to R⁴ are each H; and R¹ is selected from H, (C₁-C₅)alkyl, substituted (C₁-C₅)alkyl, (C₁-C₃)haloalkyl, (C₁-C₅)alkyl, substituted (C₁-C₅)alkyl, halogen, and hydroxyl. In some embodiments of formula (XIVb), R¹ is selected from H, F, CH₃, and CF₃.

In some embodiments of formula (XIVb), the compound is of formula (XVb): 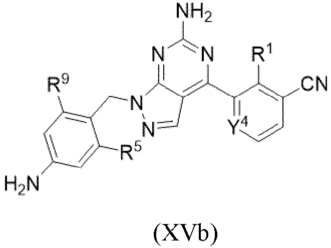 or a solvate, a hydrate and/or a stereoisomer thereof, or a pharmaceutically acceptable salt thereof.

In some embodiments of formula (XVb), R⁵ and R⁹ are independently selected from H, (C₁-C₅)alkyl, substituted (C₁-C₅)alkyl, (C₁-C₃)haloalkyl, (C₁-C₅)alkyl, substituted (C₁-C₅)alkoxy, halogen, and hydroxyl. In some embodiments of formula (XVb), R⁵ and R⁹ are independently selected from H and halogen. In some embodiments of formula (XVb), R⁵ is F. In some embodiments of formula (XVb), R⁹ is F. In some embodiments of formula (XVb), R⁵ and R⁹ are each F. In some embodiments of formula (XVb), R⁵ is H and R⁹ is F.

In some embodiments of formula (XVb), R¹ is selected from H, (C₁-C₅)alkyl, substituted (C₁-C₅)alkyl, (C₁-C₃)haloalkyl, (C₁-C₅)alkyl, substituted (C₁-C₅)alkyl, halogen, and hydroxyl. In some embodiments of formula (XVb), R¹ is selected from H, F, CH₃, and CF₃.

In formula (XVb), Y⁴ is CH.

In some embodiments of formula (XVb), the compound is: 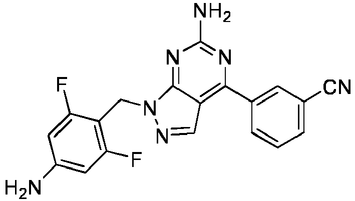 or a solvate, a hydrate and/or a stereoisomer thereof, or a pharmaceutically acceptable salt thereof.

In some embodiments of formula (XIVb), the compound is of formula (XVIb): 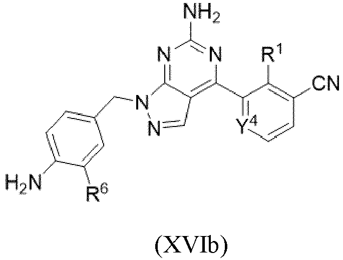 or a solvate, a hydrate and/or a stereoisomer thereof, or a pharmaceutically acceptable salt thereof.

In some embodiments of formula (XVIb), R⁶ is selected from (C₁-C₅)alkyl, substituted (C₁-C₅)alkyl, (C₁-C₃)haloalkyl, (C₁-C₅)alkyl, substituted (C₁-C₅)alkyl, halogen, and hydroxyl. In some embodiments of formula (XVIb), R⁶ is selected from (C₁-Cs)alkyl, substituted (C₁-C₅)alkyl, and (C₁-C₃)haloalkyl. In some embodiments of formula (XVIb), R⁶ is CH₃ or CF₃.

In some embodiments of formula (XVIb), R¹ is selected from H, (C₁-C₅)alkyl, substituted (C₁-C₅)alkyl, (C₁-C₃)haloalkyl, (C₁-C₅)alkyl, substituted (C₁-C₅)alkyl, halogen, and hydroxyl. In some embodiments of formula (XVIb), R¹ is selected from H, F, CH₃, and CF₃.

In formula (XVIb), Y⁴ is CH.

In some embodiments of formula (XVIb), the compound is: 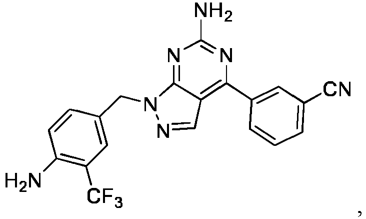 or a solvate, a hydrate and/or a stereoisomer thereof, or a pharmaceutically acceptable salt thereof.

The compound is a pyrazolo-pyrimidine compound, or a salt (e.g., a pharmaceutically acceptable salt) thereof.

In some embodiments of formula (IIb), the compound is selected from:
3-(6-amino-1-(4-aminobenzyl)-1H-pyrazolo-[3,4-d]-pyrimidine-4-yl)-benzonitrile;
3-(6-amino-1-(4-aminobenzyl)-1H-pyrazolo-[3,4-d]-pyrimidine-4-yl)-2-methylbenzonitrile; and
3-(6-amino-1-(4-aminobenzyl)-1H-pyrazolo-[3,4-d]-pyrimidine-4-yl)-2-fluorobenzonitrile.

It is understood that all variations of salts, and/or solvates, hydrates and/or stereoisomers of the compounds described herein, or shown in Table 1 are meant to be encompassed by the present disclosure. Accordingly, any of the compounds described herein may also be referred to as a compound of the invention or a solvate, a hydrate and/or a stereoisomer thereof, or a pharmaceutically acceptable salt thereof.

In some embodiments, the compound is represented by the structure of one of the compounds in Table 1, or a solvate, a hydrate and/or a stereoisomer thereof, or a salt (e.g., a pharmaceutically acceptable salt) thereof. The present disclosure is meant to encompass, a compound of any one of Table 1, or a salt thereof, and/or a solvate, a hydrate, a single stereoisomer, a mixture of stereoisomers and/or an isotopically labelled form thereof. For example, salts of a solvate, hydrate and/or stereoisomer form of any of the compounds described herein, or shown in Table 1 are meant to be encompassed by the present disclosure. However, Compounds 101-138 and 215-238 are not according to the invention and are presented for illustration purposes only.

| **Table 1: Exemplary A2A and/or A1 Receptor Antagonist Compounds** | | | |
|---|---|---|---|
| **Cmpd. No.** | **Compounds of Formula (Ia)** | **Cmpd No.** | **Compounds of Formula (Ib)** |
| 101 | 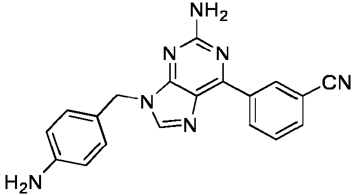 | 201 | 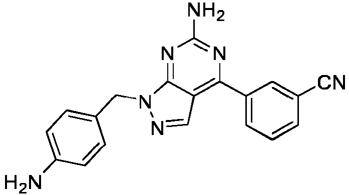 |
| 102 | 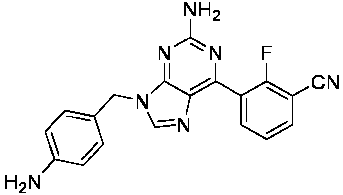 | 202 | 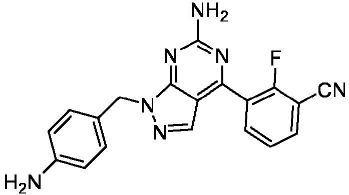 |
| 103 | 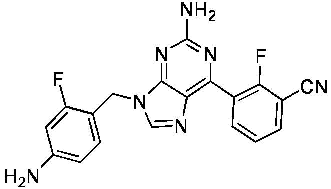 | 203 | 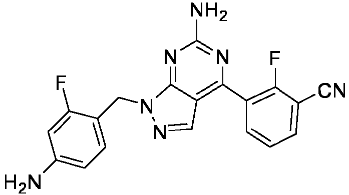 |
| 104 | 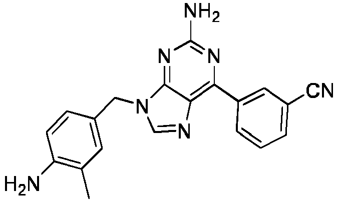 | 204 | 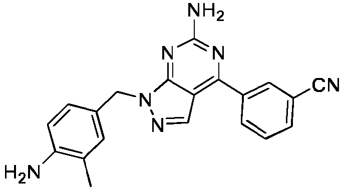 |
| 105 | 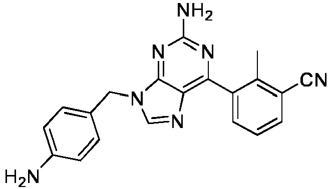 | 205 | 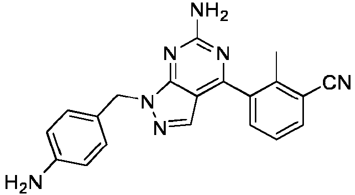 |
| 106 | 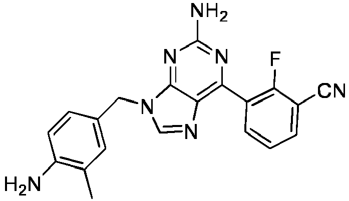 | 206 | 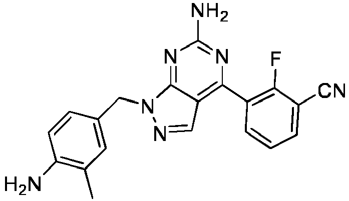 |
| 107 | 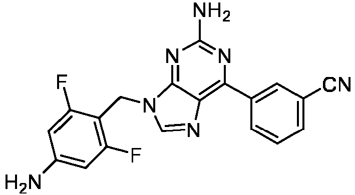 | 207 | 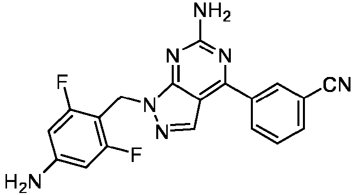 |
| 108 | 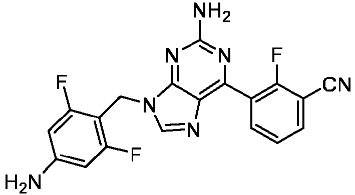 | 208 | 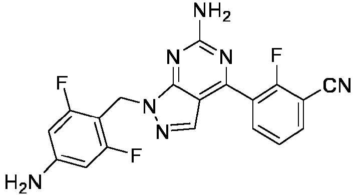 |
| 109 | 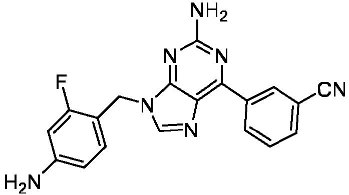 | 209 | 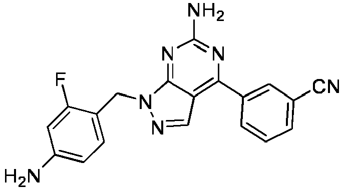 |
| 110 | 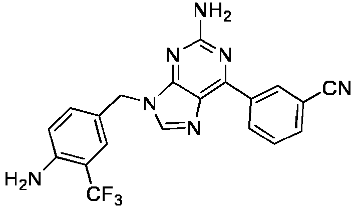 | 210 | 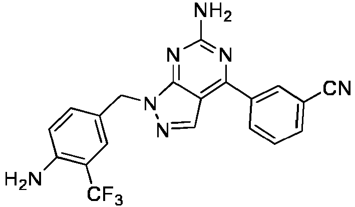 |
| 111 | 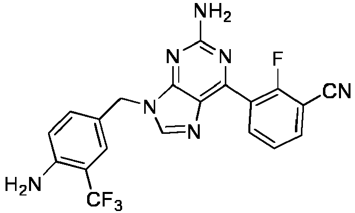 | 211 | 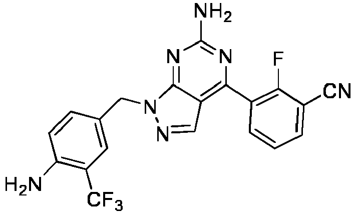 |
| 112 | 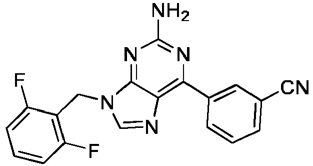 | 212 | 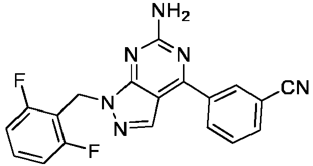 |
| 113 | 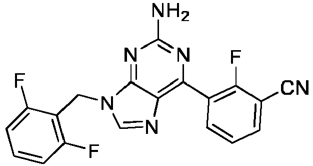 | 213 | 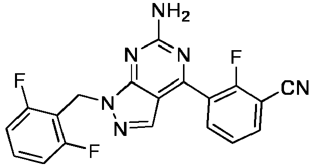 |
| 114 | 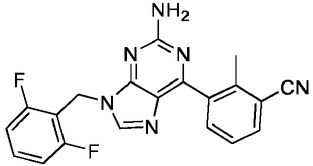 | 214 | 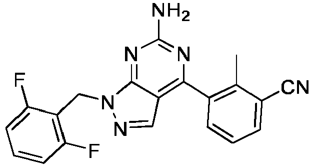 |
| 115 | 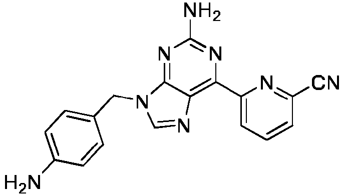 | 215 | 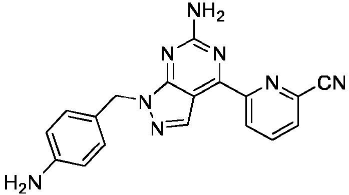 |
| 116 | 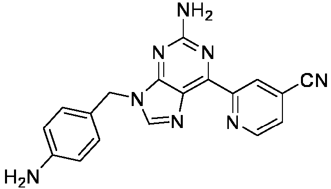 | 216 | 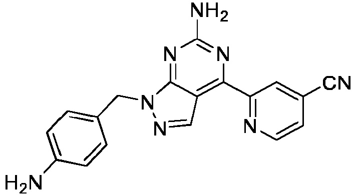 |
| 117 | 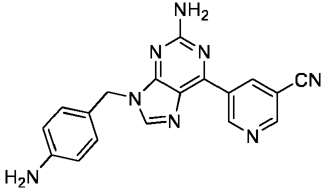 | 217 | 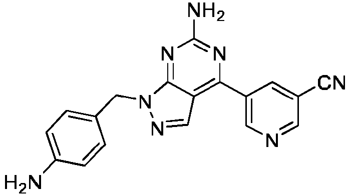 |
| 118 | 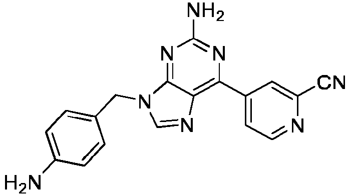 | 218 | 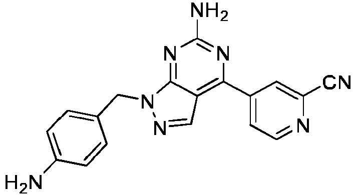 |
| 119 | 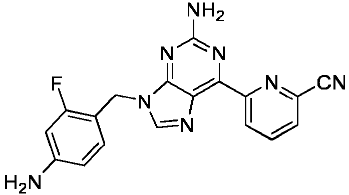 | 219 | 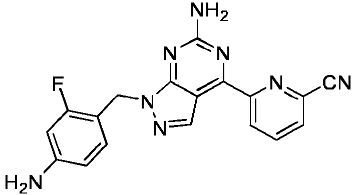 |
| 120 | 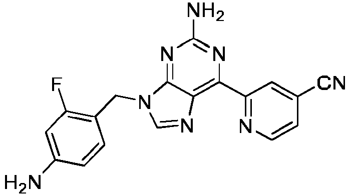 | 220 | 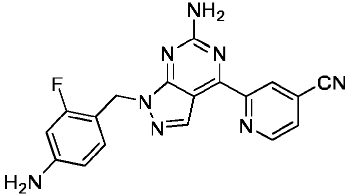 |
| 121 | 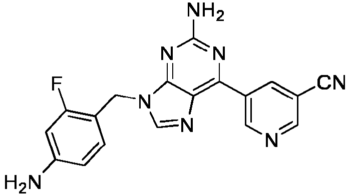 | 221 | 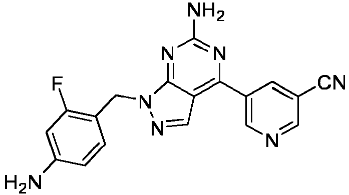 |
| 122 | 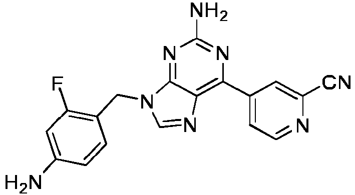 | 222 | 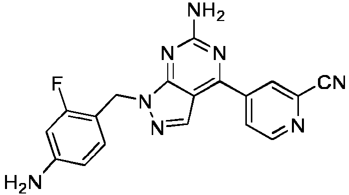 |
| 123 | 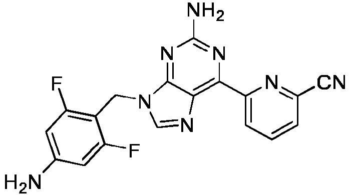 | 223 | 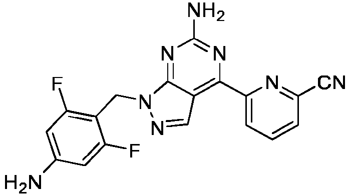 |
| 124 | 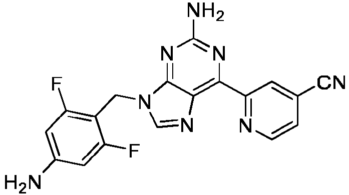 | 224 | 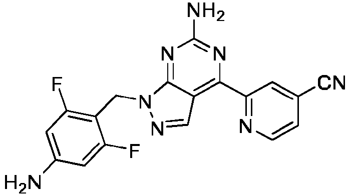 |
| 125 | 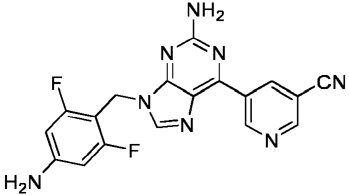 | 225 | 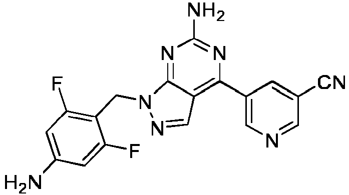 |
| 126 | 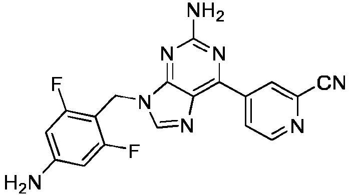 | 226 | 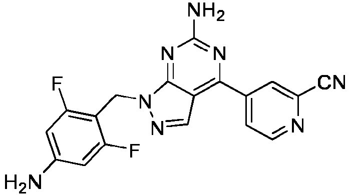 |
| 127 | 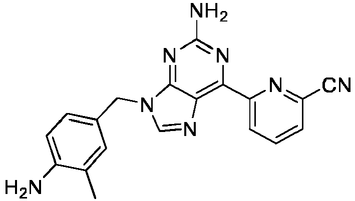 | 227 | 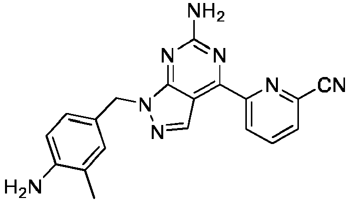 |
| 128 | 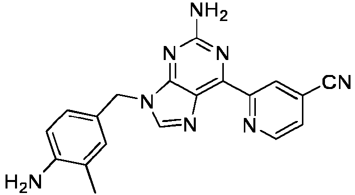 | 228 | 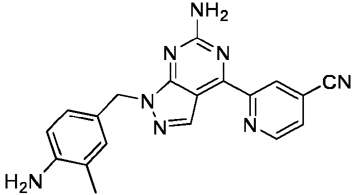 |
| 129 | 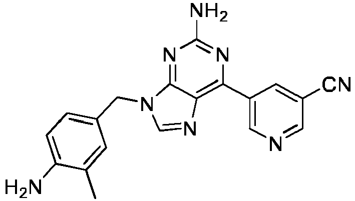 | 229 | 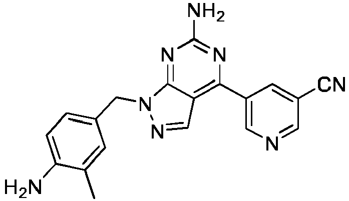 |
| 130 | 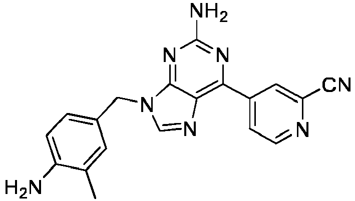 | 230 | 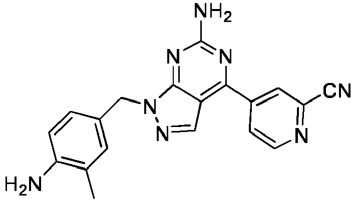 |
| 131 | 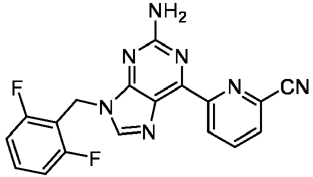 | 231 | 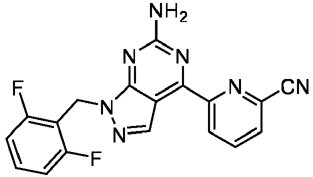 |
| 132 | 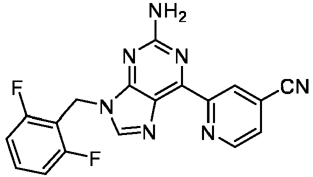 | 232 | 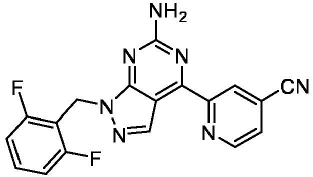 |
| 133 | 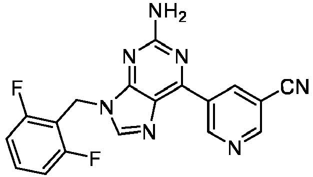 | 233 | 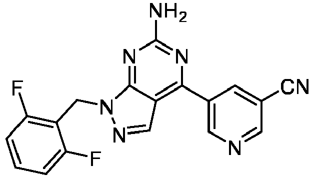 |
| 134 | 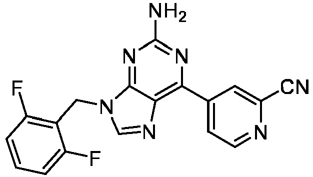 | 234 | 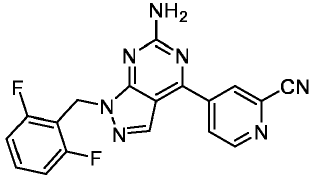 |
| 135 | 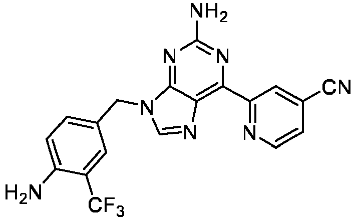 | 235 | 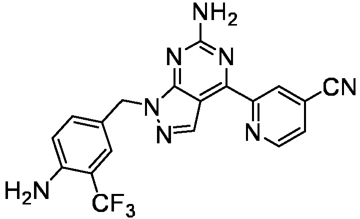 |
| 136 | 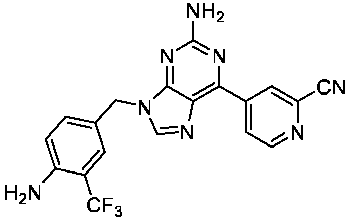 | 236 | 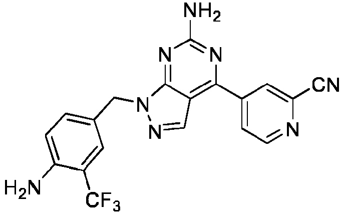 |
| 137 | 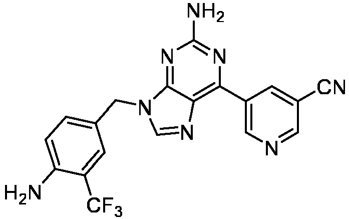 | 237 | 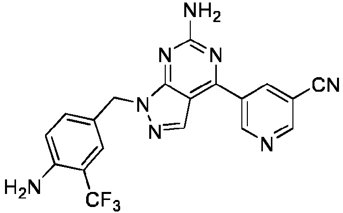 |
| 138 | 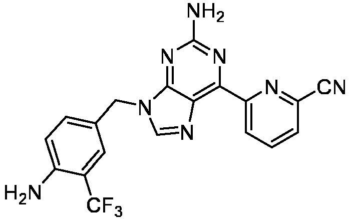 | 238 | 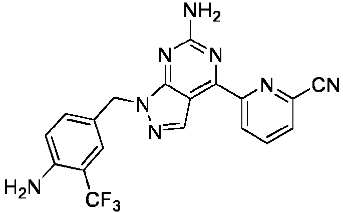 |

### Isotopically Labelled Analogs

The present disclosure also encompasses isotopically-labeled compounds which are identical to those compounds as described herein, except that one or more atoms are replaced by an atom having an atomic mass or mass number different from the atomic mass or mass number usually found in nature ("isotopologues"). The compounds of the present disclosure may also contain unnatural proportions of atomic isotopes at one or more atoms that constituted such compounds. Examples of isotopes that can be incorporated into compounds described herein include isotopes of hydrogen, carbon, nitrogen, oxygen, phosphorus, fluorine and chlorine, such as ²H ("D"), ³H, ¹³C, ¹⁴C, ¹⁵N, ¹⁸O, ¹⁷O, ³¹P, ³²P, ³⁵S, ¹⁸F, and ³⁶Cl, respectively. For example, a compound described herein can have one or more H atoms replaced with deuterium.

Generally, reference to or depiction of a certain element such as hydrogen or H is meant to include all isotopes of that element. For example, if an R group is defined to include hydrogen or H, it also includes deuterium and tritium. Compounds comprising radioisotopes such as tritium, ¹⁴C, ³²P and ³⁵S are thus within the scope of the present technology. Procedures for inserting such labels into the compounds of the present technology will be readily apparent to those skilled in the art based on the disclosure herein.

Unless otherwise stated, compounds described herein are intended to include compounds which differ only in the presence of one or more isotopically enriched atoms. For example, compounds having the present structures except for the replacement of a hydrogen by a deuterium or tritium, or the replacement of a carbon by ¹³C- or ¹⁴C- enriched carbon are within the scope of the present disclosure.

In some embodiments, certain isotopically-labeled compounds, such as those labeled with ³H and ¹⁴C, can be useful in compound and/or substrate tissue distribution assays. Tritiated (³H) and carbon-14 (¹⁴C) isotopes can be particularly preferred for their ease of preparation and detectability. Further, substitution with heavier isotopes such as deuterium can afford certain therapeutic advantages resulting from greater metabolic stability, such as increased in vivo half-life or reduced dosage requirements, and hence can be preferred in some circumstances. Isotopically-labeled compounds can generally be prepared by following procedures analogous to those disclosed herein, for example, in the Examples section, by substituting an isotopically-labeled reagent for a non-isotopically-labeled reagent.

In some embodiments, the compounds disclosed in the present disclosure are deuterated analogs of any of the compounds, or a salt thereof, as described herein. A deuterated analog of a compound of the invention is a compound where one or more hydrogen atoms are substituted with a deuterium.

Deuterium substituted compounds are synthesized using various methods such as described in: Dean, Dennis C.; Editor. Recent Advances in the Synthesis and Applications of Radiolabeled Compounds for Drug Discovery and Development. [In: Curr., Pharm. Des., 2000; 6(10)] 2000, 110 pp; George W.; Varma, Rajender S. The Synthesis of Radiolabeled Compounds via Organometallic Intermediates, Tetrahedron, 1989, 45(21), 6601-21; and Evans, E. Anthony. Synthesis of radiolabeled compounds, J. Radioanal. Chem., 1981, 64(1-2), 9-32.

Deuterated starting materials are readily available and are subjected to the synthetic methods described herein to provide for the synthesis of deuterium-containing compounds. Large numbers of deuterium-containing reagents and building blocks are available commercially from chemical vendors, such as Aldrich Chemical Co.

### Isomers

The term "compound", as used herein, is meant to include all stereoisomers, geometric isomers, tautomers, and isotopes of the structures depicted.

The compounds herein described may have asymmetric centers, geometric centers (e.g., double bond), or both. All chiral, diastereomeric, racemic forms and all geometric isomeric forms of a structure are intended, unless the specific stereochemistry or isomeric form is specifically indicated. In some embodiments, the compounds described herein have one or more chiral centers. It is understood that if an absolute stereochemistry is not expressly indicated, then each chiral center may independently be of the R-configuration or the S-configuration or a mixture thereof. Thus, compounds described herein include enriched or resolved optical isomers at any or all asymmetric atoms as are apparent from the depictions. Racemic mixtures of R-enantiomer and S-enantiomer, and enantio-enriched stereoisomeric mixtures comprising of R- and S-enantiomers, as well as the individual optical isomers can be isolated or synthesized so as to be substantially free of their enantiomeric or diastereomeric partners, and these stereoisomers are all within the scope of the present technology.

Compounds of the present disclosure containing an asymmetrically substituted atom may be isolated in optically active or racemic forms. It is well known in the art how to prepare optically active forms, such as by resolution of racemic forms, by synthesis from optically active starting materials, or through use of chiral auxiliaries.

Geometric isomers, resulting from the arrangement of substituents around a carbon-carbon double bond or arrangement of substituents around a cycloalkyl or heterocyclic ring, can also exist in the compounds of the present disclosure. Geometric isomers of olefins, C=N double bonds, or other types of double bonds may be present in the compounds described herein, and all such stable isomers are included in the present disclosure. Specifically, cis and trans geometric isomers of the compounds of the present disclosure may also exist and may be isolated as a mixture of isomers or as separated isomeric forms.

Compounds of the present disclosure also include tautomeric forms. Tautomeric forms result from the swapping of a single bond with an adjacent double bond and the concomitant migration of a proton. Tautomeric forms include prototropic tautomers which are isomeric protonation states having the same empirical formula and total charge. Examples prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, such as, 1H- and 3H-imidazole, 1H-, 2H- and 4H- 1,2,4-triazole, 1H- and 2H- isoindole, and 1H- and 2H-pyrazole. Tautomeric forms can be in equilibrium or sterically locked into one form by appropriate substitution.

### Salts and other forms

In some embodiments, the compounds described herein are present in a salt form. In some embodiments, the compounds are provided in the form of pharmaceutically acceptable salts.

Compounds included in the present compositions that are basic in nature are capable of forming a wide variety of salts with various inorganic and organic acids. The acids that can be used to prepare pharmaceutically acceptable acid addition salts of such basic compounds are those that form non-toxic acid addition salts, *i.e.,* salts containing pharmacologically acceptable anions, including but not limited to, chloride.

Compounds containing an amine functional group or a nitrogen-containing heteroaryl group may be basic in nature and may react with a variety of inorganic and organic acids to form the corresponding salts. The compounds could be used in the form of a pharmaceutically acceptable salt derived from inorganic acid or organic acid. In some embodiments, the pharmaceutically acceptable salt could be a salt derived from hydrochloric acid (i.e., a hydrochloride salt of a compound as described herein), or the like.

The pharmaceutically acceptable salts of the compounds of this disclosure could be produced by dissolving the compound in a water-miscible organic solvent, such as acetone, methanol, ethanol, or acetonitrile, and so on, and adding excessive amount of organic acid or inorganic acid aqueous solution and precipitating or crystalizing. Then, it is possible to obtain additional salt by evaporating the solvent or excessive acid from this mixture and then drying it or by produce salt by filtering extracted salt.

Other examples of salts include anions of the compounds of the present disclosure compounded with a suitable cation. For therapeutic use, salts of the compounds of the present disclosure can be pharmaceutically acceptable. However, salts of acids and bases that are non-pharmaceutically acceptable may also find use, for example, in the preparation or purification of a pharmaceutically acceptable compound.

Compounds included in the present compositions that are acidic in nature are capable of forming base salts with various pharmacologically acceptable cations. Examples of such salts include alkali metal or alkaline earth metal salts.

Compounds that include a basic or acidic moiety can also form pharmaceutically acceptable salts with various amino acids. The compounds of the disclosure can contain both acidic and basic groups; for example, one amino and one carboxylic acid group. In such a case, the compound can exist as an acid addition salt, a zwitterion, or a base salt.

The compounds described herein can be present in various forms including crystalline, powder and amorphous forms of those compounds, pharmaceutically acceptable salts, including, for example, polymorphs, pseudopolymorphs, solvates, hydrates, unsolvated polymorphs (including anhydrates), conformational polymorphs, and amorphous forms of the compounds, as well as mixtures thereof. It is understood that a salt form of any one of the subject compounds can be present in a crystalline, powder or amorphous form.

The compounds described herein may exist as solvates, especially hydrates, and unless otherwise specified, all such solvates and hydrates are intended. The term "solvate" could refer to the compound of the present disclosure containing a stoichiometric or non-stoichiometric solvent combined by non-covalent intermolecular force or its salt. Hydrates may form during manufacture of the compounds or compositions comprising the compounds, or hydrates may form over time due to the hygroscopic nature of the compounds. Compounds of the present technology may exist as organic solvates as well, including dimethylformamide (DMF), ether, and alcohol solvates, among others. The identification and preparation of any particular solvate is within the skill of the ordinary artisan of synthetic organic or medicinal chemistry.

In some embodiments, the compounds described herein are present in a solvate form. In some embodiments, the compounds described herein are present in a hydrate form when the solvent component of the solvate is water.

### Compound synthesis

Compounds of the present disclosure may be synthesized according to standard methods known in the art [see, e.g. Morrison and Boyd in "Organic Chemistry", 6th edition, Prentice Hall (1992)]. Some compounds and/or intermediates of the present disclosure may be commercially available, known in the literature, or readily obtainable by those skilled in the art using standard procedures. Some compounds of the present disclosure may be synthesized using schemes, examples, or intermediates described herein. Where the synthesis of a compound, intermediate or variant thereof is not fully described, those skilled in the art can recognize that the reaction time, number of equivalents of reagents and/or temperature may be modified from reactions described herein to prepare compounds presented or intermediates or variants thereof and that different work-up and/or purification techniques may be necessary or desirable to prepare such compounds, intermediates, or variants.

Synthesized compounds may be validated for proper structure by methods known to those skilled in the art, for example by nuclear magnetic resonance (NMR) spectroscopy and/or mass spectrometry.

### Pharmaceutical Compositions

Compounds of the present disclosure may be formulated in pharmaceutical compositions. The pharmaceutical composition can include one or more such A2A and/or A1 receptor antagonist compounds (e.g., as described herein) and at least one excipient (e.g., a pharmaceutically acceptable excipient).

The compounds described herein can find use in pharmaceutical compositions for administration to a subject in need thereof in a variety of therapeutic applications where inhibition or antagonism of the activity of A2A and/or A1 receptor is desirable.

Accordingly, in a second aspect, the present disclosure provides pharmaceutical compositions comprising at least one compound described herein, a pharmaceutically acceptable salt thereof and at least one pharmaceutically acceptable excipient. The phrase "pharmaceutically acceptable excipient," refers any ingredient other than the inventive compounds described herein (for example, a vehicle capable of suspending or dissolving the active compound, or any other convenient pharmaceutically acceptable carriers, excipients or additives) and having the properties of being substantially nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, dispensing, or dispersing agents, sweeteners, and waters of hydration. In some embodiments, the pharmaceutical composition comprises a compound as described herein, or a pharmaceutically acceptable salt thereof in a therapeutically effective amount.

The pharmaceutical composition may be formulated according to any convenient methods, and may be prepared in various forms for oral administration such as tablets, pills, powders, nanoparticles, capsules, syrups, suspensions, emulsions and microemulsions, or in forms for non-oral administration such as preparations for intramuscular, intravenous or subcutaneous administration.

In a specific example, the pharmaceutical composition could contain a pharmaceutically allowed carrier, excipient, or additive. The pharmaceutical composition could be produced as medicine in the conventional method, and could be produced as various oral medicine such as tablet, pill, powder, capsule, syrup, emulsion, micro-emulsion, and so on, or could be produced as non-oral medicine such as muscular injection, vascular injection, or subcutaneous injection.

If the pharmaceutical composition is produced in the form of an oral medicine, examples of the used additive or carrier could include cellulose, silicic calcium, corn starch, lactose, sucrose, dextrose, phosphoric acid calcium, stearic acid, stearic acid magnesium, stearic acid calcium, gelatin, talc, surfactant, suspension, emulsifying agent, diluting agent, and so on. If the pharmaceutical composition of this disclosure is produced in the form of an injection, the additives or carrier could include water, saline water, glucose aqueous solution, similar sugar-soluble solution, alcohol, glycol, ether (e.g., polyethylene glycol 400), oil, fatty acid, fatty acid ester, glyceride, surfactant, suspension, emulsifying agent, and so on.

In some embodiments, the pharmaceutical compositions are formulated for parenteral administration to a subject in need thereof. In some parenteral embodiments, the pharmaceutical compositions are formulated for intravenous administration to a subject in need thereof. In some parenteral embodiments, the pharmaceutical compositions are formulated for subcutaneous administration to a subject in need thereof.

### Methods of Modulating A2A and/or A1 Receptor

The references to methods of treatment in the subsequent paragraphs of this description are to be interpreted as references to the compounds, pharmaceutical compositions and medicaments of the present invention for use in a method for treatment of the human (or animal) body by therapy.

Aspects of the present disclosure include methods of modulating the activity of A2A and/or A1 receptor in a biological system or sample. In some embodiments, modulating the activity of A2A and/or A1 receptor refers to inhibiting an A2A and/or A1 receptor in a sample. In some embodiments, modulating the activity of A2A and/or A1 receptor refers to antagonizing the activity of an A2A and/or A1 receptor in a cell or biological system.

In some embodiments, the method includes contacting a sample with a compound of this disclosure which can modulate A2A and/or A1 receptor activity. In some embodiments, the method includes contacting a cell or biological system with a compound of this disclosure which can modulate A2A and/or A1 receptor activity.

The present disclosure provides compounds having A2A and/or A1 receptor modulating activity, e.g., A2A and/or A1 receptor inhibition and/or antagonistic activity. In some embodiments, the compound has A2A receptor inhibition and/or antagonist activity. In some embodiments, the compound has A2A and A1 receptor inhibition and/or antagonist activity. In some embodiments, the compound has A1 receptor inhibition and/or antagonist activity. The ability of the compounds to modulate A2A and/or A1 receptor activity may be characterized using a variety of assays, e.g., by an A2A and/or A1 receptor binding assay and/or an A2A receptor functional assay. For example, the experimental section describes A2A receptor binding assays. Exemplary compounds of this disclosure were assessed, and Kᵢ values determined, to show that the compounds of this disclosure can provide specific binding to human A2A receptor in an assay system. In addition, the experimental section describes A2A functional assays involving monitoring cAMP signals produced in a human recombinant A2A receptor stable cell line. Exemplary compounds of this disclosure were assessed, and IC₅₀ values obtained, indicating that the compounds can antagonize human A2A receptor in an assay system.

Aspects of the present disclosure include methods of modulating (e.g., inhibiting or antagonizing) A2A and/or A1 receptor using a compound described herein. Such methods may include methods of modulating A2A and/or A1 receptor in biological systems by contacting such systems with of the invention or the compounds of Table 1). Biological systems may include, but are not limited to, cells, tissues, organs, bodily fluids, organisms, non-mammalian subjects, and mammalian subjects (e.g., humans). Biological systems may include subjects (e.g., human subjects).

In some embodiments, the method of inhibiting or antagonizing A2A and/or A1 receptor comprises contacting a biological system or sample comprising A2A and/or A1 receptor with an effective amount of any of the compounds or a pharmaceutically acceptable salt thereof as described herein, or a pharmaceutical composition as described herein to inhibit or antagonize the A2A and/or A1 receptor. In certain embodiments, the biological system or sample is *in vitro.* In another embodiment, the biological system or sample is *in vivo.* In some embodiments, the sample is a cell sample. In some embodiments, the A2A and/or A1 receptor is cell membrane bound.

The compounds of this disclosure may antagonize an A2A receptor, e.g., as assessed by a human A2A functional assay, e.g., as described in Example 3. The A2A receptor antagonist compounds according to such methods may have IC₅₀ values (e.g., as assessed by the cAMP assay of Example 3) of 10 µM or less, such as 2µM or less, 1µM or less, 500 nM or less, 300 nM or less, 100nM or less, 30 nM or less, or 10 nM or less. Biological systems may include subjects (e.g., human subjects). The A2A receptor antagonist compounds according to such methods may have Ki values (e.g., as assessed by the binding assay of Example 2) of 1µM or less, such as 500 nM or less, 300 nM or less, 100nM or less, 30 nM or less, 10 nM or less, 3 nM or less, or 1 nM or less.

In some embodiments, the compound is an A2A receptor antagonist that exhibits a desirable level of inhibition activity at A1 receptor. In some embodiments, the compound is a dual antagonist of the A2A receptor and the A1 receptor. In some embodiments, a desirable level of A1 receptor inhibition activity refers to compound having a Ki value at a human A1 receptor in a cell model (e.g., as assessed in Example 4.1) that is 1 mM or lower, such as 300 µM or lower, 100 µM or lower, 30 µM or lower, 10 µM or lower, 3 µM or lower, or 1 µM or lower. In some embodiments, the compound of this disclosure exhibits inhibition activity for A2A receptor and A1 receptor. Example 4, Table 5, of the experimental section, provides A2A and A1 receptor inhibition data which indicates compounds of this disclosure can have potent activity as dual antagonists. In such cases, the compounds can find use in treatment of CNS and neurodegenerative diseases.

### Therapeutic Indications

Aspects of the present disclosure include methods of treating a subject for a therapeutic indication of interest using compounds and/or compositions disclosed herein. The term "therapeutic indication" refers to any symptom, condition, disorder, or disease that may be alleviated, stabilized, improved, cured, or otherwise addressed in a subject by some form of treatment or other therapeutic intervention (e.g., through A2A and/or A1 receptor antagonist administration). Therapeutic indications associated with modulation of A2A and/or A1 receptor biological activity and/or dysfunction are referred to herein as "adenosine receptor-related indications." In some embodiments, methods of the present disclosure may include treating adenosine receptor-related indications by administering an effective amount of compounds and/or compositions disclosed herein (e.g., A2A and/or A1 receptor antagonist compounds) to a subject.

The term "to treat" or "treatment" refers to inhibiting an illness, for example inhibiting an illness, condition, or disability in an subject that experiences the pathology or symptoms of the illness, condition, or disorder, namely preventing additional occurrence of pathology and/or symptoms, or improving the illness, for example improving the illness, condition or disorder in the subject that experiences or exhibits the pathology or symptoms of the illness, condition, or disorder, namely reversing the pathology and/or symptoms, for example reducing the severity of the illness. In the context of the present disclosure insofar as it relates to any of the other conditions recited herein below, the terms "treat," "treatment," and the like mean to relieve or alleviate at least one symptom associated with such condition, or to slow or reverse the progression or anticipated progression of such condition.

The term "to prevention" or "prevention" refers to preventing an illness, for example preventing an illness, condition or disorder in an entity that has not experienced or exhibited the pathology or symptoms of the illness even though the entity may have a tendency for the illness, condition or disorder.

The terms "individual", "patient", "subject" and "entity" are used interchangeably and refer to a human person or non-human animal that requires treatment of a disease. More specifically, what is referred to is a mammal such as a human, nonhuman primate, mouse, dog, cat, horse, cow, rabbit, rat, or other mammal.

### Cancer and Immuno-Oncology

The present disclosure provides a method of treating or preventing cancer in a subject using the subject compounds as therapeutic agents, and compositions including the compounds. Any cancer for which an A2A receptor antagonist are thought to be useful by those of ordinary skill in the art are contemplated as cancers treatable by this embodiment, either as a monotherapy or in combination with other therapeutic agents discussed below.

The illness subject to prevention or treatment by the aforementioned pharmaceutical composition, which is "cancer," refers collectively to the illness caused by cells wherein cells have aggressive characteristics such as dividing and growing by ignoring normal growth limit, invasive characteristics whereby cells invade the surrounding tissues, and metastatic characteristics whereby cells spread to other areas of the body. In some embodiments, the cancer is a solid tumor cancer. Cancers which are the object of prevention or therapy using the compounds and pharmaceutical compositions of this disclosure include, but are not limited to, lung cancer, breast cancer, prostate cancer, ovarian cancer, solenoma, cervical cancer, bladder cancer, head and neck cancer, renal cell carcinoma, cancer of the esophagus, pancreatic cancer, brain cancer, liver cancer, leukemia, lymphoma, melanoma, multiple myeloma, Ewing's sarcoma, osteosarcoma, colorectal neoplasm, cholangiocarcinoma, choriocarcinoma, mouth cancer, neuroblastoma, skin cancer, testicular cancer, stromal tumor, germ cell tumor, and thyroid cancer.

In some embodiments, the cancer is cancer of the kidney, breast, lung, or liver. In some embodiments, the cancer is a solid tumor cancer. In some embodiments, the liver cancer is hepatocellular carcinoma (HCC). In some embodiments, the lung cancer is non-small cell lung carcinoma (NSCLC). In some embodiments, the cancer is a solid tumor cancer.

In some embodiments, the method further comprises identifying a subject suffering from or having cancer.

The compounds of this disclosure can be administered alone or in combination with one or more additional agents described herein. Accordingly, aspects of the methods include co-administering to the subject an effective amount of a compound or composition of this disclosure and an effective amount of an additional active agent. The compound and additional active agent can be administered concurrently or sequentially.

In some embodiments of the methods, the additional active agent is an anticancer agent selected from anti-angiogenesis agent, anti-inflammatory agent, immune checkpoint inhibitor, poly ADP ribose polymerase (PARP) inhibitor, chemotherapeutic agent, and immunity anticancer agent.

Given the immunosuppressive role of adenosine, the administration of an A2A receptor antagonist of this disclosure can enhance the efficacy of an immunotherapy, such as an immune checkpoint inhibitor therapy. In some embodiments, the additional active agent is an immune checkpoint inhibitor selected from CTLA-4 inhibitor, PD-1 inhibitors, and PD-L1 inhibitor.

In some embodiments, the immune checkpoint inhibitor is an antibody or antibody fragment. In some embodiments, the additional therapeutic agent is an anti-PD-1 antibody. In another embodiment, the additional therapeutic agent is an anti-PD-L1 antibody. In some embodiments, the additional therapeutic agent is selected from pembrolizumab, nivolumab, atezolizumab, durvalumab, and avelumab.

### Inflammatory Disease

The present disclosure provides a method of treating or preventing an inflammatory disease in a subject using the subject compounds as therapeutic agents, and compositions including the compounds. Any inflammatory diseases for which an A2A receptor antagonist are thought to be useful by those of ordinary skill in the art are contemplated as diseases treatable using compounds of this disclosure, either as a monotherapy or in combination with other therapeutic agents (e.g., as described herein).

In some embodiments, the method includes administering to a subject having an inflammatory disease a therapeutically effective amount of an A2A and/or A1 receptor antagonist compound (e.g., as described herein). In some embodiments, the inflammatory disease is a chronic inflammatory disease, such as rheumatoid arthritis (RA). In some embodiments, the inflammatory disease is an acute inflammatory disease.

In some embodiments, the method further comprises identifying a subject suffering from or at risk of an inflammatory disease.

In some embodiments, the method further comprises identifying an underlying disease or condition associated with the inflammatory disease.

### CNS and Neurodegenerative Diseases

The present disclosure provides a method of treating or preventing central nervous system (CNS) or neurodegenerative diseases or disorders in a subject using the compounds of this disclosure as therapeutic agents, or pharmaceutical compositions including the compounds. In some embodiments, the CNS disease is referred to as a neurodegenerative disease or disorder of the CNS. CNS and neurodegenerative diseases, which are the object of prevention or therapy using the compounds and pharmaceutical compositions of this disclosure, are diseases that include, but are not limited to, Parkinson's disease (PD), Alzheimer's disease (AD), amyotrophic lateral sclerosis (ALS), multiple sclerosis, Huntington's disease (HD), depression, schizophrenia, and epilepsy.

In some embodiments, method is a method of treating or preventing Parkinson's disease, and includes administering to a subject having, or at risk of Parkinson's disease an effective amount of a compound of this disclosure. In some cases, the compound is one having dual inhibition activity at both A2A receptor and A1 receptor.

In some embodiments, the method further comprises identifying a subject suffering from, or at risk of, a CNS or neurodegenerative disease.

In some embodiments, the method further comprises identifying an underlying disease or condition associated with the CNS or neurodegenerative disease.

The amount of the aforementioned pharmaceutical composition administered is the effective amount to treat or prevent illness for an entity or patient, and could be administered orally or non-orally according to the purpose. When administered orally, the amount administered based on the active component is 0.01 to 1,000 mg per 1kg of body weight, more specifically 0.1 to 300mg per 1kg. When administered non-orally, 0.01 to 100mg based on the active component is administered per 1kg of body weight per day, and more specifically 0.1 to 50mg is administered once or several times. The amount administered for a specific entity or patient can be determined based on many related factors including the patient's weight, age, sex, health status, diet, time of administration, method of administration, severity of the illness, and so on, must be understood to be able to be increased or decreased appropriately by the specialist, and the aforementioned amount of administration does not limit the scope of the present disclosure in any way. A medical doctor or veterinarian with ordinary level of knowledge in the related technological field may determine and prescribe effective amount of the pharmaceutical composition. For example, a medical doctor or veterinarian may start with the amount of the compound under the present disclosure used in a pharmaceutical composition that is lower than the amount required to achieve the desired treatment effect, and may increase the amount administered gradually until the desired effect is achieved.

The compounds and compositions of this disclosure may be administered alone, in combination with a compound according to another example of the present disclosure, or in simultaneous, separate or sequential concomitant administration with at least one other therapeutic agent, for example with other pharmaceutical active ingredients described herein.

In a specific example, a pharmaceutical composition of this disclosure includes within its scope at least one of the compounds in accordance with a specific example of the effective treatment amount as effective component, or a pharmaceutical composition that is contained in combination with a pharmaceutical carrier. Arbitrarily, the compound in accordance with an embodiment of the present disclosure could be administered independently, in combination with a compound in accordance with another specific example, or simultaneously with one or more other treatment medications, for example simultaneously with an anticancer medicine (e.g., as described herein) or with an active pharmaceutical material, separately, or consecutively in conjunction.

### Definitions

Unless defined otherwise, all technical and scientific terms used herein have the same meaning as commonly understood by one of ordinary skill in the art to which this disclosure pertains.

It is understood that the definitions provided herein are not intended to be mutually exclusive. Accordingly, some chemical moieties may fall within the definition of more than one term.

The term "Cₓ-C_{y}" when used in conjunction with a chemical moiety, such as alkyl, alkenyl, or alkynyl is meant to include groups that contain from x to y carbons in the chain. For example, the term "C₁-C₆ alkyl" refers to substituted or unsubstituted saturated hydrocarbon groups, including straight-chain alkyl and branched-chain alkyl groups that contain from 1 to 6 carbons. In some embodiments, the term "(Cₓ-C_{y})alkylene" refers to a substituted or unsubstituted alkylene chain with from x to y carbons in the alkylene chain. For example "(Cₓ-C_{y})alkylene may be selected from methylene, ethylene, propylene, butylene, pentylene, and hexylene, any one of which is optionally substituted.

The term "**alkyl**" refers to an unbranched or branched saturated hydrocarbon chain. In some embodiments, alkyl as used herein has 1 to 20 carbon atoms ((C₁₋C₂₀)alkyl), 1 to 10 carbon atoms ((C₁₋C₁₀)alkyl), 1 to 8 carbon atoms ((C₁₋C₈)alkyl), 1 to 6 carbon atoms ((C₁₋C₆)alkyl), 1 to 5 carbon atoms ((C₁₋C₅)alkyl, or 1 to 3 carbon atoms (C₁₋C₅)alkyl. Examples include, but are not limited to, methyl, ethyl, n-propyl, isopropyl, n-butyl, sec-butyl, tert-butyl, n-pentyl, 2-pentyl, isopentyl, neopentyl, n-hexyl, 2-hexyl, 3-hexyl, and 3-methyl pentyl. When an alkyl residue having a specific number of carbons is named, all geometric isomers having that number of carbons may be encompassed. For example, "butyl" can include n-butyl, sec-butyl, isobutyl and t-butyl, and "propyl" can include n-propyl and isopropyl. Unless stated otherwise specifically in the specification, an alkyl chain is optionally substituted by one or more substituents such as those substituents described herein.

The term "**alkoxy**" refers to an unbranched or branched alkyl group attached to an oxygen atom (alkyl-O-). In some embodiments, alkoxy as used herein has 1 to 20 carbon atoms ((C₁₋C₂₀)alkoxy), 1 to 10 carbon atoms ((C₁₋C₁₀)alkoxy), 1 to 8 carbon atoms ((C₁₋C₈)alkoxy), 1 to 6 carbon atoms ((C₁₋C₆)alkoxy), 1 to 5 carbon atoms ((C₁₋C₅)alkyl, or 1 to 3 carbon atoms ((C₁₋C₅)alkyl. Examples include, but are not limited to, methoxy, ethoxy, n-propoxy, and butoxy. When an alkoxy residue having a specific number of carbons is named, all geometric isomers having that number of carbons may be encompassed, such as isopropoxy, isobutoxy, and t-butoxy. Unless stated otherwise specifically in the specification, an alkoxy chain is optionally substituted by one or more substituents such as those substituents described herein.

The term "**alkylene**" refers to a straight divalent hydrocarbon chain linking the rest of the molecule to a radical group, consisting solely of carbon and hydrogen, containing no unsaturation, and preferably having from 1 to 20 carbon atoms ((C₁₋C₂₀)alkylene), 1 to 10 carbon atoms ((C₁₋C₁₀)alkylene), 1 to 6 carbon atoms ((C₁₋C₆)alkylene), or 1 to 5 carbon atoms ((C₁₋C₅)alkyl, Examples include, but are not limited to, methylene, ethylene, propylene, butylene, and the like. The alkylene chain is attached to the rest of the molecule through a single bond and to the radical group through a single bond. The points of attachment of the alkylene chain to the rest of the molecule and to the radical group are through the terminal carbons respectively. Unless stated otherwise specifically in the specification, an alkylene chain is optionally substituted by one or more substituents such as those substituents described herein. Examples include, methylene (-CH₂-), ethylene (-CH₂CH₂-), propylene (-CH₂CH₂CH₂-), 2-methylpropylene (-CH₂-CH(CH₃) -CH₂-), hexylene (-(CH₂)₆-) and the like.

The term "**alkenyl**" refers to an aliphatic hydrocarbon group containing at least one carbon-carbon double bond including straight-chain, branched-chain and cyclic alkenyl groups. In some embodiments, the alkenyl group has 2-10 carbon atoms (a C₂-₁₀ alkenyl). In another embodiment, the alkenyl group has 2-4 carbon atoms in the chain (a C₂-₄ alkenyl). Exemplary alkenyl groups include, but are not limited to, ethenyl, propenyl, n-butenyl, i-butenyl, 3-methylbut-2-enyl, 3-methylbut-1-enyl, n-pentenyl, heptenyl, octenyl, cyclohexyl-butenyl, decenyl, and the like, and their isomers. An alkylalkenyl is an alkyl group as defined herein bonded to an alkenyl group as defined herein. The alkenyl group can be unsubstituted or substituted through available carbon atoms with one or more groups defined hereinabove for alkyl.

The term "**alkynyl**" refers to straight or branched monovalent hydrocarbyl groups having from 2 to 6 carbon atoms and preferably 2 to 3 carbon atoms and having at least 1 and preferably from 1 to 2 sites of acetylenic (C≡C-) unsaturation. Examples of such alkynyl groups include, but are not limited to, acetylenyl (C≡CH), propargyl (CH₂C≡CH), 1-propynyl, 1-butynyl, and 1-pentynyl.

The term "**amino-alkyl**" refers to a group formed by combining an alkyl and an amino group. Examples include -NHCH₃, -NHCH₂CH₃ -NHCH(CH₃)₂, -N(CH₃)₂, - NCH₃(CH₂CH₃) -NCH₃(CH(CH₃)₂) and similar groups, but are not restricted to these.

The term "**aryl**" refers to a monocyclic or polycyclic group having at least one hydrocarbon aromatic ring, wherein all of the ring atoms of the at least one hydrocarbon aromatic ring are carbon. Aryl may include groups with a single aromatic ring (e.g., phenyl) and multiple fused aromatic rings (e.g., naphthyl, anthryl). Aryl may further include groups with one or more aromatic hydrocarbon rings fused to one or more non-aromatic hydrocarbon rings (e.g., fluorenyl; 2,3-dihydro-1H-indene; 1,2,3,4-tetrahydronaphthalene). In certain embodiments, aryl includes groups with an aromatic hydrocarbon ring fused to a non-aromatic ring, wherein the non-aromatic ring comprises at least one ring heteroatom independently selected from the group consisting of N, O, and S. For example, in some embodiments, aryl includes groups with a phenyl ring fused to a non-aromatic ring, wherein the non-aromatic ring comprises at least one ring heteroatom independently selected from the group consisting of N, O, and S (e.g., chromane; thiochromane; 2,3-dihydrobenzofuran; indoline). In some embodiments, aryl as used herein has from 6 to 14 carbon atoms ((C₆-C₁₄)aryl), or 6 to 10 carbon atoms ((C₆-C₁₀)aryl). Where the aryl includes fused rings, the aryl may connect to one or more substituents or moieties of the formulae described herein through any atom of the fused ring for which valency permits.

The term "**cycloalkyl**" refers to a monocyclic or polycyclic saturated hydrocarbon. In some embodiments, cycloalkyl has 3 to 20 carbon atoms ((C₃₋C₂₀)cycloalkyl), 3 to 8 carbon atoms ((C₃₋C₈)cycloalkyl), 3 to 6 carbon atoms ((C₃₋C₆)cycloalkyl), or 3 to 5 carbon atoms ((C₃₋C₅)cycloalkyl). In some embodiments, cycloalkyl has 3 to 8 carbon atoms having single or multiple cyclic rings including fused, bridged, and spiro ring systems. Examples of suitable cycloalkyl groups include, but are not limited to, adamantyl, cyclopropyl, cyclobutyl, cyclopentyl, cyclooctyl, octahydropentalenyl, octahydro-1*H*-indene, decahydronaphthalene, cubane, bicyclo[3.1.0]hexane, and bicyclo[1.1.1]pentane, and the like.

The term "**carbocycle**" refers to a saturated, unsaturated or aromatic ring system in which each atom of the ring system is carbon. Carbocycle includes 3- to 10-membered monocyclic rings, 6- to 12-membered bicyclic rings, and 6- to 12-membered bridged rings. Each ring of a bicyclic carbocycle may be selected from saturated, unsaturated, and aromatic rings. In an exemplary embodiment, an aromatic ring, e.g., phenyl, may be fused to a saturated or unsaturated ring, e.g., cyclohexane, cyclopentane, or cyclohexene. A bicyclic carbocycle includes any combination of saturated, unsaturated and aromatic bicyclic rings, as valence permits. A bicyclic carbocycle includes any combination of ring sizes such as 4-5 fused ring systems, 5-5 fused ring systems, 5-6 fused ring systems, 6-6 fused ring systems, 5-7 fused ring systems, 6-7 fused ring systems, 5-8 fused ring systems, and 6-8 fused ring systems. Exemplary carbocycles include cyclopentyl, cyclohexyl, cyclohexenyl, adamantyl, phenyl, indanyl, and naphthyl.

The term "**haloalkyl**" refers to a mono haloalkyl or a polyhaloalkyl group that can be further substituted or unsubstituted. The terms halogen and alkyl are as stated herein.

The term "**heterocycle**" refers to a saturated, unsaturated or aromatic ring comprising one or more heteroatoms. Exemplary heteroatoms include N, O, Si, P, B, and S atoms. Heterocycles include 3- to 10-membered monocyclic rings, 6- to 12-membered bicyclic rings, and 6- to 12-membered bridged rings. A bicyclic heterocycle includes any combination of saturated, unsaturated and aromatic bicyclic rings, as valence permits. In an exemplary embodiment, an aromatic ring, e.g., pyridyl, may be fused to a saturated or unsaturated ring, e.g., cyclohexane, cyclopentane, morpholine, piperidine or cyclohexene. A bicyclic heterocycle includes any combination of ring sizes such as 4-5 fused ring systems, 5-5 fused ring systems, 5-6 fused ring systems, 6-6 fused ring systems, 5-7 fused ring systems, 6-7 fused ring systems, 5-8 fused ring systems, and 6-8 fused ring systems.

The term "**heteroaryl**" refers to a monocyclic or bicyclic or higher aromatic group that could be substituted or un-substituted containing at least one heteroatom (e.g., a heteroatom selected from B, N, O, S, P(=O), Si, and P. In some embodiments, the term refers to an aromatic group of from 4 to 10 carbon atoms and 1 to 4 heteroatoms selected from the group consisting of oxygen, nitrogen and sulfur within the ring. Such heteroaryl groups can have a single ring (i.e., pyridinyl or furyl) or multiple condensed rings (i.e., indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N oxide (N→O), sulfinyl, or sulfonyl moieties. Examples of monocyclic heteroaryl include pyrazolyl, pyrrolyl, thiazolyl, oxazolyl, thiophenyl, furanyl, imidazolyl, isoxazolyl, triazolyl, thiadiazolyl, tetrazolyl, oxadiazolyl, pyridinyl, pyridazinyl, pyrimidinyl, pyrazinyl, thiazolyl, and similar groups, but are not limited to the aforementioned. Examples of bicyclic heteroaryl include indolyl, benzothiophenyl, benzofuranyl, benzimidazolyl, benzoxazolyl, benzisoxazolyl, benzothiazoleil, benzothiadiazole, benzotriazolyl, quinolinyl, isoquinolinyl, purinyl, furopyridinyl, oxocromen, dioxoisoindolin, pyrazolopyridinyl, pyrazolo [1, 5-a] pyridinyl, and similar groups, but are not restricted to the aforementioned. Preferred heteroaryls include 5 or 6 membered heteroaryls such as pyridinyl, pyrrolyl, indolyl, thiophenyl, and furanyl.

The term "**heterocycloalkyl**" refers to substituted or unsubstituted monocyclic alkyl containing one or more hetero atoms selected from B, N, O, S, P(=O), Si and P. Examples include piperidinyl, piperazinyl, morpholinyl, pyrrolidinyl, thiomorpholinyl, imidazolidinyl, tetrahydrofurfuryl, and similar groups, but are not restricted to the aforementioned.

The term "**heteroalkyl**" refers to an alkyl substituent in which one or more of the carbon atoms and any attached hydrogen atoms are independently replaced with the same or different heteroatomic group. For example, 1, 2, or 3 carbon atoms may be independently replaced with the same or different heteroatomic substituent.

The term "**substituted**" refers to moieties having substituents replacing a hydrogen on one or more carbons or substitutable heteroatoms, e.g., NH or NH₂, of a compound. It will be understood that "substitution" or "substituted with" includes the implicit proviso that such substitution is in accordance with permitted valence of the substituted atom and the substituent, and that the substitution results in a stable compound. For example, stable compounds include, but is not limited to, compounds which do not spontaneously undergo transformation such as by rearrangement, cyclization, elimination, etc. In certain embodiments, substituted refers to moieties having substituents replacing two hydrogen atoms on the same carbon atom, such as substituting the two hydrogen atoms on a single carbon with an oxo, imino or thioxo group. The term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds.

It will be understood by those skilled in the art that substituents can themselves be substituted, if appropriate. Unless specifically stated as "unsubstituted," references to chemical moieties herein are understood to include substituted variants and unsubstituted variants. For example, reference to a "heteroaryl" group or moiety implicitly includes both substituted and unsubstituted variants, unless specified otherwise.

When referring to compound features, the phrase "**optionally substituted**" may be used interchangeably with the phrase "unsubstituted or substituted" and refers to when a non-hydrogen substituent may or may not be present on a given atom or group, and, thus, the description includes structures where a non-hydrogen substituent is present and structures where a non-hydrogen substituent is not present. For example, "optionally substituted alkyl" encompasses both "alkyl" and "substituted alkyl" as defined herein. It will be understood by those skilled in the art, with respect to any group containing one or more substituents, that such groups are not intended to introduce any substitution or substitution patterns that are sterically impractical, synthetically non-feasible and/or inherently unstable.

It will also be understood by those skilled in the art that when "optionally substituted" is used, any part of the following term can be substituted.

The terms "**linker**", "**linkage**" and "**linking group**" are used interchangeably and refer to a linking moiety that covalently connects two or more substituents. A linking moiety may connect two groups where the linker may be linear, branched, cyclic or a single atom. In some embodiments, the linker is divalent. In some embodiments, the linker is a branched linker. In some embodiments, the two or more substituents that are covalently connected by the linking moiety are optionally substituted alkyl or alkoxy groups. In some embodiments, the linkers are selected from -CO₂-, -O-, -OCO-, -CONH-, -NHCO-, and -NH-.

In some embodiments, substituents may include any substituents described herein, for example: halogen, hydroxy, oxo (=O), thioxo (=S), cyano (-CN), nitro (-NO₂), imino (=N-H), oximo (=N-OH), hydrazino (=N-NH₂), -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}N (R^{a})S(O)ₜR^{a} (where t is 1 or 2), -R^{b}-S(O)ₜR^{a} (where t is 1 or 2), -R^{b}-S(O)ₜOR^{a} (where t is 1 or 2), and -R^{b}-S(O)ₜN(R^{a})₂ (where t is 1 or 2). In another exemplary embodiment, substituents include alkyl, alkenyl, alkynyl, aryl, aralkyl, aralkenyl, aralkynyl, cycloalkyl, cycloalkylalkyl, heterocycloalkyl, heterocycloalkylalkyl, heteroaryl, and heteroarylalkyl, any of which may be optionally substituted by alkyl, alkenyl, alkynyl, halogen, haloalkyl, haloalkenyl, haloalkynyl, oxo, thioxo, cyano, nitro, imino, oximo, hydrazine, -R^{b}OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N (R^{a})S(O)ₜR^{a} (where t is 1 or 2), - R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2); and wherein each R^{a}, R^{b}, and R^{c} are independently selected from hydrogen, alkyl, cycloalkyl, cycloalkylalkyl, aryl, aralkyl, heterocycloalkyl, heterocycloalkylalkyl, heteroaryl, and heteroarylalkyl; and wherein each R^{a}, R^{b}, and R^{c}, valence permitting, may be optionally substituted with alkyl, alkenyl, alkynyl, halogen, haloalkyl, haloalkenyl, haloalkynyl, oxo, thioxo, cyano, nitro, imino, oximo, hydrazine, -R^{b}OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R^{b}-C(O)OR^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N (R^{a})S(O)ₜR^{a} (where t is 1 or 2), - R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2).

The term "**isomers**" or "**three-dimensional isomers**" refers to two or more compounds comprising the same numbers and types of atoms, groups or components, but with different structural arrangement and connectivity of the atoms. The term can refer to compounds that are identical in terms of chemical equation or molecular equation but are different optically or three-dimensionally, or their salts, and specifically could be partially three-dimensional isomers, enantiomers, geometrical isomers, or shape isomers.

The term "**tautomer**" refers to one of two or more structural isomers which readily convert from one isomeric form to another and which exist in equilibrium.

A "**stereoisomer**" refers to a compound made up of the same atoms bonded by the same bonds but having different three-dimensional structures, which are not interchangeable. The present disclosure contemplates various stereoisomers and mixtures thereof and includes "enantiomers", which refers to two stereoisomers whose molecules are non-superimposeable mirror images of one another.

Individual enantiomers and diastereomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, (3) direct separation of the mixture of optical enantiomers on chiral liquid chromatographic columns, or (4) kinetic resolution using stereoselective chemical or enzymatic reagents. Racemic mixtures also can be resolved into their respective enantiomers by well-known methods, such as chiral-phase gas chromatography or crystallizing the compound in a chiral solvent. Stereoselective syntheses, a chemical or enzymatic reaction in which a single reactant forms an unequal mixture of stereoisomers during the creation of a new stereocenter or during the transformation of a pre-existing one, are well known in the art. Stereoselective syntheses encompass both enantio- and diastereoselective transformations. See, for example, Carreira and Kvaerno, Classics in Stereoselective Synthesis, Wiley-VCH: Weinheim, 2009.

The symbol = denotes a bond that may be a single, double or triple bond as described herein. Substituents around a carbon-carbon double bond are designated as being in the "Z" or "E" configuration, where the terms "Z" and "E" are used in accordance with IUPAC standards. Unless otherwise specified, structures depicting double bonds encompass both the "E" and "Z" isomers.

Substituents around a carbon-carbon double bond alternatively can be referred to as "**cis**" or "**trans**," where "cis" represents substituents on the same side of the double bond and "trans" represents substituent on opposite sides of the double bond. The arrangement of substituents around a carbocyclic ring can also be designated as "cis" or "trans." The term "cis" represents substituents on the same side of the plane of the ring and the term "trans" represents substituents on opposite sides of the plane of the ring. Mixtures of compound wherein the substituents are disposed on both the same and opposite sides of the plane of the ring are designated "cis/trans."

Singular articles such as "**a**," "**an**" and "**the**" and similar referents in the context of describing the elements are to be construed to cover both the singular and the plural, unless otherwise indicated herein or clearly contradicted by context. Recitation of ranges of values herein are merely intended to serve as a shorthand method of referring individually to each separate value falling within the range, including the upper and lower bounds of the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*i.e.,* "such as") provided herein, is intended merely to better illuminate the embodiments and does not pose a limitation on the scope of the claims unless otherwise stated.

In some embodiments, where the use of the term "**about**" is before a quantitative value, the present disclosure also includes the specific quantitative value itself, unless specifically stated otherwise. As used herein, the term "about" refers to a ±10% variation from the nominal value unless otherwise indicated or inferred. Where a percentage is provided with respect to an amount of a component or material in a composition, the percentage should be understood to be a percentage based on weight, unless otherwise stated or understood from the context.

Where a molecular weight is provided and not an absolute value, for example, of a polymer, then the molecular weight should be understood to be an average molecule weight, unless otherwise stated or understood from the context.

It should be understood that the order of steps or order for performing certain actions is immaterial so long as the present disclosure remain operable. Moreover, two or more steps or actions can be conducted simultaneously.

A dash ("-") symbol that is not between two letters or symbols refers to a point of bonding or attachment for a substituent. For example, -NH₂ is attached through the nitrogen atom.

The term "**pharmaceutically acceptable salt**" refers to a salt which is acceptable for administration to a subject. It is understood that such salts, with counter ions, will have acceptable mammalian safety for a given dosage regime. Such salts can also be derived from pharmaceutically acceptable inorganic or organic bases and from pharmaceutically acceptable inorganic or organic acids, and may comprise organic and inorganic counter ions. The neutral forms of the compounds described herein may be converted to the corresponding salt forms by contacting the compound with a base or acid and isolating the resulting salts.

The terms "**pharmaceutically acceptable excipient**," "**pharmaceutically acceptable diluent**," "**pharmaceutically acceptable carrier**," and "**pharmaceutically acceptable adjuvant**" are used interchangeably and refer to an excipient, diluent, carrier, or adjuvant that is useful in preparing a pharmaceutical composition that are generally safe, non-toxic and neither biologically nor otherwise undesirable, and include an excipient, diluent, carrier, and adjuvant that are acceptable for veterinary use as well as human pharmaceutical use. The phrase "pharmaceutically acceptable excipient" includes both one and more than one such excipient, diluent, carrier, and/or adjuvant.

The term "**pharmaceutical composition**" is meant to encompass a composition suitable for administration to a subject, such as a mammal, especially a human. In general a "pharmaceutical composition" is sterile, and preferably free of contaminants that are capable of eliciting an undesirable response within the subject (*i.e*., the compound(s) in the pharmaceutical composition is pharmaceutical grade). Pharmaceutical compositions can be designed for administration to subjects or patients in need thereof *via* a number of different routes of administration including oral, buccal, rectal, parenteral, intraperitoneal, intradermal, intratracheal, intramuscular, subcutaneous, and the like.

### EXEMPLARY EMBODIMENTS

As described herein, the text refers to various embodiments of the present compounds, compositions, and methods. The various embodiments described are meant to provide a variety of illustrative examples and should not be construed as descriptions of alternative species. Rather, it should be noted that the descriptions of various embodiments provided herein may be of overlapping scope. The embodiments discussed herein are merely illustrative and are not meant to limit the scope of the present technology.

### EXAMPLES

The following examples are offered to illustrate the present disclosure and are not to be construed in any way as limiting the scope of the present technology. Any methods that are functionally equivalent are within the scope of the present technology. Various modifications of the present technology in addition to those described herein will become apparent to those skilled in the art from the foregoing description. Such modifications fall within the scope of the appended claims.

In the examples that follow, Scheme 2 and Examples 1.4-1.39, 1.49-1.69, 2 and 3 are not part of the claimed invention.

Unless otherwise stated, all temperatures are in degrees Celsius. Efforts have been made to ensure accuracy with respect to numbers used (e.g., amounts, temperatures, etc.), but some experimental errors and deviation should be allowed for.

If an abbreviation is not defined, it has its generally accepted meaning.

### General Synthetic Methods

Final compounds were confirmed by high-performance liquid chromatography/ mass spectrometry (HPLC/MS) analysis and determined to be >90% pure by weight. ¹H nuclear magnetic resonance (NMR) spectra were recorded in CDCl₃ (residual internal standard CHCl₃ = δ 7.26), dimethyl sulfoxide (DMSO)-d₆ (residual internal standard CD₃SOCD₂H = δ 2.50), methanol-d₄ (residual internal standard CD₂HOD = δ 3.30), or acetone-d₆ (residual internal standard CD₃COCD₂H = δ 2.05). The chemical shifts (δ) reported are given in parts per million (ppm) and the coupling constants (J) are in Hertz (Hz). The spin multiplicities are reported as s = singlet, bs = broad singlet, bm = broad multiplet, d = doublet, t = triplet, q = quartet, p = pentuplet, dd = doublet of doublet, ddd = doublet of doublet of doublet, dt = doublet of triplet, td = triplet of doublet, tt = triplet of triplet, and m = multiplet.

HPLC-MS analysis was carried out with gradient elution. Medium pressure liquid chromatography (MPLC) was performed with silica gel columns in both the normal phase and reverse phase.

### Example 1: Preparation of compounds

The compounds of this disclosure are produced by adapting well known chemical conversions in accordance with the method illustrated by Scheme 1 or 2 below. The synthesis of several exemplary compounds of Table 1 is described below in examples 1.1 to 1.69. Several other compounds of Table 1 were prepared by adapting the methods described herein and assessed according to the biological assays described in Examples 2-4. 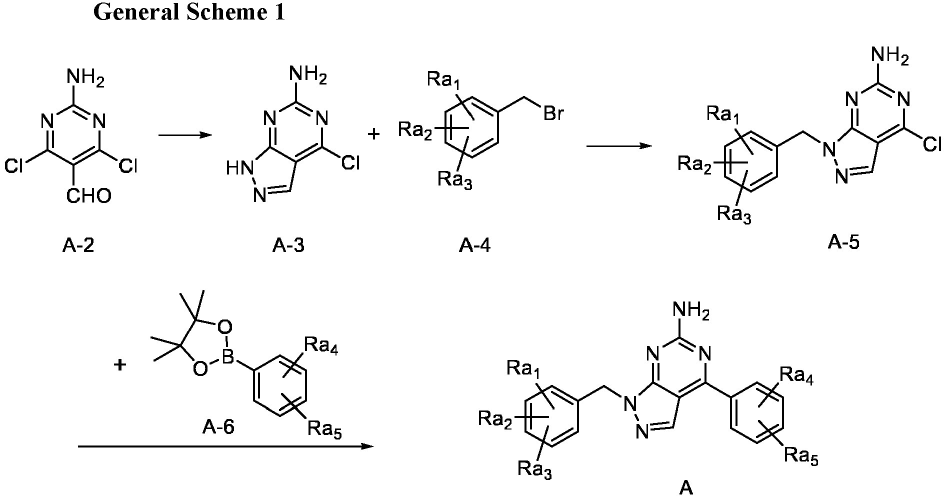 Exemplary synthetic methods for Scheme 1 are described in detail below.

To describe in more detail by referring to the aforementioned scheme 1, it is possible to produce the compound indicated by the aforementioned scheme 1 through a series of processes that includes the first stage in which the A-2 compound is dissolved in a solvent of tetrahydrofuran and H₂O, and in which the compound of scheme A-3 is produced by subjecting the solution to chain reaction with hydrazine monohydrate for 24 hours at 25°C to 60°C, the second stage in which the A-3 compound is dissolved in the *N,N-*dimethylformamide solvent, and then subjecting the solution to nucleophilic substitution reaction with the compound of scheme A-4 for 24 hours at 25°C to 60°C under the condition in which potassium carbonate or cesium carbonate is provided to produce the scheme A-5 compound; and the third stage in which the A-5 compound is dissolved in 1,4-dioxane and distilled water, and in which the solution is subject to Suzuki coupling reaction with the compound of scheme A-6 for two hours to 24 hours at 100°C to 115°C to produce the A compound under the condition in which 3-(4,4,5,5-tetramethyl-1,3,2-dioxaborolane-2-yl) benzonitrile and its derivative, and tetrakis (triphenylphosphine) palladium and sodium carbonate are provided, or in which the solution is subject to the Suzuki coupling reaction under the same conditions and in which the solution is dissolved in ethanol solvent, and in which the solution is subject to reduction reaction under the conditions of tin (II) chloride dihydrate and acid. Here, the aforementioned scheme 1's Rₐ₁, Rₐ₂, Rₐ₃, Rₐ₄, and Rₐ₅ can correspond to the substituents of the formula described herein, e.g., formula (IIb)'s R¹ to R⁹, and cyano group. 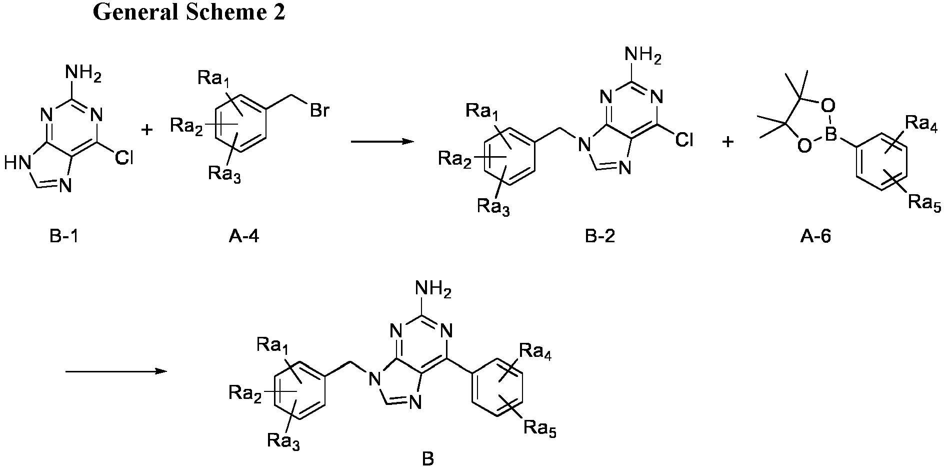 Exemplary synthetic methods for Scheme 2 are described in detail below.

It is possible to produce compounds according to the schemes above through a series of processes that includes the first stage in which the B-1 compound is dissolved in N,N-dimethylformamide solvent, and in which the B-2 compound is produced by subjecting the solution to nucleophilic substitution reaction with the compound of scheme A-4 for 24 h at 25°C to 60°C under the condition in which potassium carbonate or cesium carbonate is provided, and the second stage in which the B-2 compound is dissolved a solvent of 1,4-dioxane and distilled water, and in which the solution is subject to Suzuki coupling reaction with the compound of scheme A-6 for two hours to 24 h at 100°C to 115°C to produce compound B under the condition in which 3-(4,4,5,5-tetramethyl-1,3,2-dioxaborolane-2-yl) benzonitrile and its derivative, tetrakis (triphenylphosphine) palladium, and sodium carbonate are provided, or in which the solution is subject to the Suzuki coupling reaction under the same conditions and in which the solution is dissolved in ethanol solvent, and in which the solution is subject to reduction reaction under the conditions of tin (II) chloride dihydrate and acid.

### Example 1.1: Production of 3-(6-amino-1-(4-aminobenzyl)-1H-pyrazolo [3,4-d] pyrimidine-4-yl) benzonitrile (compound 201)

### 1.1.1. Production of 4-chloro-(4-nitrobenzyl)-1H-pyrazolo [3,4-d] pyrimidine-6-amine

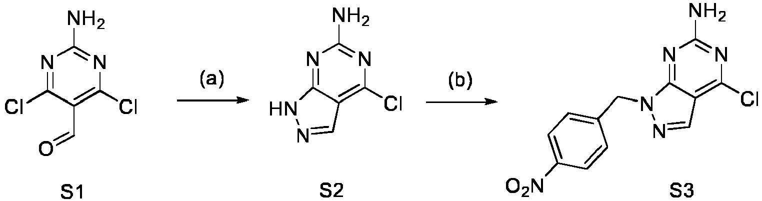

Reagents and Conditions: (a) hydrazine, TEA, THF:H₂O (3:1), r.t. to 50 °C, 3 h, quant; (b) 4-nitrobenzyl bromide, K₂CO₃, DMF, 0 °C to r.t. , 12 h, 58%.

### Stage 1: Production of 4-chloro-1H-pyrazolo [3,4-d] pyrimidine-6-amine

2-amino-4,6-dichloropyrimidine-5-carbaldehyde (S1, 4g, 20.83mmol) was melted in a solvent of tetrahydrofuran (THF) and H₂O, and to this hydrazine monohydrate (0.78ml, 24.99mmol) and triethylamine (TEA, 3.51ml, 24.99mmol) were added. Afterwards, the aforementioned reaction mixture was heated at 50°C and agitated for 3 hours, and concentrated. Then, the aforementioned concentrate was cleaned with distilled water to obtain an intermediate compound (S2, 3.45g, 100%). ¹H-NMR (DMSO-d₆, 400MHz): δ 13.25 (s, 1H), 8.02 (s, 1H), 7.14 (s, 2H).

### Stage 2: Production of 4-chloro-1-(4-nitrobenzyl)-1H-pyrazolo [3,4-d] pyrimidine-6-amine

The intermediate compound produced in the aforementioned stage 1 (S2, 3.0g, 18.12mmol) was dissolved in dimethylformamide (DMF), and 4-nitro benzyl bromide (3.92g, 18.12mmol) and potassium carbonate (K₂CO₃, 3.75g, 27.18mmol) was added to this. Then, this was agitated in room temperature for 12 hours. The aforementioned reaction mixture was diluted with ethyl acetate (EA), and was cleaned with distilled. Then, it was dried with magnesium sulfate and then filtered and concentrated. Then, the aforementioned concentrate was refined with silica gen chromatography to obtain the desired compound (S3, 3.2g, 58%). ¹H-NMR (DMSO-d₆, 400MHz): δ 8.19 (d, 2H), 8.09 (s, 1H), 7.40-7.38 (m, 4H), 5.56 (s, 2H).

### 1.1.2. Production of 3-(6-amino-1-(4-aminobenzyl)-1H-pyrazolo [3,4-d] pyrimidine-4-yl) benzonitrile (compound 201).

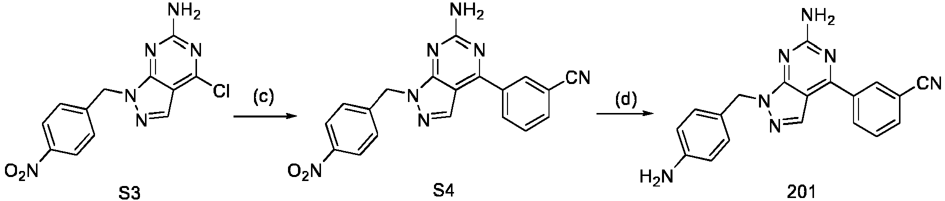

Reagents and Conditions: (c) 3-(4,4,5,5-tetramethyl-1,3,2-dioxaborolan-2-yl)benzonitrile, Pd(PPh₃)₄, Na₂CO₃, dioxane:H₂O (8:1), r.t. to 105 °C, 12 h, 70%; (d) SnCl₂.2H₂O, conc. HCl, EtOH, r.t. to 50 °C, 3 h, 45%.

### Stage 1: Production of 3-(6-amino-1-(4-nitrobenzyl)-1H-pyrazolo [3,4-d] pyrimidine-4-yl) benzonitrile

The compound of the aforementioned example 1-1 (S3, 1.0g, 3.28mmol) was dissolved in the solvent of 1,4-dioxane and H₂O, and 3-(4,4,5,5-tetramethyl-1,3,2-dioxaborolan-2-yl) benzonitrile (1.12g, 4.92mmol), tetrakis (triphenylphosphine) palladium ((0) (Pd(PPh₃)₄, 758mg, 0.65mmol), and sodium carbonate (Na₂CO₃, 695mg, 6.56mmol) were added to this. Afterwards, the aforementioned reaction mixture was agitated for 12 hours at the 105°C using a sealed tube, and then was diluted with dichloromethane (DCM), and then this was cleaned with distilled water. Afterwards, this was dried with magnesium sulfate and then filtered and concentrated, and the aforementioned concentrate was cleaned with ethyl acetate/hexane (EA/Hex) to obtain an intermediate compound (S4, 852mg, 70%). ¹H-NMR (DMSO-d₆, 400MHz): δ 8.54 (s, 1H), 8.51-8.49 (m, 2H), 8.08 (dd, 1H), 7.80 (t, 1H), 7.42 (d, 2H), 7.15 (s, 2H), 5.62 (s, 2H).

### Stage 2: Production of 3-(6-amino-1-(4-aminobenzyl)-1H-pyrazolo [3,4-d] pyrimidine-4-yl) benzonitrile

The intermediate compound produced in the aforementioned stage 1 (S4, 400mg, 1.07mmol) was dissolved in ethanol (EtOH), and then tin (II) chloride dihydrate (SnCl₂ 2H₂O, 730mg, 3.23mmol) and Conc. HCl (aq) (1.8mL, 21.54mmol) were added to this. Afterwards, the aforementioned reaction mixture was agitated for 3 hours at 50°C, and then was diluted with dichloromethane (DCM), and was cleaned with distilled water. Afterwards, this was dried with magnesium sulfate, and then dried, filtered, and concentrated. Then, the aforementioned concentrate was refined with silica gen chromatography to obtain the desired compound (201, 161mg, 45%). ¹H-NMR (DMSO-d₆, 400MHz): δ 8.50 (s, 1H), 8.48-8.45 (m, 1H), 8.35 (s, 1H), 8.07-8.04 (m, 1H), 7.79 (t, 1H), 7.07 (s, 2H), 6.95 (d, 2H), 6.47 (d, 2H), 5.23 (s, 2H), 5.04 (s, 2H).

### Example 1.2: Production of 3-(6-amino-1-(4-aminobenzyl)-1H-pyrazolo [3,4-d] pyrimidine-4-yl)-2-methylbenzonitrile (compound 205)

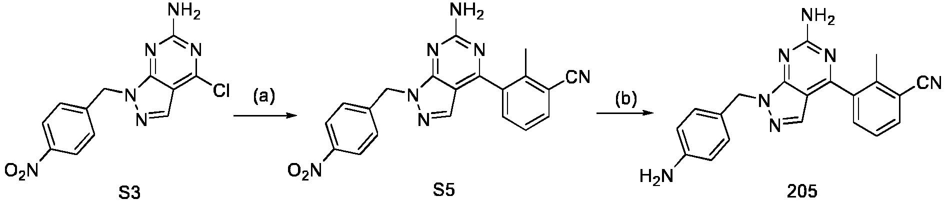

Reagents and Conditions: (a) 2-methyl-3-(4,4,5,5-tetramethyl-1,3,2-dioxaborolan-2-yl)benzonitrile, Pd(PPh₃)₄, Na₂CO₃, dioxane:H₂O (8:1), r.t. to 105 °C, 12 h, 81%; (b) SnCl₂.2H₂O, conc HCl, EtOH, r.t. to 50 °C , 3 h, 74%.

### Stage 1: Production of 3-(6-amino-1-(4-nitrobenzyl)-1H-pyrazolo [3,4-d] pyrimidine-4-yl)-2-methylbenzonitrile

In stage 1 of the aforementioned example 1.1.2, except for using 2-methyl -3-(4,4,5,5-tetramethyl-1,3,2-dioxiborolan-2-yl) benzonitrile (120mg, 0.49mmol ) instead of 3-(4,4,5,5-tetramethyl-1,3,2-dioxaborolane-2-yl) benzonitrile (1.12g, 4.92mmol), the same process as stage 1 of the aforementioned example 1.1.2 was carried out to obtain an intermediate compound (S5, 103mg, 81%). ¹H-NMR (DMSO-d₆, 400MHz): δ 8.21 (d, 2H), 7.97 (d, 1H), 7.90 (s, 1H), 7.82 (d, 1H), 7.56 (t, 1H), 7.46 (d, 2H), 7.13 (s, 2H), 5.59 (s, 2H), 2.52 (s, 3H)

### Stage 2: Production of 3-(6-amino-1-(4-aminobenzyl)-1H-pyrazolo [3,4-d] pyrimidine -4-yl)-2-methylbenzonitrile

In stage 2 of the aforementioned example 1.1.2, except for changing the amount of tin (II) chloride dihydrate (SnCl₂.2H₂O, 180mg, 0.80mmol ), and Conc. HCl (aq) (0.45ml, 5.34mmol ), the same process as stage 2 of the aforementioned example 1.1.2 was performed on the intermediate compound of the aforementioned stage 1 (S5,103mg, 0.26mmol ), to obtain a desired compound (205,71mg, 74%). ¹H-NMR (DMSO-d₆, 400MHz): δ 7.95 (d, 1H), 7.79 (d, 1H), 7.77 (s, 1H), 7.54 (t, 1H), 7.05 (s, 2H), 6.98 (d, 2H), 6.48 (d, 2H), 5.21 (s, 2H), 5.04 (s, 2H), 2.49 (s, 3H).

### Example 1.3: Production of 3-(6-amino-1-(4-aminobenzyl)-1H-pyrazolo [3,4-d] pyrimidine-4-yl)-2-fluorobenzonitrile (compound 202)

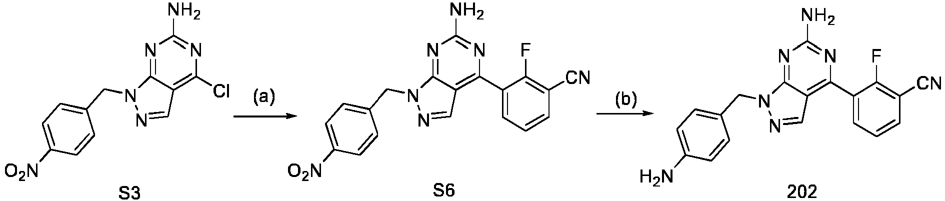

Reagents and Conditions: (a) 2-fluoro-3-(4,4,5,5-tetramethyl-1,3,2-dioxaborolan-2-yl)benzonitrile, Pd(PPh₃)₄, Na₂CO₃, dioxane:H₂O (8:1), r.t. to 105 °C, 12 h, 90%; (b) SnCl₂.2H₂O, conc. HCl, EtOH, r.t. to 50 °C, 3 h, 92%.

### Stage 1: Production of 3-(6-amino-1-(4-nitrobenzyl)-1H-pyrazolo [3,4-d] pyrimidine-4-yl)-2-fluorobenzonitrile

The same process as stage 1 of the aforementioned example 1.1.2 was performed except that 2-fluoro-3-(4,4,5,5- tetramethyl-1,3,2-dioxaborolan-2-yl) benzonitrile (1.21g, 4.92mmol) was used instead of 3-(4,4,5,5-tetramethyl-1,3,2-dioxaborolan-2-yl) benzonitrile (1.12g, 4.92mmol) in stage 1 of the aforementioned example 1.1.2, and except that the amount of tetrakis (triphenylphosphine) palladium (0) (Pd(PPh₃)₄,758mg, 0.65mmol), and the amount of sodium carbonate (Na₂CO₃,695mg, 6.56mmol) were changed, to obtain an intermediate compound (S6,1.15g, 90%). ¹H-NMR (DMSO-d₆, 400MHz): δ 8.22-8.15 (m, 4H), 8.10 (d, 1H), 7.61 (t, 1H), 7.43 (d, 2H), 7.21 (s, 2H), 5.60 (s, 2H).

### Stage 2: Production of 3-(6-amino-1-4(4-aminobenzyl)-1H-pyrazolo [3,4-d] pyrimidine-4-yl)-2-fluorobenzonitrile

The same process as stage 2 of the aforementioned example 1.1.2 was performed for the intermediate compound of the aforementioned stage 1 (S6, 144mg, 0.37mmol) except that the amount of tin (II) chloride dihydrate (SnCl₂.2H₂O, 250mg, 1.11mmol), Conc. HCl (aq) (0.62ml, 7.40mmol) in stage 2 of the aforementioned example 1.1.2 was changed to obtain the desired compound (202, 131mg, 92%). ¹H-NMR (DMSO-d₆, 400MHz): δ 8.14 (t, 2H) 7.96 (d, 1H), 7.11 (s, 2H), 6.96 (d, 2H), 6.48 (d, 2H), 5.22 (s, 2H), 5.04 (s, 2H).

### Example 1.4: Production of 3-(2-amino-9-(4-aminobenzyl)-9H-purin-6-yl) benzonitrile (compound 101)

### 1.4.1. Production of 6-chloro-9 (4-nitrobenzyl)-9H-purin-2-amine

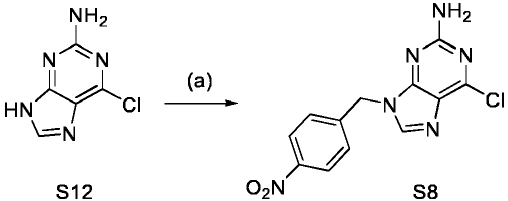

Reagent and condition: (a) 4-nitro benzyl bromide, K₂CO₃, DMF, rt.

6-chloro-9H-purin-2-amine (S12, 3g, 18.12mmol) was dissolved in *N,N-*dimethylformamide (DMF) and 4-nitro benzyl bromide (4.1g, 19.30mmol) and potassium carbonate (K₂CO₃, 3.76g, 27.18mmol) were added. Then, the mixture was agitated in room temperature. Afterwards, the aforementioned reaction mixture was diluted in dichloromethane (DCM), and was cleaned with distilled water. Then, this was dried with magnesium sulfate, filtered, and concentrated, and the aforementioned concentrate was refined with silica gel chromatography to obtain an intermediate compound (S8, 395mg, 42%). ¹H-NMR (DMSO-d₆, 400MHz): δ 8.27 (s, 1H), 8.22-8.20 (m, 2H), 7.47-7.45 (m, 2H), 6.96 (s, 2H), 5.46 (s, 2H).

### 1.4.2. Production of 3-(2-amino-9-(4-aminobenzyl)-9H-purin-6-yl) benzonitrile

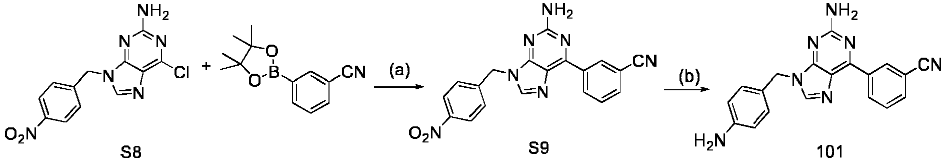

Reagents and conditions: (a) Pd(PPh₃)₄, Na₂CO₃ in H₂O, 1,4-dioxane, microwave, 105°C, 30min; (b) SnCl₂. 2H₂O, Conc. HCl(aq), EtOH, 55°C, 4h.

### Stage 1: Production of 3-(2-amino-9-(4-nitrobenzyl)-9H-purin-6-yl) benzonitrile

An intermediate compound (S8, 200mg, 0.652mmol) was dissolved in 1,4-dioxane, 3-(4,4,5,5-tetramethyl-1,3,2-dioxaborolane-2-yl) benzonitrile (224mg, 0.978mmol) and tetrakis (triphenylphosphine) palladium (0) ((Pd(PPh₃)₄, 151mg, 0.131mmol), and sodium carbonate (Na₂CO₃, 139mg, 1.304mmol) were added, and then the solution was heated at 105°C using a microwave device and agitated for 30 minutes. Afterwards, the aforementioned reaction mixture was diluted with dichloromethane (DCM) and cleaned with distilled water. Afterwards, this was dried with magnesium sulfate, filtered, and concentrated, and the aforementioned concentrate was refined with silica gel chromatography to obtain an intermediate compound (S9, 395mg, 54%). ¹H-NMR (DMSO-d₆, 400MHz): δ 9.10-9.08 (m, 1H), 9.05-9.02 (m, 1H), 8.39 (s, 1H), 8.22 (d, 2H), 8.03-8.01 (m, 1H), 7.80 (t, 1H), 7.51-7.49 (m, 2H), 6.73 (s, 2H), 5.53 (s, 2H).

### Stage 2: Production of 3-(2-amino-9-(4-aminobenzyl)-9H-purin-6-yl) benzonitrile

The intermediate compound produced in the aforementioned stage 1 (S9, 244mg, 0.624mmol) was dissolved in ethanol (EtOH), tin (II) chloride dihydrate (SnCl₂.2H₂O, 423mg, 1.872mmol) and Conc. HCl (aq) (1.1mL, 12.48mmol) were added, and the solution was agitated for 4 h at 55°C. Afterwards, the aforementioned reaction mixture was diluted with dichloromethane (DCM), and then was cleaned with distilled water. Afterwards, the solution was dried with magnesium sulfate, filtered, and concentrated, and the aforementioned concentrate was refined with silica gel chromatography to obtain the desired compound (101, 41mg, 38%). ¹H-NMR (DMSO-d₆, 400MHz): δ 9.07 (s, 1H), 9.02 (d, 1H), 8.24 (s, 1H), 8.00 (d, 1H), 7.78 (t, 1H), 7.05-7.03 (m, 2H), 6.69 (br, 2H), 6.52-6.50 (m, 2H), 5.11 (d, 4H).

### Example 1.5: Production of 3-(2-amino-9-(4-aminobenzyl)-9H-purin-6-yl)-2-fluorobenzonitrile (compound 102)

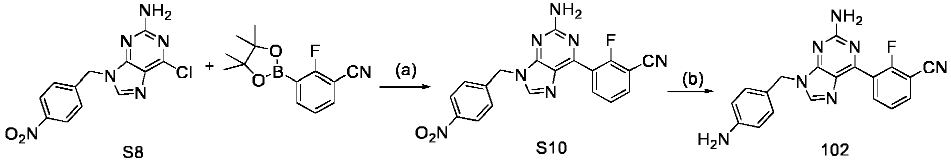

Reagents and conditions: (a) Pd(PPh₃)₄, Na₂CO₃ in H₂O, 1,4-dioxane, microwave, 105°C, 1h; (b) SnCl₂.2H₂O, Conc. HCl(aq), EtOH, 55°C, 4h.

### Stage 1: Production of 3-(2-amino-9-(4-nitrobenzyl)-9H-purin-6-yl)-2-fluorobenzonitrile

The same process as stage 1 of the aforementioned example 1.1 was performed except that 2-fluoro-3-(4,4,5,5-tetramethyl-1,3,2-dioxaborolan-2-yl)benzonitrile (224mg, 0.978mmol) instead of 3-(4,4,5,5-tetramethyl-1,3,2-dioxaborolan-2-yl)benzonitrile (224mg, 0.978mmol) in stage 1 of the aforementioned example 1.1 was used, to obtain an intermediate compound (S10, 395mg, 54%). ¹H-NMR (DMSO-d₆, 400MHz): δ 8.29 (s, 1H), 8.27-8.17 (m, 2H), 8.13-8.07 (m, 1H), 7.65-7.50(m, 4H), 6.78 (d, 2H), 5.51 (d, 2H).

### Stage 2: Production of 3-(2-amino-9-(4-aminobenzyl)-9H-purin-6-yl)-2-fluorobenzonitrile

The same process as stage 2 of the aforementioned example 1.1 was performed for the intermediate compound of the aforementioned stage 1 (S10, 244mg, 0.624mmol), to obtain the desired compound (102, 52mg, 48%). ¹H-NMR (DMSO-d₆, 400MHz): δ 8.22-8.13 (m, 2H), 8.13-8.07 (m, 1H), 7.57 (t, 1H), 7.05 (d, 2H), 6.73 (s, 2H), 6.51 (d, 2H), 5.11 (d, 4H).

### Example 1.6: Production of 3-(2-amino-9-(4-aminobenzyl)-9H-purin-6-yl)-2-methylbenzonitrile (compound 105)

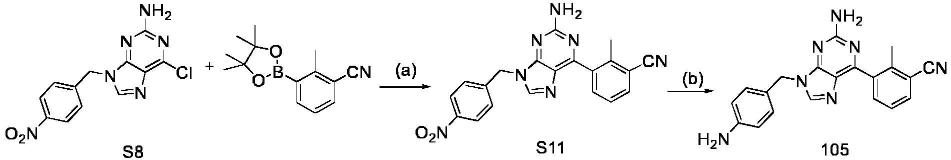

Reagents and conditions: (a) Pd(PPh₃)₄, Na₂CO₃ in H₂O, 1,4-dioxane, microwave, set T=105°C, 1h; (b) SnCl₂.2H₂O, Conc. HCl(aq), EtOH, 55°C, 4h.

### Stage 1: Production of 3-(2-amino-9-(4-nitrobenzyl)-9H-purin-6-yl)-2-methylbenzonitrile

The same process as stage 1 of the aforementioned example 1.1 was performed except that 2-methyl-3-(4,4,5,5,-tetramethyl-1,3,2-dioxaborolan-2-yl) benzonitrile (224mg, 0.978mmol) was used instead of 3-(4,4,5,5-tetramethyl-1,3,2-dioxaborolan-2-yl) benzonitrile (224mg, 0.978mmol) in stage 1 of the aforementioned example 1.1, to obtain an intermediate compound (S11, 395mg, 54%). ¹H-NMR (DMSO-d₆, 400MHz): δ 8.27-8.22 (m, 2H), 7.83 (d, 1H), 7.88 (d, 1H), 7.65-7.52 (m, 4H), 6.71 (s, 2H), 5.50 (s, 2H).

### Stage 2: Production of 3-(2-amino-9-(4-aminobenzyl)-9H-purin-6-yl)-2-methylbenzonitrile

The same process as stage 2 of the aforementioned example 1.1 was performed for the intermediate compound of the aforementioned stage 1 (S11, 244mg, 0.624mmol), to obtain the desired compound (105, 52mg, 48%). ¹H-NMR (DMSO-d₆, 400MHz): δ 8.10 (d, 1H), 7.91 (d, 1H), 7.83 (d, 1H), 7.51 (t, 1H), 7.06 (d, 2H), 6.66 (s, 2H), 6.55-6.46 (m, 2H), 5.10 (d, 4H), 3.32 (s, 3H).

### Example 1.7: Production of 3-(2-amino-9-(4-amino-2,6-difluorobenzyl)-9H-purin-6-yl)benzonitrile (compound 107)

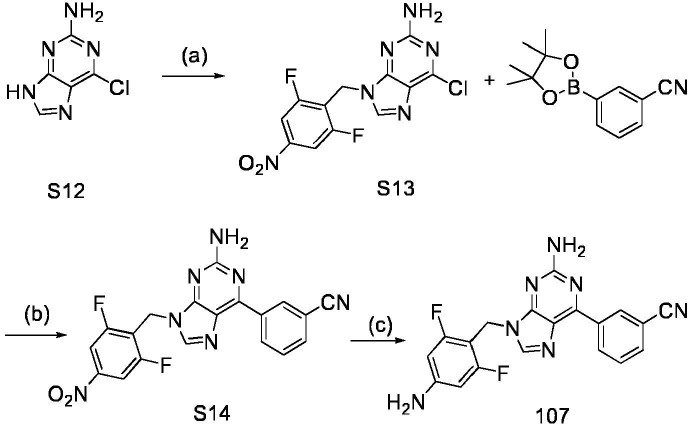

Reagents and conditions: (a) 2-(bromomethyl)-1,3-difluoro-5-nitrobenzene, K₂CO₃, DMF, rt, overnight; (b) Pd(PPh₃)₄, K₂CO₃, 1,4-dioxane, H₂O, 115°C, 4h; (c) SnCl₂.2H₂O, Conc. HCl(aq), EtOH, 55°C, 4h.

### Stage 1: Production of 6-chloro-9-(2,6-difluoro-4-nitrobenzyl)-9H-purin-2-amine

6-chloro-9H-purin-2-amine (S12, 500mg, 2.95mmol) was dissolved in dimethylformamide (DMF) and 2-(bromomethyl)-1,3-difluoro-5-nitrobenzene (817.4mg, 3.24mmol) and potassium carbonate (K₂CO₃, 611.6mg, 4.43mmol) was added, and the solution was agitated in room temperature for 1 day. Afterwards, the aforementioned reaction mixture was diluted with dichloromethane (DCM) and then cleaned with distilled water, and was dried with magnesium sulfate, filtered, and concentrated, and the aforementioned concentrate was refined with silica gen chromatography to obtain an intermediate compound (S13, 300mg, 33%). ¹H-NMR (DMSO-d₆, 400MHz): δ 8.68 (s, 1H), 7.55 (d, 2H), 6.93 (s, 2H), 4.99 (s, 2H).

### Stage 2: Production of 3-(2-amino-9-(2,6-difluoro-4-nitrobenzyl)-9H-purin-6-yl) benzonitrile

The intermediate compound produced in the aforementioned stage 1 (S13, 150mg, 0.44mmol) was dissolved in 1,4-dioxane, 3-(4,4,5,5-tetramethyl-1,3,2-dioxaborolan-2-yl) benzonitrile (151mg, 0.66mmol), tetrakis (triphenylphosphine) palladium (0) (Pd(PPh₃)₄, 25.4mg, 0.022mmol), and potassium carbonate (K₂CO₃, 121.6mg, 0.88mmol) were added, and the salutation was heated at 115°C and agitated for 4 h. Afterwards, the aforementioned reaction mixture was diluted with dichloromethane (DCM), and then cleaned with distilled water, and was dried with magnesium sulfate, filtered, and concentrated, and the aforementioned concentrate was refined with silica gel chromatography to obtain an intermediate compound (S14, 95.5mg, 53.3%). ¹H-NMR (methanol-d₄, 400MHz): δ 8.98 (s, 1H), 8.93 (dd, 1H), 8.18 (s, 1H), 8.01-7.96 (m, 2H), 7.86 (dd, 1H), 7.70 (t, 1H), 5.56 (s, 2H).

### Stage 3: Production of 3-(2-amino-9-(4-amino-2,6-difluorobenzyl)-9H-purin-6-yl) benzonitrile

The intermediate compound produced in the aforementioned stage 2 (S14, 95.1mg, 0.23mmol) was dissolved in ethanol (EtOH), and tin (II) chloride dihydrate (SnCl₂.2H₂O, 263.4mg, 1.17mmol) was added to this, and the solution was agitated for 4 h at 55°C. Afterwards, the aforementioned reaction mixture was diluted with dichloromethane (DCM), and then was cleaned with distilled water, and then was dried with magnesium sulfate, filtered, and concentrated, and the aforementioned concentrate was refined with silica gel chromatography to obtain the desired compound (107, 38.7mg, 44.6%). ¹H-NMR (DMSO-d₆, 400MHz): δ 9.06 (s, 1H), 8.99 (dd, 1H), 8.05 (s, 1H), 7.98 (dd, 1H), 7.78 (t, 1H), 6.67 (s, 2H), 6.25-6.18 (m, 2H), 5.89 (s, 2H), 5.17 (s, 2H).

### Example 1.8: Production of 3-(2-amino-9-(4-amino-2,6-difluorobenzyl)-9H-purin-6-yl)-2-fluorobenzonitrile (compound 108)

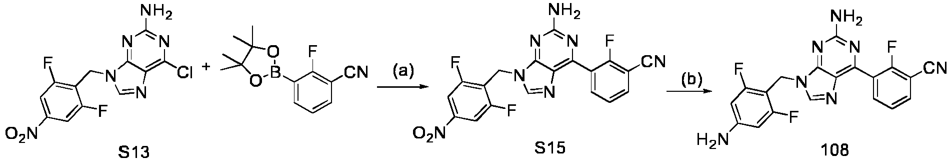

Reagents and conditions: (a) Pd(PPh₃)₄, K₂CO₃ 1,4-dioxane, H₂O, 115°C, overnight; (b) SnCl₂.2H₂O, Conc. HCl(aq), EtOH, 55°C, 3h.

### Stage 1: Production of 3-(2-amino-9-(2,6-difluoro-4-nitrobenzyl)-9H-purin-6-yl)-2-fluorobenzonitrile

The same process as stage 2 of the aforementioned example 1.4 was performed except that 2-fluoro-3-(4,4,5,5-tetramethyl-1,3,2-dioxaborolan-2-yl) benzonitrile (130.5mg, 0.53mmol) was used instead of 3-(4,4,5,5-tetramethyl-1,3,2-dioxaborolan-2-yl) benzonitrile (151mg, 0.66mmol) in stage 2 of the aforementioned example 1.4, and an intermediate compound (S15, 100mg, 53.4%) was obtained. ¹H-NMR (CDCl₃, 400MHz): δ 8.15-8.13 (m, 1H), 7.90-7.87 (m, 3H), 7.75-7.74 (m, 1H), 7.40 (t, 1H), 5.54 (s, 2H), 5.12 (s, 2H).

### Stage 2: Production of 3-(2-amino-9-(4-amino-2,6-difluorobenzyl)-9H-purin-6-yl)-2-fluorobenzonitrile

The intermediate compound produced in the aforementioned stage 1 (S15, 100mg, 0.24mmol) was dissolved in ethanol (EtOH), and tin (II) chloride dihydrate (SnCl₂.2H₂O, 265mg, 1.18mmol) was added to the solution, and the solution was agitated for 3 h at 50°C. Afterwards, the aforementioned reaction mixture was diluted with dichloromethane (DCM) and then cleaned with distilled water, and then was dried with magnesium sulfate, filtered, and concentrated, and the aforementioned concentrate was refined with silica gel chromatography to obtain the desired compound (108, 32.9mg, 34.7%). ¹H-NMR (methanol-d₄, 400MHz): δ 8.12-8.08 (m, 1H), 7.96-7.91 (m, 2H), 7.53 (t, 1H), 6.26 (d, 2H), 5.29 (s, 2H).

### Example 1.9: Production of 3-(2-amino-9-(4-amino-2-fluorobenzyl)-9H-purin-6-yl)benzonitrile (compound 109)

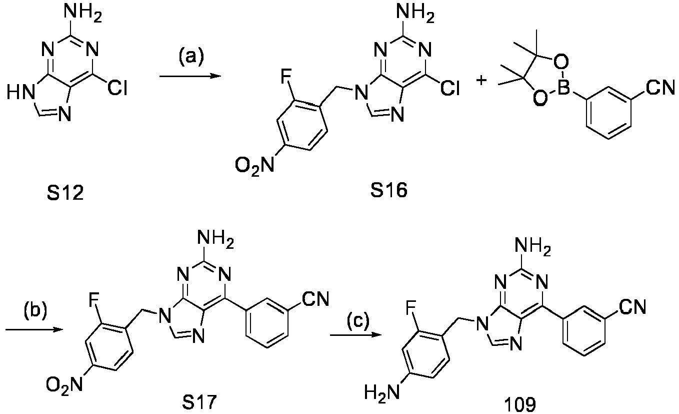

Reagents and conditions: (a) 1-(bromomethyl)-2-fluoro-4-nitrobenzene, K₂CO₃, DMF, rt, overnight; (b) Pd(PPh₃)₄, K₂CO₃, 1,4-dioxane, H₂O, 115°C, 5h; (c) SnCl₂.2H₂O, EtOH, 50°C, 1h.

### Stage 1: Production of the 6-chloro-9-(2-fluoro-4-nitrobenzyl)-9H-purin-2-amine

6-chloro-9H-purin-2-amine (S12, 500mg, 2.95mmol) was melted in dimethylformamide (DMF), and 1-(bromomethyl)-2-fluoro-4-nitrobenzene (758.2mg, 3.24mmol) and potassium carbonate (K₂CO₃, 611.6mg, 4.43mmol) was added to the solution, and the solution was agitated for 1 day. Afterwards, the aforementioned reaction mixture was diluted with dichloromethane (DCM) and then cleaned with distilled water, and was dried with magnesium sulfate, dried, filtered, and concentrated, and the aforementioned concentrate was refined with silica gel chromatography to obtain an intermediate compound (S16, 742mg, 77%). ¹H-NMR (DMSO-d₆, 400MHz): δ 8.23 (s, 1H), 8.18 (dd, 1H), 8.03 (dd, 1H), 7.30 (t, 1H), 6.97 (s, 2H), 5.49 (s, 2H).

### Stage 2: Production of 3-(2-amino-9-(2-fluoro-4-nitrobenzyl)-9H-purin-6-yl)-benzonitrile

The intermediate compound produced in the aforementioned stage 1 (S16, 300mg, 0.93mmol) was melted in a solvent of 1,4-dioxane and H₂O, 3-(4,4,5,5-tetramethyl-1,3,2-dioxaborolan-2-yl)-benzonitrile (318.4mg, 1.39mmol) and tetrakis (triphenylphosphine) palladium ((0) Pd(PPh₃)₄, 53.7mg, 0.05mmol), and potassium carbonate (K₂CO₃, 257mg, 1.86mmol) were added to the solution, and the solution was heated at 115°C and agitated for 5 h. Afterwards, the aforementioned reaction mixture was diluted with dichloromethane (DCM), was cleaned with distilled water, and then was dried with magnesium sulfate, filtered, and concentrated, and the aforementioned concentrate was refined with silica gel chromatography to obtain an intermediate compound (S17, 162mg, 44.8%). ¹H-NMR (DMSO-d₆, 400MHz): δ 9.09 (s, 1H), 9.02 (dd, 1H), 8.35 (s, 1H), 8.19 (dd, 1H), 8.06-8.01 (m, 2H), 7.81 (t, 1H), 7.31 (t, 1H), 6.75 (s, 2H), 5.55 (s, 2H).

### Stage 3: Production of 3-(2-amino-9-(4-amino-2-fluorobenzyl)-9H-purin-6-yl) benzonitrile

The intermediate compound produced in the aforementioned stage 2 (S17, 89mg, 0.23mmol) was dissolved in ethanol (EtOH), and then tin (II) chloride dihydrate (SnCl₂. 2H₂O, 257mg, 1.14mmol) was added to the solution, and the solution was agitated for 1 h at 50°C. Afterwards, the aforementioned reaction mixture was diluted with dichloromethane (DCM), and then was cleaned with distilled water, dried with magnesium sulfate, filtered, and concentrated, and the aforementioned concentrate was refined with silica gel chromatography to obtain the desired compound (109, 19mg, 23%). ¹H-NMR (DMSO-d₆, 400MHz): δ 9.06 (s, 1H), 9.01 (d, 1H), 8.14 (s, 1H), 8.00 (d, 1H), 7.79 (t, 1H), 6.98 (t, 1H), 6.69 (s, 2H), 6.32 (d, 2H), 5.48 (s, 2H), 5.16 (s, 2H).

### Example 1.10: Production of 3-(2-amino-9-(4-amino-2-fluorobenzyl)-9H-purin-6-yl)-2-fluorobenzonitrile (compound 103)

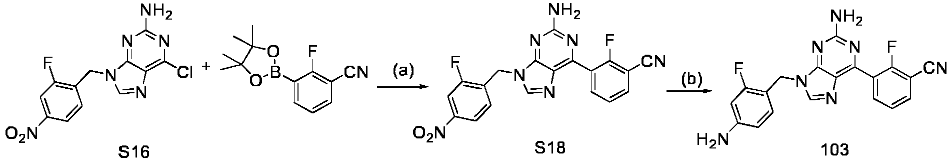

Reagents and conditions: (a) Pd(PPh₃)₄, K₂CO₃ 1,4-dioxane, H₂O, 115°C, overnight; (b) SnCl₂.2H₂O, EtOH, 50°C, 3h.

### Stage 1: Production of 3-(2-amino-9-(2-fluoro-4-nitrobenzyl)-9H-purin-6-yl)-2-fluorobenzonitrile

The same process as stage 2 of the aforementioned example 1.6 was performed except that 2-fluoro-3-(4,4,5,5-tetramethyl-1,3,2-dioxaborolan-2-yl) benzonitrile (138mg, 0.56mmol) was used instead of 3-(4,4,5,5-teramethyl-1,3,2-dioxaborolan-2-yl) benzonitrile (318mg, 1.39mmol) in stage 2 of the aforementioned example 1.6 to obtain an intermediate compound (S18, 80mg, 42.7%). ¹H-NMR (methanol-d₄, 400MHz): δ 8.21 (s, 1H), 8.15-8.07 (m, 3H), 7.98-7.93 (m, 1H), 7.59-7.52 (m, 2H), 5.60 (s, 2H).

### Stage 2: Production of 3-(2-amino-9-(4-amino-2-fluorobenzyl)-9H-purin-6-yl)-2-fluorobenzonitrile

In stage 3 of the aforementioned example 1.6, only the amount of tin(II) chloride dihydrate (SnCl₂.2H₂O, 180mg, 0.80mmol) was changed and the same process as stage 3 of the aforementioned example 1.6 was performed for the intermediate compound of the aforementioned stage 1 (S18, 65mg, 0.16mmol) to obtain the desired compound (103, 14.4mg, 24%). ¹H-NMR (DMSO-d₆, 400MHz): δ 8.17 (t, 1H), 8.10 (t, 1H), 8.05 (s, 1H), 7.58 (t, 1H), 7.01 (t, 1H), 6.74 (s, 2H), 6.36-6.32 (m, 2H), 5.50 (s, 2H), 5.15 (s, 2H).

### Example 1.11: Production of 3-(2-amino-9-(4-amino-3-methylbenzyl)-9H-purin-6-yl)benzonitrile (compound 104)

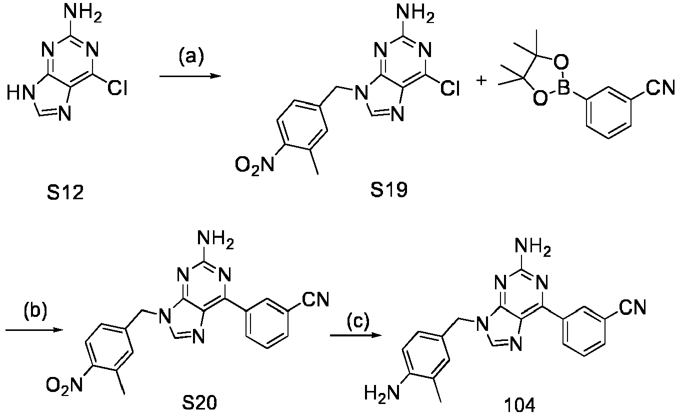

Reagent and condition: (a) 4-(bromomethyl)-2-methyl-1-nitrobenzene, K₂CO₃, DMF, rt, overnight; (b) Pd(PPh₃)₄, K₂CO₃, 1,4-dioxane, H₂O, 115°C, 2h; (c) SnCl₂.2H₂O, EtOH, 55°C, overnight.

### Stage 1: Production of 6-chloro-9-(3-methyl-4-nitrobenzyl)-9H-purin-2-amine

6-chloro-9H-purin-2-amine (S12, 846mg, 4.99mmol) was dissolved *in N,N-*dimethylformamide (DMF), 4-(bromomethyl)-2-methyl-1-nitrobenzene (1.263g, 5.49mmol) and potassium carbonate (K₂CO₃, 1.03g, 7.49mmol) was added to the solution, and then the solution was agitated for 1 day at room temperature. Afterwards, the reaction mixture was diluted with dichloromethane (DCM), and was cleaned with distilled water, and then was dried with magnesium sulfate, filtered, and concentrated, and the aforementioned concentrated was refined with silica gel chromatography to obtain an intermediate compound (S19, 1.28g, 80.5%). ¹H-NMR (CDCl₃, 400MHz): δ 7.97 (d, 1H), 7.77 (s, 1H), 7.22-7.18 (m, 2H), 5.30 (s, 2H), 2.59 (s, 3H).

### Stage 2: Production of 3-(2-amino-9-(3-methyl-4-nitrobenzyl)-9H-purin-6-yl) benzonitrile

The intermediate compound produced in the aforementioned stage 1 (S19, 278mg, 0.87mmol) was dissolved in a solvent of 1,4-dioxane and H₂O, and 3-(4,4,5,5-tetramethyl-1,3,2-dioxaborolan-2-yl)-benzonitrile (300mg, 1.31mmol) and tetrakis (triphenylphosphine) palladium (0) ((Pd(PPh₃)₄, 50.3mg, 0.043mmol), and potassium carbonate (K₂CO₃, 240.4mg, 1.74mmol) were added to the solution, and the solution was heated at 105°C and agitated for 2 h. Afterwards, the aforementioned reaction mixture was diluted with dichloromethane (DCM), and was cleaned with distilled water, and then was dried with magnesium sulfate, filtered, and concentrated, and the aforementioned concentrate was refined with silica gel chromatography to obtain an intermediate compound (S20, 143mg, 42.7%). ¹H-NMR (CDCl₃, 400MHz): δ 9.07 (s, 1H), 9.02 (d, 1H), 7.98 (d, 1H), 7.84 (s, 1H), 7.78 (d, 1H), 7.59-7.51 (m, 1H), 7.50-7.44 (m, 2H), 5.36 (s, 2H), 5.08 (s, 2H), 2.59 (s, 3H).

### Stage 3: Production of 3-(2-amino-9-(4-amino-3-methylbenzyl)-9H-purin-6-yl) benzonitrile

The intermediate compound produced in the aforementioned stage 2 (S20, 143mg, 0.37mmol) was dissolved with ethanol (EtOH), and tin (II) chloride dihydrate (SnCl₂.2H₂O, 418.6mg, 1.86mmol) was added, and the solution was agitated at 55°C for 1 day. Afterwards, the aforementioned reaction mixture was diluted with dichloromethane (DCM) and was cleaned with diluted water, and then was dried with magnesium sulfate, filtered, and concentrated, and the aforementioned concentrate was refined with silica gel chromatography to obtain the desired compound (104, 23.6mg, 18%). ¹H-NMR (DMSO-d₆, 400MHz): δ 9.08 (s, 1H), 9.02 (d, 1H), 8.23 (s, 1H), 7.99 (d, 1H), 7.79 (t, 1H), 6.96 (s, 1H), 6.91 (d, 1H), 6.86 (s, 2H), 6.55 (d, 1H), 5.11 (s, 2H), 4.87 (s, 2H), 2.01 (s, 3H).

### Example 1.12: Production of 3-(2-amino-9-(4-amino-3-methylbenzyl)-9H-purin-6-yl)-2-fluorobenzonitrile (compound 106)

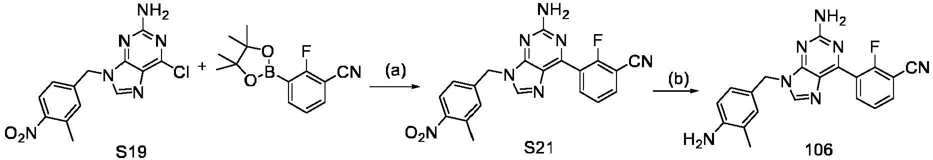

Reagent and condition: (a) Pd(PPh₃)₄, K₂CO₃, 1,4-dioxane, H₂O, 115°C, 2h; (c) SnCl₂.2H₂O, EtOH, 55°C, overnight.

### Stage 1: Production of 3-(2-amino-9-(3-methyl-4-nitrobenzyl)-9H-purin-6-yl)-2-fluorobenzonitrile

The intermediate compound (S19, 300mg, 0.94mmol) was dissolved in a solvent of 1,4-dioxane and H₂O, and then 2-fluoro-3-(4,4,5,5-tetramethyl-1,3,2-dioxaborolran-2-yl) benzonitrile (349mg, 1.41mmol) and tetrakis (triphenylphosphine) palladium (0) ((Pd(PPh₃)₄, 54.3mg, 0.05mmol), and potassium carbonate (K₂CO₃, 260mg, 1.88mmol) were added to the solution, and the solution was heated at 115°C and agitated for 2h. Afterwards, the aforementioned reaction mixture was diluted with dichloromethane (DCM), and was cleaned with distilled water, and then was dried with magnesium sulfate, filtered, and concentrated, and the aforementioned concentrate was refined with silica gel chromatography to obtain an intermediate compound (S21, 298.2mg, 78.6%). ¹H-NMR (CDCl₃, 400MHz): δ 8.22-8.16 (m, 1H), 7.99 (d, 1H), 7.83 (s, 1H), 7.79 (t, 1H), 7.70-7.64 (m, 1H), 7.43 (t, 1H), 7.25 (s, 1H), 5.13 (s, 2H), 2.60 (s, 3H).

### Stage 2: Production of 3-(2-amino-9-(4-amino-3-methylbenzyl)-9H-purin-6-yl)-2-fluorobenzonitrile

The intermediate compound produced in the aforementioned stage 1 (S21, 298.2mg, 0.74mmol) was dissolved in ethanol (EtOH), and then tin (II) chloride dihydrate (SnCl₂.2H₂O, 835mg, 3.7mmol) was added to the solution, and the solution was agitated for 1 day at 55°C. Afterwards, the aforementioned reaction mixture was diluted with dichloromethane (DCM), and was cleaned with distilled water, and then was dried with magnesium sulfate, filtered, and concentrated, and the aforementioned concentrate was refined with silica gel chromatography to obtain the desired compound (106, 98mg, 35.5%). ¹H-NMR (DMSO-d₆, 400MHz): δ 8.19-8.14 (m, 2H), 8.10-8.07 (m, 1H), 7.57 (t, 1H), 6.97 (s, 1H), 6.92 (d, 1H), 6.73 (s, 2H), 6.55 (d, 1H), 5.09 (s, 2H), 4.88 (s, 2H), 2.01 (s, 3H).

### Example 1.13: Production of 3-(2-amino-9-(2,6-difluorobenzyl)-9H-purin-6-yl) benzonitrile (compound 112)

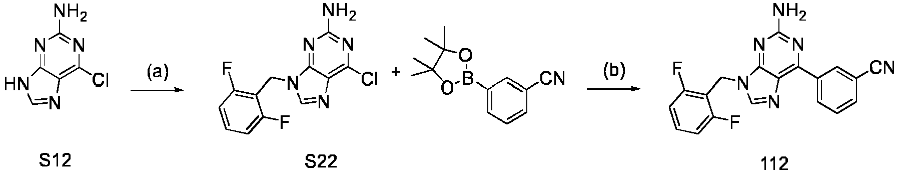

Reagent and condition: (a) 2,6-difluorobenzyl bromide, K₂CO₃, DMF, rt, 4h; (b) Pd(PPh₃)₄, K₂CO₃, 1,4-dioxane, H₂O, 115°C, overnight.

### Stage 1: Production of 6-chloro-9-(2,6-difluorobenzyl)-9H-purin-2-amine

6-chloro-9H-purin-2-amine (S12, 800mg, 4.72mmol) was dissolved in dimethylformamide (DMF), and 2,6-difluorobenzyl bromide (1.46g, 7.08mmol) and potassium carbonate (K₂CO₃, 1.95g, 14.16mmol) were added to the solution, and the solution was agitated for 4 h at room temperature. Afterwards, the aforementioned reaction mixture was filtered with a celite pad, and the remaining liquid was diluted with ethyl acetate (EA). Then, the solution was cleaned with diluted water and then was dried with sodium sulfate, filtered, and concentrated, and the aforementioned concentrate was refined with silica gel chromatography to obtain an intermediate compound (S22, 850mg, 58%). ¹H-NMR (DMSO-d₆, 400MHz): δ 8.10 (s, 1H), 7.49-7.46(m, 1H), 7.15 (t, 2H), 6.68(s, 2H), 5.35(s, 2H).

### Stage 2: Production of 3-(2-amino-9-(2,6-difluorobenzyl)-9H-purin-6-yl) benzonitrile

The intermediate compound produced in the aforementioned stage 1 (S22, 100mg, 0.31mmol) was dissolved in a solvent of 1,4-dioxane and H₂O, and 3-(4,4,5,5-tetramethyl-1,3,2-dioxabororan-2-yl) benzonitrile (85.2mg, 0.37mmol) and tetrakis (triphenylphosphine) palladium (0) ((Pd(PPh₃)₄,18mg, 0.02mmol), and potassium carbonate (K₂CO₃, 85.7mg, 0.62mmol) were added to the solution , and the solution was heated at 115°C and agitated for 1 day. Afterwards, the aforementioned reaction mixture was diluted with dichloromethane (DCM) and cleaned with distilled water, and then was dried with magnesium sulfate, filtered, and concentrated, and the aforementioned concentrate was refined with silica gel chromatography to obtain the desired compound (112, 81mg, 89%). ¹H-NMR (CDCl₃, 400MHz): δ 9.02 (s, 1H), 8.98 (dd, 1H), 7.87 (s, 1H), 7.75 (dd, 1H), 7.61 (t, 1H), 7.39-7.32 (m, 1H), 6.97 (t, 2H), 5.39 (s, 2H), 5.07 (s, 2H).

### Example 1.14: Production of 3-(2-amino-9-(2,6-difluorobenzyl)-9H-purin-6-yl)-2-fluorobenzonitrile (compound 113)

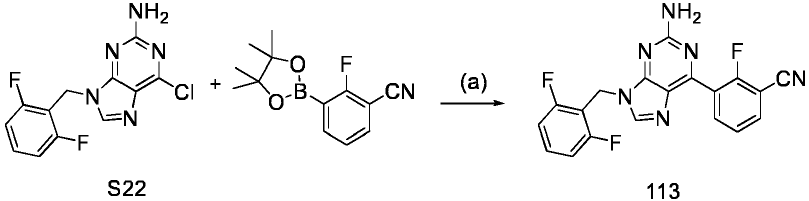

Reagent and condition: (a) Pd(PPh₃)₄, K₂CO₃, 1,4-dioxane, H₂O, 115°C, overnight.

The intermediate compound produced in stage 1 of the aforementioned example 1.13 (S22, 50mg, 0.17mmol) was dissolved in a solvent of 1,4-dioxane and H₂O, and 2-fluoro-3-(4,4,5,5-tetramethyl-1,3,2-dioxaborolan-2-yl) benzonitrile (49.4mg, 0.20mmol), tetrakis (triphenylphosphine) palladium (0) ((Pd(PPh₃)₄, 8.7mg, 0.008mmol), and potassium carbonate (K₂CO₃, 47mg, 0.34mmol) were added to the solution, and the solution was heated at 115°C and agitated for 1 day. Afterwards, the aforementioned reaction mixture was diluted with dichloromethane (DCM), and was cleaned with diluted water, and then was dried with magnesium sulfate, filtered, and concentrated, and the aforementioned concentrate was refined with silica gel chromatography to obtain the desired compound (113, 45mg, 70%). ¹H-NMR (DMSO-d₆, 400MHz): δ 8.18-8.11 (m, 1H), 8.10-8.08 (m, 2H), 7.57 (t, 1H), 7.51-7.46 (m, 1H), 7.16 (t, 2H), 6.73 (s, 2H), 5.39 (s, 2H).

### Example 1.15: Production of 3-(2-amino-9-(2,6-difluorobenzyl)-9H-purin-6-yl)-2-methylbenzonitrile (compound 114)

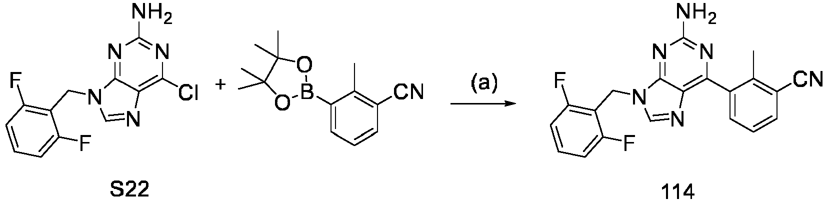

Reagent and condition: (a) Pd(PPh₃)₄, K₂CO₃, 1,4-dioxane, H₂O, 115°C, 4h.

The intermediate compound produced in stage 1 of the aforementioned example 1.13 (S22, 50mg, 0.17mmol) was dissolved in a solvent of 1,4-dioxane and H₂O, and 2-methyl-3-(4,4,5,5-tetramethyl-1,3,2-dioxaborolan-2-yl)benzonitrile (48.6mg, 0.20mmol) and tetrakis (triphenylphosphine) palladium (0) ((Pd(PPh₃)₄, 8.7mg, 0.008mmol), and potassium carbonate (K₂CO₃, 47mg, 0.34mmol) were added, and the solution was heated at 115°C and agitated for 4 h. Afterwards, the reaction mixture was diluted with dichloromethane (DCM), and was cleaned with distilled water, and then was dried with magnesium sulfate, filtered, and concentrated, and the aforementioned concentrate was refined with silica gel chromatography to obtain the desired compound (114, 47.6mg, 74%). ¹H-NMR (Methanol-d₄, 400MHz): δ 8.07 (s, 1H), 7.83 (dd, 1H), 7.77 (dd, 1H), 7.54-7.46 (m, 2H), 7.07 (t, 2H), 5.50 (s, 2H), 2.51 (s, 3H).

### Example 1.16: Production of 3-[2-amino-9-[[4-amino-3-(trifluoromethyl)phenyl]methyl]purin-6-yl]benzonitrile (compound 110)

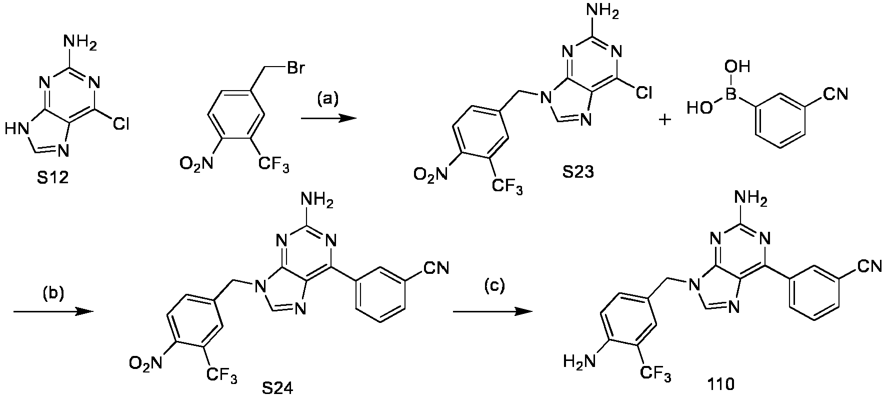

Reagents and condition: (a) K₂CO₃, DMAc, 25°C, 3h; (b) Pd(dppf)Cl₂, K₂CO₃, dioxane/H₂O, 110°C, 15h; (c) Fe, NH₄Cl, EtOH/H₂O, 80°C, 2h.

### Stage 1: Production of 6-chloro-9-[[4-nitro-3-(trifluoromethyl)phenyl]methyl]purin-2-amine .

To the solution of 4-(bromomethyl)-1-nitro-2-(trifluoromethyl)benzene (3 g, 10.56 mmol) in DMAc (50 mL) were added intermediate S12, 6-chloro-9H-purin-2-amine (1.79 g, 10.56 mmol) and K₂CO₃ (2.92 g, 21.12 mmol). Then the mixture was stirred at 25 °C for 3 h. After that the mixture was added water (80 mL) and extracted with ethyl acetate (80 mL x 3). Combined the organic phase to wash by brines (150 mL x 2), dry over anhydrous sodium sulfate, filter and concentrate in vacuum to give a residue. The residue was purified by flash silica gel chromatography (80 g SepaFlash^{®} Silica Flash Column, Eluent of 20~100% Ethyl acetate/Petroleum ether gradient @ 60 mL/min). Compound S23, 6-chloro-9-[[4-nitro-3-(trifluoromethyl)phenyl]methyl]purin-2-amine (2.5 g, 6.62 mmol, 63% yield, 98.7% purity) was obtained as a yellow solid. ¹H NMR (400 MHz, DMSO-d₆): δ 8.27 (s, 1H), 8.12 (d, *J*= 8.3 Hz, 1H), 8.05 (s, 1H), 7.67 (dd, *J=* 8.3 Hz, 1H), 6.97 (s, 2H), 5.50 (s, 2H). MS: m/z = 373.0 (M+1, ESI+).

### Stage 2: Production of 3-[2-amino-9-[[4-nitro-3-(trifluoromethyl)phenyl] methyl]purin-6-yl]benzonitrile

To the solution of compound S23, 6-chloro-9-[[4-nitro-3-(trifluoromethyl)phenyl]methyl] purin-2-amine (2.5 g, 6.71 mmol, 1 eq) in dioxane (40 mL) and water (10 mL) were added (3-cyanophenyl)boronic acid (1.97 g, 13.42 mmol, 2 eq), Pd(dppf)Cl₂ (490 mg, 670.80 µmol, 0.1eq) and K₂CO₃ (1.85 g, 13.42 mmol, 2 eq). Then the mixture was stirred at 110 °C for 15 h. The mixture was concentrated in vacuum to give a residue. The residue was purified by flash silica gel chromatography (40g SepaFlash^{®} Silica Flash Column, Eluent of 20~60% Ethyl acetate/Petroleum ether gradient @ 50 mL/min). Compound S24, 3-[2-amino-9-[[4-nitro-3-(trifluoromethyl)phenyl]methyl]purin-6-yl]benzonitrile (2.3 g, 4.56 mmol, 68% yield, 87% purity) was obtained as a yellow solid. ¹H NMR (400 MHz, DMSO-d₆): δ 9.08 (t, *J=* 1.5 Hz, 1H), 9.02 (td, *J =* 1.4, 8.0 Hz, 1H), 8.38 (s, 1H), 8.13 (d, *J=* 8.4 Hz, 1H), 8.08 (d, *J=* 1.2 Hz, 1H), 8.01 (td, *J=* 1.3, 7.8 Hz, 1H), 7.79 (t, J = 7.8 Hz, 1H), 7.71 (dd, *J =* 1.5, 8.4 Hz, 1H), 6.73 (s, 2H), 5.56 (s, 2H). MS: m/z = 440.0 (M+1, ESI+).

Stage 3: Production of 3-[2-amino-9-[[4-amino-3-(trifluoromethyl) phenyl]methyl]purin-6-yl]benzonitrile To the solution of 3-[2-amino-9-[[4-nitro-3-(trifluoromethyl)phenyl] methyl] purin-6-yl]benzonitrile (2.3 g, 4.55 mmol, 87% purity, 1 eq) in ethanol (30 mL) and water (6 mL) were added iron dust (1.27 g, 22.77 mmol, 5 eq) and NH₄Cl (1.95 g, 36.44 mmol, 8 eq). Then the mixture was stirred at 80 °C for 2 h. The reaction mixture was filtered and the filtrate was concentrated in vacuum. To the mixture was added water (80 mL) and extracted with ethyl acetate (80 mL x 3). Combined the organic phase to filter and concentrate in vacuum to give a residue. The residue was purified by prep-HPLC(column: Welch Ultimate XB-CN 250*70*10µm;mobile phase: [Hexane-EtOH (0.1% ammonia hydroxide)];B%: 30%-70%,15min) to give a product. The product was purified by re-crystallization from THF (20 mL) at 70 °C to give crude product. The crude product was purified by prep-HPLC(column: Welch Ultimate XB-SiOH 250*50*10µm;mobile phase: [Hexane-EtOH (0.1% ammonia hydroxide)];B%: 5%-45%,15min). Compound 110or 3-[2-amino-9-[[4-amino-3-(trifluoromethyl) phenyl]methyl]purin-6-yl]benzonitrile (602.34 mg, 1.44 mmol, 32% yield, 97.62% purity) was obtained as a yellow solid. ¹H NMR (400 MHz, DMSO-d₆) δ = 9.07 (s, 1H), 9.01 (br d, J = 7.9 Hz, 1H), 8.30 (s, 1H), 8.00 (br d, J = 7.7 Hz, 1H), 7.78 (t, J = 7.9 Hz, 1H), 7.41 (s, 1H), 7.29 (br d, J = 8.6 Hz, 1H), 6.79 (d, J = 8.6 Hz, 1H), 6.71 (s, 2H), 5.65 (s, 2H), 5.19 (s, 2H). HRMS-TOF: 410.1347

### Example 1.17: Production of 3-[2-amino-9-[[4-amino-3-(trifluoromethyl)phenyl]methyl]purin-6-yl]2-fluorobenzonitrile (compound 111)

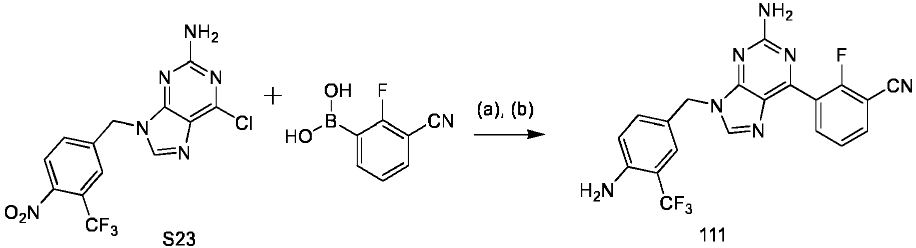

Reagents and condition: (a) Pd(PPh₃)₄, K₂CO₃, dioxane/H₂O, 110°C, 15h; (b) Fe, NH₄Cl, EtOH/H₂O, 60°C, 1h.

### Stage 1: Production of 3-[2-amino-9-[[4-nitro-3-(trifluoromethyl)phenyl]methyl]purin-6-yl]-2-fluorobenzonitrile

A mixture of (3-cyano-2-fluorophenyl)boronic acid (265 mg, 1.61 mmol, 1.5 eq), intermediate compound S23 or 6-chloro-9-[[4-nitro-3-(trifluoromethyl) phenyl]methyl]purin-2-amine (400 mg, 1.07 mmol), Pd(PPh₃)₄ (124 mg, 107.00 µmol) and K₂CO₃ (296 mg, 2.14 mmol ) in dioxane (10 mL) and water (1 mL) was degassed and purged with nitrogen for three times, and then the mixture was stirred at 100 °C for 15 h under nitrogen atmosphere. The reaction mixture was concentrated under reduced pressure to remove dioxane and water to give a residue. The residue was purified by flash silica gel chromatography (ISO^{®}; 12 g SepaFlash^{®} Silica Flash Column, Eluent of 0~100% Ethyl acetate/Petroleum ether gradient @ 35 mL/min). Intermediate compound 3-[2-amino-9-[[4-nitro-3-(trifluoromethyl)phenyl]methyl]purin-6-yl]-2-fluorobenzonitrile (200 mg, 437.31 µmol, 41%yield) was obtained as a yellow solid. MS: m/z = 440.3 (M+1, ESI+).

### Stage 2: Production of 3-[2-amino-9-[[4-amino-3-(trifluoromethyl)phenyl]methyl]purin-6-yl]2-fluorobenzonitrile.

To a solution of 3-[2-amino-9-[[4-nitro-3-(trifluoromethyl)phenyl]methyl] purin-6-yl]-2-fluorobenzonitrile (300 mg, 655.97 µmol, 1 eq) in ethanol (15 mL) and water (5 mL) was added NH₄Cl (292 mg, 5.25 mmol, 8 eq) and iron dust (183 mg, 3.28 mmol, 5 eq). The mixture was stirred at 60 °C for 1 h. The reaction mixture was concentrated under reduced pressure to remove ethanol to give a residue. The residue was purified by prep-HPLC (column: Phenomenex luna C18 150*25mm* 10µm;mobile phase: [water(0.225%FA)-ACN];B%: 40%-70%,10min). Compound 111 or 3-[2-amino-9-[[4-amino-3-(trifluoromethyl)phenyl]methyl]purin-6-yl]benzonitrile (75.32 mg, 175.81 µmol, 27%yield, 99.75% purity) was obtained as a white solid. ¹H NMR (400 MHz, DMSO-d₆): δ 8.21 (s, 1H), 8.17 (t, 1H), 8.09 (t, 1H), 7.57 (t, 1H), 7.41 (s, 1H), 7.30 (br d, 1H), 6.80 (d, 1H), 6.75 (s, 2H), 5.65 (s, 2H), 5.17 (s, 2H). HRMS-TOF: 428.1244.

### Example 1.18: Production of 6-[2-amino-9-[(4-aminophenyl)methyl]purin-6-yl] pyridine-2-carbonitrile (compound 115)

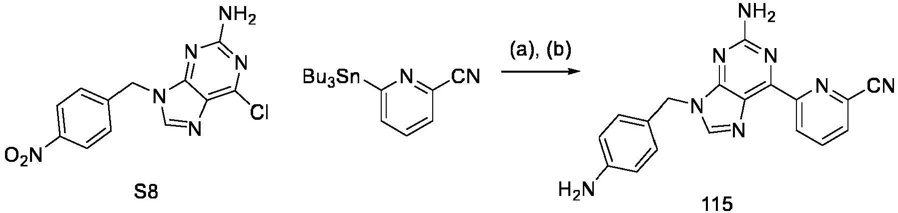

Reagents and condition: (a) XPhos-Pd-G3, THF, 90°C, 15h; (b) Fe, NH₄Cl, THF/H₂O, 80°C, 2h.

### Stage 1: Production of 6-[2-amino-9-[(4-nitrophenyl)methyl]purin-6-yl]pyridine-2-carbonitrile

To the solution of intermediate compound S8, 6-chloro-9-[(4-nitrophenyl)methyl]purin-2-amine (800 mg, 2.63 mmol, 1 eq) in THF (20 mL) were added 6-tributylstannylpyridine-2-carbonitrile (2.06 g, 5.25 mmol, 2 eq) and XPhos-Pd-G3 (222 mg, 262.56 µmol, 0.1eq). Then the mixture was stirred at 90 °C for 15 h. To the mixture was added water (30 mL) and extracted with ethyl acetate (30 mL x 3). Combined the organic phase to dry over anhydrous sodium sulfate, filter and concentrate in vacuum to give a residue. The residue was purified by flash silica gel chromatography (12 g Sepa Flash^{®} Silica Flash Column, Eluent of 0~100% Ethyl acetate/Petroleum ether gradient @ 35 mL/min). Compound 6-[2-amino-9-[(4-nitrophenyl)methyl]purin-6-yl]pyridine-2-carbonitrile (167 mg, 372.27 µmol, 14% yield, 83% purity) was obtained as a yellow solid. ¹H NMR (400 MHz, DMSO-d₆): δ 8.91 (dd, *J= 0.9,* 8.1 Hz, 1H), 8.37 (s, 1H), 8.31 - 8.25 (m, 1H), 8.22 (d, *J=* 8.7 Hz, 2H), 8.18 (dd, *J=* 0.9, 7.6 Hz, 1H), 7.50 (d, *J=* 8.7 Hz, 2H), 6.85 (br s, 1H), 5.53 (s, 2H). MS: m/z = 373.1 (M+1, ESI+).

### Stage 2: Production of 6-[2-amino-9-[(4-aminophenyl)methyl]purin-6-yl] pyridine-2-carbonitrile

To the solution of 6-[2-amino-9-[(4-nitrophenyl)methyl]purin-6-yl]pyridine-2-carbonitrile (160 mg, 429.72 µmol, 1eq) in water (3 mL) and THF (9 mL) were added NH₄Cl (184 mg, 3.44 mmol, 8 eq) and iron dust (120 mg, 2.15 mmol, 5 eq). Then the mixture was stirred at 80 °C for 2 h. The reaction mixture was filtered and the filtrate was added saturated NaHCO₃ solution (30 mL) and extracted with ethyl acetate (80 mL x 3). Combined the organic phase to dry over anhydrous sodium sulfate, filter and concentrate in vacuum to give a residue. The residue was purified by prep-HPLC (column: Waters Xbridge C18 150*50mm* 10µm;mobile phase: [water(10mM NH₄HCO₃)-ACN];B%: 5%-41%,10min). Compound 115or 6-[2-amino-9-[(4-aminophenyl)methyl]purin-6-yl]pyridine-2-carbonitrile (18.59 mg, 53.55 µmol, 12% yield, 95.94% purity) was obtained as a yellow solid. ¹H NMR (400 MHz, DMSO-d₆) δ = 8.92 (br d, *J =* 7.9 Hz, 1H), 8.34 - 8.22 (m, 2H), 8.16 (br d, *J =* 7.5 Hz, 1H), 7.05 (br d, *J =* 8.0 Hz, 2H), 6.81 (br s, 2H), 6.52 (br d, *J =* 8.0 Hz, 2H), 5.13 (br d, *J* = 7.3 Hz, 4H). HRMS-TOF: 343.1415.

### Example 1.19: Production of 6-[2-amino-9-[(4-amino-2-fluorophenyl)methyl]purin-6-yl]pyridine-2-carbonitrile (compound 119)

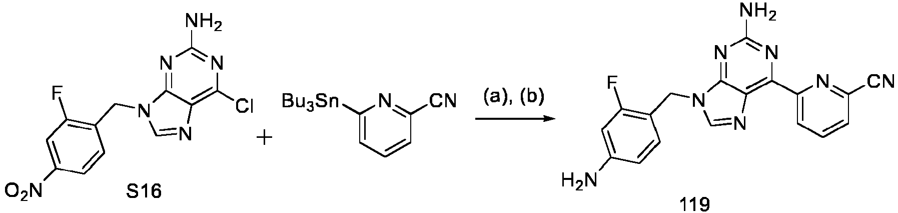

Reagents and condition: (a) XPhos-Pd-G3, THF, 90°C, 15h; (b) Fe, NH₄Cl, THF/H₂O, 80°C, 1.5h.

### Stage 1: Production of 6-[2-amino-9-[(2-fluoro-4-nitro-phenyl)methyl]purin-6-yl]pyridine-2-carbonitrile.

To a mixture of intermediate compound S16 or 6-chloro-9-[(2-fluoro-4-nitrophenyl)methyl]purin-2-amine (0.8 g, 2.48 mmol, 1 eq) in THF (10 mL) were added 6-tributylstannylpyridine-2-carbonitrile (1.8 g, 4.58 mmol, 1.85 eq) and XPhos-Pd-G3 (210 mg, 247.92 µmol, 0.1 eq). Then the mixture was stirred at 90 °C for 15 h. To the mixture was added saturated aqueous potassium fluoride solution (30 mL) and extracted with ethyl acetate (30 mL x 3). Combined the organic phase was and concentrated in vacuum to give a residue. The residue was purified by flash silica gel chromatography (25 g Sepa Flash^{®} Silica Flash Column, Eluent of 0~100% Ethyl acetate/Petroleum ether gradient 50 mL/min). Compound 6-[2-amino-9-[(2-fluoro-4-nitro-phenyl)methyl]purin-6-yl]pyridine-2-carbonitrile (260 mg, 652.78 µmol, 26% yield, 98.0% purity) was obtained as a yellow solid. ¹H NMR (400 MHz, DMSO-d₆) δ = 8.90 (d, *J =* 7.9 Hz, 1H), 8.33 (s, 1H), 8.28 (t, *J =* 7.9 Hz, 1H), 8.21 - 8.15 (m, 2H), 8.05 (dd, *J =* 1.8, 8.5 Hz, 1H), 7.33 (t, *J =* 8.1 Hz, 1H), 6.84 (br s, 2H), 5.55 (s, 2H). MS: m/z = 391.0 (M+1, ESI+).

### Stage 2: Production of 6-[2-amino-9-[(4-amino-2-fluorophenyl)methyl]purin-6-yl]pyridine-2-carbonitrile

To the solution of 6-[2-amino-9-[(2-fluoro-4-nitro-phenyl)methyl]purin-6-yl] pyridine-2-carbonitrile (150 mg, 384.29 µmol, 1 eq) in water (1.5 mL) and THF (6 mL) were added iron powder (107 mg, 1.92 mmol, 5 eq) and NH₄Cl (164 mg, 3.07 mmol, 8 eq). Then the mixture was stirred at 80 °C for 1.5 h. The reaction mixture was filtered. To the filtrate was added saturated NaHCO₃ solution (30 mL) and extracted with ethyl acetate (30 mL x 2). Combined the organic phase was dried over anhydrous Na₂SO₄, filtered and concentrated in vacuum to give a residue. The residue was purified by prep-HPLC (column: Phenomenex Gemini NX-C18(75*30mm*3µm); mobile phase: [water (0.05% ammonia hydroxide v/v)-ACN];B%: 13%-40%,7min). Compound 119 or 6-[2-amino-9-[(4-amino-2-fluorophenyl)methyl]purin-6-yl] pyridine-2-carbonitrile (21.61 mg, 58.63 µmol, 15% yield, 98.70% purity) was obtained as a yellow solid. ¹H NMR (400 MHz, DMSO-d₆) δ = 8.91 (dd, *J= 1.0,* 8.0 Hz, 1H), 8.30 - 8.24 (m, 1H), 8.16 (d, *J =* 7.8 Hz, 1H), 8.14 (s, 1H), 6.99 (t, *J =* 8.5 Hz, 1H), 6.81 (s, 2H), 6.41 - 6.28 (m, 2H), 5.49 (br s, 2H), 5.18 (s, 2H). HRMS-TOF: 361.1321.

### Example 1.20: Production of 6-[2-amino-9-[(4-amino-2,6-difluorophenyl)methyl]purin-6-yl]pyridine-2-carbonitrile (compound 123)

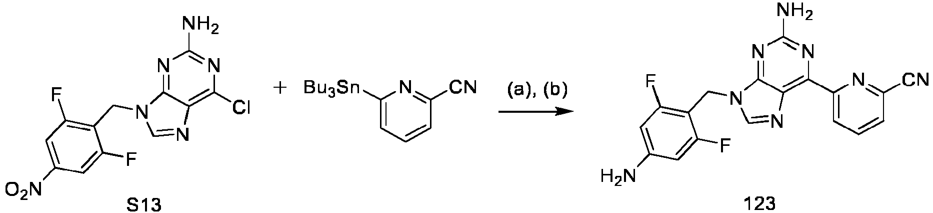

Reagents and condition: (a) XPhos-Pd-G3, THF, 90°C, 15h; (b) Fe, NH₄Cl, THF/H₂O, 80°C, 1.5h.

### Stage 1: Production of 6-[2-amino-9-[(2,6-difluoro-4-nitro-phenyl)methyl]purin-6-yl]pyirdine-2-carbonitrile

A mixture of intermediate compound S13 or 6-chloro-9-[(2,6-difluoro-4-nitrophenyl)methyl]purin-2-amine (1 g, 2.94 mmol, 1 eq), 6-tributylstannylpyridine-2-carbonitrile (2.31 g, 5.87 mmol, 2 eq) and XPhos-Pd-G3 (250 mg, 293.54 µmol, 0.1 eq) in THF (15 mL) was degassed by nitrogen and then heated to 90 °C for 15 h. To the mixture was added water (30 mL) and extracted with ethyl acetate (30 mL x 3). Combined the organic phase was dried over anhydrous sodium sulfate, filtered and concentrated in vacuum to give a residue. The residue was purified by column chromatography (SiO₂, Petroleum ether/Ethyl acetate=50/1 to 0/1). Compound 6-[2-amino-9-[(2,6-difluoro-4-nitro-phenyl)methyl]purin-6-yl]pyridine-2-carbonitrile (500 mg, 1.05 mmol, 36% yield, 85% purity) was obtained as a yellow solid. ¹H NMR (400 MHz, DMSO-d₆) δ = 8.86 (d, *J =* 8.0 Hz, 1H), 8.31 - 8.23 (m, 2H), 8.15 (d, *J*= 7.6 Hz, 1H), 8.09 (d, *J =* 7.4 Hz, 2H), 6.76 (br s, 2H), 5.52 (s, 2H)
MS: m/z = 409.1 (M+1, ESI+).

### Stage 2: Production of 6-[2-amino-9-[(4-amino-2,6-difluoro-phenyl)methyl]purin-6-yl]pyridine-2-carbonitrile

To the solution of 6-[2-amino-9-[(2,6-difluoro-4-nitro-phenyl)methyl]purin-6-yl]pyridine-2-carbonitrile (200 mg, 489.81 µmol, 1 eq) in THF (8 mL) and water (2 mL) were added iron dust (137 mg, 2.45 mmol, 5 eq) and NH₄Cl (210 mg, 3.92 mmol, 8 eq). Then the mixture was stirred at 80 °C for 1.5 h. The reaction mixture was filtered and the filtrate was added saturated NaHCO₃ aqueous solution (30 mL) and extracted with ethyl acetate (80 mL x 3). Combined the organic phase to dry over anhydrous sodium sulfate, filter and concentrate in vacuum to give a residue. The residue was purified by prep-HPLC (column: Waters Xbridge 150*25mm* 5µm; mobile phase: [water(10mM NH₄HCO₃)-ACN]; B%: 18%-38%,9min). Compound 123 or 6-[2-amino-9-[(4-amino-2,6-difluoro-phenyl)methyl]purin-6-yl]pyridine-2-carbonitrile (31.07 mg, 79.80 µmol, 16% yield, 99.33% purity) was obtained as a white solid. ¹H NMR (400 MHz, DMSO-d₆) δ = 8.88 (dd, *J* = 1.1, 8.1 Hz, 1H), 8.29 - 8.22 (m, 1H), 8.15 (dd, *J =* 1.0, 7.8 Hz, 1H), 8.03 (s, 1H), 6.77 (s, 2H), 6.22 (d, *J =* 10.5 Hz, 2H), 5.88 (s, 2H), 5.17 (s, 2H). HRMS-TOF: 379.1235.

### Example 1.21: Production of 6-[2-amino-9-[(2,6-difluorophenyl)methyl]purin-6-yl]pyridine-2-carbonitrile (compound 131)

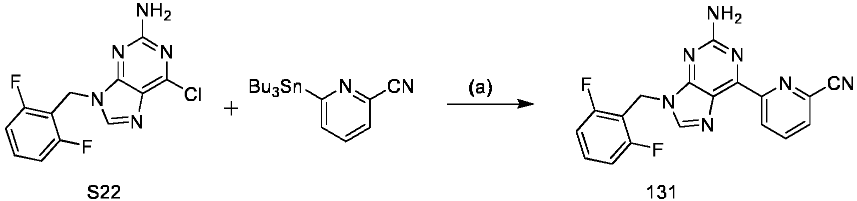

Reagents and condition: (a) XPhos-Pd-G3, THF, 90°C, 15h.

To the solution of intermediate compound S22 or 6-chloro-9-[(2,6-difluorophenyl)methyl]purin-2-amine (200 mg, 676.42 µmol, 1 eq) and 6-tributylstannylpyridine-2-carbonitrile (266 mg, 676.42 µmol, 1 eq) in THF (10 mL) was added XPhos-Pd-G3 (57.26 mg, 67.64 µmol, 0.1 eq). Then the mixture was stirred at 90 °C for 15 h. To the mixture was added potassium fluoride aqueous solution (30 mL) and extracted with ethyl acetate (30 mL x 3). Combined the organic phase was dried over anhydrous Na₂SO₄, filtered and concentrated in vacuum to give a residue. The residue was purified by flash silica gel chromatography (12 g Sepa Flash^{®} Silica Flash Column, Eluent of 0~100% Ethyl acetate/Petroleum ether gradient @ 35 mL/min). Compound 133 or 6-[2-amino-9-[(2,6-difluorophenyl)methyl]purin-6-yl]pyridine-2-carbonitrile (40.26 mg, 110.81 µmol, 16% yield, 96.84% purity) was obtained as a yellow solid. ¹H NMR (400 MHz, DMSO-d₆) δ = 8.87 (dd, *J =* 1.0, 8.1 Hz, 1H), 8.29 - 8.22 (m, 1H), 8.18 (s, 1H), 8.15 (dd, *J =* 1.0, 7.7 Hz, 1H), 7.58 - 7.39 (m, 1H), 7.16 (t, *J =* 8.1 Hz, 2H), 6.77 (s, 2H), 5.42 (s, 2H). HRMS-TOF: 364.1121.

### Example 1.22: Production of 6-[2-amino-9-[(4-amino-3-methylphenyl)methyl]purin-6-yl]pyridine-2-carbonitrile (compound 127)

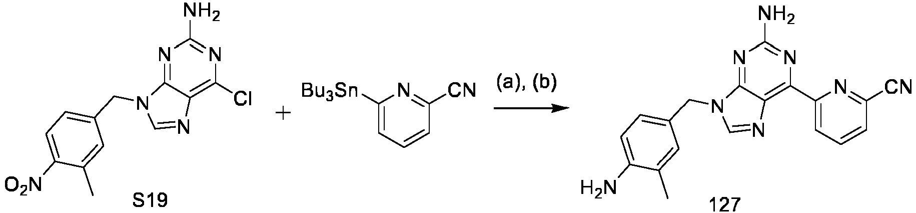

Reagents and condition: (a) XPhos-Pd-G3, THF, 90°C, 15h; (b) Fe, NH₄Cl, THF/H₂O, 80°C, 1h.

### Stage 1: Production of 6-[2-amino-9-[(3-methyl-4-nitro-phenyl)methyl] purin-6-yl]pyridine-2-carbonitrile

A mixture of intermediate compound S19 or 6-chloro-9-[(3-methyl-4-nitrophenyl)methyl]purin-2-amine (0.8 g, 2.51 mmol, 1 eq), 6-tributylstannylpyridine-2-carbonitrile (1.80 g, 4.58 mmol, 1.82 eq) and XPhos-Pd-G3 (212 mg, 251.01 µmol, 0.1 eq) in THF (10 mL) was de-gassed and then heated to 90 °C for 15 h under nitrogen. To the mixture was added saturated potassium fluoride aqueous solution (30 mL) and extracted with ethyl acetate (30 mL x 2). The combined organic phase was dried over anhydrous Na₂SO₄, filtered and concentrated in vacuum to give a residue. The residue was purified by flash silica gel chromatography (25 g Sepa Flash^{®} Silica Flash Column, Eluent of 0~100% Ethyl acetate/Petroleum ether gradient @ 50 mL/min). Compound 6-[2-amino- 9-[(3-methyl-4-nitro-phenyl)methyl]purin-6-yl]pyridine-2-carbonitrile (350 mg, 900.89 µmol, 35% yield, 99.45% purity) was obtained as a yellow solid. ¹H NMR (400 MHz, DMSO-d₆) δ = 8.91 (dd, *J=* 0.8, 8.0 Hz, 1H), 8.36 (s, 1H), 8.32 - 8.24 (m, 1H), 8.17 (dd, *J=* 0.9, 7.7 Hz, 1H), 7.98 (d, *J=* 8.4 Hz, 1H), 7.41 (s, 1H), 7.28 (br d, *J =* 8.4 Hz, 1H), 6.83 (s, 2H), 5.45 (s, 2H). MS: m/z = 387.1 (M+1, ESI+).

### Stage 2: Production of 6-[2-amino-9-[(4-amino-3-methyl-phenyl)methyl]purin-6-yl]pyridine-2-carbonitrile

To the solution of 6-[2-amino-9-[(3-methyl-4-nitro-phenyl)methyl]purin-6-yl] pyridine-2-carbonitrile (150 mg, 388.23 µmol, 1 eq) in water (1 mL) and THF (4 mL) were added iron dust (108 mg, 1.94 mmol, 5 eq) and NH₄Cl (166 mg, 3.11 mmol, 8 eq). Then the mixture was stirred at 80 °C for 1 h. The reaction mixture was filtered. To the filtrate was added saturated NaHCO₃ solution (80 mL) and extracted with ethyl acetate (80 mL x 3). The combined organic phase was dried over anhydrous Na₂SO₄, filtered and concentrated in vacuum to give a residue. The residue was purified by prep-HPLC (column: Phenomenex Gemini NX-C18(75*30mm*3µm); mobile phase: [water(10mM NH₄HCO₃)-ACN];B%: 10%-40%,8min). Compound 127 or 6-[2-amino-9-[(4-amino-3-methyl-phenyl)methyl]purin-6-yl]pyridine-2-carbonitrile (37.55 mg, 101.86 µmol, 26% yield, >99% purity) was obtained as a yellow solid. ¹H NMR (400 MHz, DMSO-d₆) δ = 8.90 (dd, *J =* 1.0, 8.1 Hz, 1H), 8.28 - 8.23 (m, 1H), 8.22 (s, 1H), 8.15 (dd, J= 1.1, 7.7 Hz, 1H), 6.95 (s, 1H), 6.91 (dd, *J* = 1.9, 8.1 Hz, 1H), 6.79 (s, 2H), 6.55 (d, *J =* 8.1 Hz, 1H), 5.11 (s, 2H), 4.89 - 4.83 (m, 2H), 2.01 (s, 3H). HRMS-TOF: 357.1571.

### Example 1.23: Production of 2-[2-amino-9-[(4-aminophenyl)methyl]purin-6-yl]pyridine-4-carbonitrile (compound 116)

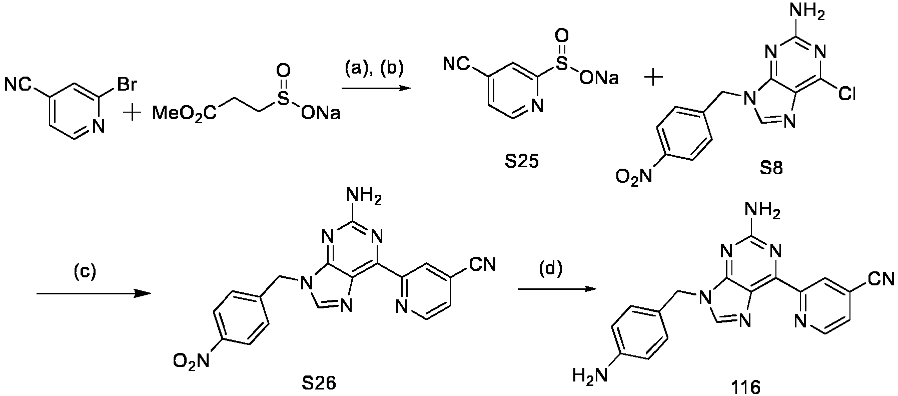

Reagents and condition: (a) CuI, DMSO, 110°C, 15h; (b) NaOMe, MeOH, 25°C, 0.5 h; (c) Pd(OAc)₂, Cy₃P, K₂CO₃, Dioxane, 130°C, 10h, MW; (d) Fe, NH₄Cl, EtOH/H₂O, 60°C, 1h.

### Stage 1: Production of methyl 3-((4-cyanopyridin-2-yl)sulfonyl)propanoate

A mixture of 2-bromopyridine-4-carbonitrile (4.4 g, 24.04 mmol, 1 eq), sodium 3-methoxy-3-oxo-propane-1-sulfinate (5.02 g, 28.85 mmol, 1.2 eq), CuI (5.49 g, 28.85 mmol, 1.2 eq) in DMSO (50 mL) was degassed and purged with nitrogen for three times, and then the mixture was stirred at 110 °C for 15 h under nitrogen atmosphere. The mixture was then cooled to room temperature, diluted with ethyl acetate (50 mL) and filtered through a pad of silica. The filtrate was washed by water (2 x 20 mL), brine (20 mL), dried over anhydrous sodium sulfate, filtered through a pad of silica and concentrated under reduced pressure. The residue was purified by flash silica gel chromatography (ISCO^{®}; 20 g Sepa Flash^{®} Silica Flash Column, Eluent of 0~100% Ethyl acetate/Petroleum ether gradient @ 35 mL/min). Compound methyl 3-[(4-cyano-2-pyridyl)sulfonyl]propanoate (3 g, 11.80 mmol, 49% yield) was obtained as a white solid. MS: m/z = 254.9 (M+1, ESI+).

### Stage 2: Production of (4-cyano-2-pyridyl)sulfinyloxysodium

To a solution of methyl 3-[(4-cyano-2-pyridyl)sulfonyl]propanoate (1.1 g, 4.33 mmol, 1 eq) in methanol (10 mL) was added sodium methoxide (233 mg, 4.33 mmol, 1 eq). The mixture was stirred at 25 °C for 0.5 h. The reaction mixture was concentrated under reduced pressure to remove methanol to give a residue. The crude product of intermediate compound S25 or (4-cyano-2-pyridyl)sulfinyloxysodium (700 mg, crude) was obtained as a yellow solid and used into the next step without further purification. MS: m/z = 167.0 (M+1, ESI+).

### Stage 3: Production of 2-[2-amino-9-[(4-nitrophenyl)methyl]purin-6-yl]pyridine-4-carbonitrile

A mixture of intermediate compound S25 or (4-cyano-2-pyridyl)sulfinyloxy sodium (374 mg, 1.97 mmol, 1.2 eq), intermediate compound S8 or 6-chloro-9-[(4-nitrophenyl)methyl] purin-2-amine (500 mg, 1.64 mmol, 1 eq), tricyclohexylphosphine (92 mg, 328.20 µmol, 0.2 eq), K₂CO₃ (453 mg, 3.28 mmol, 2 eq) and Pd(OAc)₂ (36 mg, 164.10 µmol, 0.1 eq) were taken up into a microwave tube in dioxane (2 mL) .The sealed tube was heated at 130 °C for 10 h under microwave. The reaction mixture was concentrated under reduced pressure to remove dioxane to give a residue. The residue was purified by flash silica gel chromatography (ISCO^{®}; 20 g Sepa Flash^{®} Silica Flash Column, Eluent of 0~100% Ethyl acetate/Petroleum ether gradient @ 40 mL/min). Compound 2-[2-amino-9-[(4-nitrophenyl)methyl]purin-6-yl]pyridine-4-carbonitrile (360 mg, 734.81 µmol, 45% yield, 76% purity) was obtained as a yellow solid. MS: m/z = 373.1 (M+1, ESI+).

### Stage 4: Production of 2-[2-amino-9-[(4-aminophenyl)methyl]purin-6-yl]pyridine-4-carbonitrile

To a solution of intermediate compound S26 or 2-[2-amino-9-[(4-nitrophenyl)methyl]purin-6-yl]pyridine-4-carbonitrile (150 mg, 402.86 µmol, 1 eq) and NH₄Cl (172 mg, 3.22 mmol, 8 eq) in ethanol (9 mL) and water (3 mL) was added iron dust (112 mg, 2.01 mmol, 5 eq). The mixture was stirred at 60 °C for 1 h. The reaction mixture was concentrated under reduced pressure to remove ethanol and water to give a residue. The residue was purified by prep-HPLC (column: Phenomenex luna C18 150*25mm* 10µm;mobile phase: [water(0.225%FA)-ACN];B%: 5%-25%,10min). Compound 116 or 2-[2-amino-9-[(4-aminophenyl)methyl]purin-6-yl]pyridine-4-carbonitrile (22.85 mg, 64.35 µmol, 16% yield, 96.90% purity, 0.2 FA) was obtained as a yellow solid. ¹H NMR (400 MHz, DMSO-d₆) δ = 9.01 (dd, 1H), 8.94 (s, 1H), 8.22 (s, 1H), 7.98 (dd, 1H), 7.04 (d, 2H), 6.74 (s, 2H), 6.51 (d, 2H), 5.13 (s. 4H). HRMS-TOF: 343.1415.

### Example 1.24: Production 2-[2-amino-9-[(4-amino-2-fluorophenyl)methyl]purin-6-yl]pyridine-4-carbonitrile (compound 120)

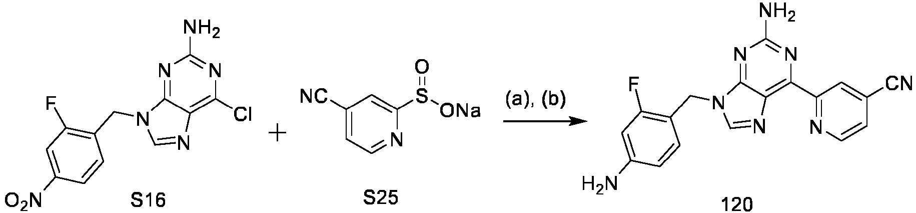

Reagents and condition: (a) Pd(OAc)₂, Cy₃P, K₂CO₃, dioxane, 130°C, 10h, MW; (b) Fe, NH₄Cl, EtOH/H₂O, 60°C, 1h.

### Stage 1: Production of 2-[2-amino-9-[(2-fluoro-4-nitro-phenyl)methyl]purin-6-yl]pyridine-4-carbonitrile.

A mixture of intermediate compound S25 or (4-cyano-2-pyridyl)sulfinyloxy sodium(424 mg, 2.23 mmol, 1.2 eq), intermediate compound S16 or 6-chloro-9-[(2-fluoro-4-nitro-phenyl)methyl]purin-2-amine (600 mg, 1.86 mmol, 1 eq), tricyclohexylphosphine (104 mg, 371.88 µmol, 0.2 eq), K₂CO₃ (513 mg, 3.72 mmol, 2 eq) and palladium acetate (42 mg, 185.94 µmol, 0.1 eq) were taken up into a microwave tube in dioxane (4 mL). The sealed tube was heated at 130 °C for 10 h under microwave. The reaction mixture was concentrated under reduced pressure to remove dioxane to give a residue. The residue was purified by flash silica gel chromatography (ISCO^{®}; 20 g Sepa Flash^{®} Silica Flash Column, Eluent of 0~100% Ethyl acetate/Petroleum ether gradient @ 35 mL/min). Compound 2-[2-amino-9-[(2-fluoro-4-nitro-phenyl)methyl]purin-6-yl]pyridine-4-carbonitrile (300 mg, 768.58 µmol, 41% yield) was obtained as a white solid. MS: m/z = 391.1 (M+1, ESI+)

### Stage 2: Production of 2-[2-amino-9-[(4-amino-2-fluoro-phenyl)methyl]purin-6-yl]pyridine-4-carbonitrile

To a solution of 2-[2-amino-9-[(2-fluoro-4-nitro-phenyl)methyl]purin-6-yl] pyridine-4-carbonitrile (150 mg, 230.57 µmol, 1 eq) and NH₄Cl (98 mg, 1.84 mmol, 8 eq) in ethanol (15 mL) and water (5 mL) was added iron powder (64 mg, 1.15 mmol, 5 eq). The mixture was stirred at 60 °C for 1 h. The reaction mixture was filtered and concentrated under reduced pressure to give a residue. The residue was purified by prep-HPLC (column: Waters Xbridge 150*25mm* 5µm; mobile phase: [water(10mM NH₄HCO₃)-ACN]; B%: 10%-40%,9min) to give the product with 87% purity. Then it was purified by prep-HPLC (column: Waters Xbridge C18 150*50mm* 10µm;mobile phase: [water(10mM NH₄HCO₃)-ACN];B%: 8%-38%,10min) again. Finally the compound 120 or 2-[2-amino-9-[(4-amino-2-fluoro-phenyl)methyl] purin-6-yl]pyridine-4-carbonitrile (9.83 mg, 26.40 µmol, 11% yield, 97.25% purity) was obtained as a white solid. ¹H NMR (400 MHz, DMSO-d₆) δ = 9.02 (d, 1H), 8.89 (s, 1H), 8.13 (s, 1H), 7.99 (d, 1H), 6.98 (t, 1H), 6.75 (s, 2H), 6.33 (d, 2H), 5.48 (s, 2H), 5.17 (s 2H). HRMS-TOF: 361.1319.

### Example 1.25: Production 2-[2-amino-9-[(4-amino-2,6-difluorophenyl)methyl]purin-6-yl]pyridine-4-carbonitrile (compound 124)

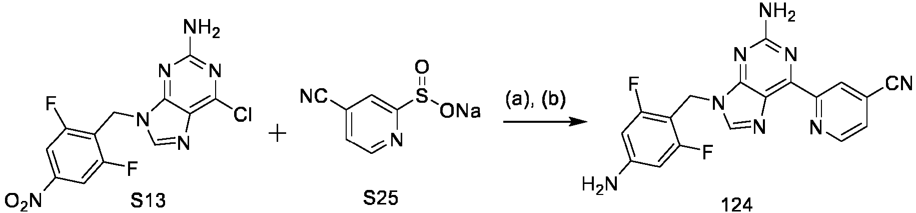

Reagents and condition: (a) Pd(OAc)₂, Cy₃P, K₂CO₃, dioxane, 130°C, 10h, MW; (b) Fe, NH₄Cl, EtOH/H₂O, 60°C, 1h.

### Stage 1: 2-[2-amino-9-[(2, 6-difluoro-4-nitro-phenyl)methyl]purin-6-yl]pyridine-4-carbonitrile

(4-cyano-2-pyridyl)sulfinyloxysodium (419 mg, 2.20 mmol, 1.5 eq), 6-chloro-9-[(2,6-difluoro-4-nitro-phenyl)methyl]purin-2-amine (500 mg, 1.47 mmol, 1 eq), tricyclohexylphosphine (123 mg, 440.31 µmol, 0.3 eq), K₂CO₃ (405 mg, 2.94 mmol, 2 eq) and palladium acetate (50 mg, 223.09 µmol, 0.15 eq) were taken up into a microwave tube in dioxane (10 mL). The sealed tube was heated at 130 °C for 10 h under microwave. The reaction mixture was concentrated under reduced pressure to remove dioxane to give a residue. The residue was purified by prep-HPLC (column: Phenomenex Luna C18 150*25mm*10µm; mobile phase: [water(0.225% FA)-ACN]; B%: 23%-53%, 10 min). Compound 2-[2-amino-9-[(2, 6-difluoro-4-nitro-phenyl)methyl]purin-6-yl]pyridine-4-carbonitrile (120 mg, 293.89 µmol, 20% yield) was obtained as a white solid.
¹H NMR (400 MHz, DMSO-d₆) δ = 9.02 (d, 1H), 8.85 (s, 1H), 8.28 (s, 1H), 8.11 - 8.07 (m, 2H), 7.99 (d, 1H), 6.72 (s, 2H), 5.52 (s, 2H). MS: m/z = 409.1 (M+1, ESI+)

### Stage 2: 2-[2-amino-9-[(4-amino-2,6-difluoro-phenyl)methyl]purin-6-yl]pyridine-4-carbonitrile

To a solution of 2-[2-amino-9-[(2,6-difluoro-4-nitro-phenyl)methyl]purin-6- yl]pyridine-4-carbonitrile (100 mg, 244.91 µmol, 1 eq) and NH₄Cl (104 mg, 1.96 mmol, 8 eq) in ethanol (9 mL) and water (3 mL) was added iron dust (68 mg, 1.22 mmol, 5 eq). The mixture was stirred at 60 °C for 1 h. The reaction mixture was filtered and concentrated under reduced pressure to give a residue. The residue was purified by prep-HPLC (column: Phenomenex luna C18 150*25mm* 10µm; mobile phase: [water (0.225% FA)-ACN]; B%: 13%-43%, 10 min) to afford the product with 90% purity. It was purified by second prep-HPLC (column: Waters Xbridge 150*25mm*5µm; mobile phase: [water (10 mM NH₄HCO₃)-ACN]; B%: 22%-52%, 9 min). Compound 2-[2-amino-9-[(4-amino-2,6-difluoro-phenyl)methyl]purin-6-yl]pyridine-4-carbonitrile (34.57 mg, 89.87 µmol, 37% yield, 97.96% purity) was obtained as a white solid. ¹H NMR (400 MHz, DMSO-d₆) δ = 9.01 (dd, 1H), 8.87 (s, 1H), 8.03 (s, 1H), 7.99 (dd, 1H), 6.72 (s, 2H), 6.22 (d, 2H), 5.88 (s, 2H), 5.17 (s, 2H). HRMS (TOF): 379.1229.

### Example 1.26: Production 2-[2-amino-9-[(4-amino-2,6-difluorophenyl)methyl]purin-6-yl]pyridine-4-carbonitrile (compound 132)

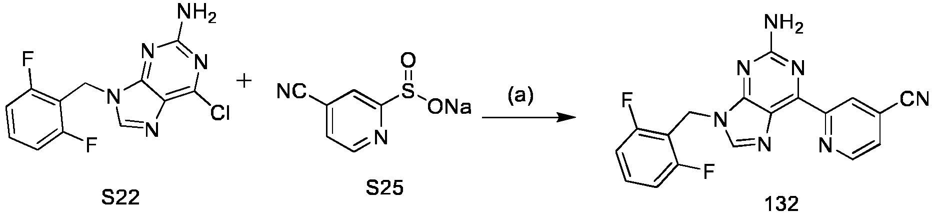

Reagents and condition: (a) Pd(OAc)₂, Cy₃P, K₂CO₃, dioxane, 130°C, 10h, MW.

A mixture of intermediate compound S25 or (4-cyano-2-pyridyl)sulfinyloxy sodium (231 mg, 1.22 mmol, 1.2 eq), intermediate compound S22 or 6-chloro-9-[(2,6-difluorophenyl) methyl]purin-2-amine (300 mg, 1.01 mmol, 1 eq) , palladium acetate (22 mg, 101.46 µmol, 0.1 eq), tricyclohexylphosphine (56 mg, 202.93 µmol, 0.2 eq) and K₂CO₃ (280 mg, 2.03 mmol, 2 eq) in dioxane (6 mL) was degassed and purged with nitrogen for three times, and then the mixture was stirred at 130 °C for 10 h under microwave condition. The mixture was concentrated under reduced pressure to remove dioxane to give a residue. The residue was purified by prep-HPLC (column: Phenomenex Gemini-NX C18 75*30mm*3µm;mobile phase: [water (0.05% ammonia hydroxide v/v)-ACN];B%: 15%-45%,7min). Compound 132 or 2-[2-amino-9-[(2,6-difluorophenyl)methyl]purin-6-yl]pyridine-4-carbonitrile (49.39 mg, 130.41 µmol, 13% yield, 93.37% purity) was obtained as a yellow solid. ¹H NMR (400 MHz, DMSO-d6) δ = 9.01 (dd, 1H), 8.87 (s, 1H), 8.18 (s, 1H), 7.99 (dd, 1H), 7.54 - 7.43 (m, 1H), 7.21 - 7.12 (m, 2H), 6.73 (s, 2H), 5.41 (s, 2H). HRMS-TOF: 364.1121.

### Example 1.27: Production 2-[2-amino-9-[(4-amino-3-methylphenyl)methyl]purin-6-yl]pyridine-4-carbonitrile (compound 128)

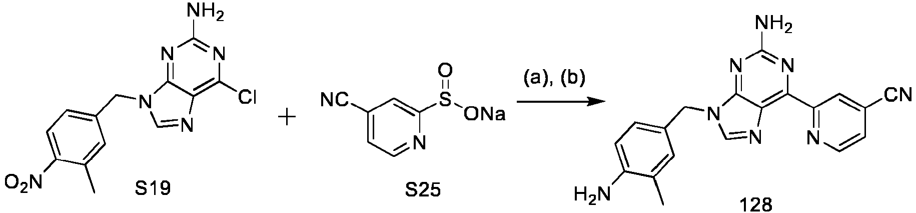

Reagents and condition: (a) Pd(OAc)₂, Cy₃P, K₂CO₃, dioxane, 130°C, 10h, MW; (b) Fe, NH₄Cl, EtOH/H₂O, 60°C, 1h.

### Stage 1: Production of 2-[2-amino-9-[(3-methyl-4-nitro-phenyl)methyl]purin-6-yl]pyridine-4-carbonitrile

A mixture of intermediate compound S25 or sodium 4-cyanopyridine-2-sulfinate (357mg, 1.88 mmol, 1.2 eq), intermediate compound S19 or 6-chloro-9-[(3-methyl-4-nitrophenyl)methyl] purin-2-amine (500 mg, 1.57 mmol, 1 eq), palladium acetate (35 mg, 157.00 µmol,0.1 eq), tricyclohexylphosphine (87 mg, 314.00 µmol, 0.2 eq) and K₂CO₃ (433 mg, 3.14 mmol, 2 eq) in dioxane (2 mL) was degassed and purged with nitrogen for three times, and then the mixture was stirred at 130 °C for 10 h under microwave condition. The reaction mixture was concentrated under reduced pressure to remove dioxane to give a residue. The residue was purified by flash silica gel chromatography (ISCO^{®}; 20 g Sepa Flash^{®} Silica Flash Column, Eluent of 0~10% Ethyl acetate/Petroleum ether gradient @ 30 mL/min). Compound 2-[2-amino-9-[(3-methyl-4-nitro-phenyl)methyl]purin-6-yl]pyridine-4-carbonitrile (300 mg, 535.76 µmol, 34% yield, 69% purity) was obtained as a yellow solid. MS: m/z = 387.3 (M+1, ESI+).

### Stage 2: Production of 2-[2-amino-9-[(4-amino-3-methyl-phenyl)methyl]purin-6-yl]pyridine-4-carbonitrile

To a solution of 2-[2-amino-9-[(3-methyl-4-nitro-phenyl)methyl]purin-6-yl] pyridine-4-carbonitrile (150 mg, 388.23 µmol, 1 eq) and NH₄Cl (7 M, 444 µL, 8 eq) in water (5 mL) and ethanol (15 mL) was added iron powder (108 mg, 1.94 mmol, 5 eq). The mixture was stirred at 60 °C for 1 h. The reaction mixture was concentrated under reduced pressure to remove ethanol and water to give a residue. The residue was purified by prep-HPLC (column: Phenomenex luna C18 150*25mm* 10µm; mobile phase: [water(0.225%FA)-ACN];B%: 8%-28%,9min). Compound 128 or 2-[2-amino-9-[(4-amino-3-methyl-phenyl)methyl]purin-6-yl]pyridine-4-carbonitrile (21.82 mg, 58.15 µmol, 15% yield, 96.70% purity) was obtained as a yellow solid. ¹H NMR (400 MHz, DMSO-d₆) δ = 9.02 (d, 1H), 8.90 (s, 1H), 8.23 (s, 1H), 7.99 (d, 1H), 6.95 (s, 1H), 6.93 - 6.90 (m, 1H), 6.75 (s, 2H), 6.55 (d, 1H), 5.12 (s, 2H), 4.87 (s, 2H), 2.06 (s, 3H). HRMS-TOF: 357.1569.

### Example 1.28: Production 2-[2-amino-9-[[4-amino-3-(trifluoromethyl)phenyl]methyl]purin-6-yl]pyridine-4-carbonitrile (compound 135)

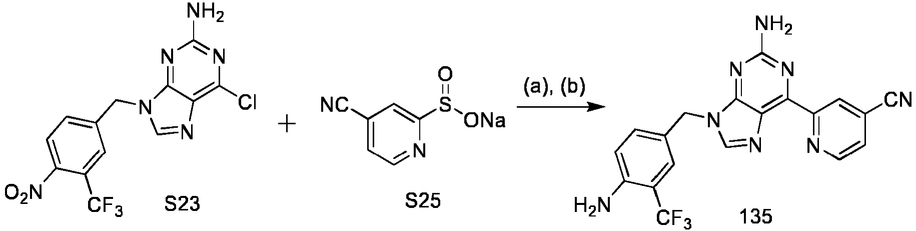

Reagents and condition: (a) Pd(OAc)₂, Cy₃P, K₂CO₃, dioxane, 130°C, 10h, MW; (b) Fe, NH₄Cl, EtOH/H₂O, 60°C, 1h.

### Stage 1: Production of 2-[2-amino-9-[[4-nitro-3-(trifluoromethyl)phenyl]methyl]purin-6-yl]pyridine-4-carbonitrile.

A mixture of intermediate compound S25 or sodium 4-cyanopyridine-2-sulfinate (230 mg, 1.21 mmol, 1.5 eq), intermediate compound S23 or 6-chloro-9-[[4-nitro-3-(trifluoromethyl)phenyl]methyl]purin-2-amine (300 mg, 804.96 µmol, 1 eq), potassium carbonate (222 mg, 1.61 mmol, 2 eq) and Cy₃P-Pd-G3 (52 mg, 80.50 µmol, 0.1 eq) in dioxane (5 mL) was degassed and purged with nitrogen for three times, and then the mixture was stirred at 120 °C for 16 h under nitrogen atmosphere. The reaction mixture was concentrated under reduced pressure to remove dioxane to give a residue. The residue was purified by prep-HPLC (column: Shim-pack C18 150*25*10µm;mobile phase: [water(0.225%FA)-ACN];B%: 34%-54%, 10 min). Compound 135 or 2-[2-amino-9-[[4-nitro-3-(trifluoromethyl)phenyl]methyl]purin-6-yl]pyridine-4-carbonitrile (300 mg, 647.23 µmol, 80% yield, 95% purity) was obtained as a white solid. ¹H NMR (400 MHz, DMSO-d₆) δ = 9.04 (dd, *J =* 0.7, 4.9 Hz, 1H), 8.90 (s, 1H), 8.37 (s, 1H), 8.13 (s, 1H), 8.08 (s, 1H), 8.01 (dd, *J =* 1.5*,* 5.0 Hz, 1H), 7.71 (dd, *J =* 1.2, 8.4 Hz, 1H), 6.80 (s, 2H), 5.57 (s, 2H).

### Stage 2: Production of 2-[2-amino-9-[[4-amino-3-(trifluoromethyl)phenyl]methyl]purin-6-yl]pyridine-4-carbonitrile.

To a solution of 2-[2-amino-9-[[4-nitro-3-(trifluoromethyl)phenyl]methyl]purin-6-yl]pyridine-4-carbonitrile (300 mg, 681.29 µmol, 1 eq) in ethanol (15 mL) and water (5 mL) were added iron dust (190 mg, 3.41 mmol, 5 eq) and NH₄Cl (292 mg, 5.45 mmol, 8 eq). The mixture was stirred at 60 °C for 1 h. The reaction mixture was filtered and concentrated under reduced pressure to give a residue. The residue was purified by prep-HPLC (column: Shim-pack C18 150*25*10µm;mobile phase: [water(0.225% FA)-ACN];B%: 17%-47%, 10 min). Compound 135 or 2-[2-amino-9-[[4-amino-3-(trifluoromethyl)phenyl]methyl]purin-6-yl]pyridine-4-carbonitrile (40.01 mg, 96.51 µmol, 14% yield, 98.98% purity) was obtained as a yellow solid. ¹H NMR (400 MHz, DMSO-d₆) δ = 9.02 (d, 1H), 8.89 (s, 1H), 8.29 (s, 1H), 7.99 (dd, 1H), 7.40 (d, 1H), 7.29 (dd, 1H), 6.82-6.74 (m, 3H), 5.64 (s, 2H), 5.20 (s, 2H) HRMS-TOF: 411.1281.

### Example 1.29: Production 5-[2-amino-9-[(4-aminophenyl)methyl]purin-6-yl]pyridine-3-carbonitrile (compound 117)

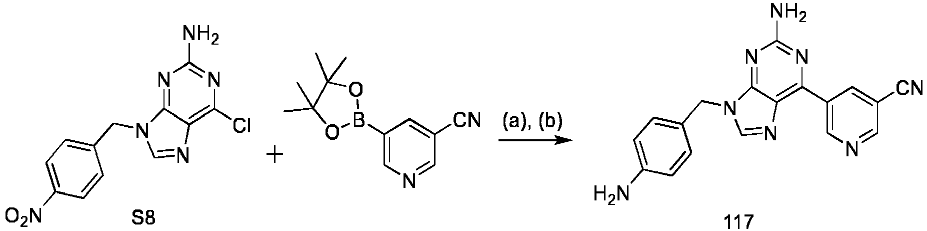

Reagents and condition: (a) Pd(dppf)Cl₂, K₂CO₃, dioxane/H₂O, 110°C, 15h; (b) Fe, NH₄Cl, EtOH/H₂O, 80°C, 1h.

### Stage 1: Production of 5-[2-amino-9-[(4-nitrophenyl)methyl]purin-6-yl]pyridine-3-carbonitrile.

A mixture of intermediate compound S8 or 6-chloro-9-[(4-nitrophenyl)methyl]purin-2-amine (200 mg, 656.40 µmol, 1 eq), 5-(4,4,5,5-tetramethyl-1,3,2-dioxaborolan-2-yl)pyridine-3-carbonitrile (181 mg, 787.68 µmol, 1.2 eq), Pd(dppf)Cl₂ (48 mg, 65.64 µmol, 0.1 eq) and K₂CO₃ (181 mg, 1.31 mmol, 2 eq) in dioxane (10 mL) and water (0.5 mL) was de-gassed and then heated to 110 °C for 15 h under nitrogen. To the mixture was added water (130 mL) and extracted with ethyl acetate (130 mL x 2). Combined the organic phase was dried over anhydrous sodium sulfate, filtered and concentrated in vacuum to give a residue. The residue was purified by prep-TLC (SiO₂, PE:EA=1:1). Compound 5-[2-amino-9-[(4-nitrophenyl)methyl]purin-6-yl]pyridine-3-carbonitrile (150 mg, 330.34 µmol, 50% yield, 82% purity) was obtained as yellow solid. MS: m/z = 373 (M+1, ESI+).

### Stage 2: Production of 5-[2-amino-9-[(4-aminophenyl)methyl]purin-6-yl]pyridine-3-carbonitrile

To the solution of 5-[2-amino-9-[(4-nitrophenyl)methyl]purin-6-yl]pyridine- 3-carbonitrile (150 mg, 330.34 µmol, 1 eq) in ethanol (9 mL) and water (3 mL) were added iron powder (92 mg, 1.65 mmol, 5 eq) and NH₄Cl (141 mg, 2.64 mmol, 8 eq). Then the mixture was stirred at 80 °C for 1 h. The reaction mixture was filtered and the filtrate was concentrated in vacuum. Then to the mixture was added water (50 mL) and extracted with ethyl acetate (80 mL x 3). Combined the organic phases to dry over anhydrous sodium sulfate, filter and concentrate in vacuum to give a residue. The residue was purified by prep-HPLC (column: Waters Xbridge 150*25mm* 5µm; mobile phase: [water (0.05% ammonia hydroxide v/v)-ACN]; B%: 14%-44%,10min). Compound 117 or 5-[2-amino-9-[(4-aminophenyl)methyl]purin-6-yl] pyridine-3-carbonitrile (20.59 mg, 58.88 µmol, 18% yield, 98.45% purity) was obtained as a yellow solid. ¹H NMR (400 MHz, DMSO-d₆) δ = 10.01 (d, *J=* 1.8 Hz, 1H), 9.30 (s, 1H), 9.16 (d, *J=* 1.8 Hz, 1H), 8.29 (s, 1H), 7.05 (br d, *J=* 8.3 Hz, 2H), 6.77 (s, 2H), 6.52 (d, *J=* 8.3 Hz, 2H), 5.14 (s, 2H), 5.11 (s, 2H). HRMS-TOF: 343.1410.

### Example 1.30: Production 5-[2-amino-9-[(4-amino-2-fluorophenyl)methyl]purin-6-yl]pyridine-3-carbonitrile (compound 121)

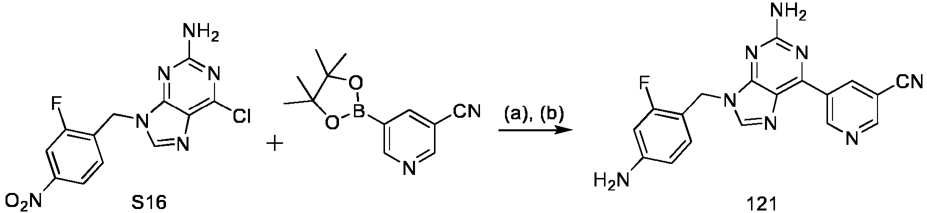

Reagents and condition: (a) Pd(dppf)Cl₂, K₂CO₃, dioxane/H₂O, 110°C, 15h; (b) Fe, NH₄Cl, EtOH/H₂O, 80°C, 1h.

### Stage 1: Production of 5-[2-amino-9-[(2-fluoro-4-nitro-phenyl)methyl]purin-6-yl]pyridine-3-carbonitrile.

A mixture of intermediate compound S16 or 6-chloro-9-[(2-fluoro-4-nitrophenyl)methyl]purin-2-amine (300 mg, 929.71 µmol, 1 eq), 5-(4,4,5,5-tetramethyl-1,3,2-dioxaborolan-2-yl)pyridine-3-carbonitrile (320 mg, 1.39 mmol, 1.5 eq), K₂CO₃ (257 mg, 1.86 mmol, 2 eq) and Pd(dppf)Cl₂ (68 mg, 92.97 µmol, 0.1 eq) in dioxane (10 mL) and water (0.5 mL) was de-gassed and then heated to 110 °C for 15 h under nitrogen. To the mixture was added water (30 mL) and then extracted with ethyl acetate (30 mL x 3). Combined the organic phase to wash with brines (50 mL x 2), dry over anhydrous Na₂SO₄, filter and concentrate in vacuum to give a residue. The residue was purified by flash silica gel chromatography (20 g Sepa Flash^{®} Silica Flash Column, Eluent of 0~80% Ethyl acetate/Petroleum ether gradient @ 35 mL/min). Compound 121 or 5-[2-amino-9-[(2-fluoro-4-nitro-phenyl)methyl]purin-6-yl]pyridine-3-carbonitrile (200 mg, 502.14 µmol, 54% yield, 98% purity) was obtained as a yellow solid. MS: m/z = 391.2 (M+1, ESI+).

### Stage 2: Production of 5-[2-amino-9-[(4-amino-2-fluoro-phenyl)methyl]purin-6-yl]pyridine-3-carbonitrile.

To the solution of 5-[2-amino-9-[(2-fluoro-4-nitro-phenyl)methyl]purin-6-yl]pyridine-3-carbonitrile (200 mg, 512.39 µmol, 1 eq) and NH₄Cl (220 mg, 4.10 mmol, 8 eq) in ethanol (9 mL) and water (3 mL) was added iron powder (143 mg, 2.56 mmol, 5 eq). Then the mixture was stirred at 80 °C for 1 h. The mixture was concentrated in vacuum to give a residue. To the residue was added water (30 mL) and extracted with ethyl acetate (30 mL x 3). Combined the organic phase and dried over anhydrous Na₂SO₄, filtered and concentrated in vacuum to give a residue. The crude product was triturated with methanol (10 mL) and THF (1 mL) at 60 °C for 30 min. Compound 121 or 5-[2-amino-9-[(4-amino-2-fluorophenyl)methyl]purin-6-yl] pyridine-3-carbonitrile (60 mg, 166.51 µmol, 32% yield, 95.08% purity) was obtained as yellow solid. ¹H NMR (400 MHz, DMSO-d₆) δ = 10.01 (d, *J= 2.0* Hz, 1H), 9.30 (t, *J*= 2.1 Hz, 1H), 9.17 (d, *J =* 2.0 Hz, 1H), 8.20 (s, 1H), 6.99 (t, *J =* 8.4 Hz, 1H), 6.79 (s, 2H), 6.44 - 6.28 (m, 2H), 5.50 (s, 2H), 5.18 (s, 2H). HRMS-TOF: 361.1319.

### Example 1.31: Production 5-[2-amino-9-[(4-amino-2,6-difluorophenyl)methyl]purin-6-yl]pyridine-3-carbonitrile (compound 125)

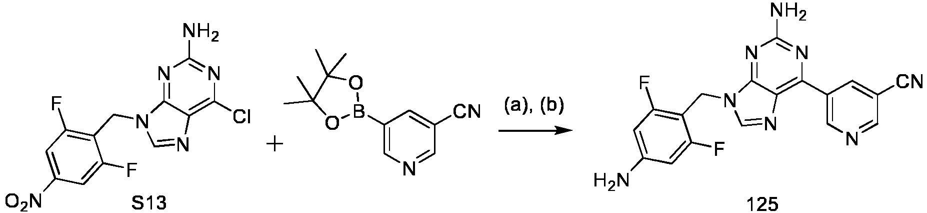

Reagents and condition: (a) Pd(dppf)Cl₂, K₂CO₃, dioxane/H₂O, 110°C, 2h; (b) Fe, NH₄Cl, EtOH/H₂O, 80°C, 1h.

### Stage 1: Production of 5-[2-amino-9-[(2,6-difluoro-4-nitro-phenyl)methyl]purin-6-yl]pyridine-3-carbonitrile.

A mixture of intermediate compound S13 or 6-chloro-9-[(2,6-difluoro-4-nitrophenyl)methyl]purin-2-amine (200 mg, 587.07 µmol, 1 eq), 5-(4,4,5,5-tetramethyl-1,3,2-dioxaborolan-2-yl)pyridine-3-carbonitrile (270 mg, 1.17 mmol, 2 eq), Pd(dppf)Cl₂ (43 mg, 58.71 µmol, 0.1 eq) and K₂CO₃ (162 mg, 1.17 mmol, 2 eq) were taken up into a microwave tube in dioxane (10 mL) and water (2 mL). The sealed tube was heated at 110 °C for 2 h under microwave condition. After that the mixture was added water (130 mL) and extracted with ethyl acetate (130 mL x 2). Combined the organic phases to dry over anhydrous Na₂SO₄, filter and concentrate in vacuum to give a residue. The residue was purified by flash silica gel chromatography (ISCO^{®}; 20 g Sepa Flash^{®} Silica Flash Column, Eluent of 0~100% Ethyl acetate/Petroleum ether gradient @ 40 mL/min). Compound 125 or 5-[2-amino-9-[(2,6-difluoro-4-nitro-phenyl)methyl]purin-6-yl]pyridine-3-carbonitrile (200 mg, 426.14 µmol, 72% yield, 87% purity) was obtained as a yellow solid. MS: m/z = 408.9 (M+1, ESI+).

### Stage 2: Production of 5-[2-amino-9-[(4-amino-2,6-difluoro-phenyl)methyl]purin-6-yl]pyridine-3-carbonitrile.

To the solution of 5-[2-amino-9-[(2,6-difluoro-4-nitro-phenyl)methyl] purin-6-yl]pyridine-3-carbonitrile (200 mg, 489.81 µmol, 1 eq) in ethanol (8 mL) and water (2 mL) were added iron powder (137 mg, 2.45 mmol, 5 eq) and NH₄Cl (210 mg, 3.92 mmol, 8 eq). Then the mixture was stirred at 80 °C for 1 h. After that the reaction mixture was filtered and the filtrate was concentrated in vacuum, then the residue was added water (50 mL) and extracted with ethyl acetate (50 mL x 3). Combined the organic phases to dry over anhydrous Na₂SO₄, filter and concentrate in vacuum to give a residue. The residue was purified by prep-HPLC (column: Waters Vinridis Silica 2-EP OBD 50*150mm*5µm;mobile phase: [Heptane-EtOH (0.1% NH₃•H₂O)];B%: 5%-45%,10min). Compound 125 or 5-[2-amino-9-[(4-amino-2,6-difluoro-phenyl)methyl]purin-6-yl]pyridine-3-carbonitrile (16.22 mg, 40.31 µmol, 8% yield, 96.64% purity) was obtained as a yellow solid. ¹H NMR (400 MHz, DMSO-d₆) δ = 9.99 (d, *J=* 2.0 Hz, 1H), 9.28 (t, *J=* 2.1 Hz, 1H), 9.15 (d, *J =* 2.1 Hz, 1H), 8.09 (s, 1H), 6.74 (s, 2H), 6.22 (d, *J =* 10.4 Hz, 2H), 5.88 (s, 2H), 5.17 (s, 2H). HRMS-TOF: 379.1229.

### Example 1.32: Production 5-[2-amino-9-[(2,6-difluorophenyl)methyl]purin-6-yl]pyridine-3-carbonitrile (compound 133)

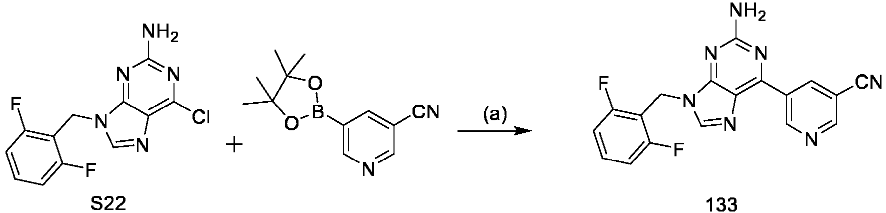

Reagents and condition: (a) Pd(dppf)Cl₂, K₂CO₃, dioxane/H₂O, 110°C, 15h.

A mixture of intermediate compound S22 or 6-chloro-9-[(2,6-difluorophenyl)methyl]purin-2-amine (150 mg, 507.31 µmol, 1 eq), 5-(4,4,5,5-tetramethyl-1,3,2-dioxaborolan-2-yl)pyridine-3-carbonitrile (140 mg, 608.78 µmol, 1.2 eq), Pd(dppf)Cl₂ (37 mg, 50.73 µmol, 0.1 eq) and K₂CO₃ (140 mg, 1.01 mmol, 2 eq) in dioxane (10 mL) and water (1 mL) was de-gassed and then heated to 110 °C for 15 h under nitrogen. The mixture was concentrated in vacuum to give a residue. The residue was purified by prep-HPLC (column: Xtimate C18 150*40mm*10µm; mobile phase: [water (0.05% ammonia hydroxide v/v)-ACN];B%: 30%-60%,10min). Compound 133 or 5-[2-amino-9-[(2,6-difluorophenyl)methyl]purin-6-yl]pyridine-3-carbonitrile (60.03 mg, 161.42 µmol, 32% yield, 96.75% purity) was obtained as a yellow solid. ¹H NMR (400 MHz, DMSO-d₆) δ = 9.98 (d, *J=* 1.7 Hz, 1H), 9.27 (s, 1H), 9.16 (d, *J =* 1.7 Hz, 1H), 8.24 (s, 1H), 7.55 - 7.42 (m, 1H), 7.16 (t, *J =* 8.1 Hz, 2H), 6.74 (s, 2H), 5.42 (s, 2H). HRMS-TOF: 364.1121.

### Example 1.33: Production 5-[2-amino-9-[(4-amino-3-methylphenyl)methyl]purin-6-yl]pyridine-3-carbonitrile (compound 129)

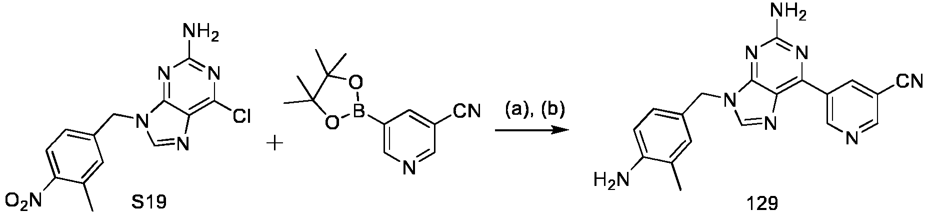

Reagents and condition: (a) Pd(dppf)Cl₂, K₂CO₃, dioxane/H₂O, 110°C, 15h. (b) Fe, NH₄Cl, EtOH/H₂O, 80°C, 1 h.

### Stage 1: Production of 5-[2-amino-9-[(3-methyl-4-nitro-phenyl)methyl]purin-6-yl]pyridine-3-carbonitrile

A mixture of intermediate compound S19 or 6-chloro-9-[(3-methyl-4-nitrophenyl)methyl]purin-2-amine (300 mg, 941.27 µmol, 1 eq), 5-(4,4,5,5-tetramethyl-1,3,2-dioxaborolan-2-yl)pyridine-3-carbonitrile (325 mg, 1.41 mmol, 1.5 eq), Pd(dppf)Cl₂ (69 mg, 94.13 µmol, 0.1 eq) and K₂CO₃ (260 mg, 1.88 mmol, 2 eq) in dioxane (10 mL) and water (0.5 mL) was de-gassed and then heated to 110 °C for 15 h under nitrogen. After that the mixture was added water (80 mL) and extracted with ethyl acetate (80 mL x 3). Combined the organic phase to dry over anhydrous Na₂SO₄, filter and concentrate in vacuum to give a residue. The residue was purified by flash silica gel chromatography (12 g Sepa Flash^{®} Silica Flash Column, Eluent of 0~80% Ethyl acetate/Petroleum ether gradient @ 30 mL/min). Compound 5-[2-amino-9-[(3-methyl-4-nitro-phenyl)methyl]purin-6-yl]pyridine-3-carbonitrile (300 mg, 750.84 µmol, 79% yield, 96.7% purity) was obtained as a yellow solid. ¹H NMR (400 MHz, DMSO-d₆) δ = 10.02 (d, J = 2.0 Hz, 1H), 9.31 (t, *J =* 2.1 Hz, 1H), 9.17 (d, *J =* 2.0 Hz, 1H), 8.41 (s, 1H), 7.97 (d, *J=* 8.4 Hz, 1H), 7.41 (s, 1H), 7.28 (dd, *J =* 1.5, 8.4 Hz, 1H), 6.80 (s, 2H), 5.45 (s, 2H), 2.49 (br s, 3H). MS: m/z = 387 (M+1, ESI+).

### Stage 2: Production of5-[2-amino-9-[(4-amino-3-methyl-phenyl)methyl]purin-6-yl]pyridine-3 -carbonitrile

To the solution of 5-[2-amino-9-[(3-methyl-4-nitro-phenyl)methyl]purin-6-yl]pyridine-3-carbonitrile (300 mg, 722.1 µmol, 1 eq) in ethanol (9 mL) and water (3 mL) were added iron powder (202 mg, 3.61 mmol, 5 eq) and NH₄Cl (310 mg, 5.78 mmol, 8 eq). Then the mixture was stirred at 80 °C for 1 h. The reaction mixture was filtered and the filtrate was concentrated in vacuum. Then the residue was added water (50 mL) and extracted with ethyl acetate (50 mL x 3). The combined organic phase was dried over anhydrous Na₂SO₄, filtered and concentrated in vacuum to give a residue. The crude product was triturated with methanol (8 mL) at 25 °C for 30 min, then filtered and the solid was dried under reduced pressure. Compound 129 or 5-[2-amino-9-[(4-amino-3-methylphenyl)methyl]purin-6-yl]pyridine-3-carbonitrile (36.5 mg, 99.14 µmol, 13% yield, 96.26% purity) was obtained as a yellow solid. ¹H NMR (400 MHz, DMSO-d₆) δ = 10.01 (d, *J* = 2.0 Hz, 1H), 9.29 (t, *J =* 2.0 Hz, 1H), 9.15 (d, *J =* 2.0 Hz, 1H), 8.28 (s, 1H), 6.96 (s, 1H), 6.92 (dd, *J* = 1.7, 8.1 Hz, 1H), 6.76 (s, 2H), 6.56 (d, *J =* 8.1 Hz, 1H), 5.12 (s, 2H), 2.01 (s, 3H). HRMS-TOF: 357.1555.

### Example 1.34: Production 4-[2-amino-9-[(4-aminophenyl)methyl]purin-6-yl]pyridine-2-carbonitrile (compound 118)

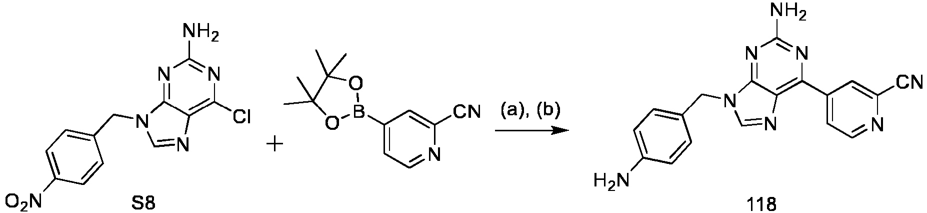

Reagents and condition: (a) Pd(dppf)Cl₂, K₂CO₃, dioxane/H₂O, 110°C, 2h, MW. (b) Fe, NH₄Cl, EtOH/H₂O, 80°C, 1 h.

### Stage 1: Production of 4-[2-amino-9-[(4-nitrophenyl)methyl]purin-6-yl] pyridine-2-carbonitrile.

A mixture of intermediate compound S8 or 6-chloro-9-[(4-nitrophenyl)methyl]purin-2-amine (200 mg, 656.40 µmol, 1 eq), 4-(4,4,5,5-tetramethyl-1,3,2-dioxaborolan-2-yl)pyridine-2-carbonitrile (755 mg, 3.28 mmol, 5 eq), K₂CO₃ (181 mg, 1.31 mmol, 2 eq) and Pd(dppf)Cl₂ (48 mg, 65.64 µmol, 0.1 eq) were taken up into a microwave tube in dioxane (10 mL) and water (2 mL). The sealed tube was heated at 110 °C for 2 h under microwave condition. After that, to the mixture was added water (130 mL) and extracted with ethyl acetate (130 mL x 3). The combined organic phase was dried over anhydrous sodium sulfate, filtered and concentrated in vacuum to give a residue. The residue was purified by flash silica gel chromatography (12 g Sepa Flash^{®} Silica Flash Column, Eluent of 0~80% Ethyl acetate/Petroleum ether gradient @ 35 mL/min). Compound 4-[2-amino-9-[(4-nitrophenyl)methyl]purin-6-yl]pyridine-2-carbonitrile (200 mg, 504.92 µmol, 77% yield, 94% purity) was obtained as yellow solid. MS: m/z = 373.1 (M+1, ESI+).

### Stage 2: Production of 4-[2-amino-9-[(4-aminophenyl)methyl]purin-6-yl]pyridine-2-carbonitrile

To the solution of 4-[2-amino-9-[(4-nitrophenyl)methyl]purin-6-yl]pyridine-2-carbonitrile (200 mg, 537.14 µmol, 1 eq) and NH₄Cl (230 mg, 4.30 mmol, 8 eq) in ethanol (8 mL) and water (2 mL) was added iron powder (150 mg, 2.69 mmol, 5 eq). Then the mixture was stirred at 80 °C for 1 h. The reaction mixture was filtered and the filtrate was concentrated in vacuum, then the residue was added water (50 mL) and extracted with ethyl acetate (50 mL x 3). Combined the organic phases to concentrate in vacuum to give a residue. The residue was purified by prep-HPLC (column: Waters Vinridis Silica 2-EP OBD 50*150mm*5µm;mobile phase: [Heptane-EtOH(0.1%NH3H2O)];B%: 5%-45%,10min). Compound 118 or 4-[2-amino-9-[(4-aminophenyl)methyl]purin-6-yl] pyridine-2-carbonitrile (38.42 mg, 107.37 µmol, 20% yield, 92.28% purity) was obtained as a yellow solid. ¹H NMR (400 MHz, DMSO-d₆) δ = 9.05 (s, 1H), 8.98 (d, *J =* 5.3 Hz, 1H), 8.89 (dd, *J =* 1.4, 5.2 Hz, 1H), 8.33 (s, 1H), 7.05 (br d, *J =* 8.3 Hz, 2H), 6.82 (s, 2H), 6.52 (d, *J =* 8.4 Hz, 2H), 5.14 (s, 2H), 5.11 (s, 2H). HRMS-TOF: 343.1413.

### Example 1.35: Production 4-[2-amino-9-[(4-amino-2-fluorophenyl)methyl]purin-6-yl]pyridine-2-carbonitrile (compound 122)

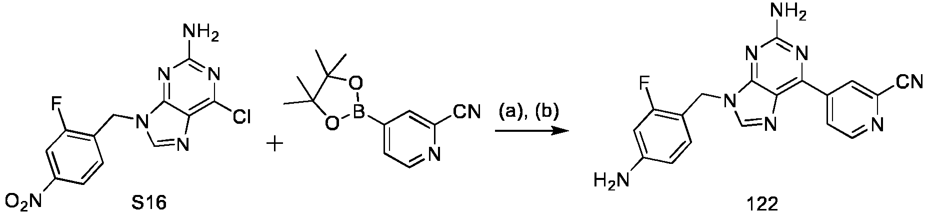

Reagents and condition: (a) Pd(dppf)Cl₂, K₂CO₃, dioxane/H₂O, 110°C, 2h, MW. (b) Fe, NH₄Cl, THF/H₂O, 80°C, 1 h.

### Stage 1: Production of 4-[2-amino-9-[(2-fluoro-4-nitro-phenyl)methyl]purin-6-yl] pyridine-2-carbonitrile

A mixture of intermediate compound S16 or 6-chloro-9-[(2-fluoro-4-nitrophenyl)methyl]purin-2-amine (300 mg, 929.71 µmol, 1 eq), 4-(4,4,5,5-tetramethyl-1,3,2-dioxaborolan-2-yl)pyridine-2-carbonitrile (428 mg, 1.86 mmol, 2 eq), K₂CO₃ (257 mg, 1.86 mmol, 2 eq) and Pd(dppf)Cl₂ (68 mg, 92.97 µmol, 0.1 eq) were taken up into a microwave tube in dioxane (8 mL) and water (2 mL). The sealed tube was heated at 110 °C for 2 h under microwave condition. The mixture was concentrated in vacuum to give a residue. The residue was purified by flash silica gel chromatography (12 g Sepa Flash^{®} Silica Flash Column, Eluent of 0~50% Ethyl acetate/Petroleum ether gradient @ 60 mL/min). Compound 4-[2-amino-9-[(2-fluoro-4-nitro-phenyl)methyl]purin-6-yl] pyridine-2-carbonitrile (200 mg, 478.83 µmol, 52% yield, 93.45% purity) was obtained as a yellow solid. ¹H NMR (400 MHz, DMSO-d₆) δ = 9.04 (s, 1H), 8.99 (d, *J =* 4.6 Hz, 1H), 8.88 (dd, *J= 1.5,* 5.1 Hz, 1H), 8.41 (s, 1H), 8.18 (dd, *J=* 2.1, 9.9 Hz, 1H), 8.04 (dd, *J =* 1.9, 8.6 Hz, 1H), 7.35 (t, *J =* 8.0 Hz, 1H), 6.86 (s, 2H), 5.56 (s, 2H). MS: m/z = 391.1 (M+1, ESI+).

### Stage 2: Production of 4-[2-amino-9-[(4-amino-2-fluoro-phenyl)methyl]purin-6-yl]pyridine-2-carbonitrile

To the solution of 4-[2-amino-9-[(2-fluoro-4-nitro-phenyl)methyl]purin-6-yl] pyridine-2-carbonitrile (200 mg, 512.39 µmol, 1 eq) in ethanol (9 mL) and water (3 mL) were added iron powder (143 mg, 2.56 mmol, 5 eq) and NH₄Cl (219 mg, 4.10 mmol, 8 eq). Then the mixture was stirred at 80 °C for 1 h. The reaction mixture was filtered and the filtrate was concentrated in vacuum. To the residue was added water (80 mL) and extracted with ethyl acetate (80 mL x 3). Combined the organic phase was washed with brines (100 mL x 2), dried over anhydrous Na₂SO₄, filtered and concentrated in vacuum to give a residue. The residue was purified by prep-HPLC (column: Phenomenex Gemini NX-C18(75*30mm*3µm); mobile phase:[water(10mM NH₄HCO₃)-ACN];B%: 18%-48%,8min). Compound 122 or 4-[2-amino-9-[(4-amino-2-fluoro-phenyl)methyl]purin-6-yl]pyridine-2-carbonitrile (20.14 mg, 54.73 µmol, 11% yield, 98.80% purity) was obtained as a yellow solid. ¹H NMR (400 MHz, DMSO-d₆) δ = 9.04 (s, 1H), 8.97 (d, *J=* 5.1 Hz, 1H), 8.87 (dd, *J* = 1.5, 5.1 Hz, 1H), 8.22 (s, 1H), 6.99 (t, *J =* 8.5 Hz, 1H), 6.81 (s, 2H), 6.34 (s, 1H), 6.32 (br d, *J=* 1.2 Hz, 1H), 5.48 (s, 2H), 5.18 (s, 2H). HRMS-TOF: 361.1317.

### Example 1.36: Production 4-[2-amino-9-[(4-amino-2,6-difluorophenyl)methyl]purin-6-yl]pyridine-2-carbonitrile (compound 126)

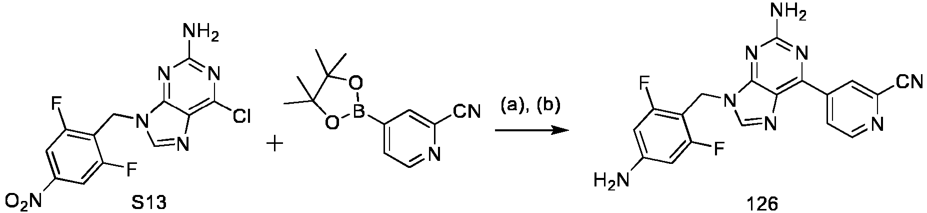

Reagents and condition: (a) Pd(dppf)Cl₂, K₂CO₃, dioxane/H₂O, 110°C, 2h. (b) Fe, NH₄Cl, THF/H₂O, 80°C, 0.5 h.

### Stage 1: Production of 4-[2-amino-9-[(2,6-difluoro-4-nitro-phenyl)methyl]purin-6-yl]pyridine-2-carbonitrile

To a solution of intermediate compound S13 or 6-chloro-9-[(2,6-difluoro-4-nitrophenyl)methyl]purin-2-amine (160 mg, 469.66 µmol, 1 eq), 4-(4,4,5,5-tetramethyl-1,3,2-dioxaborolan-2-yl) pyridine-2-carbonitrile (540 mg, 2.35 mmol, 5 eq) in dioxane (14 mL) and water (1.4 mL) were added K₂CO₃ (130 mg, 939.32 µmol, 2 eq) and Pd(dppf)Cl₂ (34 mg, 46.97 µmol, 0.1 eq). The mixture was stirred at 110 °C for 2 h. The mixture was added water (30 mL) and extracted by ethyl acetate (30 mL x 3), the combined organic phase was dried over anhydrous Na₂SO₄, filtered and concentrated in vacuum to give a residue. The residue was purified by flash silica gel chromatography (ISCO^{®}; 12 g Sepa Flash^{®} Silica Flash Column, Eluent of 0~42% Ethyl acetate/Petroleum ether gradient @ 40 mL/min). Compound 4-[2-amino-9-[(2,6-difluoro-4-nitro-phenyl)methyl]purin-6-yl]pyridine-2-carbonitrile (75 mg, 174.50 µmol, 37% yield, 95% purity) was obtained as a yellow solid. ¹H NMR (400 MHz, DMSO-d₆) δ = 9.02 - 9.00 (m, 1H), 8.97 (dd, *J =* 0.6*,* 5.1 Hz, 1H), 8.85 (dd, *J=* 1.6, 5.1 Hz, 1H), 8.38 (s, 1H), 8.10 (d, *J =* 7.5 Hz, 2H), 6.79 (s, 2H), 5.53 (s, 2H). MS: m/z = 409.1 (M+1, ESI+)

### Stage 2: Production of 4-[2-amino-9-[(4-amino-2,6-difluoro-phenyl)methyl]purin-6-yl]pyridine-2-carbonitrile

To a solution of 4-[2-amino-9-[(2,6-difluoro-4-nitro-phenyl)methyl]purin- 6-yl]pyridine-2-carbonitrile (92 mg, 225.31 µmol, 1 eq) in ethanol (8 mL) and water (2 mL) were added iron dust (63 mg, 1.13 mmol, 5 eq) and NH₄Cl (96 mg, 1.80 mmol, 8 eq). The mixture was stirred at 80 °C for 0.5 h. The mixture was filtered and the filtrate was concentrated in vacuum to give a residue. The residue was added water (40 mL) and extracted with ethyl acetate (30 mL x 3). The combined organic layers were concentrated under reduced pressure to give a residue. The residue was purified by prep-HPLC (column: Welch Ultimate XB-CN 250*25*10µm;mobile phase: [Heptane-EtOH (0.1%NH₃•H₂O)];B%: 25%-65%,15min). Compound 126 or 4-[2-amino-9-[(4-amino-2,6-difluorophenyl)methyl]purin-6-yl]pyridine-2-carbonitrile (38 mg, 99.43 µmol, 44% yield, 97.67% purity) was obtained as a yellow solid. ¹H NMR (400 MHz, DMSO-d₆) δ = 9.03 (dd, *J= 0.8,* 1.6 Hz, 1H), 8.97 (dd, *J=* 0.9, 5.1 Hz, 1H), 8.86 (dd, *J =* 1.6, 5.1 Hz, 1H), 8.13 (s, 1H), 6.79 (s, 2H), 6.21 (d, *J =* 10.5 Hz, 2H), 5.88 (s, 2H), 5.18 (s, 2H). HRMS-TOF: 379.1231.

### Example 1.37: Production 4-[2-amino-9-[(2,6- difluorophenyl)methyl]purin-6-yl]pyridine-2-carbonitrile (compound 134)

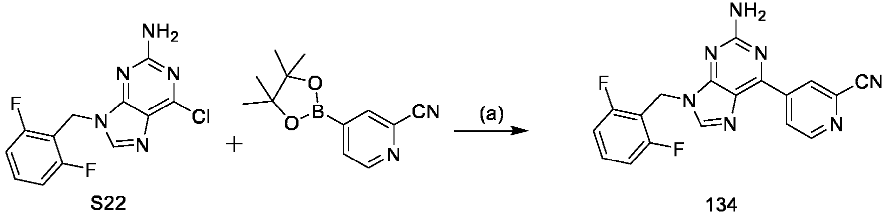

Reagents and condition: (a) Pd(dppf)Cl₂, K₂CO₃, dioxane/H₂O, 110°C, 15h.

A mixture of intermediate compound S22 or 6-chloro-9-[(2,6-difluorophenyl)methyl]purin-2-amine (100 mg, 338.21 µmol, 1 eq), 4-(4,4,5,5-tetramethyl-1,3,2-dioxaborolan-2-yl)pyridine-2-carbonitrile (117 mg, 507.31 µmol, 1.5 eq), K₂CO₃ (94 mg, 676.42 µmol, 2 eq) and Pd(dppf)Cl₂ (25 mg, 33.82 µmol, 0.1 eq) in dioxane (5 mL) and water (0.5 mL) was de-gassed and then heated to 110 °C for 15 h under nitrogen. To the mixture was added water (20 mL) and extracted with ethyl acetate (30 mL x 3). The combined organic phase was dried over anhydrous sodium sulfate, filtered and concentrated in vacuum to give a residue. The residue was purified by flash silica gel chromatography (4 g SepaFlash^{®} Silica Flash Column, Eluent of 0~100% Ethyl acetate/Petroleum ether gradient @ 30 mL/min) to give impure product. The impure product was purified by prep-HPLC(column: Xtimate C18 150*40mm*10µm;mobile phase: [water (0.05% ammonia hydroxide v/v)-ACN]; B%: 30%-60%,10min). Compound 134 or 4-[2-amino-9-[(2,6-difluorophenyl)methyl]purin-6-yl]pyridine-2-carbonitrile (18.9 mg, 50.98 µmol, 15% yield, 98.03% purity) was obtained as a yellow solid. ¹H NMR (400 MHz, DMSO-d₆) δ = 9.03 (s, 1H), 8.98 (dd, *J=* 0.6, 5.1 Hz, 1H), 8.86 (dd, *J=* 1.6, 5.1 Hz, 1H), 8.29 (s, 1H), 7.54 - 7.43 (m, 1H), 7.17 (t, *J =* 8.1 Hz, 2H), 6.80 (s, 2H), 5.44 (s, 2H). HRMS-TOF: 364.1120.

### Example 1.38: Production 4-[2-amino-9-[(4-amino-3-methylphenyl)methyl]purin-6-yl]pyridine-2-carbonitrile (compound 130)

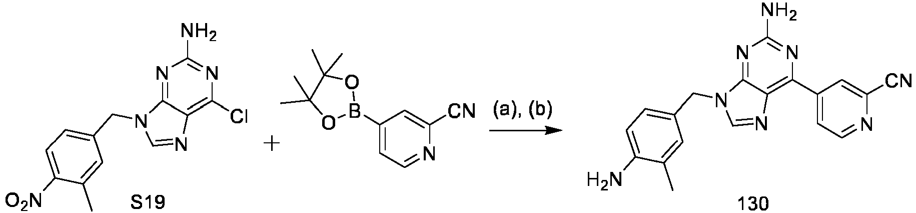

Reagents and condition: (a) Pd(dppf)Cl₂, K₂CO₃, dioxane/H₂O, 110°C, 2h, MW. (b) Fe, NH₄Cl, THF/H₂O, 80°C, 1 h.

### Stage 1: Production of 4-[2-amino-9-[(3-methyl-4-nitro-phenyl)methyl]purin-6-yl]pyridine-2-carbonitrile

A mixture of intermediate compound S19 or 6-chloro-9-[(3-methyl-4-nitrophenyl)methyl]purin-2-amine (100 mg, 313.76 µmol, 1 eq), 4-(4,4,5,5-tetramethyl-1,3,2-dioxaborolan-2-yl)pyridine-2-carbonitrile (144 mg, 627.51 µmol, 2 eq), Pd(dppf)Cl₂ (23 mg, 31.38 µmol, 0.1 eq) and K₂CO₃ (87 mg, 627.51 µmol, 2 eq) in dioxane (3 mL) and water (0.3 mL) was degassed and purged with nitrogen for three times, and then the mixture was stirred under microwave irradiation at 110 °C for 2 h. The mixture was added water (30 mL) and extracted with ethyl acetate (30 mL x 3). The combined organic phase was dried over anhydrous Na₂SO₄, filtered and concentrated in vacuum to give a residue. The residue was purified by flash silica gel chromatography (ISCO^{®}; 12 g Sepa Flash^{®} Silica Flash Column, Eluent of 0~80% Ethyl acetate/Petroleum ether gradient @ 35 mL/min). Compound 4-[2-amino-9-[(3-methyl-4-nitro-phenyl)methyl]purin-6-yl]pyridine-2-carbonitrile (104 mg, 242.26 µmol, 77% yield, 90% purity) was obtained as a yellow solid. ¹H NMR (400 MHz, DMSO-d₆) δ = 9.05 (s, 1H), 8.99 (d, *J =* 5.1 Hz, 1H), 8.89 (dd, *J =* 1.6, 5.1 Hz, 1H), 8.44 (s, 1H), 7.97 (d, *J =* 8.4 Hz, 1H), 7.41 (s, 1H), 7.28 (dd, *J =* 1.4, 8.5 Hz, 1H), 6.85 (s, 2H), 5.45 (s, 2H), 2.51 (s, 3H). MS: m/z = 387.1 (M+1, ESI+)

### Stage 2: Production of 4-[2-amino-9-[(4-amino-3-methyl-phenyl)methyl]purin-6-yl]pyridine-2-carbonitrile

To a solution of 4-[2-amino-9-[(3-methyl-4-nitro-phenyl)methyl]purin-6-yl] pyridine-2-carbonitrile (200 mg, 517.64 µmol, 1 eq) in ethanol (6 mL) and water (2 mL) were added iron dust (145 mg, 2.59 mmol, 5 eq) and NH₄Cl (221 mg, 4.14 mmol, 8 eq). The mixture was stirred at 80 °C for 1 h. The mixture was filtered and the filtrate was concentrated in vacuum to give a residue. The residue was added water (40 mL) and extracted with ethyl acetate (30 mL x 3). The combined organic layers were concentrated under reduced pressure to give a residue. The crude product was triturated with THF at 70 °C for 30 min. Compound 130 or 4-[2-amino-9-[(4-amino-3-methyl-phenyl) methyl]purin-6-yl]pyridine-2-carbonitrile (62 mg, 167.01 µmol, 32% yield, 96.73% purity) was obtained as a yellow solid. ¹H NMR (400 MHz, DMSO-d₆) δ = 9.04 (s, 1H), 8.97 (d, *J =* 5.1 Hz, 1H), 8.87 (dd, *J* = 1.5, 5.1 Hz, 1H), 8.32 (s, 1H), 6.99 - 6.88 (m, 2H), 6.85 (s, 2H), 6.54 (d, *J =* 8.0 Hz, 1H), 5.12 (s, 2H), 4.87 (s, 2H), 2.00 (s, 3H). HRMS-TOF: 357.1568.

### Example 1.39: Production 4-[2-amino-9-[[4-amino-3-(trifluoromethyl)phenyl]methyl]purin-6-yl]pyridine-2-carbonitrile (compound 136)

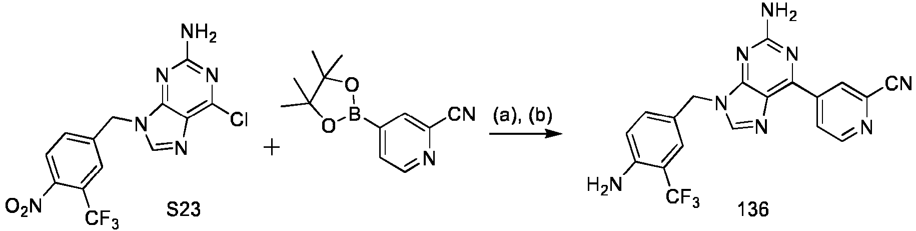

Reagents and condition: (a) APhos-Pd-G3, K₃PO₄, DMAc, 60°C, 16h (b) Fe, NH₄Cl, EtOH/H₂O, 60°C, 1 h.

### Stage 1: Production of 4-[2-amino-9-[[4-nitro-3-(trifluoromethyl)phenyl]methyl]purin-6-yl]pyridine-2-carbonitrile

A mixture of intermediate compound S23 or 6-chloro-9-[[4-nitro-3-(trifluoromethyl)phenyl]methyl]purin-2-amine (50 mg, 134.16 µmol, 1 eq), 4-(4,4,5,5-tetramethyl-1,3,2-dioxaborolan-2-yl)pyridine-2-carbonitrile (34 mg, 147.58 µmol, 1.1 eq), K₃PO₄ (1.5 M aqueous solution, 90 µL, 1 eq) and APhos-Pd-G3 (8.52 mg, 13.42 µmol, 0.1 eq) in DMAc (2 mL) was degassed and purged with nitrogen for three times, and then the mixture was stirred at 60 °C for 16 h under nitrogen atmosphere. The reaction mixture was concentrated under reduced pressure to remove solvent. The residue was purified by prep-TLC (SiO₂, Ethyl acetate/Petroleum ether= 1:1). Compound 4-[2-amino-9-[[4-nitro-3-(trifluoromethyl)phenyl]methyl]purin-6-yl]pyridine-2-carbonitrile (40 mg, 90.84 µmol, 68% yield) was obtained as a yellow oil. MS: m/z = 441.1 (M+1, ESI+).

### Stage 2: Production of 4-[2-amino-9-[[4-amino-3-(trifluoromethyl)phenyl]methyl]purin-6-yl]pyridine-2-carbonitrile

To a solution of 4-[2-amino-9-[[4-nitro-3-(trifluoromethyl)phenyl]methyl]purin-6-yl]pyridine-2-carbonitrile (40 mg, 90.84 µmol, 1 eq) in ethanol (15 mL) and water (5 mL) were added iron dust (25 mg, 454.2 µmol, 5 eq) and NH₄Cl (39 mg, 726.72 µmol, 8 eq). The mixture was stirred at 60 °C for 1 h. The reaction mixture was filtered and concentrated under reduced pressure to give a residue. The residue was purified by prep-HPLC (column: Welch Ultimate XB-SiOH 250*50*10µm; mobile phase: [Hexane-EtOH (0.1% ammonia hydroxide)];B%: 10%-50%,15min). Compound 136 or 4-[2-amino-9-[[4-amino-3-(trifluoromethyl)phenyl]methyl]purin-6-yl]pyridine-2-carbonitrile (8.9 mg, 20.71 µmol, 23% yield, 95.51% purity) was obtained as a white solid. ¹H NMR (400 MHz, DMSO-d₆) δ = 9.03 (s, 1H), 8.97 (d, *J =* 5.1 Hz, 1H), 8.87 (dd, *J =* 1.3, 5.1 Hz, 1H), 8.38 (s, 1H), 7.41 (s, 1H), 7.29 (br d, *J* = 8.4 Hz, 1H), 6.84 (s, 2H), 6.79 (d, *J =* 8.5 Hz, 1H), 5.66 (s, 2H), 5.20 (s, 2H). HRMS-TOF: 411.1305.

### Example 1.40: Production 3-(6-amino-1-(4-amino-3-methylbenzyl)-1H-pyrazolo[3,4-d]pyrimidin-4-yl)benzonitrile (compound 204)

1.40.1. Production of 3-(6-amino-1H-pyrazolo[3,4-d]pyrimidin-4-yl)benzonitrile (intermediate compound S107) 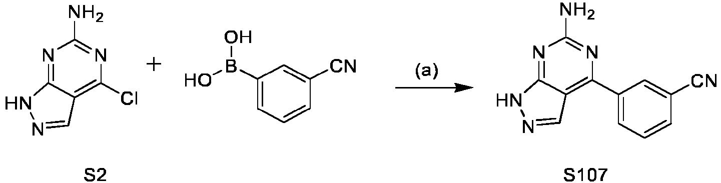 Reagents and Conditions: (a) Pd(PPh₃)₄, Na₂CO₃, dioxane/H₂O, 100°C, 16 h.

A mixture of 4-chloro-1H-pyrazolo[3,4-d]pyrimidin-6-amine (500 mg, 1 eq), (3-cyanophenyl)boronic acid (519 mg, 1.2 eq), Pd(PPh₃)₄ (340 mg, 0.1 eq) and Na₂CO₃ (625 mg, 2 eq) in dioxane (20 mL) was degassed and purged with nitrogen for three times, and then the mixture was stirred at 100 °C for 16hr under nitrogen atmosphere. The reaction mixture was partitioned between ethyl acetate (100 mL) and water (30 mL). The organic phase was separated, washed by brine (20 mL), dried over sodium sulfate, filtered and concentrated under reduced pressure to give a residue. The crude product 3-(6-amino-1H-pyrazolo[3,4-d]pyrimidin-4-yl)benzonitrile (500 mg, crude) was obtained as a yellow solid and used into the next step without further purification. MS: m/z = 237.1 (M+1, ESI+).

### 1.40.2. Production of 3-(6-amino-1-(4-amino-3-methylbenzyl)-1H-pyrazolo[3,4-d]pyrimidin-4-yl)benzonitrile (compound 204).

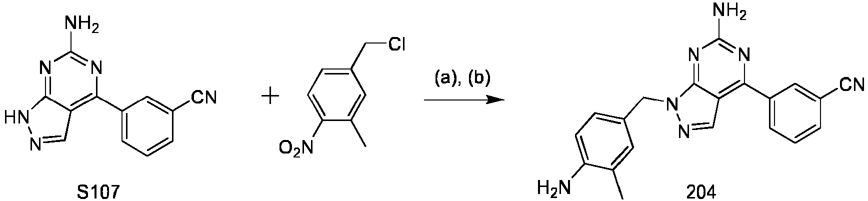

Reagents and Conditions: (a) K₂CO₃, DMF, 80°C, 16h; (b) Fe, NH₄Cl, EtOH/H₂O, 60°C, 1h.

### Stage 1: Production of 3-[6-amino-1-[(3-methyl-4-nitrophenyl)methyl]pyrazolo[3,4-d]pyrimidin-4-yl]benzonitrile

To a solution of 4-(chloromethyl)-2-methyl-1-nitro-benzene (500 mg, 1.27 eq) and intermediate compound S107 or 3-(6-amino-1H-pyrazolo[3,4-d]pyrimidin-4-yl)benzonitrile (500 mg, 1 eq) in DMF (10 mL) was added K₂CO₃ (585 mg, 2 eq). The mixture was stirred at 80 °C for 16hr. The reaction mixture was concentrated under reduced pressure to give a residue. The residue was purified by prep-HPLC (column: Phenomenex Luna C18 150*25mm *10µm;mobile phase: [water(0.225%FA)-ACN];B%: 40%-70%,10min). Compound 3-[6-amino-1-[(3-methyl-4-nitro-phenyl)methyl]pyrazolo[3,4-d] pyrimidin-4-yl]benzonitrile (200 mg, 24% yield) was obtained as a yellow solid. ¹H NMR (400 MHz, DMSO-d₆) δ = 8.56 - 8.42 (m, 3H), 8.10 - 8.06 (m, 1H), 8.00 - 7.92 (m, 1H), 7.86 - 7.76 (m, 1H), 7.40 - 7.30 (m, 1H), 7.24 - 7.08 (m, 3H), 5.65 - 5.43 (m, 2H), 2.49 (s, 3H). MS: m/z = 386.0 (M+1, ESI+).

### Stage 2: Production of 3-(6-amino-1-(4-amino-3-methylbenzyl)-1H-pyrazolo[3,4-d]pyrimidin-4-yl)benzonitrile (compound 204)

To a solution of 3-[6-amino-1-[(3-methyl-4-nitro-phenyl)methyl]pyrazolo [3,4-d]pyrimidin-4-yl]benzonitrile (150 mg, 1 eq) in ethanol (12 mL) and water (4 mL) were added iron dust (108 mg, 1.95 mmol, 5 eq) and NH₄Cl (166 mg, 8 eq). The mixture was stirred at 60 °C for 1 hr. The reaction mixture was filtered and concentrated under reduced pressure to give a residue. The residue was purified by prep-HPLC (column: Phenomenex Luna C18 150*25mm*10µm;mobile phase: [water(0.225% FA)-ACN];B%: 23%-53%,10min). Compound 204 or 3-[6-amino-1-[(4-amino-3-methylphenyl)methyl]pyrazolo[3,4-d]pyrimidin-4-yl]benzonitrile (54.26 mg, 38% yield, 98.27% purity) was obtained as a white solid. ¹H NMR (400 MHz, DMSO-d₆) δ = 8.54 - 8.49 (m, 1H), 8.49 - 8.45 (m, 1H), 8.37 - 8.31 (m, 1H), 8.09 - 8.01 (m, 1H), 7.84 - 7.70 (m, 1H), 7.16 - 6.99 (m, 2H), 6.91 - 6.86 (m, 1H), 6.86 - 6.80 (m, 1H), 6.58 - 6.45 (m, 1H), 5.33 - 5.14 (m, 2H), 4.98 - 4.57 (m, 2H), 2.02 - 1.96 (m, 3H).

### Example 1.41: Production 3-(6-amino-1-(4-amino-3-methylbenzyl)-1H-pyrazolo[3,4-d]pyrimidin-4-yl)-2-fluorobenzonitrile (compound 206)

### 1.41.1. Production of 4-chloro-1-(3-methyl-4-nitrobenzyl)-1H-pyrazolo[3,4-d]pyrimidin-6-amine (intermediate compound S108)

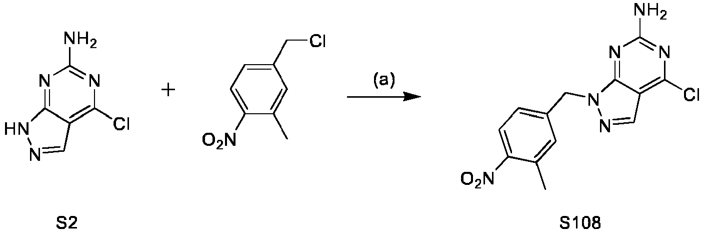

Reagents and Conditions: (a) K₂CO₃, DMAc, 80°C, 16 h.

To a solution of 4-chloro-1H-pyrazolo[3,4-d]pyrimidin-6-amine (0.8 g, 1 eq) and 4-(chloromethyl)-2-methyl-1-nitro-benzene (720 mg, 0.8) in DMAc (50 mL) was added K₂CO₃ (1.30 g, 2 eq). The mixture was stirred at 80 °C for 16hr. The reaction mixture filtered and concentrated under reduced pressure to give a residue. The crude product 4-chloro-1-[(3-methyl-4-nitro-phenyl)methyl]pyrazolo[3,4-d]pyrimidin-6-amine (800 mg, crude) was obtained as a yellow solid and used into the next step without further purification. MS: m/z = 354.9 (M+1, ESI+).

### 1.41.2. Production of 3-(6-amino-1-(4-amino-3-methylbenzyl)-1H-pyrazolo[3,4-d]pyrimidin-4-yl)-2-fluorobenzonitrile (compound 206)

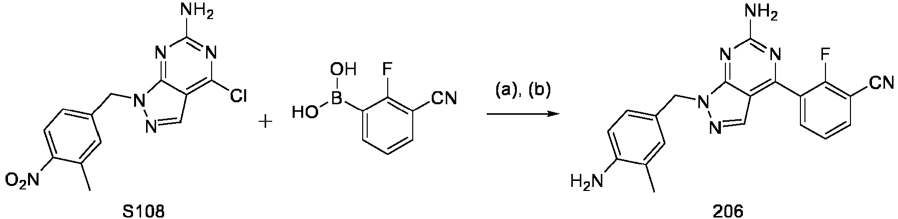

Reagents and condition: (a) Pd(PPh₃)₄, K₂CO₃, dioxane/H₂O, 100°C, 16h; (b) Fe, NH₄Cl, EtOH/H₂O, 60 °C, 1 h.

### Stage 1: Production of 3-(6-amino-1-(3-methyl-4-nitrobenzyl)-1H-pyrazolo[3,4-d]pyrimidin-4-yl)-2-fluorobenzonitrile

A mixture of (3-cyano-2-fluoro-phenyl)boronic acid (434 mg, 1.2 eq), intermediate compound S108 or 4-chloro-1-[(3-methyl-4-nitro-phenyl)methyl]pyrazolo[3,4-d]pyrimidin-6-amine (700 mg, 1 eq), Pd(PPh₃)₄ (253 mg, 219.63 µmol, 0.1 eq) and Na₂CO₃ (465 mg, 2 eq) in dioxane (10 mL) and water (2 mL) was degassed and purged with nitrogen for three times, and then the mixture was stirred at 110 °C for 16hr under nitrogen atmosphere. The reaction mixture was filtered and concentrated under reduced pressure to give a residue. The residue was purified by flash silica gel chromatography (ISCO^{®}; 40 g SepaFlash^{®} Silica Flash Column, Eluent of 0~80% Ethyl acetate/Petroleum ether gradient @ 40 mL/min). Compound 3-[6-amino-1-[(3-methyl-4-nitro-phenyl)methyl]pyrazolo[3,4-d]pyrimidin-4-yl]-2-fluoro-benzonitrile (800 mg, 90% yield) was obtained as a yellow solid. MS: m/z = 386.0 (M+1, ESI+).

### Stage 2: Production of 3-(6-amino-1-(4-amino-3-methylbenzyl)-1H-pyrazolo[3,4-d]pyrimidin-4-yl)-2-fluorobenzonitrile (compound 206)

To a solution of 3-[6-amino-1-[(3-methyl-4-nitro-phenyl)methyl]pyrazolo [3,4-d]pyrimidin-4-yl]-2-fluoro-benzonitrile (200 mg, 1 eq) in water (4 mL) and ethanol (12 mL) were added iron dust (138 mg, 5 eq) and NH₄Cl (212 mg, 8 eq). The mixture was stirred at 60 °C for 1 hr. The reaction mixture was filtered and concentrated under reduced pressure to give a residue. The residue was purified by prep-HPLC (column: Phenomenex Synergi C18 150*25mm* 10µm;mobile phase: [water(0.225%FA)-ACN];B%: 16%-46%,11min), the 89% purity compound was obtained after first purification. The residue was purified by prep-HPLC (column: Phenomenex Luna C18 150*25mm*10µm;mobile phase: [water(0.225%FA)-ACN];B%: 18%-48%,10min). Compound 206 or 3-[6-amino-1-[(4-amino-3-methyl-phenyl)methyl]pyrazolo[3,4-d]pyrimidin-4-yl]-2-fluoro-benzonitrile (111.35 mg, 59% yield, 97.78% purity) was obtained as a white solid. ¹H NMR (400 MHz, DMSO-d6) δ = 8.21 - 8.10 (m, 2H), 8.00 - 7.94 (m, 1H), 7.66 - 7.52 (m, 1H), 7.16 - 7.07 (m, 2H), 6.92 - 6.80 (m, 2H), 6.56 - 6.46 (m, 1H), 5.34 - 5.15 (m, 2H), 4.93 - 4.67 (m, 2H), 2.06 - 1.88 (m, 3H).

### Example 1.42: Production 3-(6-amino-1-(4-amino-3-(trifluoromethyl)benzyl)-1H-pyrazolo[3,4-d]pyrimidin-4-yl)benzonitrile (compound 210)

1.42.1. Production of 4-chloro-1-(4-nitro-3-(trifluoromethyl)benzyl)-1H-pyrazolo[3,4-d]pyrimidin-6-amine (intermediate compound S109) 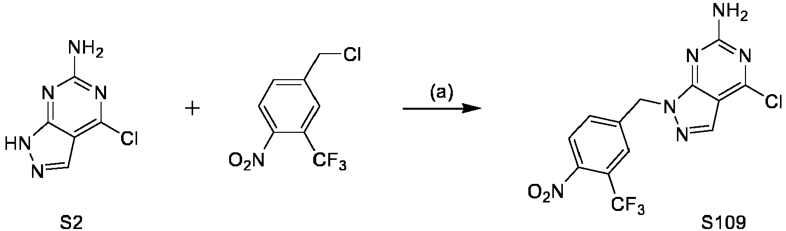 Reagents and Conditions: (a) K₂CO₃, DMAc, 80°C, 16 h.

To a solution of 4-(chloromethyl)-1-nitro-2-(trifluoromethyl)benzene (3.39 g, 1.2 eq) and 4-chloro-1H-pyrazolo[3,4-d]pyrimidin-6-amine (2 g, 1 eq) in DMF (20 mL) was added K₂CO₃ (3.26 g, 2 eq). The mixture was stirred at 80 °C for 16hr. The reaction mixture was concentrated under reduced pressure to remove DMF to give a residue. The residue was purified by flash silica gel chromatography (ISCO^{®}; 40 g SepaFlash^{®} Silica Flash Column, Eluent of 0~100% Ethyl acetate/Petroleum ether gradient @ 45 mL/min). Compound 4-chloro-1-[[4-nitro-3-(trifluoromethyl)phenyl]methyl]pyrazolo[3,4-d]pyrimidin-6-amine (2 g, 45% yield) was obtained as a yellow solid. MS: m/z = 373.3 (M+1, ESI+)

### 1.42.2. Production of 3-(6-amino-1-(4-amino-3-(trifluoromethyl)benzyl)-1H-pyrazolo[3,4-d]pyrimidin-4-yl)benzonitrile (compound 210)

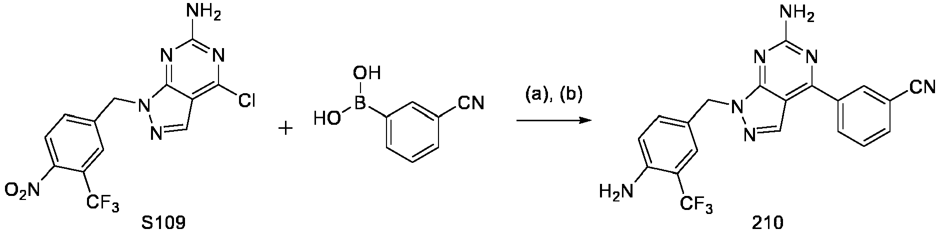

Reagents and condition: (a) Pd(PPh₃)₄, K₂CO₃, dioxane/H₂O, 100°C, 15h; (b) Fe, NH₄Cl, EtOH/H₂O, 60 °C, 2 h.

### Stage 1: Production of 3-(6-amino-1-(4-nitro-3-(trifluoromethyl)benzyl)-1H-pyrazolo[3,4-d]pyrimidin-4-yl)benzonitrile

A mixture of (3-cyanophenyl)boronic acid (236 mg, 1.5 eq), intermediate compound S109 or 4-chloro-1-[[4-nitro-3-(trifluoromethyl) phenyl]methyl]pyrazolo[3,4-d]pyrimidin-6-amine (400 mg, 1 eq), Pd(PPh₃)₄ (124 mg, 0.1 eq), K₂CO₃ (296 mg, 2 eq) in dioxane (10 mL) and water (1 mL) was degassed and purged with nitrogen for three times, and then the mixture was stirred at 100 °C for 15 hr under nitrogen atmosphere. The reaction mixture was concentrated under reduced pressure to remove dioxane and water to give a residue. The residue was purified by flash silica gel chromatography (ISCO^{®}; 12 g SepaFlash^{®} Silica Flash Column, Eluent of 0~100% Ethyl acetate/Petroleum ether gradient @ 35 mL/min). Compound 3-[6-amino-1-[[4-nitro-3-(trifluoromethyl)phenyl]methyl] pyrazolo[3,4-d]pyrimidin-4-yl]benzonitrile (300 mg, 63% yield) was obtained as a yellow oil. MS: m/z = 440.1 (M+1, ESI+).

### Stage 2: Production of 3-(6-amino-1-(4-amino-3-(trifluoromethyl)benzyl)-1H-pyrazolo[3,4-d]pyrimidin-4-yl)benzonitrile (compound 210)

To a solution of 3-[6-amino-1-[[4-nitro-3-(trifluoromethyl)phenyl]methyl]pyrazolo[3,4-d]pyrimidin-4-yl]benzonitrile (290 mg, 1 eq) in water (5 mL) and ethanol (15 mL) was added iron dust (190 mg, 5 eq) and NH₄Cl (292 mg, 8 eq). The mixture was stirred at 60 °C for 2 hr. The reaction mixture was concentrated under reduced pressure to remove ethanol to give a residue. The residue was purified by prep-HPLC (column: Unisil 3-100 C18 Ultra 150*50mm*3 µm;mobile phase: [water(0.225%FA)-ACN];B%: 43%-63%,10min). Compound 210 or 3-[6-amino-1-[[4-amino-3-(trifluoromethyl)phenyl]methyl] pyrazolo[3,4-d]pyrimidin-4-yl]benzonitrile (59.98 mg, 21% yield, 99.14% purity) was obtained as a white solid. ¹H NMR (400 MHz, DMSO-d₆) δ = 8.52 (s, 1H), 8.47 (d, *J =* 8.1 Hz, 1H), 8.38 (s, 1H), 8.06 (d, *J =* 7.7 Hz, 1H), 7.79 (t, *J =* 7.8 Hz, 1H), 7.30 (d, *J* = 1.5 Hz, 1H), 7.23 - 7.17 (m, 1H), 7.10 (s, 2H), 6.78 (d, *J =* 8.4 Hz, 1H), 5.59 (s, 2H), 5.30 (s, 2H).

### Example 1.43: Production of 3-(6-amino-1-(4-amino-3-(trifluoromethyl)benzyl)-1H-pyrazolo [3,4-d] pyrimidin-4-yl)-2-fluorobenzonitrile (compound 211)

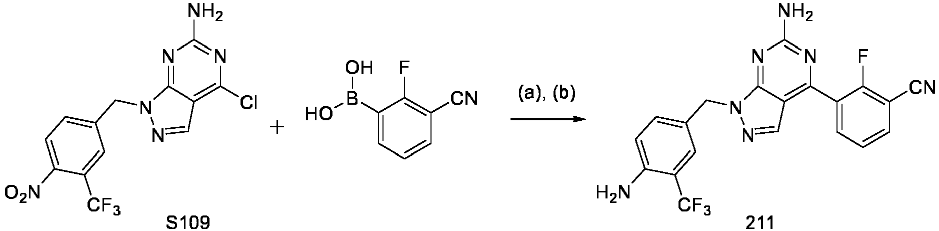

Reagents and condition: (a) Pd(PPh₃)₄, K₂CO₃, dioxane/H₂O, 100°C, 15h; (b) Fe, NH₄Cl, EtOH/H₂O, 60 °C, 2 h.

### Stage 1: Production of 3-(6-amino-1-(4-nitro-3-(trifluoromethyl)benzyl)-1H-pyrazolo[3,4-d]pyrimidin-4-yl)-2-fluorobenzonitrile

A mixture of (3-cyano-2-fluoro-phenyl)boronic acid (265 mg, 1.5 eq), intermediate compound S109 or 4-chloro-1-[[4-nitro-3-(trifluoromethyl)phenyl]methyl]pyrazolo[3,4-d]pyrimidin-6-amine (400 mg, 1 eq), Pd(PPh₃)₄ (124 mg, 0.1 eq), K₂CO₃ (296 mg, 2 eq) in dioxane (10 mL) and water (1 mL) was degassed and purged with nitrogen for three times, and then the mixture was stirred at 100 °C for 15 hr under nitrogen atmosphere. The reaction mixture was concentrated under reduced pressure to remove dioxane and water to give a residue. The residue was purified by flash silica gel chromatography (ISCO^{®}; 12 g Sepa Flash^{®} Silica Flash Column, Eluent of 0~100% Ethyl acetate/Petroleum ether gradient @ 35 mL/min). Compound 3-[6-amino-1-[[4-nitro-3-(trifluoromethyl)phenyl]methyl]pyrazolo[3,4-d]pyrimidin-4-yl]-2-fluoro-benzonitrile (350 mg, 71% yield) was obtained as a yellow oil. MS: m/z = 458.3 (M+1, ESI+).

### Stage 2: Production of 3-(6-amino-1-(4-amino-3-(trifluoromethyl)benzyl)-1H-pyrazolo[3,4-d]pyrimidin-4-yl)-2-fluorobenzonitrile (compound 211)

To a solution of 3-[6-amino-1-[[4-nitro-3-(trifluoromethyl)phenyl]methyl] pyrazolo[3,4-d]pyrimidin-4-yl]-2-fluoro-benzonitrile (300 mg, 1 eq) in ethanol (15 mL) and water (5 mL) was added iron dust (183 mg, 5 eq) and NH₄Cl (280 mg, 8 eq). The mixture was stirred at 60 °C for 2 hr. The reaction mixture was concentrated under reduced pressure to remove ethanol to give a residue. The residue was purified by prep-HPLC (column: Unisil 3-100 C18 Ultra 150*50mm*3 µm;mobile phase: [water(0.225%FA)-ACN];B%: 43%-63%,10min). Compound 211 or 3-[6-amino-1-[[4-amino-3-(trifluoromethyl)phenyl]methyl]pyrazolo[3,4-d]pyrimidin-4-yl]-2-fluoro-benzonitrile (41.58 mg, 15% yield, 98.23% purity) was obtained as a solid off-white. ¹H NMR (400 MHz, DMSO-d₆) δ = 8.20 - 8.11 (m, 2H), 8.00 (d, *J =* 3.5 Hz, 1H), 7.60 (t, *J=* 7.8 Hz, 1H), 7.30 (d, *J=* 1.6 Hz, 1H), 7.23 - 7.17 (m, 1H), 7.15 (s, 2H), 6.78 (d, *J =* 8.4 Hz, 1H), 5.60 (s, 2H), 5.29 (s, 2H).

### Example 1.44: Production of 3-(6-amino-1-(2,6-difluorobenzyl)-1H-pyrazolo[3,4-d]pyrimidin-4-yl)-2-fluorobenzonitrile (compound 213)

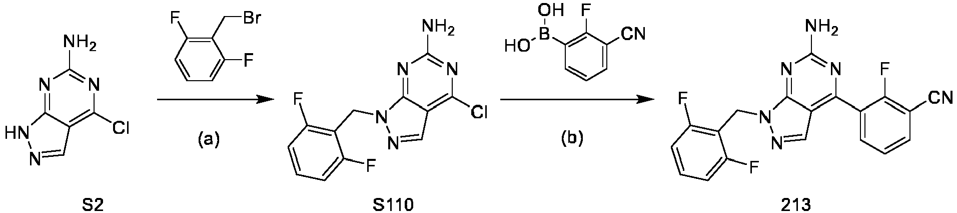

Reagents and Conditions: (a) K₂CO₃, DMAc, 80°C, 16 h; (b) Pd(PPh₃)₄, K₂CO₃, dioxane/H₂O, 80°C, 16 h.

### Stage 1: Production of 4-chloro-1-[(2,6-difluorophenyl)methyl]pyrazolo[3,4-d]pyrimidin-6-amine (intermediate compound S110)

To a solution of 4-chloro-1H-pyrazolo[3,4-d]pyrimidin-6-amine (8 g, 47.18 mmol, 1 eq) and 2-(bromomethyl)-1,3-difluoro-benzene (11.72 g, 56.61 mmol, 1.2 eq) in DMAc (100 mL) was added K₂CO₃ (13.04 g, 94.36 mmol, 2 eq). The mixture was stirred at 80 °C for 16hr. The reaction mixture was filtered and concentrated under reduced pressure to give a residue. The residue was purified by flash silica gel chromatography (ISCO^{®}; 80 g Sepa Flash^{®} Silica Flash Column, Eluent of 0~100% Ethyl acetate/Petroleum ether gradient @ 60 mL/min). Intermediate compound S110 or 4-chloro-1-[(2,6-difluorophenyl)methyl]pyrazolo[3,4-d]pyrimidin-6-amine (4 g, 13.53 mmol, 29% yield) was obtained as a white solid. ¹H NMR (400 MHz, DMSO-d₆) δ = 8.35 - 7.77 (m, 1H), 7.53 - 7.41 (m, 1H), 7.40 - 7.31 (m, 2H), 7.19 - 6.98 (m, 2H), 5.60 - 5.20 (m, 2H).

### Stage 2: Production of 3-(6-amino-1-(2,6-difluorobenzyl)-1H-pyrazolo[3,4-d]pyrimidin-4-yl)-2-fluorobenzonitrile (compound 213)

A mixture of (3-cyano-2-fluoro-phenyl)boronic acid (251 mg, 1.5 eq), intermediate compound S110 or 4-chloro-1-[(2,6-difluorophenyl) methyl]pyrazolo[3,4-d]pyrimidin-6-amine (300 mg, 1 eq), Pd(PPh₃)₄ (117 mg, 0.1 eq), K₂CO₃ (280 mg, 2 eq) in dioxane (10 mL) and water (2 mL) was degassed and purged with nitrogen for three times, and then the mixture was stirred at 80 °C for 16hr under nitrogen atmosphere. The reaction mixture was concentrated under reduced pressure to give a residue. The residue was purified by prep-HPLC (column: Unisil 3-100 C18 Ultra 150*50mm*3 µm;mobile phase: [water(0.225%FA)-ACN];B%: 45%-65%,10min). Compound 213 or 3-[6-amino-1-[(2,6-difluorophenyl)methyl]pyrazolo[3,4-d]pyrimidin-4-yl]-2-fluoro-benzonitrile (118.51 mg, 30% yield, 98.93% purity) was obtained as a white solid. ¹H NMR (400 MHz, DMSO-d₆) δ = 8.22 - 8.09 (m, 2H), 7.95 (d, *J=* 3.5 Hz, 1H), 7.60 (t, *J =* 7.8 Hz, 1H), 7.52 - 7.39 (m, 1H), 7.19 (s, 2H), 7.13 (t, *J =* 8.0 Hz, 2H), 5.45 (s, 2H).

### Example 1.45: Production of 3-(6-amino-1-(4-amino-2-fluorobenzyl)-1H-pyrazolo[3,4-d]pyrimidin-4-yl)benzonitrile (compound 209)

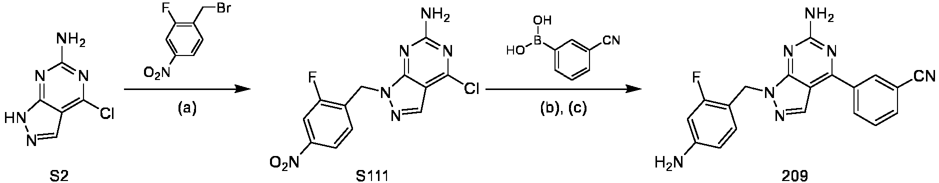

Reagents and Conditions: (a) K₂CO₃, DMAc, 60°C, 16 h; (b) Pd(PPh₃)₄, K₂CO₃, dioxane/H₂O, 80°C, 16 h; (c) Fe, NH₄Cl, EtOH/H₂O, 80°C, 3h.

### Stage 1: Production of 4-chloro-1-(2-fluoro-4-nitrobenzyl)-1H-pyrazolo[3,4-d] pyrimidin-6-amine (intermediate compound S111)

To a solution of 4-chloro-1H-pyrazolo[3,4-d]pyrimidin-6-amine (5.1 g, 1 eq) and 1-(bromomethyl)-2-fluoro-4-nitro-benzene (7 g, 1 eq) in DMF (20 mL) was added K₂CO₃ (8.3 g, 2 eq). The mixture was stirred at 60 °C for 16hr. The reaction mixture was filtered and concentrated under reduced pressure to give a residue. The residue was purified by flash silica gel chromatography (ISCO^{®}; 40 g SepaFlash^{®} Silica Flash Column, Eluent of 0~100% Ethyl acetate/Petroleum ether gradient @ 40 mL/min). Intermediate compound S111 or 4-chloro-1-[(2-fluoro-4-nitro-phenyl) methyl]pyrazolo[3,4-d]pyrimidin-6-amine (5 g, 52% yield) was obtained as a yellow solid. ¹H NMR (400 MHz, DMSO-d₆) δ = 8.15 - 8.11 (m, 1H), 8.08 - 8.06 (m, 1H), 8.05 - 8.01 (m, 1H), 7.47 - 7.38 (m, 2H), 7.33 (t, *J =* 8.0 Hz, 1H), 5.57 (s, 2H). MS: m/z = 323.3 (M+1, ESI+).

### Stage 2: Production of 3-(6-amino-1-(2-fluoro-4-nitrobenzyl)-1H-pyrazolo [3,4-d]pyrimidin-4-yl)benzonitrile

A mixture of (3-cyanophenyl)boronic acid (341 mg, 1.5 eq), intermediate compound S111 or 4-chloro-1-[(2-fluoro-4-nitro-phenyl)methyl]pyrazolo[3,4-d]pyrimidin-6-amine (500 mg, 1 eq), Pd(PPh₃)₄ (179 mg, 0.1 eq), K₂CO₃ (428 mg, 2 eq) in dioxane (10 mL) and water (2 mL) was degassed and purged with nitrogen for three times, and then the mixture was stirred at 100 °C for 16hr under nitrogen atmosphere. The reaction mixture was concentrated under reduced pressure to remove dioxane to give a residue. The residue was purified by prep-HPLC (column: Unisil 3-100 C18 Ultra 150*50mm*3 µm;mobile phase: [water(0.225%FA)-ACN];B%: 45%-65%,10min). Compound 3-[6-amino-1-[(2-fluoro-4-nitro-phenyl)methyl] pyrazolo[3,4-d]pyrimidin-4-yl]benzonitrile (200 mg, 33% yield, 99% purity) was obtained as a yellow solid. ¹H NMR (400 MHz, DMSO-d₆) δ = 8.55 - 8.51 (m, 1H), 8.49 - 8.46 (m, 1H), 8.31 - 8.26 (m, 1H), 8.18 - 8.01 (m, 4H), 7.38 - 7.28 (m, 1H), 7.22 - 7.05 (m, 2H), 5.62 (s, 2H). MS: m/z = 389.9 (M+1, ESI+).

### Stage 3: Production of 3-(6-amino-1-(4-amino-2-fluorobenzyl)-1H-pyrazolo[3,4-d]pyrimidin-4-yl)benzonitrile (compound 209)

To a solution of 3-[6-amino-1-[(2-fluoro-4-nitro-phenyl)methyl]pyrazolo [3,4-d]pyrimidin-4-yl]benzonitrile (200 mg, 513.69 µmol, 1 eq) in ethanol (15 mL) and water (5 mL) were added iron dust (143 mg, 5 eq) and NH₄Cl (219 mg, 8 eq). The mixture was stirred at 80 °C for 3 hr. The reaction mixture was concentrated under reduced pressure to remove ethanol and water to give a residue. The residue was purified by prep-HPLC (column: Unisil 3-100 C18 Ultra 150*50mm*3 µm;mobile phase: [water(0.225%FA)-ACN];B%: 30%-50%,10min). Compound 209 or 3-[6-amino-1-[(4-amino-2-fluorophenyl)methyl]pyrazolo[3,4-d]pyrimidin-4-yl]benzonitrile (36.42 mg, 19% yield, 97.66% purity) was obtained as an off-white solid. ¹H NMR (400 MHz, DMSO-d₆) δ = 8.51 (s, 1H), 8.49 - 8.44 (m, 1H), 8.35 (s, 1H), 8.08 - 8.01 (m, 1H), 7.79 (t, *J =* 7.8 Hz, 1H), 7.07 (s, 2H), 6.88 (t, *J =* 8.5 Hz, 1H), 6.36 - 6.22 (m, 2H), 5.40 (s, 2H), 5.28 (s, 2H).

### Example 1.46: Production of 3-(6-amino-1-(4-amino-2,6-difluorobenzyl)-1H-pyrazolo[3,4-d]pyrimidin-4-yl)benzonitrile (compound 207)

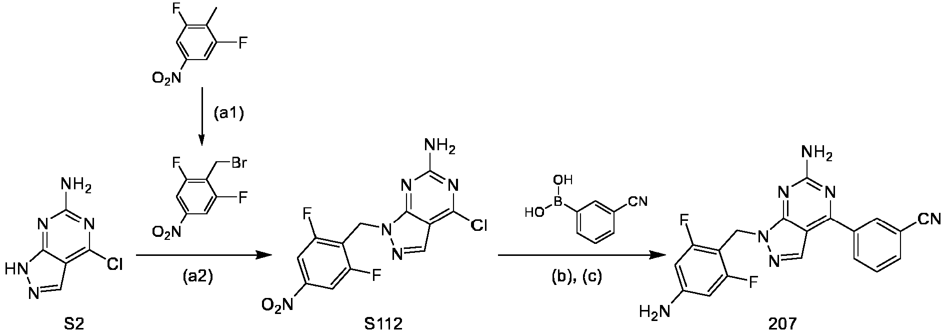

Reagents and Conditions: (a1) NBS, AIBN, CCl₄, 90°C, 15h; (a2) K₂CO₃, DMAc, 80°C, 3 h; (b) Pd(dppf)Cl₂, K₂CO₃, dioxane/H₂O, 110°C, 2 h, MW; (c) Fe, NH₄Cl, EtOH/H₂O, 80°C, 1h.

### Stage 1: Production of 4-chloro-1-[(2,6-difluoro-4-nitro-phenyl)methyl] pyrazolo[3,4-d]pyrimidin-6-amine (intermediate compound S112)

To the solution of 1,3-difluoro-2-methyl-5-nitro-benzene (2 g, 11.55 mmol, 1 eq) in tetrachloromethane (50 mL) were added NBS (3.08 g, 17.33 mmol, 1.5 eq) and AIBN (190 mg, 1.16 mmol, 0.1 eq). Then the mixture was stirred at 90 °C for 15 h. The reaction mixture was concentrated in vacuum to give a residue. To the residue was added DMAc (50 mL), 4-chloro-1H-pyrazolo[3,4-d]pyrimidin-6-amine (1.96 g, 11.55 mmol, 1 eq) and K₂CO₃ (3.19 g, 23.11 mmol, 2 eq). Then the mixture was stirred at 80 °C for 3 h. To the mixture was added water (130 mL) and ethyl acetate (130 mL), and the layers were separated. The aqueous was extracted with ethyl acetate (130 mL x 2). Combined the organic phase to wash by brines (150 mL x 2), dry over anhydrous sodium sulfate, filter and the filtrate was concentrated in vacuum to give a residue. The residue was purified by flash silica gel chromatography (40 g SepaFlash^{®} Silica Flash Column, Eluent of 0~30% Ethyl acetate/Petroleum ether gradient @ 60 mL/min). Intermediate compound S112 or 4-chloro-1-[(2,6-difluoro-4-nitro-phenyl) methyl]pyrazolo[3,4-d]pyrimidin-6-amine (1.5 g, 4.26 mmol, 37% yield, 96.7% purity) was obtained as a yellow solid.

### Stage 2: Production of 3-[6-amino-1-[(2,6-difluoro-4-nitro-phenyl) methyl]pyrazolo[3,4-d]pyrimidin-4-yl]benzonitrile

Intermediate compound S112 or 4-chloro-1-[(2,6-difluoro-4-nitrophenyl)methyl]pyrazolo[3,4-d]pyrimidin-6-amine (500 mg, 1.47 mmol, 1 eq), (3-cyanophenyl)boronic acid (323 mg, 2.20 mmol, 1.5 eq), K₂CO₃ (405 mg, 2.94 mmol, 2 eq) and Pd(dppf)Cl₂ (107 mg, 146.77 µmol, 0.1 eq) were taken up into a microwave tube in dioxane (6 mL) and water (2 mL). The sealed tube was heated at 110 °C for 2 h under microwave. After that the mixture was added water (80 mL) and ethyl acetate (80 mL), and the layers were separated. The aqueous was extracted with ethyl acetate (80 mL x 3). Combined the organic phase to dry over anhydrous sodium sulfate, filter and the filtrate was concentrated in vacuum to give a residue. The residue was purified by flash silica gel chromatography (20 g SepaFlash^{®} Silica Flash Column, Eluent of 0~100% Ethyl acetate/Petroleum ether gradient @ 50 mL/min). Compound 3-[6-amino-1-[(2,6-difluoro-4-nitro-phenyl)methyl]pyrazolo[3,4-d]pyrimidin-4-yl]benzonitrile (200 mg, 441.90 µmol, 30% yield, 90% purity) was obtained as a yellow solid. ¹H NMR (400 MHz, DMSO-d₆) δ = 8.49 (d, *J =* 1.2 Hz, 1H), 8.47 - 8.41 (m, 1H), 8.35 (s, 1H), 8.16 - 8.07 (m, 2H), 8.06 (td, *J* = 1.3, 7.8 Hz, 1H), 7.79 (t, *J =* 7.9 Hz, 1H), 7.16 (s, 2H), 5.55 (s, 2H). MS: m/z = 408.0 (M+1, ESI+).

### Stage 3: Production of 3-[6-amino-1-[(4-amino-2,6-difluoro-phenyl) methyl]pyrazolo[3,4-d]pyrimidin-4-yl]benzonitrile (compound 207)

To the solution of 3-[6-amino-1-[(2,6-difluoro-4-nitro-phenyl)methyl]pyrazolo[3,4-d]pyrimidin-4-yl] benzonitrile (200 mg, 491.00 µmol, 1 eq) in ethanol (8 mL) and water (2 mL) were added iron dust (137 mg, 2.45 mmol, 5 eq) and NH₄Cl (210 mg, 3.93 mmol, 8 eq). Then the mixture was stirred at 80 °C for 1 h. The reaction mixture was filtered and the filtrate was concentrated in vacuum. After that, to the mixture was added water (30 mL) and then extracted with ethyl acetate (30 mL x 3). Combined the organic phase to dry over anhydrous sodium sulfate, filter and the filtrate was concentrated in vacuum to give a residue. The residue was purified by prep-HPLC(column: Welch Ultimate XB-CN 250*70*10µm;mobile phase: [Hexane-EtOH (0.1% ammonia hydroxide)];B%: 30%-70%,15min). Compound 207 or 3-[6-amino-1-[(4-amino-2,6-difluorophenyl)methyl]pyrazolo[3,4-d]pyrimidin-4-yl]benzonitrile (62.95 mg, 163.33 µmol, 33% yield, 97.91% purity) was obtained as a yellow solid. ¹H NMR (400 MHz, DMSO-d₆) δ = 8.49 (t, *J =* 1.4 Hz, 1H), 8.45 (td, *J =* 1.4, 7.9 Hz, 1H), 8.29 (s, 1H), 8.05 (td, *J =* 1.3, 7.7 Hz, 1H), 7.79 (t, *J =* 7.8 Hz, 1H), 7.07 (s, 2H), 6.30 - 6.06 (m, 2H), 5.80 (s, 2H), 5.25 (s, 2H).

### Example 1.47: Production of 3-(6-amino-1-(4-amino-2-fluorobenzyl)-1H-pyrazolo[3,4-d]pyrimidin-4-yl)-2-fluorobenzonitrile (compound 203)

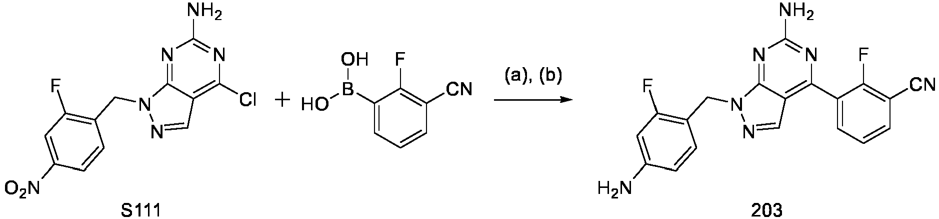

Reagents and condition: (a) Pd(dppf)Cl₂, K₂CO₃, dioxane/H₂O, 110°C, 16h; (b) Fe, NH₄Cl, EtOH/H₂O, 80°C, 3h.

### Stage 1: Production of 3-(6-amino-1-(2-fluoro-4-nitrobenzyl)-1H-pyrazolo[3,4-d]pyrimidin-4-yl)-2-fluorobenzonitrile

A mixture of (3-cyano-2-fluoro-phenyl)boronic acid (383 mg, 2.33 mmol, 1.5 eq), intermediate compound S111 or 4-chloro-1-[(2-fluoro-4-nitro-phenyl)methyl]pyrazolo[3,4-d]pyrimidin- 6-amine (500 mg, 1 eq), Pd(PPh₃)₄ (179 mg, 0.1 eq), K₂CO₃ (428 mg, 2 eq) in dioxane (10 mL) and water (2 mL) was degassed and purged with nitrogen for three times, and then the mixture was stirred at 100 °C for 16hr under nitrogen atmosphere. The reaction mixture was concentrated under reduced pressure to remove dioxane to give a residue. The residue was purified by flash silica gel chromatography (ISCO^{®}; 12 g Sepa Flash^{®} Silica Flash Column, Eluent of 0~80% Ethyl acetate/Petroleum ether gradient @ 30 mL/min). Compound 3-[6-amino-1-[(2-fluoro-4-nitro-phenyl)methyl]pyrazolo[3,4-d]pyrimidin-4-yl]-2-fluoro-benzonitrile (300 mg, 48% yield) was obtained as a yellow solid. MS: m/z = 407.9 (M+1, ESI+).

### Stage 2: Production of 3-(6-amino-1-(4-amino-2-fluorobenzyl)-1H-pyrazolo [3,4-d]pyrimidin-4-yl)-2-fluorobenzonitrile (compound 203)

To a solution of 3-[6-amino-1-[(2-fluoro-4-nitro-phenyl)methyl]pyrazolo [3,4-d]pyrimidin-4-yl]-2-fluoro-benzonitrile (209 mg, 1 eq) in ethanol (15 mL) and water (5 mL) was added iron dust (143 mg, 5 eq) and NH₄Cl (219 mg, 8 eq). The mixture was stirred at 80 °C for 3 hr. The reaction mixture was concentrated under reduced pressure to remove ethanol to give a residue. The residue was purified by prep-HPLC (column: Unisil 3-100 C18 Ultra 150*50mm*3 µm;mobile phase: [water(0.225%FA)-ACN];B%: 33%-53%,10min), 80% purity desired compound was obtained, then the residue was purified by second prep-HPLC (column: Phenomenex Gemini-NX C18 75*30mm*3µm;mobile phase: [water(0.225%FA)-ACN];B%: 22%-52%,7min). Compound 203 or 3-[6-amino-1-[(4-amino-2-fluoro-phenyl)methyl]pyrazolo[3,4-d] pyrimidin-4-yl]-2-fluoro-benzonitrile (62.37 mg, 31% yield, 97.40% purity) was obtained as a white solid. ¹H NMR (400 MHz, DMSO-d₆) δ = 8.21 - 8.10 (m, 2H), 7.98 (d, *J =* 3.7 Hz, 1H), 7.61 (t, *J =* 7.8 Hz, 1H), 7.14 (s, 2H), 6.97 - 6.84 (m, 1H), 6.32 (s, 1H), 6.31 - 6.28 (m, 1H), 5.42 (s, 2H), 5.27 (s, 2H).

### Example 1.48: Production of 3-(6-amino-1-(4-amino-2,6-difluorobenzyl)-1H-pyrazolo[3,4-d]pyrimidin-4-yl)-2-fluorobenzonitrile (compound 208)

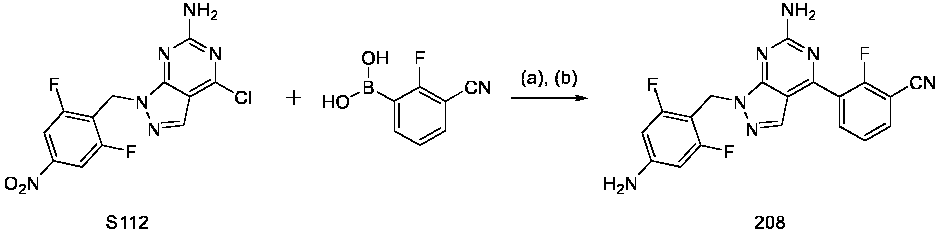

Reagents and condition: (a) Pd(dppf)Cl₄, K₂CO₃, dioxane/H₂O, 110°C, 15h; (b) Fe, NH₄Cl, EtOH/H₂O, 80°C, 1h.

### Stage 1: Production of 3-[6-amino-1-[(2,6-difluoro-4-nitro-phenyl)methyl] pvyrazolo[3,4-d]pyrimidin-4-yl]-2-fluoro-benzonitrile

To the solution of intermediate compound S112 or 4-chloro-1-[(2,6-difluoro-4-nitro-phenyl)methyl]pyrazolo[3,4-d]pyrimidin-6-amine (400 mg, 1.17 mmol, 1 eq) in water (2 mL) and dioxane (8 mL) were added (3-cyano-2-fluoro-phenyl)boronic acid (300 mg, 1.82 mmol, 1.55 eq), Pd(dppf)Cl₂ (86 mg, 117.41 µmol, 0.1 eq) and K₂CO₃ (325 mg, 2.35 mmol, 2 eq). Then the mixture was stirred at 110 °C for 15 h. After that the mixture was concentrated in vacuum to give a residue, and it was purified by flash silica gel chromatography (12 g SepaFlash^{®} Silica Flash Column, Eluent of 20~100% Ethyl acetate/Petroleum ether gradient @ 50 mL/min). Compound 3-[6-amino-1-[(2,6-difluoro-4-nitro-phenyl)methyl]pyrazolo[3,4-d]pyrimidin-4-yl]-2-fluoro-benzonitrile (140 mg, 322.25 µmol, 27% yield, 97.9% purity) was obtained as a yellow solid. ¹H NMR (400 MHz, DMSO-d₆) δ = 8.20 - 8.12 (m, 2H), 8.12 - 8.05 (m, 2H), 7.99 (d, *J =* 3.6 Hz, 1H), 7.60 (t, *J =* 7.8 Hz, 1H), 7.22 (s, 2H), 5.54 (s, 2H). MS: m/z = 426.1 (M+1, ESI+).

### Stage 2: Production of 3-(6-amino-1-(4-amino-2,6-difluorobenzyl)-1H-pyrazolo[3,4-d]pyrimidin-4-yl)-2-fluorobenzonitrile (compound 208)

To the solution of 3-[6-amino-1-[(2,6-difluoro-4-nitro-phenyl)methyl]pyrazolo [3,4-d]pyrimidin-4-yl]-2-fluoro-benzonitrile (140 mg, 329.16 µmol, 1 eq) in ethanol (8 mL) and water (2 mL) were added iron dust (92 mg, 1.65 mmol, 5 eq) and NH₄Cl (140 mg, 2.63 mmol, 8 eq). Then the mixture was stirred at 80 °C for 1 h. The reaction mixture was filtered and the filtrate was concentrated in vacuum. To the mixture were added water (50 mL) and then extracted with ethyl acetate (30 mL x 3). Combined the organic phase to dry over anhydrous sodium sulfate, filter and concentrate in vacuum to give a residue. The residue was purified by prep-HPLC(column: Welch Ultimate XB-CN 250*70*10µm;mobile phase: [Hexane-EtOH (0.1% NH₃·H₂O)];B%: 35%-75%,15min). Compound 208 or 3-[6-amino-1-[(4-amino-2,6-difluoro-phenyl)methyl]pyrazolo[3,4-d]pyrimidin-4-yl]-2-fluoro-benzonitrile (44.01 mg, 109.41 µmol, 33% yield, 98.28% purity) was obtained as a yellow solid. ¹H NMR (400 MHz, DMSO-d₆) δ = 8.14 (dd, *J* = 6.7, 7.7 Hz, 2H), 7.93 (d, *J* = 3.6 Hz, 1H), 7.60 (t, *J =* 7.8 Hz, 1H), 7.13 (s, 2H), 6.27 - 6.07 (m, 2H), 5.80 (s, 2H), 5.24 (s, 2H).

### Example 1.49: Production of 4-[6-amino-1-[(2,6-difluorophenyl)methyl] pyrazolo[3,4-d]pyrimidin-4-yl]pyridine-2-carbonitrile (compound 234)

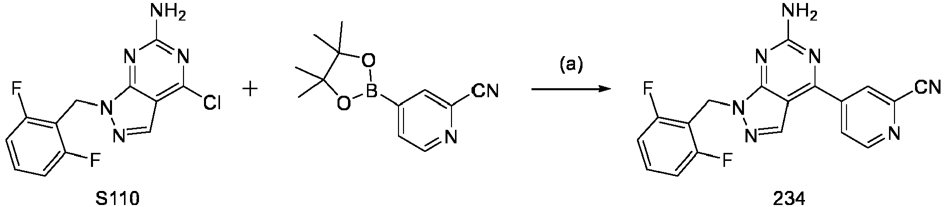

Reagents and condition: (a) Pd(dppf)Cl₂, K₂CO₃, dioxane/H₂O, 80°C, 16h.

A mixture of intermediate compound S110 or 4-chloro-1-[(2,6-difluorophenyl)methyl]pyrazolo[3,4-d] pyrimidin-6-amine (300 mg, 1 eq) , 4-(4,4,5,5-tetramethyl-1,3,2-dioxaborolan- 2-yl)pyridine-2-carbonitrile (279 mg, 1.2 eq), K₂CO₃ (279 mg, 2 eq), Pd(dppf)Cl₂ (74 mg, 0.1 eq) in dioxane (10 mL) and water (2 mL) was degassed and purged with nitrogen for three times, and then the mixture was stirred at 80 °C for 16hr under nitrogen atmosphere. The reaction mixture was concentrated under reduced pressure to remove dioxane to give a residue. The residue was purified by prep-HPLC (column: Unisil 3-100 C18 Ultra 150*50mm*3 µm;mobile phase: [water(0.225% FA)-ACN];B%: 38%-58%,10min). Compound 234 or 4-[6-amino-1-[(2,6-difluorophenyl)methyl]pyrazolo[3,4-d]pyrimidin-4-yl]pyridine-2-carbonitrile (124.39 mg, 34% yield, 99.96% purity) was obtained as a white solid. ¹H NMR (400 MHz, DMSO-d₆) δ = 8.96 (d, *J =* 5.1 Hz, 1H), 8.58 (d, *J =* 0.6 Hz, 1H), 8.41 - 8.39 (m, 1H), 8.39 - 8.36 (m, 1H), 7.51 - 7.41 (m, 1H), 7.25 (s, 2H), 7.17 - 7.08 (m, 2H), 5.47 (s, 2H).

### Example 1.50: Production of 4-(6-amino-1-(4-amino-2,6-difluorobenzyl)-1H-pyrazolo[3,4-d]pyrimidin-4-yl)picolinonitrile (compound 226)

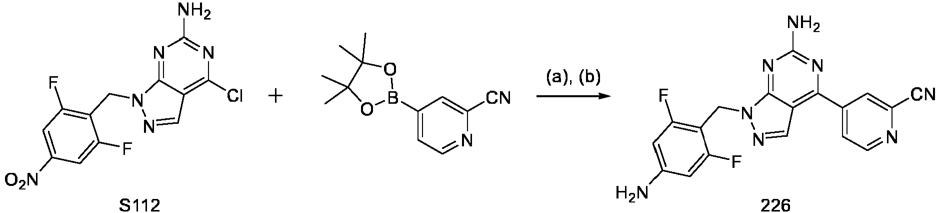

Reagents and condition: (a) Pd(dppf)Cl₂, K₂CO₃, dioxane/H₂O, 80°C, 16h; (b) Fe, NH₄Cl, EtOH/H₂O, 80°C, 2h.

### Stage 1: Production of 4-[6-amino-1-[(2,6-difluoro-4-nitro-phenyl)methyl] pyrazolo[3,4-d]pyrimidin-4-yl]pyridine-2-carbonitrile

To the solution of intermediate compound S112 or 4-chloro-1-[(2,6-difluoro-4-nitro-phenyl)methyl]pyrazolo [3,4-d]pyrimidin-6-amine (400 mg, 1.17 mmol, 1 eq) in dioxane (10 mL) and water (2 mL) were added 4-(4,4,5,5-tetramethyl-1,3,2-dioxaborolan-2-yl) pyridine-2-carbonitrile (540 mg, 2.35 mmol, 2 eq), Pd(dppf)Cl₂ (86 mg, 117.41 µmol, 0.1 eq) and K₂CO₃ (325 mg, 2.35 mmol, 2 eq). Then the mixture was stirred at 100 °C for 15 h. The mixture was concentrated in vacuum to give a residue. The residue was purified by flash silica gel chromatography (20 g SepaFlash^{®} Silica Flash Column, Eluent of 0~60% Ethyl acetate/Petroleum ether gradient @ 50 mL/min). Compound 4-[6-amino-1-[(2,6-difluoro-4-nitro-phenyl)methyl] pyrazolo[3,4-d]pyrimidin-4-yl]pyridine-2-carbonitrile (300 mg, 625.68 µmol, 53% yield, 85% purity) was obtained as a yellow solid. ¹H NMR (400 MHz, DMSO-d₆) δ = 8.96 (d, *J =* 5.1 Hz, 1H), 8.58 (s, 1H), 8.43 (s, 1H), 8.38 (dd, *J =* 1.7, 5.1 Hz, 1H), 8.09 (br d, *J =* 7.1 Hz, 2H), 7.28 (br s, 2H), 5.56 (s, 2H). MS: m/z = 409.1 (M+1, ESI+).

### Stage 2: Production of 4-[6-amino-1-[(4-amino-2,6-difluoro-phenyl) methyl]pyrazolo[3,4-d]pyrimidin-4-yl]pyridine-2-carbonitrile (compound 226)

To the mixture of 4-[6-amino-1-[(2,6-difluoro-4-nitro-phenyl)methyl]pyrazolo [3,4-d]pyrimidin-4-yl]pyridine-2-carbonitrile (300 mg, 734.72 µmol, 1 eq) in water (3 mL) and ethanol (9 mL) were added iron dust (205 mg, 3.67 mmol, 5 eq) and NH₄Cl (314 mg, 5.88 mmol, 8 eq). Then the mixture was stirred at 80 °C for 2 h. The reaction mixture was filtered and the filtrate was concentrated in vacuum. To the mixture was added water (30 mL) and extracted with ethyl acetate (30 mL x 3). Combined the organic phase to dry over anhydrous sodium sulfate, filter and the filtrate was concentrated in vacuum to give a residue. The residue was purified by prep-HPLC (column: Welch Ultimate XB-CN 250*70*10µm;mobile phase: [Hexane-ethanol (0.1% NH₃·H₂O)];B%: 30%-70%,15min). Compound 226 or 4-[6-amino-1-[(4-amino-2,6-difluoro-phenyl)methyl]pyrazolo[3,4-d]pyrimidin-4-yl]pyridine-2-carbonitrile (70.80 mg, 178.73 µmol, 24% yield, 95.51% purity) was obtained as a yellow solid. ¹H NMR (400 MHz, DMSO-d₆) δ = 8.96 (dd, *J =* 0.8, 5.1 Hz, 1H), 8.58 (dd, *J* = 0.8, 1.6 Hz, 1H), 8.46 - 8.24 (m, 2H), 7.19 (s, 2H), 6.29 - 6.08 (m, 2H), 5.80 (s, 2H), 5.26 (s, 2H).

### Example 1.51: Production of 4-(6-amino-1-(4-amino-3-methylbenzyl)-1H-pyrazolo[3,4-d]pyrimidin-4-yl)picolinonitrile (compound 230)

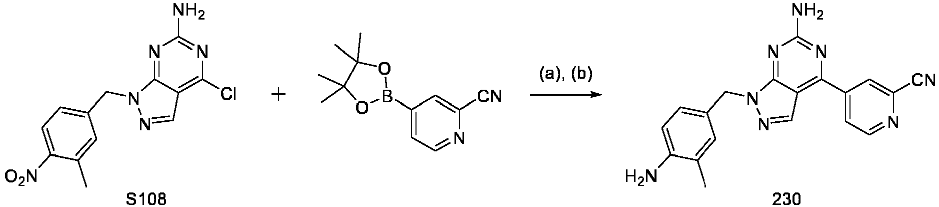

Reagents and condition: (a) Pd(dppf)Cl₂, K₂CO₃, dioxane/H₂O, 110°C, 16h; (b) Fe, NH₄Cl, EtOH/H₂O, 60°C, 3h.

### Stage 1: Production of 4-(6-amino-1-(3-methyl-4-nitrobenzyl)-1H-pyrazolo[3,4-d]pyrimidin-4-yl)picolinonitrile

A mixture of intermediate compound S108 or 4-chloro-1-[(3-methyl-4-nitrophenyl)methyl]pyrazolo[3,4-d] pyrimidin-6-amine (500 mg, 1 eq), 4-(4,4,5,5-tetramethyl-1,3,2-dioxaborolan- 2-yl)pyridine-2-carbonitrile (541 mg, 1.5 eq), K₂CO₃ (433 mg, 2 eq), Pd(dppf)Cl₂ (114 mg, 0.1 eq) in dioxane (10 mL) and water (2 mL) was degassed and purged with N₂ for three times, and then the mixture was stirred at 110°C for 16hr under N₂ atmosphere. The reaction mixture was filtered and concentrated under reduced pressure to give a residue. The residue was purified by flash silica gel chromatography (ISCO^{®}; 12 g SepaFlash^{®} Silica Flash Column, Eluent of 0~100% Ethyl acetate/Petroleum ether gradient @ 30 mL/min). Compound 4-[6-amino-1-[(3-methyl-4-nitro-phenyl)methyl] pyrazolo[3,4-d]pyrimidin-4-yl]pyridine-2-carbonitrile (500 mg, 82.49% yield) was obtained as a yellow solid. ¹H NMR (400 MHz, DMSO-d₆) δ = 8.99 (dd, *J* = 0.7*,* 5.1 Hz, 1H), 8.56 (s, 1H), 8.43 (dd, *J=* 1.7, 5.1 Hz, 1H), 7.98 - 7.91 (m, 1H), 7.39 - 7.31 (m, 1H), 7.30 - 7.07 (m, 3H), 5.68 - 5.49 (m, 2H), 2.49 - 2.47 (m, 3H). MS: m/z = 387.1 (M+1, ESI+).

### Stage 2: Production of 4-(6-amino-1-(4-amino-3-methylbenzyl)-1H-pyrazolo[3,4-d]pyrimidin-4-yl)picolinonitrile (compound 230)

To a solution of 4-[6-amino-1-[(3-methyl-4-nitro-phenyl)methyl] pyrazolo[3,4-d]pyrimidin-4-yl]pyridine-2-carbonitrile (400 mg, 1 eq) in ethanol (15 mL) and water (5 mL) were added iron dust (289 mg, 5 eq) and NH₄Cl (443 mg, 8 eq). The mixture was stirred at 60 °C for 3 h. The reaction mixture was filtered and concentrated under reduced pressure to give a residue. The residue was purified by prep-HPLC (column: Phenomenex Gemini-NX C18 75*30mm*3µm;mobile phase: [water (0.05% ammonia hydroxide v/v)-ACN];B%: 15%-45%,11.5min). Compound 230 or 4-[6-amino-1-[(4-amino-3-methyl-phenyl) methyl]pyrazolo[3,4-d]pyrimidin-4-yl]pyridine-2-carbonitrile (41.4 mg, 7% yield, 95.38% purity) was obtained as a yellow solid. ¹H NMR (400 MHz, DMSO-d₆) δ = 8.97 (dd, *J =* 0.6, 5.1 Hz, 1H), 8.60 (d, *J =* 0.9 Hz, 1H), 8.44 (s, 1H), 8.40 (dd, *J =* 1.7, 5.1 Hz, 1H), 7.17 (s, 2H), 6.93 - 6.79 (m, 2H), 6.52 (d, *J=* 8.1 Hz, 1H), 5.24 (s, 2H), 4.81 (s, 2H), 1.99 (s, 3H).

### Example 1.52: Production of 6-(6-amino-1-(4-aminobenzyl)-1H-pyrazolo[3,4-d]pyrimidin-4-yl)picolinonitrile (compound 215)

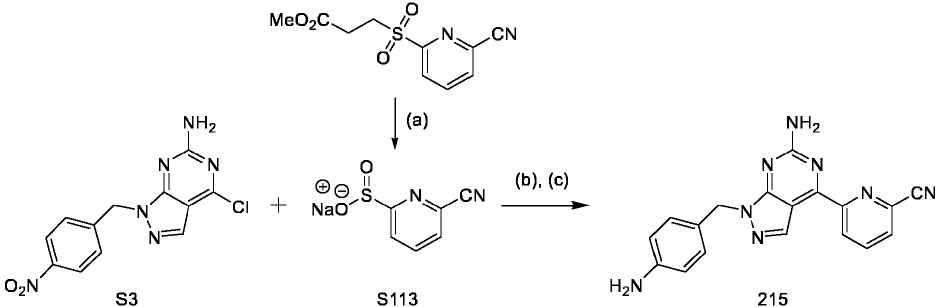

Reagents and condition: (a) NaOMe, MeOH, 25°C, 0.5 h; (b) Pd(OAc)₂, K₂CO₃, Cy₃P, dioxane, 120°C, 16h; (c) Fe, NH₄Cl, EtOH/H₂O, 60 °C, 3h.

### Stage 1: Production of (6-cyano-2-pyridyl)sulfinyloxy sodium (intermediate compound S113)

To a solution of sodium methoxide (212 mg, 1 eq) in methanol (10 mL) was added methyl 3-[(6-cyano-2-pyridyl)sulfonyl]propanoate (1 g, 1 eq). The mixture was stirred at 25 °C for 0.5 hr. The reaction mixture was concentrated under reduced pressure to remove methanol to give a residue. The crude product of intermediate compound S113 or (6-cyano-2-pyridyl)sulfinyloxy sodium (1 g, crude) was obtained as a yellow solid and used into the next step without further purification. MS: m/z = 167.0 (M+1, ESI+).

### Stage 2: Production of 6-[6-amino-1-[(4-nitrophenyl)methyl]pyrazolo[3,4-d] pyrimidin-4-yl]pyridine-2-carbonitrile

A mixture of intermediate compound S113 or 4-chloro-1-[(4-nitrophenyl) methyl]pyrazolo[3,4-d]pyrimidin-6-amine (500 mg, 1 eq), (6-cyano-2-pyridyl)sulfinyloxy sodium (374 mg, 1.2 eq), palladium acetate (36 mg, 0.1 eq), Cy₃P (92 mg, 0.2 eq) and K₂CO₃ (453 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by flash silica gel chromatography (ISCO^{®}; 12 g SepaFlash^{®} Silica Flash Column, Eluent of 70~100% Ethyl acetate/Petroleum ether gradient @ 35 mL/min). Compound 6-[6-amino-1-[(4-nitrophenyl)methyl]pyrazolo[3,4-d]pyrimidin-4-yl]pyridine-2-carbonitrile (200 mg, 504.92 µmol, 31% yield, 94% purity) was obtained as a white solid. ¹H NMR (400 MHz, DMSO-d₆) δ = 8.66 (dd, *J =* 1.3, 8.0 Hz, 1H), 8.50 (s, 1H), 8.35 - 8.30 (m, 1H), 8.28 - 8.24 (m, 1H), 8.20 (d, *J =* 8.9 Hz, 2H), 7.41 (d, *J =* 8.9 Hz, 2H), 7.20 (s, 2H), 5.64 (s, 2H). MS: m/z = 373.1 (M+1, ESI+).

### Stage 3: Production of 6-(6-amino-1-(4-aminobenzyl)-1H-pyrazolo[3,4-d]pyrimidin-4-yl)picolinonitrile (compound 215)

To a solution of 6-[6-amino-1-[(4-nitrophenyl)methyl]pyrazolo[3,4-d] pyrimidin-4-yl]pyridine-2-carbonitrile (200 mg, 1 eq) in water (5 mL) and ethanol (15 mL) was added iron dust (150 mg, 5 eq) and NH₄Cl (230 mg, 8 eq). The mixture was stirred at 60 °C for 3 hr. The reaction mixture was concentrated under reduced pressure to remove ethanol and water to give a residue. The residue was purified by prep-HPLC (column: Phenomenex Gemini-NX C18 75*30mm*3µm;mobile phase: [water (0.05% ammonia hydroxide v/v)-ACN];B%: 10%-40%,11.5min), the 89% purity desired compound was obtained, the residue was re-purified by prep-HPLC (column: Welch Ultimate XB-CN 250*50* 10µm;mobile phase: [Hexane-EtOH (0.1% NH₃·H₂O];B%: 20%-60%,15min), the 91% purity desired compound was obtained. Then the residue was purified by prep-HPLC (column: Phenomenex Luna C18 150*25mm* 10µm;mobile phase: [water(0.225%FA)-ACN];B%: 16%-46%,10min). Compound 215 or 6-[6-amino-1-[(4-aminophenyl)methyl]pyrazolo[3,4-d]pyrimidin-4-yl]pyridine-2-carbonitrile (21.55 mg, 11% yield, 98.25% purity) was obtained as a yellow solid. ¹H NMR (400 MHz, DMSO-d₆) δ = 8.65 (d, *J =* 7.6 Hz, 1H), 8.39 (s, 1H), 8.35 - 8.27 (m, 1H), 8.26 - 8.22 (m, 1H), 7.12 (s, 2H), 6.94 (br d, *J=* 8.3 Hz, 2H), 6.48 (br d, *J=* 8.3 Hz, 2H), 5.25 (s, 2H), 5.03 (s, 2H).

### Example 1.53: Production of 6-[6-amino-1-[(4-amino-2-fluorophenyl)methyl]pyrazolo[3,4-d]pyrimidin-4-yl]pyridine-2-carbonitrile (compound 219)

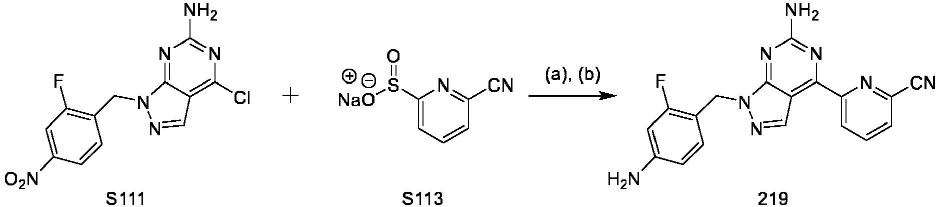

Reagents and condition: (a) Pd(OAc)₂, K₂CO₃, Cy₃P, dioxane, 120 °C, 16h; (c) Fe, NH₄Cl, THF/H₂O, 80 °C, 4h.

### Stage 1: Production of 6-[6-amino-1-[(2-fluoro-4-nitro-phenyl)methyl]pyrazolo[3,4-d]pyrimidin-4-yl]pyridine-2-carbonitrile

A mixture of intermediate compound S113 or (6-cyano-2-pyridyl)sulfinyloxy sodium (495.01 mg, 2.60 mmol, 1.2 eq), intermediate compound S111 or 4-chloro-1-[(2-fluoro-4-nitro-phenyl)methyl]pyrazolo[3,4-d]pyrimidin-6-amine (700 mg, 2.17 mmol, 1 eq), palladium acetate (48.70 mg, 216.93 µmol, 0.1 eq), Cy₃P (121.67 mg, 433.86 µmol, 140.66 µL, 0.2 eq) and K₂CO₃ (599.64 mg, 4.34 mmol, 2 eq) in dioxane (5 mL) was degassed and purged with nitrogen for three times, and then the mixture was stirred at 120 °C for 16hr under nitrogen atmosphere. The mixture was concentrated in vacuum to give a residue. The residue was purified by flash silica gel chromatography (12 g SepaFlash^{®} Silica Flash Column, Eluent of 10~50% Ethyl acetate/Petroleum ether gradient @ 40 mL/min). Compound 6-[6-amino-1-[(2-fluoro-4-nitro-phenyl)methyl]pyrazolo[3,4-d]pyrimidin-4-yl]pyridine-2-carbonitrile (220 mg, 503.88 µmol, 26% yield, 89.4% purity) was obtained as a yellow solid. ¹H NMR (400 MHz, DMSO-d₆) δ = 8.66 (dd, *J=* 1.1, 7.9 Hz, 1H), 8.49 (s, 1H), 8.36 - 8.29 (m, 1H), 8.28 - 8.23 (m, 1H), 8.16 (dd, *J=* 2.3, 9.8 Hz, 1H), 8.04 (dd, *J =* 1.8, 8.6 Hz, 1H), 7.29 (t, *J =* 8.0 Hz, 1H), 7.22 (s, 2H), 5.64 (s, 2H).

### Stage 2: Production of 6-[6-amino-1-[(4-amino-2-fluorophenyl)methyl]pyrazolo[3,4-d]pyrimidin-4-yl]pyridine-2-carbonitrile (compound 219)

To the mixture of 6-[6-amino-1-[(2-fluoro-4-nitro-phenyl)methyl]pyrazolo[3,4-d]pyrimidin-4-yl]pyridine-2-carbonitrile (200 mg, 512.39 µmol, 1 eq) in THF (10 mL) and water (2 mL) was added iron dust (143 mg, 2.56 mmol, 5 eq) and NH₄Cl (219.27 mg, 4.10 mmol, 8 eq). Then the mixture was stirred at 80 °C for 4 h. The reaction mixture was filtered and the filtrate was added 50 mL saturated NaHCO₃ aqueous solution and extracted with ethyl acetate (50 mL x 3). Combined the organic layers to dry over anhydrous sodium sulfate, filter and concentrate in vacuum to give a residue. The residue was purified by prep-HPLC(column: Welch Ultimate XB-CN 250*70*10µm;mobile phase: [Hexane-EtOH (0.1% NH₃·H₂O)];B%: 35%-75%,15min). Compound 219 or 6-[6-amino-1-[(4-amino-2-fluorophenyl)methyl]pyrazolo[3,4-d]pyrimidin-4-yl]pyridine-2-carbonitrile (56.69 mg, 151.63 µmol, 30% yield, 96.38% purity) was obtained as a yellow solid. ¹H NMR (400 MHz, DMSO-d₆) δ = 8.65 (dd, *J =* 1.2, 8.0 Hz, 1H), 8.39 (s, 1H), 8.34 - 8.29 (m, 1H), 8.26 - 8.22 (m, 1H), 7.13 (s, 2H), 6.95 - 6.79 (m, 1H), 6.31 (dd, *J=* 1.8, 5.4 Hz, 1H), 6.28 (s, 1H), 5.40 (s, 2H), 5.29 (s, 2H).

### Example 1.54: Production of 6-[6-amino-1-[(4-amino-2,6-difluorophenyl)methyl]pyrazolo[3,4-d]pyrimidin-4-yl]pyridine-2-carbonitrile (compound 223)

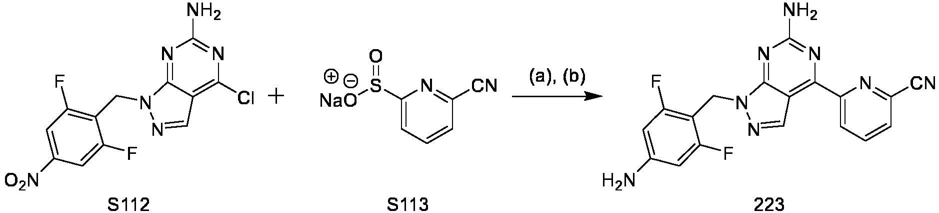

Reagents and condition: (a) Pd(OAc)₂, K₂CO₃, Cy₃P, dioxane, 120 °C, 16h; (c) Fe, NH₄Cl, THF/H₂O, 80 °C, 5h.

### Stage 1: Production of 6-[6-amino-1-[(2,6-difluoro-4-nitrophenyl)methyl]pyrazolo[3,4-d]pyrimidin-4-yl]pyridine-2-carbonitrile

A mixture of intermediate compound S113 or sodium 6-cyanopyridine-2-sulfinate (530 mg, 2.79 mmol, 2 eq), intermediate compound S112 or 4-chloro-1-[(2,6-difluoro-4-nitro-phenyl)methyl]pyrazolo[3,4-d]pyrimidin-6-amine (470 mg, 1.38 mmol, 0.99 eq), palladium acetate (31 mg, 139.36 µmol, 0.1 eq), Cy₃P (78 mg, 278.72 µmol, 0.2 eq) and K₂CO₃ (385 mg, 2.79 mmol, 2 eq) in dioxane (15 mL) was degassed and purged with nitrogen for three times, and then the mixture was stirred at 120 °C for 16hr under nitrogen atmosphere. The mixture was concentrated in vacuum to give a residue. The residue was purified by flash silica gel chromatography (20 g SepaFlash^{®} Silica Flash Column, Eluent of 30~60% Ethyl acetate/Petroleum ether gradient @ 50 mL/min). Compound 6-[6-amino-1-[(2,6-difluoro-4-nitro-phenyl)methyl] pyrazolo[3,4-d]pyrimidin-4-yl]pyridine-2-carbonitrile (180 mg, 290.95 µmol, 21% yield, 66% purity) was obtained as a yellow solid. ¹H NMR (400 MHz, DMSO-d₆) δ = 8.65 (dd, *J=* 1.1, 8.0 Hz, 1H), 8.38 (s, 1H), 8.35 - 8.33 (m, 1H), 8.26 - 8.21 (m, 1H), 8.12 - 8.06 (m, 2H), 7.22 (s, 2H), 5.57 (s, 2H). MS: m/z = 409.0 (M+1, ESI+).

### Stage 2: Production of 6-[6-amino-1-[(4-amino-2,6-difluorophenyl)methyl]pyrazolo[3,4-d]pyrimidin-4-yl]pyridine-2-carbonitrile (compound 223)

To the mixture of 6-[6-amino-1-[(2,6-difluoro-4-nitro-phenyl)methyl]pyrazolo[3,4-d]pyrimidin-4-yl]pyridine-2-carbonitrile (180 mg, 290.95 µmol, 66% purity, 1 eq) in THF (10 mL) and water (3 mL) was added iron dust (81 mg, 1.45 mmol, 5 eq) and NH₄Cl (124 mg, 2.33 mmol, 8 eq). Then the mixture was stirred at 80 °C for 5 hr. The reaction mixture was filtered and the filtrate was added saturated NaHCO₃ aqueous solution (50 mL) and extracted with ethyl acetate (80 mL x 3). Combined the organic phase to dry over anhydrous sodium sulfate, filter and concentrate in vacuum to give a residue. The residue was purified by prep-HPLC (column: Welch Ultimate XB-CN 250*70*10µm;mobile phase: [Hexane-EtOH (0.1% ammonia hydroxide)];B%: 40%-80%,10min). Compound 223 or 6-[6-amino-1-[(4-amino-2,6-difluoro-phenyl)methyl]pyrazolo[3,4-d]pyrimidin-4-yl]pyridine-2-carbonitrile (24.24 mg, 60.94 µmol, 21% yield, 95.11% purity) was obtained as a yellow solid. ¹H NMR (400 MHz, DMSO-d₆) δ = 8.65 (dd, *J =* 0.9, 8.0 Hz, 1H), 8.35 (s, 1H), 8.34 - 8.29 (m, 1H), 8.27 - 8.21 (m, 1H), 7.13 (s, 2H), 6.18 (d, *J =* 10.3 Hz, 2H), 5.80 (s, 2H), 5.27 (s, 2H).

### Example 1.55: Production of 6-(6-amino-1-(2,6-difluorobenzyl)-1H-pyrazolo[3,4-d]pyrimidin-4-yl)picolinonitrile (compound 231)

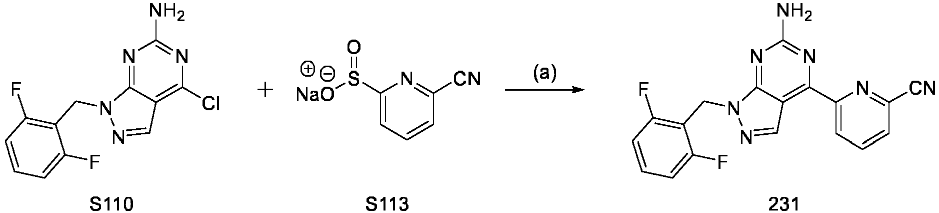

Reagents and condition: (a) Pd(OAc)₂, K₂CO₃, Cy₃P, dioxane, 120°C, 10h, MW.

A mixture of (6-cyano-2-pyridyl)sulfinyloxysodium (231 mg, 1.2 eq), intermediate compound S110 or 4-chloro-1-[(2,6-difluorophenyl) methyl]pyrazolo[3,4-d]pyrimidin-6-amine (300 mg, 1 eq), palladium acetate (23 mg, 0.1 eq), Cy₃P (57 mg, 0.2 eq) and K₂CO₃ (280 mg, 2 eq) in dioxane (2 mL) was degassed and purged with nitrogen for three times, and then the mixture was stirred at 120 °C for 10 hr under microwave condition. The reaction mixture was filtered and concentrated under reduced pressure to give a residue. The residue was purified by prep-HPLC (column: Phenomenex Luna C18 150*25mm* 10µm;mobile phase: [water(0.225%FA)-ACN];B%: 37%-67%,11min). Compound 231 or 6-[6-amino-1-[(2,6-difluorophenyl)methyl]pyrazolo[3,4-d]pyrimidin-4-yl]pyridine-2-carbonitrile (70.52 mg, 18% yield, 95.65% purity) was obtained as a yellow solid. ¹H NMR (400 MHz, DMSO-d₆) δ = 8.65 (dd, *J=* 1.0, 8.0 Hz, 1H), 8.36 (s, 1H), 8.33 - 8.27 (m, 1H), 8.26 - 8.20 (m, 1H), 7.51 - 7.41 (m, 1H), 7.18 (s, 2H), 7.12 (t, *J =* 8.0 Hz, 2H), 5.48 (s, 2H).

### Example 1.56: Production of 6-(6-amino-1-(4-amino-3-methylbenzyl)-1H-pyrazolo[3,4-d]pyrimidin-4-yl)picolinonitrile (compound 227)

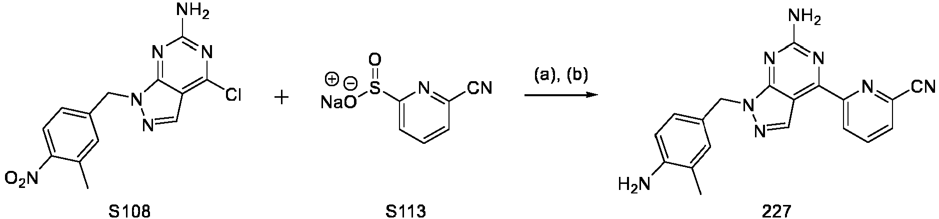

Reagents and condition: (a) Pd(OAc)₂, K₂CO₃, Cy₃P, dioxane, 120°C, 16h; (b) Fe, NH₄Cl, EtOH/H₂O, 60°C, 2h.

### Stage 1: Production of 6-[6-amino-1-[(3-methyl-4-nitrophenyl)methyl]pyrazolo[3,4-d]pyrimidin-4-yl]pyridine-2-carbonitrile

A mixture of intermediate compound S113 or (6-cyano-2-pyridyl)sulfinyloxy sodium (356 mg, 1.2 eq), intermediate compound S108 or 4-chloro-1-[(3-methyl-4-nitrophenyl)methyl]pyrazolo[3,4-d]pyrimidin-6-amine (500 mg, 1 eq), palladium acetate (35 mg, 0.1 eq), Cy₃P (88 mg, 0.2 eq) and K₂CO₃ (433 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by flash silica gel chromatography (ISCO^{®}; 24 g Sepa Flash^{®} Silica Flash Column, Eluent of 50~80% Ethyl acetate/Petroleum ether gradient @ 35 mL/min). Compound 6-[6-amino-1-[(3-methyl-4-nitrophenyl)methyl]pyrazolo[3,4-d]pyrimidin-4-yl]pyridine-2-carbonitrile (300 mg, 40% yield, 81% purity) was obtained as a yellow solid. ¹H NMR (400 MHz, DMSO-d₆) δ = 8.66 (dd, *J=* 1.1, 7.9 Hz, 1H), 8.48 (s, 1H), 8.37 - 8.29 (m, 1H), 8.28 - 8.22 (m, 1H), 7.95 (d, *J =* 8.5 Hz, 1H), 7.32 (s, 1H), 7.24 - 7.14 (m, 3H), 5.55 (s, 2H), 2.47 (s, 3H). MS: m/z = 387.1 (M+1, ESI+).

### Stage 2: Production of 6-(6-amino-1-(4-amino-3-methylbenzyl)-1H-pyrazolo[3,4-d]pyrimidin-4-yl)picolinonitrile (compound 227)

To a solution of 6-[6-amino-1-[(3-methyl-4-nitro-phenyl)methyl] pyrazolo[3,4-d]pyrimidin-4-yl]pyridine-2-carbonitrile (311 mg, 1 eq) in water (5 mL) and ethanol (15 mL) were added iron dust (225 mg, 5 eq) and NH₄Cl (344 mg, 8 eq). The mixture was stirred at 60 °C for 2 hr. The reaction mixture was concentrated under reduced pressure to remove ethanol and water to give a residue. The residue was purified by prep-HPLC (column: Phenomenex Gemini-NX C18 75*30mm*3µm;mobile phase: [water (0.05% ammonia hydroxide v/v)-ACN];B%: 10%-40%,11.5min), the 88 % purity desired compound was obtained, the residue was re-purified by prep-HPLC (column: Welch Ultimate XB-SiOH 250*50*10µm;mobile phase: [Hexane-EtOH (0.1% NH₃·H₂O];B%: 15%-55%,15min), the 90% purity desired compound was obtained. Then the residue was purified by prep-HPLC (column: Welch Ultimate XB-SiOH 250*50*10µm;mobile phase: [Hexane-EtOH (0.1% NH₃·H₂O];B%: 15%-55%,15min), 91% purity desired compound was obtained. Finally the residue was purified by prep-HPLC (column: Phenomenex Luna C18 150*25mm* 10µm;mobile phase: [water(0.225%FA)-ACN];B%: 21%-51%,10min). Compound 227 or 6-[6-amino-1-[(4-amino-3-methyl-phenyl)methyl]pyrazolo[3,4-d]pyrimidin-4-yl]pyridine-2-carbonitrile (30.69 mg, 10% yield, 98.87% purity) was obtained as a yellow solid. ¹H NMR (400 MHz, DMSO-d₆) δ = 8.65 (dd, *J =* 1.1, 8.0 Hz, 1H), 8.39 (s, 1H), 8.34 - 8.27 (m, 1H), 8.26 - 8.20 (m, 1H), 7.11 (s, 2H), 6.86 (s, 1H), 6.82 (dd, *J* = 1.9, 8.1 Hz, 1H), 6.52 (d, *J =* 8.0 Hz, 1H), 5.24 (s, 2H), 4.80 (s, 2H), 1.99 (s, 3H).

### Example 1.57: Production of 2-(6-amino-1-(4-aminobenzyl)-1H-pyrazolo[3,4-d]pyrimidin-4-yl)isonicotinonitrile (compound 216)

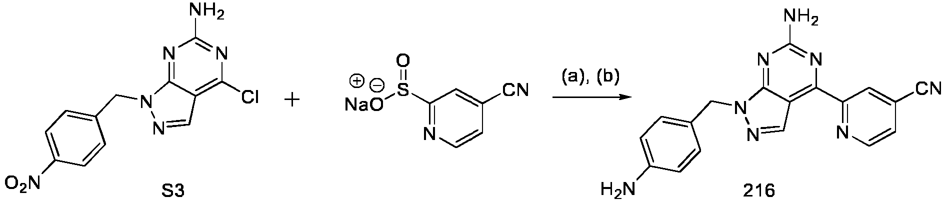

Reagents and condition: (a) Pd(OAc)₂, K₂CO₃, Cy₃P, dioxane, 120°C, 16h; (b) Fe, NH₄Cl, EtOH/H₂O, 60°C, 2h.

### Stage 1: Production of 2-(6-amino-1-(4-nitrobenzyl)-1H-pyrazolo[3,4-d]pyrimidin-4-yl)isonicotinonitrile

A mixture of sodium 4-cyanopyridine-2-sulfinate (298 mg, 1.2 eq), intermediate compound S3 or 4-chloro-1-[(4-nitrophenyl)methyl] pyrazolo[3,4-d]pyrimidin-6-amine (400 mg, 1 eq), palladium acetate (29 mg, 0.1 eq), Cy₃P (73 mg, 0.2 eq) and K₂CO₃ (362 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by flash silica gel chromatography (ISCO^{®}; 12 g SepaFlash^{®} Silica Flash Column, Eluent of 70-90% Ethyl acetate/Petroleum ether gradient @ 30 mL/min). Compound 2-[6-amino-1-[(4-nitrophenyl) methyl]pyrazolo[3,4-d]pyrimidin-4-yl]pyridine-4-carbonitrile (300 mg, 55% yield, 89% purity) was obtained as a yellow solid. MS: m/z = 373.1 (M+1, ESI+).

### Stage 2: Production of 2-(6-amino-1-(4-aminobenzyl)-1H-pyrazolo[3,4-d]pyrimidin-4-yl)isonicotinonitrile (compound 216)

To a solution of 2-[6-amino-1-[(4-nitrophenyl)methyl]pyrazolo[3,4-d] pyrimidin-4-yl]pyridine-4-carbonitrile (300 mg, 1 eq) in water (5 mL) and ethanol (15 mL) was added iron dust (225 mg, 5 eq) and NH₄Cl (345 mg, 8 eq). The mixture was stirred at 60 °C for 2 hr. The reaction mixture was concentrated under reduced pressure to remove ethanol and water to give a residue. The residue was purified by prep-HPLC (column: Phenomenex Gemini-NX C18 75*30mm*3µm;mobile phase: [water(10mM NH₄HCO₃)-ACN];B%: 18%-48%,8min), 80% purity desired compound was obtained. Then the residue was purified by the 2nd prep-HPLC (column: Welch Ultimate XB-SiOH 250*50*10µm;mobile phase: [Hexane-EtOH (0.1% NH₃·H₂O)];B%: 15%-55%,15min), 80% purity desired compound was obtained. Finally the residue was purified by the 3rd prep-HPLC (column: Phenomenex Luna C18 150*25mm* 10µm;mobile phase: [water(0.225%FA)-ACN];B%: 17%-47%,10min). Compound 216 or 2-[6-amino-1-[(4-aminophenyl)methyl]pyrazolo[3,4-d]pyrimidin-4-yl]pyridine-4-carbonitrile (23.18 mg, 8% yield, 95.02% purity) was obtained as a yellow solid. ¹H NMR (400 MHz, DMSO-d₆) δ = 9.54 (d, *J=* 2.1 Hz, 1H), 9.22 (d, *J=* 2.0 Hz, 1H), 8.92 (t, *J=* 2.1 Hz, 1H), 8.45 (s, 1H), 7.13 (s, 2H), 6.96 (d, *J =* 8.4 Hz, 2H), 6.63 - 6.37 (m, 2H), 5.25 (s, 2H), 5.04 (s, 2H). HRMS-TOF: 343.1408.

### Example 1.58: Production of 2-(6-amino-1-(4-amino-2-fluorobenzyl)-1H-pyrazolo[3,4-d]pyrimidin-4-yl)isonicotinonitrile (compound 220)

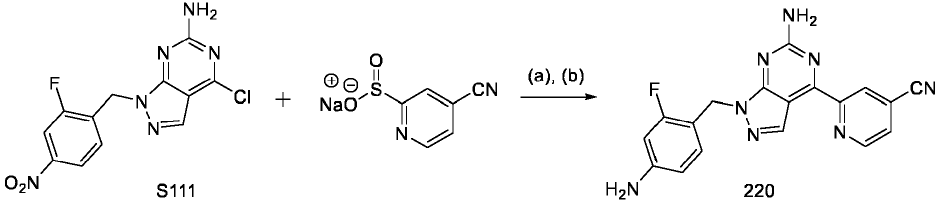

Reagents and condition: (a) Pd(OAc)₂, K₂CO₃, Cy₃P, dioxane, 120°C, 16h; (b) Fe, NH₄Cl, EtOH/H₂O, 60°C, 1h.

### Stage 1: Production of 2-(6-amino-1-(2-fluoro-4-nitrobenzyl)-1H-pyrazolo[3,4-d]pyrimidin-4-yl)isonicotinonitrile

A mixture of sodium 4-cyanopyridine-2-sulfinate (424 mg, 1.2 eq), intermediate compound S111 or 4-chloro-1-[(2-fluoro-4-nitro-phenyl)methyl]pyrazolo[3,4-d]pyrimidin-6-amine (600 mg, 1 eq), palladium acetate (41 mg, 0.1 eq), Cy₃P (104 mg, 0.2 eq) and K₂CO₃ (513 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by flash silica gel chromatography (ISCO^{®}; 12 g SepaFlash^{®} Silica flash column, eluent of 70-90% ethyl acetate/petroleum ether gradient @ 30 mL/min). Compound 2-[6-amino-1-[(2-fluoro-4-nitro-phenyl)methyl]pyrazolo[3,4-d]pyrimidin-4-yl]pyridine-4-carbonitrile (300 mg, 41% yield, 99% purity) was obtained as a yellow solid. ¹H NMR (400 MHz, DMSO-d₆) δ = 9.13 - 9.06 (m, 1H), 8.68 (dd, *J=* 0.9, 1.5 Hz, 1H), 8.55 (s, 1H), 8.16 (dd, *J* = 2.3*,* 9.9 Hz, 1H), 8.09 (dd, *J =* 1.6, 4.9 Hz, 1H), 8.03 (dd, *J* = 2.1*,* 8.5 Hz, 1H), 7.30 (t, *J =* 8.0 Hz, 1H), 7.21 (s, 2H), 5.63 (s, 2H).

### Stage 2: Production of 2-(6-amino-1-(4-amino-2-fluorobenzyl)-1H-pyrazolo[3,4-d]pyrimidin-4-yl)isonicotinonitrile (compound 220)

To a solution of 2-[6-amino-1-[(2-fluoro-4-nitro-phenyl)methyl]pyrazolo[3, 4-d]pyrimidin-4-yl]pyridine-4-carbonitrile (300 mg, 1 eq) in water (5 mL) and ethanol (15 mL) was added iron dust (214 mg, 5 eq) and NH₄Cl (328 mg, 8 eq). The mixture was stirred at 60 °C for 1 hr. The reaction mixture was concentrated under reduced pressure to remove ethanol and water to give a residue. The residue was purified by prep-HPLC (column: Welch Ultimate XB-CN 250*70*10µm;mobile phase: [Hexane-EtOH column: Welch Ultimate XB-SiOH 250*50*10µm;mobile phase: [Hexane-EtOH (0.1% NH₃·H₂O];B%: 15%-55%, 15 min (0.1% NH₃·H₂O)];B%: 30%-70%, 15 min), 90% purity desired compound was obtained after prep-HPLC, the residue was purified by 2^{nd} prep-HPLC (column: Welch Ultimate XB-CN 250*70*10µm;mobile phase: [Hexane-EtOH (0.1% ammonia hydroxide)];B%: 30%-70%, 15 min). Compound 220 or 2-[6-amino-1-[(4-amino-2-fluorophenyl)methyl]pyrazolo[3,4-d]pyrimidin-4-yl]pyridine-4-carbonitrile (33.67 mg, 89.81 µmol, 96.12% purity) was obtained as a yellow solid. ¹H NMR (400 MHz, DMSO-d₆) δ = 9.08 (dd, *J=* 0.7, 5.0 Hz, 1H), 8.68 (d, *J=* 1.3 Hz, 1H), 8.46 (s, 1H), 8.08 (dd, *J =* 1.6, 5.0 Hz, 1H), 7.13 (s, 2H), 6.88 (t, *J =* 8.4 Hz, 1H), 6.33 - 6.30 (m, 1H), 6.29 (s, 1H), 5.40 (s, 2H), 5.29 (s, 2H).

### Example 1.59: Production of 2-[6-amino-1-[(4-amino-2,6-difluorophenyl)methyl]pyrazolo[3,4-d]pyrimidin-4-yl]pyridine-4-carbonitrile (compound 224)

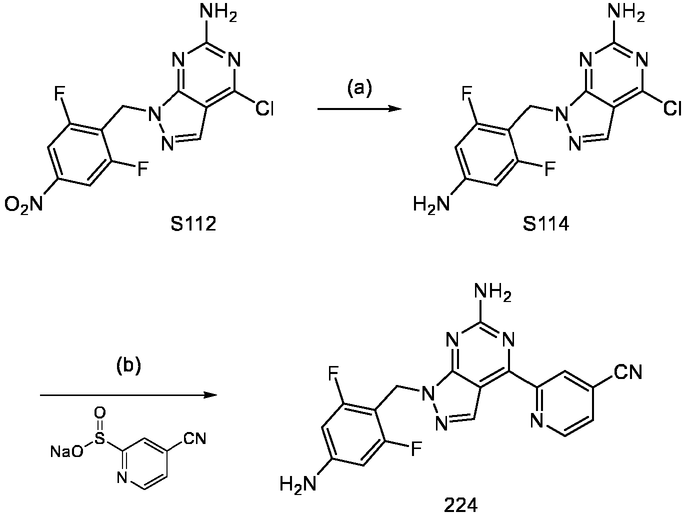

Reagents and condition: (a) Fe, NH₄Cl, EtOH/H₂O, 80°C, 3h; (b) Pd(OAc)₂, K₂CO₃, PCy₃, dioxane, 120°C, 16h.

### Stage 1: Production of 1-(4-amino-2,6-difluorobenzyl)-4-chloro-1H-pyrazolo[3,4-d]pyrimidin-6-amine (intermediate compound S114)

To the mixture of intermediate compound S112 or 4-chloro-1-[(2,6-difluoro-4-nitrophenyl)methyl]pyrazolo[3,4-d]pyrimidin-6-amine (700 mg, 1.38 mmol, 67% purity, 1 eq) in water (3 mL) and ethanol (10 mL) were added iron dust (385 mg, 6.88 mmol, 5 eq) and NH₄Cl (590 mg, 11.01 mmol, 8 eq). Then the mixture was stirred at 80 °C for 3 h. The reaction mixture was filtered and the filtrate was concentrated in vacuum. The mixture was added water (30 mL) and extracted with ethyl acetate (30 mL x 3). Combined the organic layers to dry over anhydrous sodium sulfate, filter and concentrate in vacuum. The residue was purified by prep-HPLC(column: Welch Ultimate XB-CN 250*70*10µm;mobile phase: [Hexane-EtOH (0.1% NH₃·H₂O)];B%: 20%-60%,15min). Intermediate compound S114 or 1-(4-amino-2,6-difluorobenzyl)-4-chloro-1*H*-pyrazolo[3,4-*d*]pyrimidin-6-amine (400 mg, 1.03 mmol, 75% yield, 88% purity) was obtained as a yellow solid. ¹H NMR (400 MHz, DMSO-d₆) δ = 7.92 (s, 1H), 7.32 (s, 2H), 6.25 - 6.09 (m, 2H), 5.80 (s, 2H), 5.18 (s, 2H).

### Stage 2: Production of 2-[6-amino-1-[(4-amino-2,6-difluorophenyl)methyl]pyrazolo[3,4-d]pyrimidin-4-yl]pyridine-4-carbonitrile (compound 224)

A mixture of (4-cyano-2-pyridyl)sulfinyloxy sodium (550 mg, 2.89 mmol, 2.55 eq), intermediate compounds S114 or 1-[(4-amino-2,6-difluoro-phenyl)methyl]-4-chloro-pyrazolo[3,4-d]pyrimidin-6-amine (400 mg, 1.13 mmol, 88% purity, 1 eq) , palladium acetate (25 mg, 113.30 µmol, 0.1 eq), Cy₃P (63 mg, 226.59 µmol, 73.46 µL, 0.2 eq) and K₂CO₃ (313 mg, 2.27 mmol, 2 eq) in dioxane (10 mL) was degassed and purged with nitrogen for three times, and then the mixture was stirred at 120 °C for 16hr under nitrogen atmosphere. The reaction mixture was filtered and the filtrate was concentrated in vacuum. The residue was purified by flash silica gel chromatography (20 g Sepa Flash^{®} Silica Flash Column, Eluent of 20~40% Ethyl acetate/Petroleum ether gradient @ 40 mL/min). Compound 224 or 2-[6-amino-1-[(4-amino-2,6-difluoro-phenyl)methyl]pyrazolo[3,4-d]pyrimidin-4-yl]pyridine-4-carbonitrile (70 mg, 181.87 µmol, 16% yield, 98.30% purity) was obtained as a yellow solid. ¹H NMR (400 MHz, DMSO-d₆) δ = 9.07 (dd, *J =* 0.8, 5.0 Hz, 1H), 8.67 (dd, *J =* 0.9, 1.5 Hz, 1H), 8.41 (s, 1H), 8.06 (dd, *J=* 1.7, 5.0 Hz, 1H), 7.11 (s, 2H), 6.27 - 6.11 (m, 2H), 5.79 (s, 2H), 5.26 (s, 2H).

### Example 1.60: Production of 2-(6-amino-1-(2,6-difluorobenzyl)-1H-pyrazolo[3,4-d]pyrimidin-4-yl)isonicotinonitrile (compound 232)

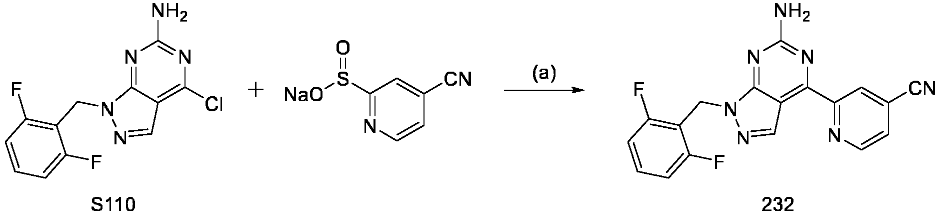

Reagents and condition: (a) Pd(OAc)₂, K₂CO₃, Cy₃P, dioxane, 120°C, 16h.

A mixture of (4-cyano-2-pyridyl)sulfinyloxy sodium (232 mg, 1.2 eq), intermediate compound S110 or 4-chloro-1-[(2,6-difluorophenyl) methyl]pyrazolo[3,4-d]pyrimidin-6-amine (300 mg, 1 eq), palladium acetate (23 mg, 0.1 eq), Cy₃P (57 mg, 0.2 eq) and K₂CO₃ (281 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by prep-HPLC (column: Unisil 3-100 C18 Ultra 150*50mm*3 µm;mobile phase: [water(0.225% FA)-ACN];B%: 43%-63%,10min). Compound 232 or 2-[6-amino-1-[(2,6-difluorophenyl)methyl]pyrazolo[3,4-d]pyrimidin-4-yl]pyridine-4-carbonitrile (66.33 mg, 18% yield, 99.34% purity) was obtained as a white solid. ¹H NMR (400 MHz, DMSO-d₆) δ = 9.06 (d, *J =* 5.0 Hz, 1H), 8.67 (s, 1H), 8.44 - 8.38 (m, 1H), 8.09 - 8.03 (m, 1H), 7.51 - 7.39 (m, 1H), 7.22 - 7.15 (m, 2H), 7.14 - 7.09 (m, 2H), 5.47 (s, 2H).

### Example 1.61: 2-(6-amino-1-(4-amino-3-methylbenzyl)-1H-pyrazolo[3,4-d]pyrimidin-4-yl)isonicotinonitrile (compound 228)

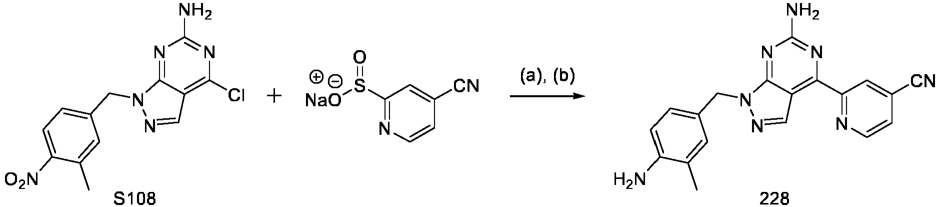

Reagents and condition: (a) Pd(OAc)₂, K₂CO₃, Cy₃P, dioxane, 120°C, 16h; (b) Fe, NH₄Cl, EtOH/H₂O, 60°C, 2h.

### Stage 1: Production of 2-(6-amino-1-(3-methyl-4-nitrobenzyl)-1H-pyrazolo[3,4-d]pyrimidin-4-yl)isonicotinonitrile

A mixture of (4-cyano-2-pyridyl)sulfinyloxy sodium (215 mg, 1.2 eq), intermediate compound S108 or 4-chloro-1-[(3-methyl-4-nitro-phenyl)methyl]pyrazolo[3,4-d]pyrimidin-6-amine (300 mg, 1 eq), palladium acetate (21 mg, 0.1 eq), Cy₃P (52 mg, 0.2 eq) and K₂CO₃ (260 mg, 2 eq) in dioxane (5 mL) was degassed and purged with nitrogen for three times, and then the mixture was stirred at 120 °C for 16hr under nitrogen atmosphere. The reaction mixture was filtered and concentrated under reduced pressure to give a residue. The residue was purified by flash silica gel chromatography (ISCO^{®}; 24 g SepaFlash^{®} Silica Flash Column, Eluent of 50~80% Ethyl acetate/Petroleum ether gradient @ 35 mL/min). Compound 2-[6-amino-1-[(3-methyl-4-nitro- phenyl)methyl]pyrazolo[3,4-d]pyrimidin-4-yl]pyridine-4-carbonitrile (200 mg, 55% yield) was obtained as a yellow solid. MS: m/z = 387.1 (M+1, ESI+).

### Stage 2: Production of 2-(6-amino-1-(4-amino-3-methylbenzyl)-1H-pyrazolo[3,4-d]pyrimidin-4-yl)isonicotinonitrile (compound 228)

To a solution of 2-[6-amino-1-[(3-methyl-4-nitro-phenyl)methyl] pyrazolo[3,4-d]pyrimidin-4-yl]pyridine-4-carbonitrile (200 mg, 1 eq) in ethanol (15 mL) and water (5 mL) was added iron dust (145 mg, 5 eq) and NH₄Cl (221 mg, 8 eq). The mixture was stirred at 60 °C for 2 hr. The reaction mixture was filtered and concentrated under reduced pressure to give a residue. The residue was purified by prep-HPLC (column: Phenomenex Gemini-NX C18 75*30mm*3µm;mobile phase: [water(10 mM NH₄HCO₃)-ACN];B%: 20%-50%,8min), 80% purity desired compound was obtained after prep-HPLC, the residue was purified by 2^{nd} prep-HPLC (column: Shim-pack C18 150*25*10µm;mobile phase: [water(0.225%FA)-ACN];B%: 16%-46%,10min). Compound 228 or 2-[6-amino-1-[(4-amino-3-methylphenyl)methyl]pyrazolo[3,4-d] pyrimidin-4-yl]pyridine-4-carbonitrile (19.65 mg, 10% yield, 96.15% purity) was obtained as a yellow solid. ¹H NMR (400 MHz, DMSO-d₆) δ = 9.08 (dd, *J=* 0.8, 4.9 Hz, 1H), 8.67 (dd, *J* = 0.9, 1.6 Hz, 1H), 8.45 (s, 1H), 8.07 (dd, *J* = 1.6*,* 5.0 Hz, 1H), 7.10 (s, 2H), 6.86 (s, 1H), 6.82 (dd, *J* = 1.9, 8.1 Hz, 1H), 6.51 (d, *J =* 8.0 Hz, 1H), 5.23 (s, 2H), 4.80 (s, 2H), 1.98 (s, 3H).

### Example 1.62: Production of 5-(6-amino-1-(4-aminobenzyl)-1H-pyrazolo[3,4-d]pyrimidin-4-yl)nicotinonitrile (compound 217)

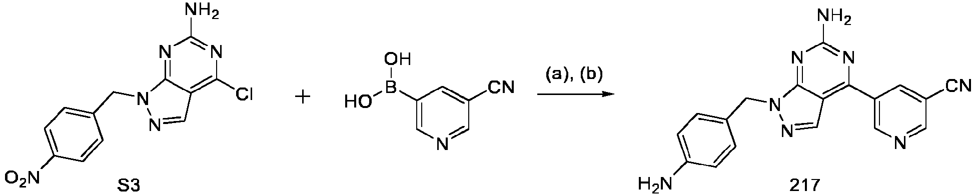

Reagents and condition: (a) Pd(dppf)Cl₂, K₂CO₃, dioxane/H₂O, 110°C, 16h; (b) Fe, NH₄Cl, THF/H₂O, 60°C, 3h.

### Stage 1: Production of 5-(6-amino-1-(4-nitrobenzyl)-1H-pyrazolo[3,4-d] pyrimidin-4-yl)nicotinonitrile

A mixture of (5-cyano-3-pyridyl)boronic acid (583 mg, 1.5 eq), intermediate compound S3 or 4-chloro-1-[(4-nitrophenyl)methyl] pyrazolo[3,4-d]pyrimidin-6-amine (800 mg, 1 eq), K₂CO₃ (726 mg, 2 eq), Pd(dppf)Cl₂ (192 mg, 0.1 eq) in dioxane (10 mL) and water (2 mL) was degassed and purged with nitrogen for three times, and then the mixture was stirred at 110 °C for 16hr under nitrogen atmosphere. The reaction mixture was filtered and concentrated under reduced pressure to give a residue. The residue was purified by flash silica gel chromatography (ISCO^{®}; 24 g Sepa Flash^{®} Silica Flash Column, Eluent of 0~100% Ethyl acetate/Petroleum ether gradient @ 30 mL/min). Compound 5-[6-amino-1-[(4-nitrophenyl)methyl]pyrazolo[3,4-d]pyrimidin-4-yl]pyridine-3-carbonitrile (800 mg, 82% yield) was obtained as a yellow solid. ¹H NMR (400 MHz, DMSO-d₆) δ = 9.63 - 9.52 (m, 1H), 9.37 - 9.31 (m, 2H), 9.25 - 9.23 (m, 1H), 9.15 - 9.12 (m, 2H), 7.46 - 7.42 (m, 2H), 5.65 - 5.60 (m, 2H). MS: m/z = 373.1 (M+1, ESI+).

### Stage 2: Production of 5-(6-amino-1-(4-aminobenzyl)-1H-pyrazolo[3,4-d] pyrimidin-4-yl)nicotinonitrile (compound 217)

To a solution of 5-[6-amino-1-[(4-nitrophenyl)methyl]pyrazolo[3,4-d] pyrimidin-4-yl]pyridine-3-carbonitrile (300 mg, 1 eq) in THF (15 mL) and water (5 mL) was added iron dust (224 mg, 5 eq) and NH₄Cl (344mg, 8 eq). The mixture was stirred at 60 °C for 3 hr. The reaction mixture was concentrated under reduced pressure to remove THF and water to give a residue. The residue was purified by prep-HPLC (column: Phenomenex Gemini-NX C18 75*30mm*3µm;mobile phase: [water (0.05% ammonia hydroxide v/v)-ACN];B%: 10%-40%,11.5min). Compound 217 or 5-[6-amino-1-[(4-aminophenyl)methyl]pyrazolo[3,4-d]pyrimidin-4-yl]pyridine-3-carbonitrile (27.63 mg, 7% yield, 98.17% purity) was obtained as a yellow solid. ¹H NMR (400 MHz, DMSO-d₆) δ = 9.54 (d, *J=* 2.1 Hz, 1H), 9.22 (d, *J =* 2.0 Hz, 1H), 8.92 (t, *J =* 2.1 Hz, 1H), 8.45 (s, 1H), 7.13 (s, 2H), 6.96 (d, *J =* 8.4 Hz, 2H), 6.63 - 6.37 (m, 2H), 5.25 (s, 2H), 5.04 (s, 2H).

### Example 1.63: Production of 5-[6-amino-1-[(4-amino-2-fluorophenyl)methyl]pyrazolo[3,4-d]pyrimidin-4-yl]pyridine-3-carbonitrile (compound 221)

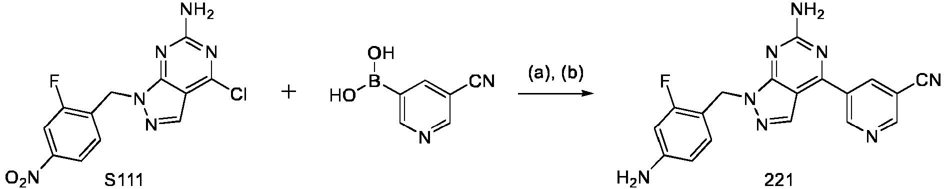

Reagents and condition: (a) Pd(dppf)Cl₂, K₂CO₃, dioxane/H₂O, 110°C, 16h; (b) Fe, NH₄Cl, EtOH/H₂O, 60°C, 2h.

### Stage 1: Production of 5-(6-amino-1-(2-fluoro-4-nitrobenzyl)-1H-pyrazolo [3,4-d]pyrimidin-4-yl)nicotinonitrile

A mixture of (5-cyano-3-pyridyl)boronic acid (343 mg, 1.5 eq), intermediate compound S111 or 4-chloro-1-[(2-fluoro-4-nitro-phenyl)methyl]pyrazolo[3,4-d]pyrimidin-6-amine (500 mg, 1 eq), K₂CO₃ (428 mg, 2 eq), Pd(dppf)Cl₂ (113 mg, 0.1 eq) in dioxane (10 mL) and water (2 mL) was degassed and purged with nitrogen for three times, and then the mixture was stirred at 110 °C for 16hr under nitrogen atmosphere. The reaction mixture was concentrated under reduced pressure to remove dioxane to give a residue. The residue was purified by flash silica gel chromatography (ISCO^{®}; 12 g SepaFlash^{®} Silica Flash Column, Eluent of 0~80% Ethyl acetate/Petroleum ether gradient @ 30mL/min). Compound 5-[6-amino-1-[(2-fluoro-4-nitro-phenyl)methyl]pyrazolo[3,4-d]pyrimidin-4-yl]pyridine-3-carbonitrile (300 mg, 50% yield) was obtained as a yellow solid. MS: m/z = 390.9 (M+1, ESI+).

### Stage 2: Production of 5-(6-amino-1-(4-amino-2-fluorobenzyl)-1H-pyrazolo [3,4-d]pyrimidin-4-yl)nicotinonitrile (compound 221)

To a solution of 5-[6-amino-1-[(2-fluoro-4-nitro-phenyl)methyl]pyrazolo[3,4-d]pyrimidin-4-yl]pyridine-3-carbonitrile (300 mg, 1 eq) in ethanol (15 mL) and water (5 mL) was added iron dust (214 mg, 5 eq) and NH₄Cl (328 mg, 8 eq). The mixture was stirred at 60 °C for 2 hr. The reaction mixture was filtered and concentrated under reduced pressure to give a residue. The residue was purified by prep-HPLC (column: Phenomenex Gemini-NX C18 75*30mm*3µm;mobile phase: [water (0.05% ammonia hydroxide v/v)-ACN];B%: 13%-43%,11.5min). Compound 221 or 5-[6-amino-1-[(4-amino-2-fluorophenyl)methyl]pyrazolo[3,4-d]pyrimidin-4-yl]pyridine-3-carbonitrile (37.61 mg, 13% yield, 94.57% purity) was obtained as a yellow solid. ¹H NMR (400 MHz, DMSO-d₆) δ = 9.54 (d, *J=* 2.1 Hz, 1H), 9.23 (d, *J=* 2.0 Hz, 1H), 8.91 (t, *J=* 2.0 Hz, 1H), 8.45 (s, 1H), 7.14 (s, 2H), 6.90 (t, *J =* 8.4 Hz, 1H), 6.31 (d, *J =* 10.8 Hz, 2H), 5.41 (s, 2H), 5.29 (s, 2H).

### Example 1.64: Production of 5-[6-amino-1-[(4-amino-2,6-difluorophenyl)methyl]pyrazolo[3,4-d]pyrimidin-4-yl]pyridine-3-carbonitrile (compound 225)

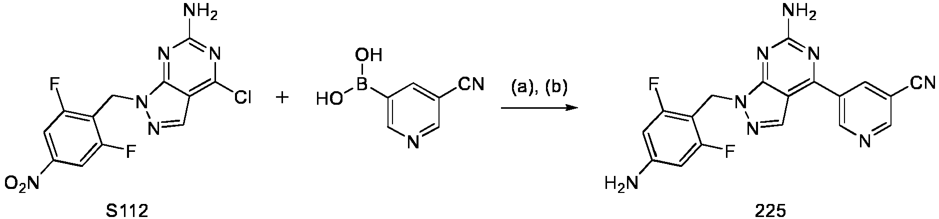

Reagents and conditions: (a) Pd(dppf)Cl₂, K₂CO₃, dioxane/H₂O, 100°C, 15h; (b) Fe, NH₄Cl, EtOH/H₂O, 80°C, 2h.

### Stage 1: Production of 5-[6-amino-1-[(2,6-difluoro-4-nitro-phenyl)methyl] pyrazolo[3,4-d]pyrimidin-4-yl]pyridine-3-carbonitrile

To the solution of intermediate compound S112 or 4-chloro-1-[(2,6-difluoro-4-nitro-phenyl)methyl]pyrazolo [3,4-d]pyrimidin-6-amine (400 mg, 1.17 mmol, 1 eq) in dioxane (10 mL) and water (2 mL) were added (5-cyano-3-pyridyl)boronic acid (346 mg, 2.34 mmol, 2 eq), Pd(dppf)Cl₂ (86 mg, 117.00 µmol, 0.1 eq) and K₂CO₃ (323 mg, 2.34 mmol, 2 eq). Then the mixture was stirred at 100 °C for 15 h. The mixture was concentrated in vacuum to give a residue. The residue was purified by flash silica gel chromatography (20 g Sepa Flash^{®} Silica Flash Column, Eluent of 0~60% Ethyl acetate/Petroleum ether gradient @ 50 mL/min). Compound 5-[6-amino- 1-[(2,6-difluoro-4-nitro-phenyl)methyl]pyrazolo[3,4-d]pyrimidin-4-yl]pyridine-3-carbonitrile (400 mg, 852.27 µmol, 73% yield, 87% purity) was obtained as a yellow solid. ¹H NMR (400 MHz, DMSO-d₆) δ = 9.51 (d, *J =* 2.0 Hz, 1H), 9.21 (d, *J=* 1.9 Hz, 1H), 8.89 (t, *J =* 2.1 Hz, 1H), 8.44 (s, 1H), 8.09 (d, *J =* 7.3 Hz, 2H), 7.23 (s, 2H), 5.55 (s, 2H). MS: m/z = 409.0 (M+1, ESI+).

### Stage 2: Production of 5-[6-amino-1-[(4-amino-2,6-difluoro-phenyl) methyl]pyrazolo[3,4-d]pyrimidin-4-yl]pyridine-3-carbonitrile (compound 225)

To the mixture of 5-[6-amino-1-[(2,6-difluoro-4-nitro-phenyl)methyl] pyrazolo[3,4-d]pyrimidin-4-yl]pyridine-3-carbonitrile (400 mg, 979.62 µmol, 1 eq) in water (3 mL) and ethanol (9 mL) were added iron dust (274 mg, 4.90 mmol, 5 eq) and NH₄Cl (420 mg, 7.84 mmol, 8 eq). Then the mixture was stirred at 80 °C for 2 h. The reaction mixture was filtered and the filtrate was concentrated in vacuum. Then the mixture was added water (30 mL) and then extracted with ethyl acetate (30 mL x 3). Combined the organic phase to dry over anhydrous sodium sulfate, filter and concentrate in vacuum to give a residue. The residue was purified by prep-HPLC(column: Welch Ultimate XB-CN 250*70*10µm;mobile phase: [Hexane-EtOH (0.1% NH₃·H₂O)];B%: 30%-70%, 15min) to give a impure product. The impure product was re-purified by prep-HPLC(column: Welch Ultimate XB-CN 250*50*10µm;mobile phase: [Hexane-EtOH (0.1% NH₃·H₂O)];B%: 25%-65%,15min). Compound 225 or 5-[6-amino-1-[(4-amino-2,6-difluoro-phenyl)methyl]pyrazolo[3,4-d]pyrimidin-4-yl]pyridine-3-carbonitrile (113.05 mg, 297.07 µmol, 30% yield, 99.42% purity) was obtained as a yellow solid. ¹H NMR (400 MHz, DMSO-d₆) δ = 9.52 (d, *J* = 2.1 Hz, 1H), 9.21 (d, *J* = 1.8 Hz, 1H), 8.89 (t, *J =* 2.1 Hz, 1H), 8.38 (s, 1H), 7.13 (s, 2H), 6.18 (d, *J=* 10.1 Hz, 2H), 5.25 (s, 2H).

### Example 1.65: Production of 5-(6-amino-1-(2,6-difluorobenzyl)-1H-pyrazolo[3,4-d]pyrimidin-4-yl)nicotinonitrile (compound 233)

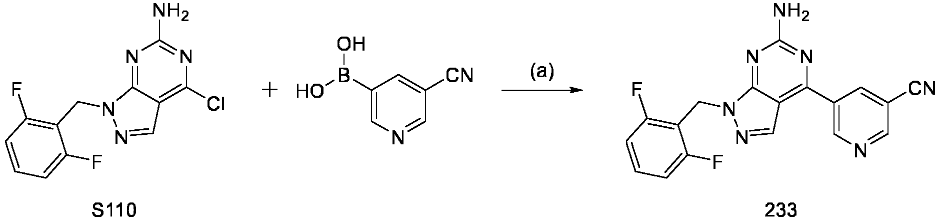

### Reagents and condition: (a) Pd(dppf)Cl₂, K₂CO₃, dioxane/H₂O, 110°C, 16h.

A mixture of intermediate compound S110 or 4-chloro-1-[(2,6-difluorophenyl)methyl]pyrazolo[3,4-d]pyrimidin-6-amine (400 mg, 1 eq), (5-cyano-3-pyridyl)boronic acid (300 mg, 1.5 eq), K₂CO₃ (373 mg, 2 eq), Pd(dppf)Cl₂ (99 mg, 0.1 eq) in dioxane (10 mL) and water (2 mL) was degassed and purged with nitrogen for three times, and then the mixture was stirred at 110 °C for 16hr under nitrogen atmosphere. The reaction mixture was concentrated under reduced pressure to remove dioxane to give a residue. The residue was purified by prep-HPLC (column: Unisil 3-100 C18 Ultra 150*50mm*3µm; mobile phase: [water(0.225%FA)-ACN];B%: 45%-65%,10min), 80% purity desired compound was obtained, then it was purified by second prep-HPLC (column: Phenomenex Luna C18 150*25mm* 10µm;mobile phase: [water(0.225%FA)-ACN];B%: 31%-61%,10min). Compound 233 or 5-[6-amino-1-[(2,6-difluorophenyl)methyl]pyrazolo[3,4-d]pyrimidin-4-yl]pyridine-3-carbonitrile (69.15 mg, 14% yield, 97.76% purity) was obtained as a yellow solid. ¹H NMR (400 MHz, DMSO-d₆) δ = 8.96 (d, *J =* 5.1 Hz, 1H), 8.58 (d, *J =* 0.6 Hz, 1H), 8.41 - 8.39 (m, 1H), 8.39 - 8.36 (m, 1H), 7.51 - 7.41 (m, 1H), 7.25 (s, 2H), 7.17 - 7.08 (m, 2H), 5.47 (s, 2H).

### Example 1.66: Production of 5-(6-amino-1-(4-amino-3-methylbenzyl)-1H-pyrazolo[3,4-d]pyrimidin-4-yl)nicotinonitrile (compound 229)

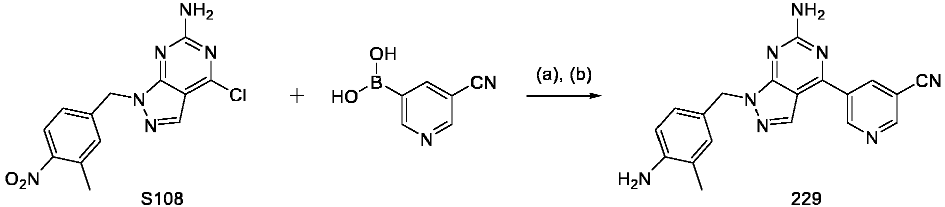

Reagents and condition: (a) Pd(dppf)Cl₂, K₂CO₃, dioxane, 110°C, 16h; (b) Fe, NH₄Cl, EtOH/H₂O, 60°C, 2h.

### Stage 1: Production of 5-(6-amino-1-(3-methyl-4-nitrobenzyl)-1H-pyrazolo [3,4-d]pyrimidin-4-yl)nicotinonitrile

A mixture of (5-cyano-3-pyridyl)boronic acid (208 mg, 1.5 eq), intermediate compound S108 or 4-chloro-1-[(3-methyl-4-nitro-phenyl)methyl] pyrazolo[3,4-d]pyrimidin-6-amine (300 mg, 1 eq), K₂CO₃ (260 mg, 2 eq), Pd(dppf)Cl₂ (68.87 mg, 0.1 eq) in dioxane (10 mL) was degassed and purged with nitrogen for three times, and then the mixture was stirred at 110 °C for 16hr under nitrogen atmosphere. The reaction mixture was filtered and concentrated under reduced pressure to give a residue. The residue was purified by flash silica gel chromatography (ISCO^{®}; 24 g SepaFlash^{®} Silica Flash Column, Eluent of 0~100% Ethyl acetate/Petroleum ether gradient @ 35 mL/min). Compound 5-[6-amino-1-[(3-methyl-4-nitro-phenyl)methyl]pyrazolo[3,4-d]pyrimidin-4-yl]pyridine-3-carbonitrile (300 mg, 82% yield) was obtained as a yellow solid. MS: m/z = 386.9 (M+1, ESI+).

### Stage 2: Production of 5-(6-amino-1-(4-amino-3-methylbenzyl)-1H-pyrazolo [3,4-d]pyrimidin-4-yl)nicotinonitrile (compound 229)

To a solution of 5-[6-amino-1-[(3-methyl-4-nitro-phenyl)methyl]pyrazolo[3,4-d]pyrimidin-4-yl]pyridine-3-carbonitrile (400 mg, 1 eq) in ethanol (15 mL) and water (5 mL) was added iron dust (289 mg, 5 eq) and NH₄Cl (443 mg, 8 eq). The mixture was stirred at 60 °C for 2 hr. The reaction mixture was filtered and concentrated under reduced pressure to give a residue. The residue was purified by prep-HPLC (column: Shim-pack C18 150*25*10µm;mobile phase: [water(0.225%FA)-ACN];B%: 12%-42%,10min), 92% purity desired compound was obtained, then the mixture was purified by 2^{nd} prep-HPLC (column: Phenomenex Gemini-NX C18 75*30mm*3µm;mobile phase: [water (0.05% ammonia hydroxide v/v)-ACN];B%: 14%-44%,11.5min). Compound 229 or 5-[6-amino-1-[(4-amino-3-methyl-phenyl)methyl]pyrazolo[3,4-d]pyrimidin-4-yl]pyridine-3-carbonitrile (58.12 mg, 15% yield, 97.57% purity) was obtained as a yellow solid. ¹H NMR (400 MHz, DMSO-d₆) δ = 9.53 (d, *J=* 2.1 Hz, 1H), 9.22 (d, *J* = 2.1 Hz, 1H), 8.91 (t, *J =* 2.1 Hz, 1H), 8.43 (s, 1H), 7.12 (s, 2H), 6.91 - 6.77 (m, 2H), 6.52 (d, *J =* 8.1 Hz, 1H), 5.23 (s, 2H), 4.80 (s, 2H), 1.99 (s, 3H).

### Example 1.67: Production of 4-(6-amino-1-(4-aminobenzyl)-1H-pyrazolo[3,4-d]pyrimidin-4-yl)picolinonitrile (compound 218)

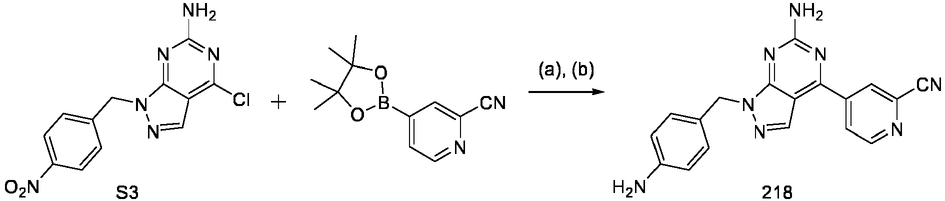

Reagents and condition: (a) Pd(dppf)Cl₂, K₂CO₃, dioxane/H₂O, 110°C, 16h; (b) Fe, NH₄Cl, EtOH/H₂O, 60°C, 2h.

### Stage 1: Production of 4-(6-amino-1-(4-nitrobenzyl)-1H-pyrazolo[3,4-d]pyrimidin-4-yl)picolinonitrile

A mixture of intermediate compound S3 or 4-chloro-1-[(4-nitrophenyl)methyl]pyrazolo[3,4-d]pyrimidin- 6-amine (800 mg, 1 eq), 4-(4,4,5,5-tetramethyl-1,3,2-dioxaborolan-2-yl)pyridine-2-carbonitrile (906 mg, 1.5 eq), K₂CO₃ (725 mg, 2 eq), Pd(dppf)Cl₂ (192 mg, 0.1 eq) in dioxane (10 mL) and water (2 mL) was degassed and purged with nitrogen for three times, and then the mixture was stirred at 110 °C for 16hr under nitrogen atmosphere. The reaction mixture was filtered and concentrated under reduced pressure to give a residue. The residue was purified by flash silica gel chromatography (ISCO^{®}; 12 g Sepa Flash^{®} Silica Flash Column, Eluent of 30~80% Ethyl acetate/Petroleum ether gradient @ 35 mL/min). Compound 4-[6-amino-1-[(4-nitrophenyl)methyl]pyrazolo[3,4-d]pyrimidin-4-yl]pyridine-2-carbonitrile (500 mg, 51% yield) was obtained as a yellow solid. ¹H NMR (400 MHz, DMSO-d₆) δ = 9.05 - 8.94 (m, 1H), 8.84 - 8.71 (m, 2H), 8.58 (s, 1H), 8.43 (dd, *J=* 1.8, 5.1 Hz, 1H), 8.09 - 8.07 (m, 1H), 7.46 - 7.41 (m, 2H), 7.31 - 7.24 (m, 2H), 5.67 - 5.61 (m, 2H). MS: m/z = 372.9 (M+1, ESI+).

### Stage 2: Production of 4-(6-amino-1-(4-aminobenzyl)-1H-pyrazolo[3,4-d] pyrimidin-4-yl)picolinonitrile (compound 218)

To a solution of 4-[6-amino-1-[(4-nitrophenyl)methyl]pyrazolo[3,4-d] pyrimidin-4-yl]pyridine-2-carbonitrile (300 mg, 1 eq) in water (5 mL) and ethanol (15 mL) was added iron dust (224 mg, 5 eq) and NH₄Cl (344 mg, 8 eq). The mixture was stirred at 60 °C for 2 hr. After that, the reaction mixture was concentrated under reduced pressure to remove ethanol and water to give a residue. The residue was purified by prep-HPLC (column: Phenomenex Gemini-NX C18 75*30mm*3µm;mobile phase: [water (0.05% ammonia hydroxide v/v)-ACN];B%: 12%-42%,11.5min), 80% purity desired compound was obtained, then the residue was purified by the 2^{nd} prep-HPLC (column: Phenomenex Gemini-NX C18 75*30mm*3µm;mobile phase: [water(10mM NH₄HCO₃)-ACN];B%: 16%-46%,8min). Compound 218 or 4-[6-amino-1-[(4- aminophenyl)methyl]pyrazolo[3,4-d]pyrimidin-4-yl]pyridine-2-carbonitrile (52.04 mg, 18% yield, 95.28% purity) was obtained as a white solid. ¹H NMR (400 MHz, DMSO-d₆) δ = 8.97 (d, *J =* 5.1 Hz, 1H), 8.60 (d, *J =* 0.7 Hz, 1H), 8.44 (s, 1H), 8.40 (dd, *J=* 1.6, 5.1 Hz, 1H), 7.18 (s, 2H), 6.96 (d, *J =* 8.3 Hz, 2H), 6.48 (d, *J=* 8.4 Hz, 2H), 5.25 (s, 2H), 5.04 (s, 2H).

### Example 1.68: Production of 4-(6-amino-1-(4-amino-2-fluorobenzyl)-1H-pyrazolo[3,4-d]pyrimidin-4-yl)picolinonitrile (compound 222)

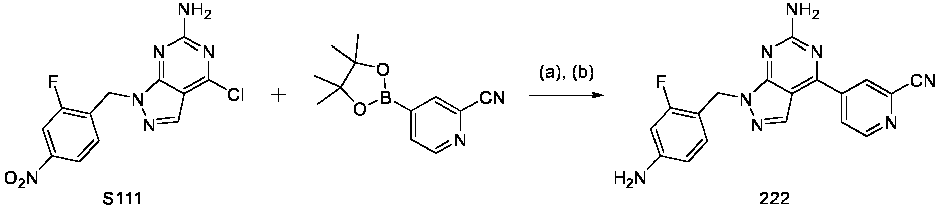

Reagents and condition: (a) Pd(dppf)Cl₂, K₂CO₃, dioxane/H₂O, 110°C, 16h; (b) Fe, NH₄Cl, EtOH/H₂O, 60°C, 2h.

### Stage 1: Production of 4-(6-amino-1-(2-fluoro-4-nitrobenzyl)-1H-pyrazolo [3,4-d]pyrimidin-4-yl)picolinonitrile

A mixture of intermediate compound S111 or 4-chloro-1-[(2-fluoro-4-nitrophenyl)methyl]pyrazolo [3,4-d]pyrimidin-6-amine (1 g, 1 eq), 4-(4,4,5,5-tetramethyl-1,3,2-dioxaborolan-2-yl)pyridine-2-carbonitrile (784 mg, 1.1 eq), K₂CO₃ (856 mg, 2 eq), Pd(dppf)Cl₂ (226 mg, 0.1 eq) in dioxane (10 mL) and water (2 mL) was degassed and purged with nitrogen for three times, and then the mixture was stirred at 110 °C for 16hr under nitrogen atmosphere. The reaction mixture was concentrated under reduced pressure to remove dioxane to give a residue. The residue was purified by flash silica gel chromatography (ISCO^{®}; 12 g SepaFlash^{®} Silica Flash Column, Eluent of 0~80% Ethyl acetate/Petroleum ether gradient @ 35 mL/min). Compound 4-[6-amino-1-[(2-fluoro-4-nitrophenyl) methyl]pyrazolo[3,4-d]pyrimidin-4-yl]pyridine-2-carbonitrile (1 g, 83% yield) was obtained as a yellow solid. ¹H NMR (400 MHz, DMSO-d₆) δ = 8.98 (dd, *J =* 0.7, 5.1 Hz, 1H), 8.65 - 8.59 (m, 2H), 8.56 (s, 1H), 8.41 (dd, *J =* 1.7, 5.1 Hz, 1H), 7.61 - 7.54 (m, 1H), 7.34 (t, *J=* 8.0 Hz, 1H), 7.31 - 7.23 (m, 2H), 5.63 (s, 2H). MS: m/z = 391.1 (M+1, ESI+).

### Stage 2: Production of 4-(6-amino-1-(4-amino-2-fluorobenzyl)-1H-pyrazolo [3,4-d]pyrimidin-4-yl)picolinonitrile (compound 222)

To a solution of 4-[6-amino-1-[(2-fluoro-4-nitro-phenyl)methyl]pyrazolo [3,4-d]pyrimidin-4-yl]pyridine-2-carbonitrile (500 mg, 1 eq) in ethanol (15 mL) and water (5 mL) was added iron dust (357 mg, 5 eq) and NH₄Cl (548 mg, 8 eq) . The mixture was stirred at 60 °C for 2 hr. The reaction mixture was filtered and concentrated under reduced pressure to give a residue. The residue was purified by prep-HPLC (column: Phenomenex Gemini-NX C18 75*30mm*3µm;mobile phase: [water (0.05% ammonia hydroxide v/v)-ACN];B%: 15%-45%,11.5min), 79% purity desired compound obtained, the residue was purified by the 2^{nd} prep-HPLC (column: Phenomenex Gemini-NX C18 75*30mm*3µm;mobile phase: [water(10mM NH₄HCO₃)-ACN];B%: 18%-48%,8min), 81% purity desired compound obtained. Finally the residue was purified by the 3^{rd} prep-HPLC (column: Phenomenex Gemini-NX C18 75*30mm*3µm;mobile phase: [water (0.05% ammonia hydroxide v/v)-ACN];B%: 16%-46%,11.5min). Compound 222 or 4-[6-amino-1-[(4-amino-2-fluoro-phenyl) methyl]pyrazolo[3,4-d]pyrimidin-4-yl]pyridine-2-carbonitrile (31.41 mg, 95.76% purity) was obtained as a yellow solid. ¹H NMR (400 MHz, DMSO-d₆) δ = 8.97 (dd, *J=* 0.8, 5.1 Hz, 1H), 8.60 (dd, *J =* 0.8, 1.7 Hz, 1H), 8.44 (s, 1H), 8.40 (dd, *J =* 1.8, 5.1 Hz, 1H), 7.19 (s, 2H), 6.89 (t, *J =* 8.4 Hz, 1H), 6.37 - 6.24 (m, 2H), 5.46 - 5.37 (m, 2H), 5.32 - 5.22 (m, 2H).

### Example 1.69: Production of 2-[6-amino-1-[[4-amino-3-(trifluoromethyl)phenyl]methyl]pyrazolo[3,4-d]pyrimidin-4-yl]pyridine-4-carbonitrile (compound 235)

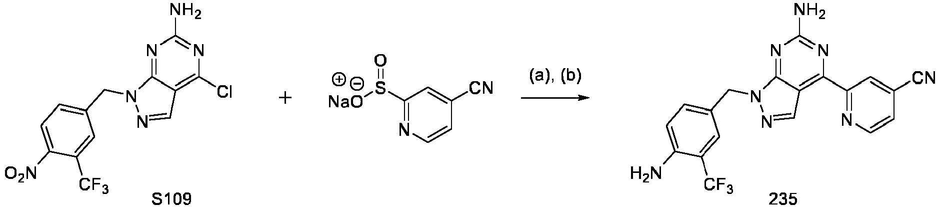

Reagents and condition: (a) Pd(OAc)₂, Cy₃P, K₂CO₃, dioxane, 120°C, 16h; (b) Fe, NH₄Cl, EtOH/H₂O, 60 °C, 8h.

### Stage 1: Production of 2-[6-amino-1-[[4-nitro-3-(trifluoromethyl)phenyl]methyl]pyrazolo[3,4-d]pyrimidin-4-yl]pyridine-4-carbonitrile

A mixture of sodium 4-cyanopyridine-2-sulfinate (420 mg, 2.21 mmol, 2 eq), intermediate compound S109 or 4-chloro-1-[[4-nitro-3-(trifluoromethyl)phenyl]methyl]pyrazolo[3,4-d]pyrimidin-6-amine (435 mg, 1.10 mmol, 94.74% purity, 1 eq), palladium acetate (25 mg, 110.49 µmol, 0.1 eq), Cy₃P (62 mg, 220.98 µmol, 71.64 µL, 0.2 eq) and K₂CO₃ (305 mg, 2.21 mmol, 2 eq) in dioxane (10 mL) was degassed and purged with nitrogen for three times, and then the mixture was stirred at 120 °C for 16hr under nitrogen atmosphere. The mixture was concentrated in vacuum to give a residue. The residue was purified by flash silica gel chromatography (12 g SepaFlash^{®} Silica Flash Column, Eluent of 30~60% Ethyl acetate/Petroleum ether gradient @ 40 mL/min). Compound 2-[6-amino-1-[[4-nitro-3-(trifluoromethyl)phenyl]methyl]pyrazolo[3,4-d]pyrimidin-4-yl]pyridine-4-carbonitrile (160 mg, 354.13 µmol, 32% yield, 97.46% purity) was obtained as a yellow solid. ¹H NMR (400 MHz, DMSO-d₆) δ = 9.09 (d, *J=* 4.9 Hz, 1H), 8.68 (s, 1H), 8.57 (d, *J =* 1.0 Hz, 1H), 8.17 - 8.04 (m, 2H), 7.96 (s, 1H), 7.60 (dd, *J =* 1.2, 8.4 Hz, 1H), 7.20 (s, 2H), 5.69 (s, 2H). MS: m/z = 441.1 (M+1, ESI+).

### Stage 2: Production of 2-[6-amino-1-[[4-amino-3-(trifluoromethyl)phenyl]methyl]pyrazolo[3,4-d]pyrimidin-4-yl]pyridine-4-carbonitrile (compound 235)

To the solution of 2-[6-amino-1-[[4-nitro-3-(trifluoromethyl)phenyl]methyl]pyrazolo[3,4-d]pyrimidin-4-yl]pyridine-4-carbonitrile (150 mg, 340.65 µmol, 1 eq) in ethanol (10 mL) and water (3 mL) was added iron dust (95 mg, 1.70 mmol, 5 eq) and NH₄Cl (146 mg, 2.73 mmol, 8 eq). Then the mixture was stirred at 60 °C for 8 h. The reaction mixture was filtered, to the filtrate was added saturated NaHCO₃ solution (50 mL) and extracted with ethyl acetate (80 mL x 3). Combined the organic phase to dry over anhydrous sodium sulfate, filter and concentrate in vacuum to give a residue. The residue was purified by prep-HPLC(column: Welch Ultimate XB-CN 250*70*10µm;mobile phase: [Hexane-ethanol (0.1% ammonia hydroxide)];B%: 20%-60%,15min). Compound 235 or 2-[6-amino-1-[[4-amino-3-(trifluoromethyl)phenyl]methyl]pyrazolo[3,4-d]pyrimidin-4-yl]pyridine-4-carbonitrile (76.29 mg, 180.52 µmol, 53% yield, 97.10% purity) was obtained as a yellow solid. ¹H NMR (400 MHz, DMSO-d₆) δ = 9.08 (dd, *J=* 0.7, 5.0 Hz, 1H), 8.67 (d, *J =* 0.9 Hz, 1H), 8.47 (s, 1H), 8.07 (dd, *J=* 1.6, 5.0 Hz, 1H), 7.28 (d, *J =* 1.6 Hz, 1H), 7.18 (dd, *J=* 1.3, 8.6 Hz, 1H), 7.14 (s, 2H), 6.77 (d, *J =* 8.4 Hz, 1H), 5.59 (s, 2H), 5.31 (s, 2H).

### Example 2: Assessment of purine compounds

### 2.1 A2A Receptor (A2AR) SPA binding assay

### General Method

Test compound is weighed, then dissolved in 100% DMSO to prepare 20 mM stock solution. Dilute the stock solution with DMSO into 100X final concentration, and then use this DMSO solution to perform 3-fold serial dilution for totally 12 concentrations with DMSO. 10-fold diluting the serial dilutions with assay buffer to the working concentrations. Compound IC₅₀ values are determined in an A2AR (Human recombinant adenosine A2A receptor from HKE293 cells) SPA (scintillation proximity assay) binding assay with [3H] SCH58261 as the radioligand. The assay buffer is prepared with DPBS containing 10 mM MgCl₂. 5 µL of compound working solutions (a total of 12 concentrations starting from 1 or 3 µM, 1/3 dilution) is transferred to the wells by the 12-channel pipette. A2AR membrane is pre-incubated with adenosine deaminase for 15 min at room temperature. Then SPA beads are added and the membrane-bead suspension mixture is incubated for 30 min at room temperature with the rotary mixer. The reaction is started by the addition to each well of 25 µL of membrane-bead suspension containing 0.04 mg/mL A2aR membrane, 0.024 mg/mL adenosine deaminase and 4 mg/mL SPA beads, and 20 µL of the 7.5 nM [3H] SCH58261 radioligand solution. Final concentration of DMSO in the A2AR SPA binding assay is 1%. Sealing the plates and incubate for 1 hr at 27°C with vigorous mixing on a plate shaker. After 1 hr, centrifuge the plate (1000rpm, 1 min), and settle the plate for 5 min prior to counting. The signal of radiation is measured by the Microbeta2. The readout of the signal is used to calculate compound IC₅₀ values. Non-specific binding refers to the wells containing reaction system with the presence of 100 µM SCH58261. The dose-response curve is fitted with GraphPad Prism 5 and IC₅₀ values are calculated by log(inhibitor) vs. response -Variable slope program with the following formula: $Y = Bottom + \left(Top-Bottom\right) / \left(1+{10}^{∧}\left(\left({LogIC}_{50}-X\right)*Hill Slope\right)\right)$ ${K}_{i} = {IC}_{50} / \left(1+radio ligand concentration used in assay/radio ligand {K}_{d}\right)$

The binding affinity of selected compounds against A2A receptor was assessed using the SPA binding assay described above. The binding affinity of reference compounds Taminadenant (CAS No. 1337962-47-6) and AZD4635 (CAS No. 1321514-06-0) was also measured in this assay.

**Table 2: Binding affinity of exemplary compounds against human A2A receptor**

| A : Kᵢ ≤ 10nM | |
|---|---|
| B : 10nM < Kᵢ ≤ 100 nM | |
| C : 100nM < Kᵢ ≤ 500nM | |
| D : Kᵢ > 500nM | |
| **Table 2** | |
| **Compd. No.** | **human A2A receptor Kᵢ** |
| 101 | B |
| 102 | C |
| 103 | B |
| 104 | A |
| 105 | D |
| 106 | B |
| 107 | A |
| 108 | B |
| 109 | A |
| 110 | A |
| 111 | B |
| 112 | A |
| 113 | B |
| 114 | B |
| 118 | D |
| 119 | C |
| 120 | A |
| 124 | A |
| 125 | C |
| 126 | B |
| 128 | A |
| 132 | A |
| 134 | B |
| Taminadenant | B |
| AZD4635 | A |

Several tested compounds bound with high affinity to human A2A receptor and were superior to Taminadenant

### 2.2 Human adenosine A1 Receptor binding assay

### General Method

Test compound is weighed, dissolved in DMSO to make a stock solution of 10 mM, diluted with DMSO to prepare working solutions, then 100-fold diluted to the indicated concentrations. Human recombinant adenosine A₁ receptors expressed in CHO-K1 cells were used to prepare membranes in incubation buffer (20 mM HEPES (4-(2-hydroxyethyl)-1-piperazineethanesulfonic acid) pH 7.4, 10 mM MgCl₂, 100 mM NaCl). Test compound or vehicle (2.2 µL) was added in 200 µL of membranes and incubated with 20 µL of 1 nM [³H] DPCPX (8-cyclopentyl-1,3-dipropylxanthine) for 90 minutes at 25°C. Non-specific binding was estimated in the presence of 100 µM R(-)-PIA ((*R*)-*N*-(1-methyl-2-phenylethyl)adenosine). The incubation was stopped by vacuum filtration onto 0.3 % PEI (polyethylenimine) presoaked GF/B filters using a harvester followed by four washes with ice-cold 50 mM Tris-HCl, pH 7.4, and the radioactivity on GF/B filtermats counted in a scintillation counter (PerkinElmer Topcount^{™}) to determine [³H] DPCPX specifically bound. Final concentration of vehicle DMSO is 1%. Data is fitted using the non-linear curve fitting routines in Meth-IQ software (ID Business Solutions Ltd., UK). IC₅₀ is converted to Kᵢ by Cheng-Prusoff equation: ${K}_{i} = {IC}_{50} / \left(1+\left[L\right]/{K}_{D}\right)$ where [L] is concentration of radiolabeled ligand used in the assay.

The binding affinity of selected compounds against A1 receptor was assessed using the binding assay described above.

**Table 3: Binding affinity of exemplary compounds against human A1 receptor**

| A : Kᵢ > 1000nM | |
|---|---|
| B : 300nM < Kᵢ ≤ 1000nM | |
| C: Kᵢ ≤ 300nM | |
| **Table 3** | |
| **Compd. No.** | **human A1 receptor Kᵢ** |
| 101 | A |
| 103 | A |
| 104 | A |
| 106 | A |
| 107 | C |
| 108 | B |
| 109 | B |
| 110 | B |
| 111 | A |
| 112 | C |
| 113 | B |
| 114 | A |
| 120 | B |
| 124 | C |
| 128 | B |
| 132 | C |

Several tested compounds bound with low affinity to human A1 receptor. This result indicates that some compounds of this disclosure exhibit selectivity for A2A receptor over human A1 receptor.

### Example 3: Assessment of purine compounds using a human A2A functional assay

### General Method

cAMP assay was performed on human recombinant A2AR stable cell line (HEK293). The cells were cultured at 37 °C and 5% CO₂ with culture medium (90% DMEM+10% FBS+500µg/mL G418). The compounds and reference compound ZM241385 were diluted in 3-fold started from high concentration. Then, 100 nL compounds were transferred by Echo machine with 12 doses respectively. Cells were collected with stimulation buffer (1×HBSS with 5 mM HEPES+0.05 mM IBMX+0.1% BSA) and plated the cells at the density of 5,000 cells/well, centrifuged at 600 rpm for 3 min and then incubation with compounds at room temperature for 60 min. After that, 5µL 4X Eu-cAMP tracer solution and 5µL 4X ULight^{™}-anti-cAMP solution were added into the cells, centrifuged at 600 rpm for 3 min and incubated for 60 min. cAMP signal was detected with Envision. GraphPad Prism (version 6.0) was used for the data analysis.

The A2A receptor antagonistic activity of selected compounds was assessed using the cAMP assay described above. The antagonistic activity of reference compounds Taminadenant (CAS No. 1337962-47-6) and AZD4635 (CAS No. 1321514-06-0) was also measured in this assay.

**Table 4: A2A Receptor antagonistic activity of exemplary compounds in a hA2A functional assay.**

| A : IC₅₀ ≤ 300nM | |
|---|---|
| B : 300nM < IC₅₀ ≤ 1000nM | |
| C : IC₅₀ > 1000nM | |
| **Table 4** | |
| **Compd. No.** | **cAMP IC₅₀** |
| 101 | C |
| 103 | C |
| 104 | A |
| 107 | A |
| 108 | B |
| 109 | A |
| 110 | A |
| 111 | C |
| 112 | A |
| 113 | B |
| 120 | A |
| 124 | A |
| 128 | B |
| 132 | A |
| Taminadenant | B |
| AZD4635 | A |

Several compounds of this disclosure showed strong antagonistic activity against human A2A receptor and were superior to Taminadenant

### Example 4: Assessment of activity of pyrazolo-pyrimidine compounds

### 4.1 Human adenosine A1 receptor binding assay

### General Method

Test compound is weighed, dissolved in DMSO to make a stock solution of 10 mM, diluted with DMSO to prepare working solutions, then 100-fold diluted to the indicated concentrations. Human recombinant adenosine A₁ receptors expressed in CHO-K1 cells were used to prepare membranes in incubation buffer (20 mM HEPES (4-(2-hydroxyethyl)-1-piperazineethanesulfonic acid) pH 7.4, 10 mM MgCl₂, 100 mM NaCl). Test compound or vehicle (2.2 µL) was added in 200 µL of membranes and incubated with 20 µL of 1 nM [³H] DPCPX (8-cyclopentyl-1,3-dipropylxanthine) for 90 minutes at 25°C. Non-specific binding was estimated in the presence of 100 µM R(-)-PIA ((*R*)-*N*-(1-methyl-2-phenylethyl)adenosine). The incubation was stopped by vacuum filtration onto 0.3 % PEI (polyethylenimine) presoaked GF/B filters using a harvester followed by four washes with ice-cold 50 mM Tris-HCl, pH 7.4, and the radioactivity on GF/B filtermats counted in a scintillation counter (PerkinElmer Topcount^{™}) to determine [³H] DPCPX specifically bound. Final concentration of vehicle DMSO is 1%.

Data is fitted using the non-linear curve fitting routines in Meth-IQ software (ID Business Solutions Ltd., UK). IC₅₀ is converted to Kᵢ by Cheng-Prusoff equation:${K}_{i} = {IC}_{50} / \left(1+\left[L\right]{/ K}_{D}\right)$ where [L] is concentration of radiolabeled ligand used in the assay.

### 4.2 Human adenosine A2A receptor binding assay

### General Method

Test compound is weighed, dissolved in DMSO to make a stock solution of 10 mM, diluted with DMSO to prepare working solutions, then 100-fold diluted to the indicated concentrations. Human recombinant adenosine A_{2A} receptors expressed in HEK-293 cells were used to prepare membranes in incubation buffer (50 mM Tris-HCl, pH 7.4, 10 mM MgCl₂, 1 mM EDTA, 2 U/ml Adenosine Deaminase). Test compound or vehicle (2.2 µL) was added in 200 µL of membranes and incubated with 20 µL of 50 nM [³H] CGS-21680 for 90 minutes at 25°C. Non-specific binding was estimated in the presence of 50 µM NECA (5'-*N*-ethylcarboxamidoadenosine). The incubation was stopped by vacuum filtration onto 0.3 % PEI (polyethylenimine) presoaked GF/B filters using a harvester followed by four washes with ice-cold 50 mM Tris-HCl, pH 7.4, and the radioactivity on GF/B filtermats counted in a scintillation counter (PerkinElmer Topcount^{™}) to determine [³H] CGS-21680 specifically bound. Final concentration of vehicle DMSO is 1%.

Data is fitted using the non-linear curve fitting routines in Meth-IQ software (ID Business Solutions Ltd., UK). IC₅₀ is converted to Kᵢ by Cheng-Prusoff equation:${K}_{i} = {IC}_{50} / \left(1+\left[L\right]{/ K}_{D}\right)$ where [L] is concentration of radiolabeled ligand used in the assay.

### 4.3 Results

The A2A and A1 receptor inhibition activity of select compounds was assessed using the binding assay described above in Examples 4.1 and 4.2. The activity of reference adenosine receptor an antagonist compound Istradefylline (CAS No. 155270-99-8) was also measured in this assay.

Table 5: A2A and A1 receptor inhibition activity of exemplary compounds.

For A2A receptor inhibition:
A: **Kᵢ** ≤ 20 nM,
B: 20 nM < **Kᵢ** ≤ 200 nM,
C: 200 nM < **Kᵢ** ≤ 1000nM,
D: 1000 nM < **Kᵢ** ≤ 5000nM.

For A1 receptor inhibition:
A: **Kᵢ** ≤ 0.1 µM,
B: 0.1 µM < **Kᵢ** ≤ 1 µM,
C: 1 µM < **Kᵢ** ≤ 10 µM,
D: **Kᵢ** > 10 µM.

| **Table 5** | | |
|---|---|---|
| **Cmpd No.** | **hA1 Kᵢ (µM)** | **hA2A Kᵢ (nM)** |
| 203 | A | A |
| 204 | B | A |
| 206 | A | A |
| 207 | A | A |
| 208 | A | A |
| 209 | B | A |
| 210 | A | A |
| 211 | A | A |
| 213 | A | A |
| 215 | B | B |
| 216 | C | C |
| 217 | D | D |
| 218 | C | D |
| 219 | B | A |
| 220 | B | B |
| 221 | D | D |
| 222 | D | D |
| 223 | B | A |
| 224 | B | B |
| 225 | C | D |
| 226 | D | D |
| 227 | B | A |
| 228 | B | B |
| 229 | D | D |
| 230 | D | D |
| 231 | B | A |
| 232 | B | B |
| 233 | C | C |
| 234 | C | C |
| 235 | B | A |
| Istradefylline | B | A |

The A2A receptor binding assay results of Table 5 indicate that the compounds of this disclosure can act as A2A receptor antagonists. Several compounds exhibited potent activity as dual antagonists of both A2A and A1 receptors. See e.g., compounds 203, 206, 207, 208, 211, and 213 in Table 5.

### EQUIVALENTS AND INCORPORATION BY REFERENCE

While the invention has been particularly shown and described with reference to a preferred embodiment and various alternate embodiments, it will be understood by persons skilled in the relevant art that various changes in form and details can be made therein without departing from the spirit and scope of the invention. Therefore, it must be understood that the embodiments described above are for illustrative purposes and do not limit the present invention.

All references, issued patents and patent applications cited within the body of the instant specification, are herein incorporated by reference in their entirety for all purposes.

## Claims

1. A compound of formula (IIb), or a solvate, a hydrate, and/or a stereoisomer thereof, or a pharmaceutically acceptable salt thereof: 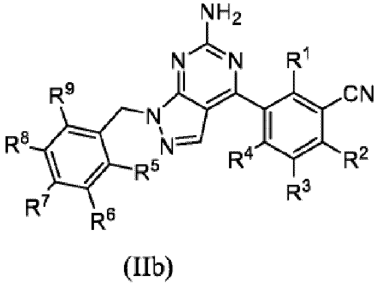 wherein:
R¹ to R⁹ are independently selected from H, (C₁-C₈)alkyl, substituted (C₁-C₈)alkyl, (C₂-C₈)alkenyl, substituted (C₂-C₈)alkenyl, (C₂-C₈)alkynyl, substituted (C₂-C₈)alkynyl, (C₁-C₃)haloalkyl, (C₁-C₈)alkoxy, substituted (C₁-C₈)alkoxy, -CONH₂, substituted amido, -NH₂, substituted amino, -CO₂H, cyano, halogen, hydroxyl, -NO₂, -SO₃H, -SO₂NH₂, substituted sulfonamide, and thiol.

2. The compound, or a solvate, a hydrate, and/or a stereoisomer thereof, or a pharmaceutically acceptable salt thereof of claim 1, wherein R¹ to R⁹ are independently selected from H, NH₂, F, CH₃, and CF₃.

3. The compound, or a solvate, a hydrate, and/or a stereoisomer thereof, or a pharmaceutically acceptable salt thereof of claim 1, wherein the compound is of formula (IIIb): 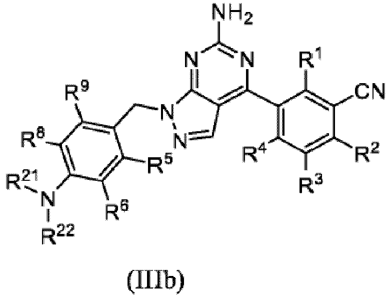 wherein:
R²¹ and R²² are independently selected from H, (C₁-C₈)alkyl, substituted (C₁-C₈)alkyl, SO₂R³⁰, and COR³⁰, wherein R³⁰ is (C₁-C₈)alkyl, or substituted (C₁-C₈)alkyl.

4. The compound, or a solvate, a hydrate, and/or a stereoisomer thereof, or a pharmaceutically acceptable salt thereof of claim 3, wherein
R² to R⁴ are each H; and
R¹ is selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl, for example is selected from H, F, CH₃, and CF₃.

5. The compound, or a solvate, a hydrate, and/or a stereoisomer thereof, or a pharmaceutically acceptable salt thereof of claim 4, wherein
R⁵, R⁶, R⁸ and R⁹ are independently selected from H, (C₁-C₅)alkyl, substituted (C₁-C₅)alkyl, (C₁-C₃)haloalkyl, (C₁-C₅)alkoxy, substituted (C₁-C₅)alkoxy, halogen, and hydroxyl, for example are independently selected from H, F, CH₃, and CF₃.

6. (Currently Amended) The compound, or a solvate, a hydrate, and/or a stereoisomer thereof, or a pharmaceutically acceptable salt thereof of claim 1, wherein the compound of formula (IIb) is selected from:

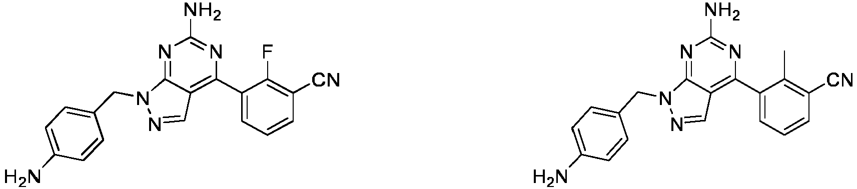

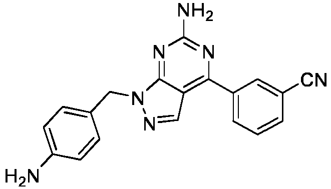

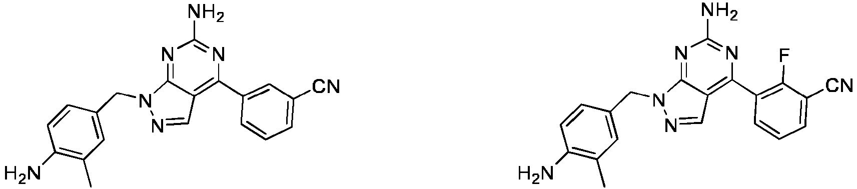

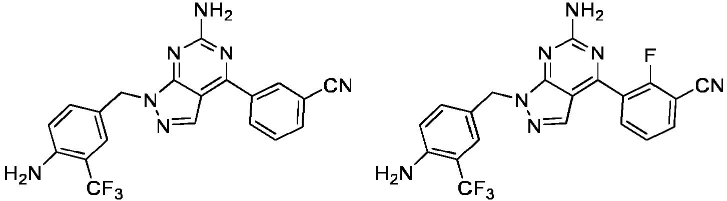

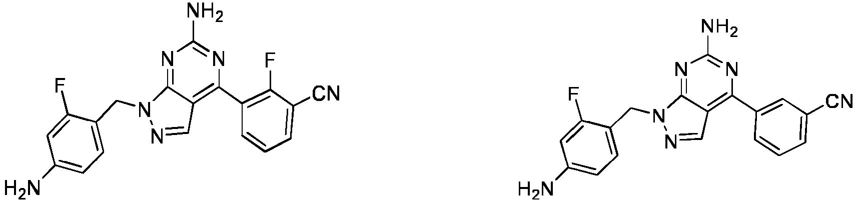

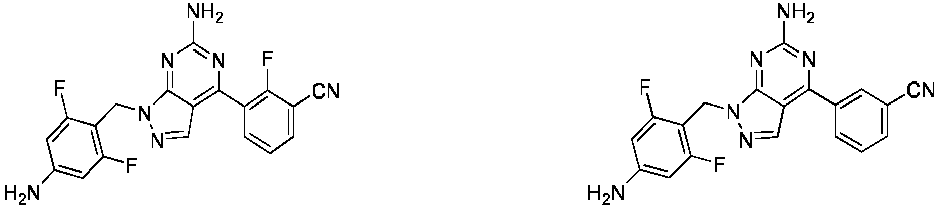

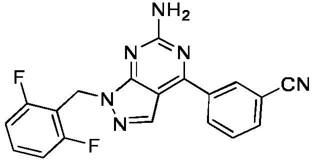

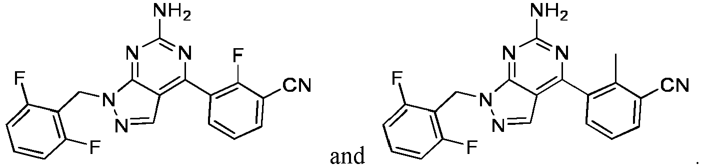

7. A pharmaceutical composition comprising:
a compound or a solvate, a hydrate, and/or a stereoisomer thereof, or a pharmaceutically acceptable salt thereof according to any one of claims 1 to 6; and
a pharmaceutically acceptable excipient.

8. A compound or a solvate, a hydrate, and/or a stereoisomer thereof, or a pharmaceutically acceptable salt thereof according to any one of claims 1 to 6, or a pharmaceutical composition according to claim 7, for use in therapy.

9. An *in vitro* method of inhibiting or antagonizing an adenosine A2A and/or A1 receptor,
the method of inhibiting comprising contacting a sample comprising the adenosine A2A and/or A1 receptor with an effective amount of a compound or a solvate, a hydrate, and/or a stereoisomer thereof, or a pharmaceutically acceptable salt thereof according to any one of claims 1 to 6 to inhibit the adenosine A2A and/or A1 receptor; and
the method of antagonizing comprising contacting a cell comprising the adenosine A2A and/or A1 receptor with an effective amount of a compound or a solvate, a hydrate, and/or a stereoisomer thereof, or a pharmaceutically acceptable salt thereof according to any one of claims 1 to 6 to antagonize the adenosine A2A and/or A1 receptor.

10. A compound or a pharmaceutical composition for use in treating cancer, wherein the compound is according to any one of claims 1 to 6, and the pharmaceutical composition is according to claim 7.

11. The compound or a pharmaceutical composition for the use of claim 10, wherein the cancer is selected from lung cancer, breast cancer, prostate cancer, ovarian cancer, solenoma, cervical cancer, bladder cancer, head and neck cancer, renal cell carcinoma, cancer of the esophagus, pancreatic cancer, brain cancer, liver cancer, leukemia, lymphoma, melanoma, multiple myeloma, Ewing's sarcoma, osteosarcoma, colorectal neoplasm, cholangiocarcinoma, choriocarcinoma, mouth cancer, neuroblastoma, skin cancer, testicular cancer, stromal tumor, germ cell tumor, and thyroid cancer.

12. The compound or composition for the use of claim 10, wherein the compound or composition is administered in combination with an additional active agent,
wherein the additional active agent is selected from anti-angiogenesis agent, antiinflammatory agent, immune checkpoint inhibitor, PARP inhibitor, chemotherapeutic agent, and immunity anticancer agent.

13. A compound or pharmaceutical composition for use in treating an inflammatory disease or central nervous system disease, wherein the compound is according to any one of claims 1 to 6, and the pharmaceutical composition is according to claim 7.

## Patentansprüche

1. Verbindung von Formel (IIb) oder Solvat, Hydrat und/oder Stereoisomer davon oder pharmazeutisch akzeptables Salz davon: 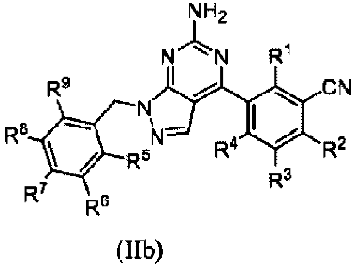 wobei:
R¹ bis R⁹ unabhängig voneinander ausgewählt sind aus H, (C₁-C₈)-Alkyl, substituiertem (C₁-C₈)-Alkyl, (C₂-C₈)-Alkenyl, substituiertem (C₂-C₈)-Alkenyl, (C₂-C₈)-Alkinyl, substituiertem (C₂-C₈)-Alkinyl, (C₁-C₃)-Haloalkyl, (C₁-C₈)-Alkoxy, substituiertem (C₁-C₈)-Alkoxy, -CONH₂, substituiertem Amido, -NH₂, substituiertem Amino, -CO₂H, Cyano, Halogen, Hydroxyl, -NO₂, -SO₃H, -SO₂NH₂, substituiertem Sulfonamid und Thiol.

2. Verbindung oder Solvat, Hydrat und/oder Stereoisomer davon oder pharmazeutisch akzeptables Salz davon nach Anspruch 1, wobei R¹ bis R⁹ unabhängig voneinander aus H, NH₂, F, CH₃und CF₃ ausgewählt sind.

3. Verbindung oder Solvat, Hydrat und/oder Stereoisomer davon oder pharmazeutisch akzeptables Salz davon nach Anspruch 1, wobei die Verbindung Formel (IIIb) aufweist: 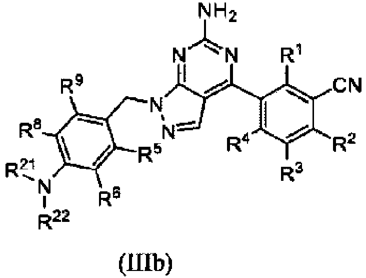 wobei:
R²¹ und R²² unabhängig voneinander ausgewählt sind aus H, (C₁-C₈)-Alkyl, substituiertem (C₁-C₈)-Alkyl, SO₂R³⁰und COR³⁰, wobei R³⁰ (C₁-C₈)-Alkyl oder substituiertes (C₁-C₈)-Alkyl ist.

4. Verbindung oder Solvat, Hydrat und/oder Stereoisomer davon oder pharmazeutisch akzeptables Salz davon nach Anspruch 3, wobei
R² bis R⁴ jeweils H sind; und
R¹ ausgewählt ist aus H, (C₁-C₅)-Alkyl, substituiertem (C₁-C₅)-Alkyl, (C₁-C₃)-Haloalkyl, (C₁-C₅)-Alkoxy, substituiertem (C₁-C₅)-Alkoxy, Halogen und Hydroxyl, zum Beispiel ausgewählt ist aus H, F, CH₃und CF₃.

5. Verbindung oder Solvat, Hydrat und/oder Stereoisomer davon oder pharmazeutisch akzeptables Salz davon nach Anspruch 4, wobei
R⁵, R⁶, R⁸ und R⁹ unabhängig voneinander ausgewählt sind aus H, (C₁-C₅)-Alkyl, substituiertem (C₁-C₅)-Alkyl, (C₁-C₃)-Haloalkyl, (C₁-C₅)-Alkoxy, substituiertem (C₁-C₅)-Alkoxy, Halogen und Hydroxyl, zum Beispiel unabhängig voneinander ausgewählt sind aus H, F, CH₃und CF₃.

6. (Aktuell geändert) Verbindung oder Solvat, Hydrat und/oder Stereoisomer davon oder pharmazeutisch akzeptables Salz davon nach Anspruch 1, wobei die Verbindung von Formel (IIb) ausgewählt ist aus:

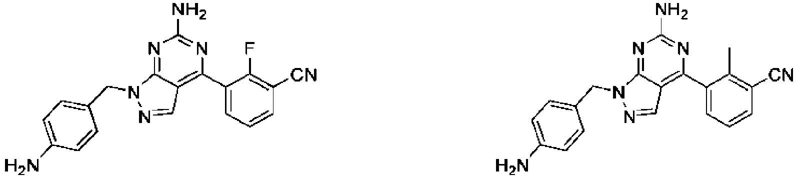

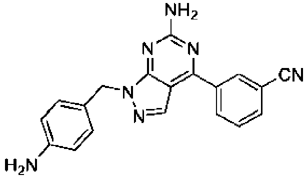

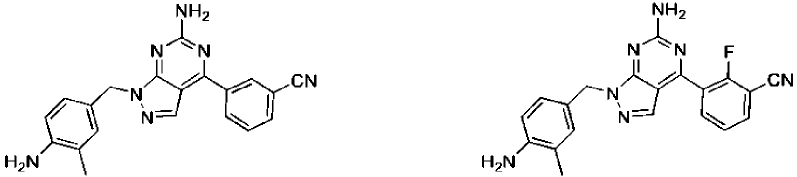

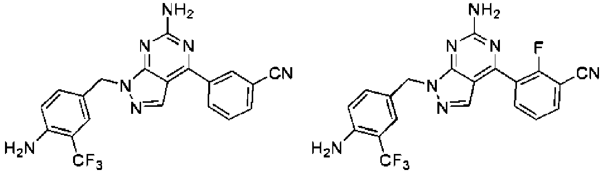

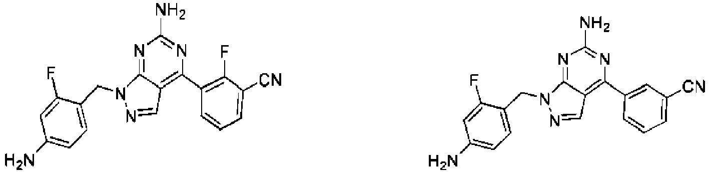

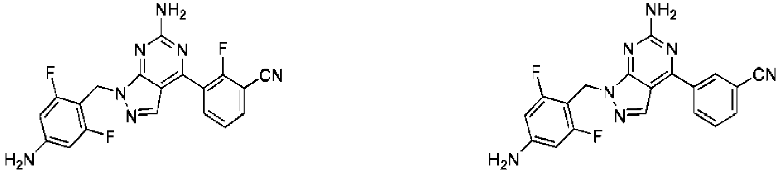

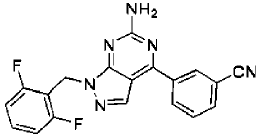

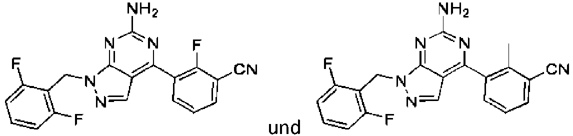

7. Pharmazeutische Zusammensetzung, umfassend:
eine Verbindung oder ein Solvat, ein Hydrat und/oder ein Stereoisomer davon oder ein pharmazeutisch akzeptables Salz davon nach einem der Ansprüche 1 bis 6; und
einen pharmazeutisch akzeptablen Hilfsstoff.

8. Verbindung oder Solvat, Hydrat und/oder Stereoisomer davon oder pharmazeutisch akzeptables Salz davon nach einem der Ansprüche 1 bis 6 oder pharmazeutische Zusammensetzung nach Anspruch 7 zur Verwendung in Therapie.

9. *In-vitro-* Verfahren zur Hemmung oder Antagonisierung eines Adenosin-A2A- und/oder -A1-Rezeptors,
wobei das Verfahren zur Hemmung In-Kontakt-Bringen einer Probe, die den Adenosin-A2A- und/oder -A1-Rezeptor umfasst, mit einer wirksamen Menge einer Verbindung oder eines Solvats, eines Hydrats und/oder eines Stereoisomers davon oder eines pharmazeutisch akzeptablen Salzes davon nach einem der Ansprüche 1 bis 6 zur Hemmung des Adenosin-A2A- und/oder -A1-Rezeptors umfasst; und
das Verfahren zur Antagonisierung In-Kontakt-Bringen einer Zelle, die den Adenosin-A2A- und/oder -A1-Rezeptor umfasst, mit einer wirksamen Menge einer Verbindung oder eines Solvats, eines Hydrats und/oder eines Stereoisomers davon oder eines pharmazeutisch akzeptablen Salzes davon nach einem der Ansprüche 1 bis 6 zur Antagonisierung des Adenosin-A2A- und/oder -A1-Rezeptors umfasst.

10. Verbindung oder pharmazeutische Zusammensetzung zur Verwendung beim Behandeln von Krebs, wobei die Verbindung nach einem der Ansprüche 1 bis 6 ist und die pharmazeutische Zusammensetzung nach Anspruch 7 ist.

11. Verbindung oder pharmazeutische Zusammensetzung zur Verwendung nach Anspruch 10, wobei der Krebs ausgewählt ist aus Lungenkrebs, Brustkrebs, Prostatakrebs, Eierstockkrebs, Solenom, Gebärmutterhalskrebs, Blasenkrebs, Kopf-Hals-Tumoren, Nierenzellkarzinom, Speiseröhrenkrebs, Bauchspeicheldrüsenkrebs, Hirntumor, Leberkrebs, Leukämie, Lymphom, Melanom, Multiplem Myelom, Ewing-Sarkom, Osteosarkom, kolorektale Neoplasie, Cholangiokarzinom, Chorionkarzinom, Mundhöhlenkrebs, Neuroblastom, Hautkrebs, Hodenkrebs, Stromatumor, Keimzelltumor und Schilddrüsenkrebs.

12. Verbindung oder Zusammensetzung zur Verwendung nach Anspruch 10, wobei die Verbindung oder Zusammensetzung in Kombination mit einem zusätzlichen Wirkstoff verabreicht wird,
wobei der zusätzliche Wirkstoff ausgewählt ist aus Anti-Angiogenese-Mittel, entzündungshemmendem Mittel, Immun-Checkpoint-Inhibitor, PARP-Inhibitor, Chemotherapeutikum und Immunitäts-Antikrebsmittel.

13. Verbindung oder pharmazeutische Zusammensetzung zur Verwendung beim Behandeln einer entzündlichen Erkrankung oder einer Erkrankung des zentralen Nervensystems, wobei die Verbindung nach einem der Ansprüche 1 bis 6 ist und die pharmazeutische Zusammensetzung nach Anspruch 7 ist.

## Revendications

1. Composé de formule (IIb), ou solvate, hydrate, et/ou stéréoisomère de celui-ci, ou sel pharmaceutiquement acceptable de celui-ci : 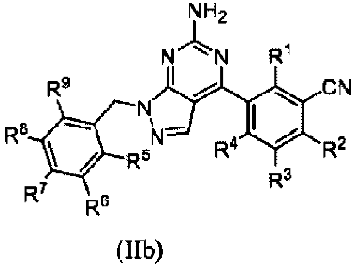 dans lequel :
R¹ à R⁹ sont sélectionnés indépendamment parmi H, alkyle en C₁-C₈, alkyle en C₁-C₈ substitué, alcényle en C₂-C₈, alcényle en C₂-C₈ substitué, alcynyle en C₂-C₈, alcynyle en C₂-C₈ substitué, haloalkyle en C₁-C₃, alcoxy en C₁-C₈, alcoxy en C₁-C₈ substitué, -CONH₂, amido substitué, -NH₂, amino substitué, -CO₂H, cyano, halogène, hydroxyle, -NO₂, -SO₃H, -SO₂NH₂, sulfonamide substitué et thiol.

2. Composé, ou solvate, hydrate, et/ou stéréoisomère de celui-ci, ou sel pharmaceutiquement acceptable de celui-ci selon la revendication 1, dans lequel R¹ à R⁹ sont sélectionnés indépendamment parmi H, NH₂, F, CH₃ et CF₃.

3. Composé, ou solvate, hydrate, et/ou stéréoisomère de celui-ci, ou sel pharmaceutiquement acceptable de celui-ci selon la revendication 1, dans lequel le composé est de formule (IIIb) : 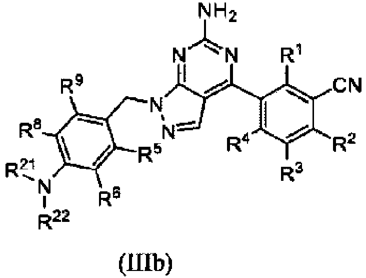 dans lequel :
R²¹ et R²² sont sélectionnés indépendamment parmi H, alkyle en C₁-C₈, alkyle en C₁-C₈ substitué, SO₂R³⁰, et COR³⁰, dans lequel R³⁰ est alkyle en C₁-C₈, ou alkyle en C₁-C₈ substitué.

4. Composé ou solvate, hydrate, et/ou stéréoisomère de celui-ci, ou sel pharmaceutiquement acceptable de celui-ci selon la revendication 3, dans lequel
R² à R⁴ sont chacun H ; et
R¹ est sélectionné parmi H, alkyle en C₁-C₅, alkyle en C₁-C₅ substitué, haloalkyle en C₁-C₃, alcoxy en C₁-C₅, alcoxy en C₁-C₅ substitué, halogène et hydroxyle, par exemple est sélectionné parmi H, F, CH₃ et CF₃.

5. Composé ou solvate, hydrate, et/ou stéréoisomère de celui-ci, ou sel pharmaceutiquement acceptable de celui-ci selon la revendication 4, dans lequel
R⁵, R⁶, R⁸ et R⁹ sont sélectionnés indépendamment parmi H, alkyle en C₁-C₅, alkyle en C₁-C₅ substitué, haloalkyle en C₁-C₃, alcoxy en C₁-C₅, alcoxy en C₁-C₅ substitué, halogène et hydroxyle, par exemple sont sélectionnés indépendamment parmi H, F, CH₃ et CF₃.

6. (Actuellement modifié) Composé, ou solvate, hydrate, et/ou stéréoisomère de celui-ci, ou sel pharmaceutiquement acceptable de celui-ci selon la revendication 1, dans lequel le composé de formule (IIb) est sélectionné parmi :

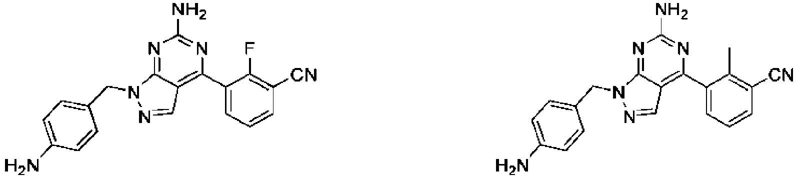

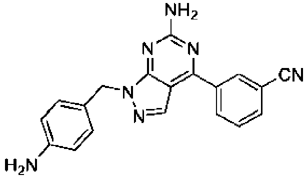

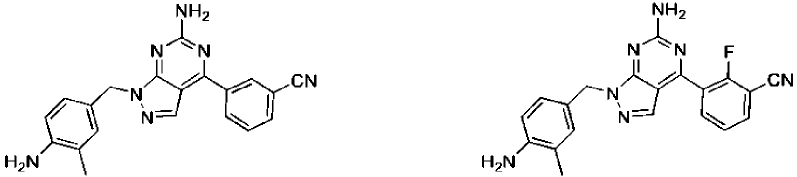

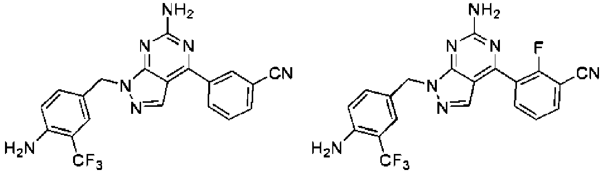

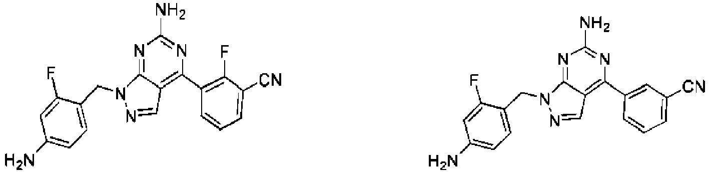

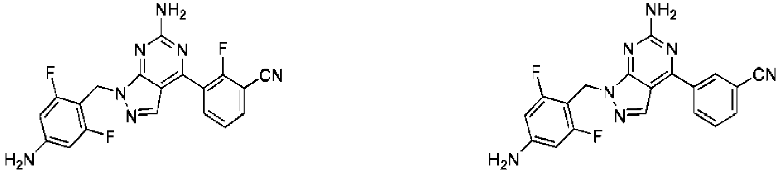

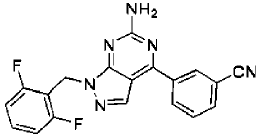

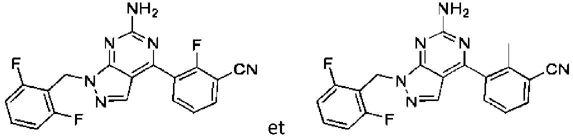

7. Composition pharmaceutique comprenant :
un composé, ou un solvate, un hydrate, et/ou un stéréoisomère de celui-ci, ou un sel pharmaceutiquement acceptable de celui-ci selon l'une quelconque des revendications 1 à 6 ; et
un excipient pharmaceutiquement acceptable.

8. Composé ou solvate, hydrate, et/ou stéréoisomère de celui-ci, ou sel pharmaceutiquement acceptable de celui-ci selon l'une quelconque des revendications 1 à 6, ou composition pharmaceutique selon la revendication 7, pour utilisation en thérapie.

9. Procédé *in vitro* d'inhibition ou d'antagonisme d'un récepteur A2A et/ou A1 de l'adénosine,
le procédé d'inhibition comprenant la mise en contact d'un échantillon contenant le récepteur A2A et/ou A1 de l'adénosine avec une quantité efficace d'un composé ou d'un solvate, d'un hydrate, et/ou d'un stéréoisomère de celui-ci, ou d'un sel pharmaceutiquement acceptable de celui-ci selon l'une quelconque des revendications 1 à 6, pour inhiber le récepteur A2A et/ou A1 de l'adénosine ; et
le procédé d'antagonisme comprenant la mise en contact d'une cellule contenant le récepteur A2A et/ou A1 de l'adénosine avec une quantité efficace d'un composé ou d'un solvate, d'un hydrate, et/ou d'un stéréoisomère de celui-ci, ou d'un sel pharmaceutiquement acceptable de celui-ci selon l'une quelconque des revendications 1 à 6, pour antagoniser le récepteur A2A et/ou A1 de l'adénosine.

10. Composé ou composition pharmaceutique pour utilisation dans le traitement du cancer, dans lequel le composé est conforme à l'une quelconque des revendications 1 à 6, et la composition pharmaceutique est conforme à la revendication 7.

11. Composé ou composition pharmaceutique pour utilisation selon la revendication 10, dans lequel ou laquelle le cancer est sélectionné parmi le cancer du poumon, le cancer du sein, le cancer de la prostate, le cancer de l'ovaire, le solénome, le cancer du col de l'utérus, le cancer de la vessie, le cancer de la tête et du cou, le carcinome à cellules rénales, le cancer de l'œsophage, le cancer du pancréas, le cancer du cerveau, le cancer du foie, la leucémie, le lymphome, le mélanome, le myélome multiple, le sarcome d'Ewing, l'ostéosarcome, le néoplasme colorectal, le cholangiocarcinome, le choriocarcinome, le cancer de la bouche, le neuroblastome, le cancer de la peau, le cancer des testicules, la tumeur stromale, la tumeur germinale et le cancer de la thyroïde.

12. Composé ou composition pour utilisation selon la revendication 10, dans lequel ou laquelle le composé ou la composition est administré(e) en combinaison avec un agent actif supplémentaire,
dans lequel ou laquelle l'agent actif supplémentaire est sélectionné parmi les agents anti-angiogéniques, les agents anti-inflammatoires, les inhibiteurs de points de contrôle immunitaire, les inhibiteurs de PARP, les agents chimiothérapeutiques et les agents d'immunité anticancéreux.

13. Composé ou composition pharmaceutique pour utilisation dans le traitement d'une maladie inflammatoire ou d'une maladie du système nerveux central, dans lequel ou laquelle le composé est conforme à l'une quelconque des revendications 1 à 6, et la composition pharmaceutique est conforme à la revendication 7.
